(19) Europäisches Patentamt
European Patent Office
Office européen des brevets

(11) EP 4 537 832 A1

(12) **EUROPEAN PATENT APPLICATION**
published in accordance with Art. 153(4) EPC

(43) Date of publication:
**16.04.2025 Bulletin 2025/16**

(21) Application number: **23819243.9**

(22) Date of filing: **09.06.2023**

(51) International Patent Classification (IPC):
***A61K 31/675*** (2006.01) ***A61K 31/495*** (2006.01)
***A61K 31/496*** (2006.01) ***A61K 31/472*** (2006.01)
***A61K 31/4375*** (2006.01) ***A61K 31/445*** (2006.01)
***A61K 31/5377*** (2006.01) ***A61K 31/18*** (2006.01)
***A61K 31/255*** (2006.01) ***A61K 31/551*** (2006.01)
***A61K 31/136*** (2006.01) ***A61K 31/4709*** (2006.01)
***A61K 31/7068*** (2006.01) ***A61K 31/337*** (2006.01)
***A61K 31/4523*** (2006.01) ***A61K 31/7072*** (2006.01)
***A61K 31/704*** (2006.01) ***A61K 31/4745*** (2006.01)
***A61K 31/517*** (2006.01) ***A61K 31/519*** (2006.01)
***A61K 31/404*** (2006.01)

(52) Cooperative Patent Classification (CPC):
**A61K 31/136; A61K 31/14; A61K 31/18; A61K 31/255; A61K 31/337; A61K 31/404; A61K 31/4375; A61K 31/445; A61K 31/4523; A61K 31/4709; A61K 31/472; A61K 31/4745; A61K 31/495; A61K 31/496; A61K 31/517;** (Cont.)

(86) International application number:
**PCT/CN2023/099294**

(87) International publication number:
**WO 2023/237080 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.06.2022 CN 202210657718**

(71) Applicant: **Ascentawits Pharmaceuticals, Ltd.**
**Shenzhen, Guangdong 518118 (CN)**

(72) Inventors:
- **LI, Anrong**
  **Shenzhen, Guangdong 518118 (CN)**
- **QI, Tianyang**
  **Shenzhen, Guangdong 518118 (CN)**
- **DUAN, Jianxin**
  **Shenzhen, Guangdong 518118 (CN)**
- **MENG, Fanying**
  **San Francisco 94121 California (US)**
- **LIU, Xing**
  **Shenzhen, Guangdong 518118 (CN)**
- **WANG, Yizhi**
  **Shenzhen, Guangdong 518118 (CN)**

(74) Representative: **Studio Torta S.p.A.**
**Via Viotti, 9**
**10121 Torino (IT)**

# (54) METHOD FOR TREATING CANCER PATIENT WITH AKR1C3 ENZYME-ACTIVATED PRODRUG

(57) Provided is treatment of cancer patients with an AKR1C3 enzyme-activated prodrug, characterized in that: the tumor or cancer tissue of the patients is detected to have a gene mutation capable of up-regulating or activating NRF2; or the patients are detected to have a gene mutation capable of up-regulating or activating NRF2.

EP 4 537 832 A1

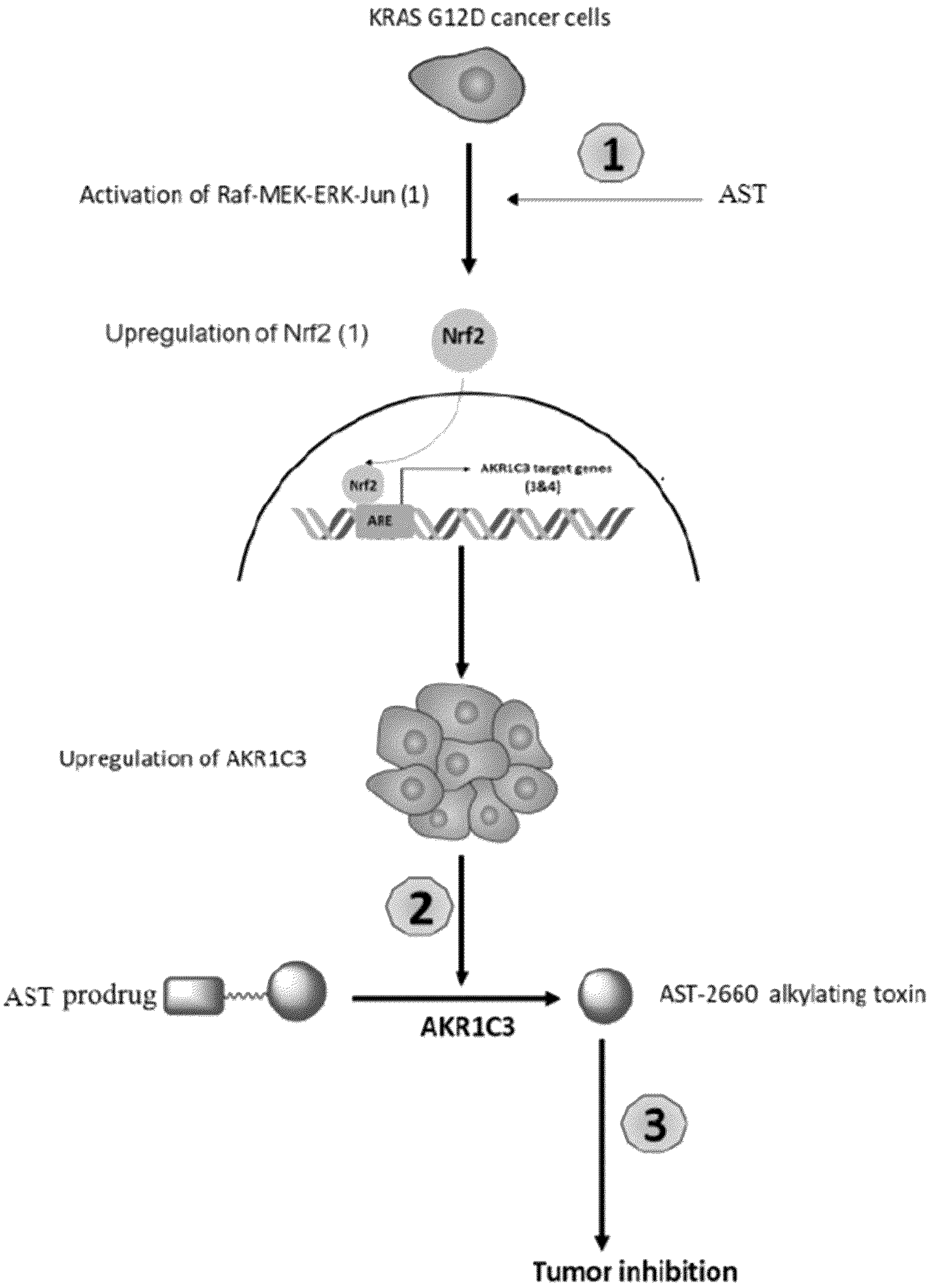


Fig. 28

(52) Cooperative Patent Classification (CPC): (Cont.)
**A61K 31/519; A61K 31/5377; A61K 31/551; A61K 31/675; A61K 31/704; A61K 31/7048; A61K 31/7068; A61K 31/7072; A61K 38/07; A61K 38/08; A61P 35/00**

## Description

### TECHNICAL FIELD

The present invention relates to a treatment method for cancer, and in particular to a treatment method for cancer due to specific genes.

### BACKGROUND

AST-3424 is an AKR1C3 enzyme-activated DNA alkylating agent prodrug that has entered phase II clinical trials (clinical registrations in China are CTR20201915, CTR20201908, CTR20191399, and CTR20191371; clinical registrations in the United States of America are NCT04315324, and NCT03592264) in both China and the United States of America, and it is specifically activated by AKR1C3 enzyme highly expressed in a variety of tumors and releases DNA alkylating agent to exert antitumor effects.

Preclinical studies have shown that the efficacy of AST-3424 is highly correlated with the expression of AKR1C3 enzyme, and small differences in the enzyme expression will greatly affect the final therapeutic efficacy of the drug (AACR 2016, Abstract# 1369: In vitro and in vivo antitumor activity of TH3424: Preclinical rationale for a highly selective AKR1C3 prodrug for treating hepatocellular carcinomas; AACR-NCI-EORTC Annual Meeting, 2017, Abstract: LB-B16: The AKR1C3-Activated Prodrug OBI-3424 Exerts Profound In Vivo Efficacy Against Preclinical Models of T-Cell Acute Lymphoblastic Leukemia (T-ALL); a Pediatric Preclinical Testing Consortium Study; Clin Cancer Res 2019;25:4493-503:OBI-3424, a Novel AKR1C3-Activated Prodrug, Exhibits Potent Efficacy against Preclinical Models of T-ALL; Am J Cancer Res 2021;11(7):3645-3659, A novel selective AKR1C3-activated prodrug AST-3424/OBI-3424 exhibits broad anti-tumor activity).

The above-mentioned Phase II clinical trials in China and the United States of America will investigate the relevance between AKR1C3 expression levels and therapeutic efficacy in cancer patients, and determine quantitative values of AKR1C3 expression levels for screening patients who would benefit from the experimental drug.

That is, there is a need to detect the expression level of AKR1C3 enzyme in patients before the administration of the AKR1C3 enzyme-activated anti-cancer prodrug described above, and the drug may have better therapeutic effect only for patients whose expression level of AKR1C3 enzyme has achieved the predetermined expression level.

Based on the Phase I clinical results of the drug, researchers have determined that the patient may enroll in Phase II clinical patients where his/her AKR1C3 enzyme expression level is H-score≥135 (IHC) (Safety, Pharmacokinetics, and Clinical Activity of OBI-3424, an AKR1C3-Activated Prodrug, in Patients with Advanced or Metastatic Solid Tumors: A Phase 1 Dose-Escalation Study, J Clin Oncol 40, 2022, suppl 16; abstr 3030, DOI: 10.1200/JCO.2022.40.16_suppl.3030).

### SUMMARY **OF THE INVENTION**

In the further preclinical efficacy studies, the inventors found that AKR1C3 enzyme-activated prodrug AST-3424, AST have significant inhibitory effects on PDX and CDX tumor models with Kras-G12D/G12C mutation (Parts 1, 2, 3, 4 of examples and subsequent research section). The inventors speculated that the Kras-G12D/G12C mutation is somehow associated with an AKR1C3 enzyme high expression: the Kras-G12D/G12C mutation will result in a high expression of AKR1C3 enzyme.

Based on the above speculation, the inventors conducted an AKR1C3 RNA detection and an AKR1C3 protein expression level detection on the tissues of the PDX and CDX models as described above (Part 5 and Part 9 of examples). The results demonstrated that RNA and protein expression levels of AKR1C3 in the above models with Kras-G12D/G12C mutation were high, which confirmed the above speculation: the Kras-G12D/G12C mutation is associated with the high expression of AKR1C3 enzyme; Kras-G12D/G12C mutation is accompanied with the high expression of AKR1C3 enzyme.

For verifying the conclusion that Kras-G12D mutation is accompanied with the high expression of AKR1C3, the inventors collected the AKR1C3 expression data of 179 PDX models with Kras-G12D mutation and found that:
The distribution trend of AKR1C3 expression level in the KRAS G12D PDX models was mainly concentrated in medium and high expressions, which account for 90.4%.

Further, by analyzing the above statistical data, it was found that in the KRAS G12D PDX models, the distribution trend of NRF2 expression level was also concentrated in medium and high expression, which was consistent with the trend of AKR1C3 protein expression and had a certain correlation (Part 6 of the examples).

For verifying the conclusion that Kras-G12C mutation is accompanied with the high expression of AKR1C3 enzyme, the inventors collected the AKR1C3 expression data of 51 PDX models with Kras-G12C mutation and found that:
In the KRAS G12C PDX models, AKR1C3 expression level was equally distributed in low, medium and high levels, wherein

the expression levels over medium account for 66.7%.

Further, by analyzing above statistical data, it was found that in the KRAS G12C PDX models, the overall correlation between the distribution trends of NRF2 expression level and AKR1C3 protein expression level was weak (Part 7 of the examples).

For verifying the conclusion that Kras-G13D mutation is accompanied with the high expression of AKR1C3 enzyme, the inventors collected the AKR1C3 expression data of 48 PDX models with Kras-G13D mutation and found that: The distribution trend of AKR1C3 expression level in the KRAS G13D PDX models was mainly concentrated in medium and high expressions, wherein the expression levels over medium account for 83.3%.

In the KRAS G13D PDX models, the distribution trend of NRF2 expression level was somewhat correlated with that of an AKR1C3 protein expression level, and both AKR1C3 and NRF2 were mostly highly expressed (Part 8 of the examples).

The inventors further learned from the literature that KRAS G12D mutant tumor cells can directly up-regulate and activate NRF2 (Nature, 2011, 475: 106; Cancer Res, 2014, 74: 7430) through the RAF-MEK-ERK-Jun signal transduction pathway (Nature, 2011, 475: 106). Activated NRF2 can further up-regulate and activate a series of downstream genes, including AKR1C3 (Chem Res Toxicol, 2017, 30:162; Cancer, 2019, 11: 1715). Based on above regulatory pathways, it can be seen that a gene mutation that can up-regulate or activate NRF2, such as G12D mutation, can eventually up-regulate AKR1C3 gene, resulting in increased expression level of AKR1C3 enzyme in cancer cells.

Based on the above experiments and literature research, the inventors creatively proposed that on the basis of the known manner for selecting/screening of patients by detecting the expression level of AKR1C3 enzyme, we could screen the AKR1C3 enzyme-activated prodrug by directly using it to detect whether the tumor or cancer tissue of the patients has a gene mutation that can up-regulate or activate NRF2; or whether the patients are detected to have a gene mutation that can up-regulate or activate NRF2.

For this purpose, the following technical solutions are provided in the present application.

Solution 1

A method for treating cancer patients with an AKR1C3 enzyme-activated prodrug, characterized in that: the tumor or cancer tissue of the patients is detected to have a gene mutation capable of up-regulating or activating NRF2; or the patients are detected to have a gene mutation capable of up-regulating or activating NRF2.

Solution 2

Pharmaceutical use of AKR1C3 enzyme-activated prodrug in the manufacture of a medicament for treating cancer, characterized in that:

the tumor or cancer tissue of the patient is detected to have a gene mutation capable of up-regulating or activating NRF2; or

the patient is detected to have a gene mutation capable of up-regulating or activating NRF2.

Solution 3

A medicament comprising AKR1C3 enzyme-activated prodrug compound, which is used to treat cancer patients,

wherein the tumor or cancer tissue of the patients is detected to have a gene mutation capable of up-regulating or activating NRF2; or

the patients are detected to have a gene mutation capable of up-regulating or activating NRF2.

Solution 4

A method for treating cancer patients with an AKR1C3 enzyme-activated prodrug, which comprises the step of administrating a drug or preparation containing AKR1C3 enzyme-activated prodrug; and the step of detecting NRF2 content or expression level in a cancer cell or tissue of the patient,

where the NRF2 content or expression level is measured to be equal to or greater than the predetermined value, the patients are administered with a medicament or preparation containing AKR1C3 enzyme-activated prodrug.

Solution 5

A method for treating cancer or tumor, which comprises the step of administrating a medicament or preparation

containing AKR1C3 enzyme-activated prodrug; and the step of regulating NRF2 content or expression level, where the NRF2 content or expression level is regulated to be equal to or greater than the predetermined value, the patient is administered with a medicament or preparation containing AKR1C3 enzyme-activated prodrug.

Solution 6

Pharmaceutical use of AKR1C3 enzyme-activated prodrug in the manufacture of a medicament for treating cancer, characterized in that:

the tumor or cancer tissue is detected to comprise a NRF2 content or expression level that is equal to or greater than the predetermined value; or
the patient is detected to comprise a NRF2 content or expression level that is equal to or greater than the predetermined value.

Solution 7

A medicament comprising AKR1C3 enzyme-activated prodrug compound, which is used to treat cancer patients,

wherein the tumor or cancer tissue of the patients is detected to comprise a NRF2 content or expression level that is equal to or greater than the predetermined value; or
the patients are detected to comprise a NRF2 content or expression level that is equal to or greater than the predetermined value.

Generally speaking, NRF2 content or expression level, and a gene mutation that can up-regulate or activate NRF2 are detected using tumor or cancer tissue samples of the patients. However, for various reasons, such as the patients cannot provide qualified tumor or cancer tissue sample, or current detection methods cannot detect accurately, or the tumor or cancer tissue cannot reflect the patient's recent condition, it may be necessary to detect or diagnose the NRF2 content or expression level, and a gene mutation that can up-regulate or activate NRF2 of the patients themselves. For example, blood can be used to detect a gene mutation, and the detection results can represent the condition of tumor or cancer tissue of the patient; circulating tumor cell (CTC) detection can be used to detect the NRF2 content or expression level of specific cancer cells (all tumor cells free in the peripheral blood) and a gene mutation that can up-regulate or activate NRF2 through patient's peripheral blood test sample. In some cases, a gene mutation can be diagnosed or adjunctively diagnosed by gene mutations of parents in biological parent-child relationships.

The meaning of "predetermined value" is that the NRF2 content or expression level of patients that is detected by a certain method (such as in vitro diagnosis) is equal to or greater than a certain predetermined value, and medicaments or formulas containing the compounds of the present application are administrated to the patients to achieve the purpose of accurately targeted treatment of cancer/tumor patients. The determination of the predetermined value requires to determine the cancer type and specific medicaments or preparations before conducting clinical trials in different patient populations and giving statistical analysis according to the final clinical trial results and data at least. This predetermined value cannot be given at present, but it can be finally determined through clinical trials.

In the present application, a gene mutation that can up-regulate or activate NRF2 is selected from the following gene mutations:
KRAS, KEAP1, CUL3, NRF2, p21, p62, FTL1, FTH1, HMOX1, GSR, SLC7A11, GCLM, GCLC, GPX2, TXN1, TXNRD1, PRDX1, SRXN1, ABCC1, ABCC2, G6PD, PGD, ME1, IDH1, EGFR.

The transcription factor NRF2 protein is a recombinant and synthetic protein, and its full name is nuclear factor erythroid 2 p45 related factor 2. It is a member of the Cap'n'collar (CNC) transcription factor family, and consists of seven Neh domains (Nrf2 ECH homeodomains), each with a different function:

Neh1 CNC-bZIP domain is responsible for binding to small Maf (sMAF) protein and the dimerization;
Neh2 domain mediates the interaction with Keap1 through DLG and ETGE motifs;
Neh4, Neh5, and Neh3 domains are important for the trans-activation of NRF2; and
Neh6 domain is a serine-rich region and can regulate the stability.

A gene mutation that up-regulate or activate NRF2 refers to a gene mutation that can activate the activity of NRF2 protein or directly up-regulate the expression level of NRF2 protein. There have been numerous studies showing that, at the upstream of NRF2, namely in the NRF2 signal transduction classical pathway (Wu S, et al. Nrf2 in cancers: A double-edged sword. Cancer Med. 2019; 8(5): 2252-2267.) and Nrf2 signal transduction nonclassical pathway (Jiang T, et al. p62 links autophagy and Nrf2 signaling. Free Radic Biol Med. 2015; 88 (Pt B): 199-204.), various genes can activate or up-regulate NRF2 protein after mutation, and these gene mutations include:

KRAS mutation. The full name of the KRAS gene is Kirsten ratsarcoma viral oncogene homolog, which belongs to the RAS superprotein family. The protein encoded by the KRAS gene is a small GTPase. In human genome, there are two KRAS genes, namely KRAS 1, located on the short arm of chromosome 6; and KRAS2, located on the short arm of chromosome 12. Among them, KRAS1 is a "pseudogene" that cannot be transcribed into RNA, so it is nonfunctional; while KRAS2 is a "true gene" that can be transcribed and translated into proteins that have biological activity. The KRAS genes and proteins studied in patents and literature reports usually refer to the "KRAS2" gene and its protein products, and so does this application.

Among the gene mutations in KRAS, 97% occur at amino acid residues 12 or 13. Structural studies have shown that most of these gene mutations interfere with the ability of KRAS to hydrolyze GTP. KRAS gene mutations are mainly concentrated in codon positions 12, 13 and 61, wherein mutations at codon position 12 account for more than 80% of all mutations, including G12A, G12C, G12D, G12R, G12S and G12V, the most important of which are the three mutations of G12C, G12V and G13D.

KEAP1, Recombinant Kelch Like ECH Associated Protein 1.

CUL3, Cullin family protein 3.

NRF2, nuclear factor erythroid 2 p45 related factor 2.

p21, which is a negative regulator of the cell cycle, a member of CDKIs family, and a cyclin-dependent kinase inhibitor.

p62, i.e., p62 protein, which is encoded by the SQSTM1 gene and can also be written as p62/SQSTM1, formerly known as SequestoSOM-1. P62 protein is a selective autophagy receptor.

FTL1, Ferritin, Light Polypeptide 1.

FTH1, Ferritin, heavy polypeptide 1.

HMOX1, i.e., heme oxygenase 1 (Abbreviate as HMOX1 or HO-1), which is a heme oxygenase (EC 1.14.99.3) that is an important enzyme in the metabolism of heme that converts heme into biliverdin. GSR, Recombinant Glutathione Reductase.

SLC7A11, Recombinant Solute Carrier Family 7, Member 11.

GCLM, Recombinant Glutamate Cysteine Ligase, Modifier.

GCLC, Glutamate Cysteine Ligase, Catalytic.

GPX2, Glutathione Peroxidase 2.

TXN1, Thioredoxin 1, also known as His; Trx; ADF; TRX1; etc.

TXNRD1, thioredoxin reductase 1.

PRDX1, Prdx1 (peroxiredoxin 1), peroxiredoxin (Prdx) family member 1.

SRXN1, Sulfiredoxin-1.

ABCC1, ATP binding cassette sub-family C member 1.

ABCC2, ATP binding cassette sub-family C member 2.

G6PD, 6-Phosphogluconic dehydrogenase.

PGD, 6-phosphogluconate dehydrogenase, 6-PGDH.

ME1, malic enzyme (ME).

IDH1, isocitrate dehydrogenase soluble 1.

EGFR, epidermal growth factor receptor, abbreviate as EGFR, ErbB-1 or HER1, which is a member of human epidermal growth factor receptor (HER) family. The family includes HER1 (erbB1, EGFR), HER2 (erbB2, NEU), HER3 (erbB3) and HER4 (erbB4). HER family plays an important regulatory role in cellular physiological processes.

In particular, furthermore, KRAS mutation is selected from the six subtypes: G12D, G12V, G12R, G12C, G12A, and G13D.

Any one or two gene mutations in the genes corresponding to KRAS can be detected and diagnosed with commercially available (companion) diagnostic kits. In January 2012, the FDA approved KRAS mutation detection method of Qiagen can be used as a companion diagnosis for cetuxib. In May 2015, the FDA approved that KRAS mutation detection method of Roche can be used for the diagnosis of metastatic colon cancer.

At present, Amoy Dx, Shanghai Tellgen Life, Wuhan YZYMED, Wuhan Hygeianey, Suzhou Microdiag and other companies in China have provided detection kits for KRAS gene activated mutations. Above detection methods for KRAS mutations are all based on the principle of real-time fluorescence quantitative PCR method. The information on some diagnostic kits approved in China is as follows:

Amoy Dx, National Equipment Registration 20153401126, Human KRAS gene mutation detection kit (fluorescent PCR method)

Shanghai Tellgen Life, National Equipment Registration 20163401341, Human K-RAS gene 7 mutations detection kit (fluorescence PCR method).

Evidently, NGS sequencing (YS 450 gene NGS large panel) can also be used to determine the specific KRAS mutation subtype.

More preferably, the KRAS mutation is selected from the KRAS-G12D mutation.

The TMB (Tumor Mutation Load (burden)) level of the described gene mutation is medium.

Since the levels of TMB (Tumor Mutation Load (burden)) are different between different tumor types, it is generally believed that: a TMB of more than 20 mutations/Mb (Mb represents bases per million) is high; a TMB of less than 10 mutations/Mb is low, and a TMB in the middle is medium. At the World Conference on Lung Cancer in 2017, Squibb Inc. had announced the results of a clinical trial named CheckMate-032. This was a phase II clinical trial that enrolled 401 patients with advanced lung cancer who had failed firstline therapy and were treated with a PD-1 inhibitor alone or in combination with Ipilimumab. The patients were divided into three categories of patients with high TMB, medium TMB, and low TMB according to their TMB levels, then among those who received the combination therapy, the efficiency of the three groups was 62%, 20%, and 23%, respectively, and the efficiency of the group with high TMB was three times higher than the remaining two groups. The median overall survival of the three groups was: 22.0 months, 3.6 months, and 3.4 months, respectively, and 22.0 months and 3.4 months differ by six times! This trial demonstrated that for different cancer treatment drugs, different TMB levels have a significant impact on the efficacy of the drugs.

AKR1C3 enzyme-activated prodrug refers to a compound that is a prodrug, and the prodrug molecule reacts with AKR1C3 enzyme to release cytotoxic anti-tumor compounds.

Broadly speaking, AKR1C3 enzyme-activated prodrugs meet, but are not limited to, at least one of the following conditions:

A. The inhibitory effect of a compound on cancer cell proliferation detected in the presence of AKR1C3 inhibitor (for example, compound 36, i.e.

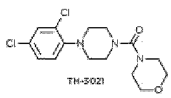

TH-3021

in Bioorganic and Medicinal Chemistry, 2014: 962-977) is smaller than that detected in the absence of AKR1C3 inhibitor. When the inhibitory effect on cancer cell proliferation is quantified with $IC_{50}$, if the $IC_{50}$ of a compound on a cancer cell line detected in the presence of AKR1C3 inhibitor is greater than the $IC_{50}$ detected in the absence of AKR1C3 inhibitor, it can be determined that the compound is an AKR1C3-activated anticancer drug (Lysis-Prodrug). Specific compounds are as the compounds disclosed in the following patent documents:

PCT/US2016/021581 with Publication No. WO2016145092A1, corresponding to the Chinese Application No. 2016800150788 with Publication No. CN107530556A;
PCT/US2016/062114 with Publication No. WO2017087428, corresponding to the Chinese Application No. 2016800446081 with Publication No. CN108290911A;
PCT/US2016/025665 with Publication No. WO2016161342, corresponding to the Chinese Application No. 2016800200132 with Publication No. CN108136214A; and
PCT/NZ2019/050030 with Publication No. WO2019190331, corresponding to the Chinese Application No. CN2019800234236 with Publication No. CN111918864A. The entire contents of above patent documents are incorporated herein by reference in its entirety.

Wherein, the compounds disclosed in PCT/US2016/021581 with Publication No. WO2016145092A1, corresponding to the Chinese Application No. 2016800150788 with Publication No. CN107530556A; PCT/US2016/062114 with Publication No. WO2017087428, corresponding to the Chinese Application No. 2016800446081 with Publication No. CN108290911A; and PCT/US2016/025665 with Publication No. WO2016161342, corresponding to the Chinese Application No. 2016800200132 with Publication No. CN108136214A are lysis-prodrugs that ultimately cleave and is metabolized to primary drugs that play a role being

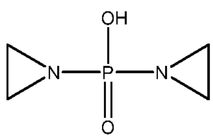

, paclitaxel, camptothecin and other drugs; the compounds disclosed in PCT/NZ2019/050030 with Publication No. WO2019190331, corresponding to the Chinese Application No. CN2019800234236 with Publication No.

CN111918864A are lysis-prodrugs that ultimately lyze and is metabolized to primary drugs that play a role being nitrogen mustard structural drugs.

B. The inhibitory effect of a compound on the proliferation of cancer cells with different expression levels of AKR1C3 enzyme is significantly different, and the inhibitory effect on the proliferation of cancer cells with high expression of AKR1C3 enzyme is much greater than that of cancer cells with low expression of AKR1C3 enzyme. When the inhibitory effect on cancer cell proliferation is quantified with $IC_{50}$, if the $IC_{50}$ value of a compound on cancer cells with high expression of AKR1C3 enzyme is lower than the $IC_{50}$ value on cancer cells with low expression of AKR1C3 enzyme, it can be determined that the compound is an AKR1C3-activated anticancer drug (Lysis-Prodrug). Specific compounds are as the compounds disclosed in Patent PCT/CN2020/120281 with Publication No. WO2021068952A1, the entire contents of which are incorporated herein by reference in its entirety.

The AKR1C3 enzyme-activated prodrug is selected from the group consisting of the compound of formula (1), (2), (3), (4), (5), (6), (7), (8), (9), (10), (11) and the salt, ester, solvate and isotope isomer thereof.

The compound of formula (1) is an AKR1C3 enzyme-activated alkylating agent prodrug compound, and more specifically, it is an AKR1C3 enzyme-activated DNA alkylating agent prodrug compound;

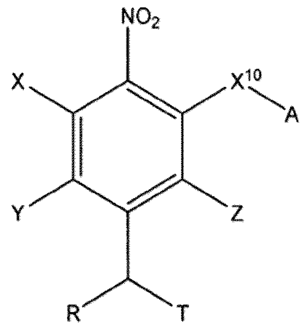

(1),

wherein the definitions of X, Y, Z, R, T, A and $X^{10}$ are described in the claims of Patent Application PCT/US2016/021581 with Publication No. WO2016145092A1 (corresponding to the Chinese Application No. 201680015078.8 with Publication No. CN107530556A), which describes the synthesis and preparation methods of specific compounds as well and is incorporated herein by reference in its entirety, with detailed definitions being :

$X^{10}$ is O, S, SO, or $SO_2$;
A is $C_6$-$C_{10}$ aryl, 5-15-membered heteraryl or -N=$CR^1R^2$;
$R^1$ and $R^2$ are each independently hydrogen, $C_1$-$C_6$ alkyl, $C_3$-$C_8$ cycloalkyl, $C_6$-$C_{10}$ aryl, 4-15-membered heterocycle, ether, -$CONR^{13}R^{14}$ or -$NR^{13}COR^{14}$;
X, Y and Z are independently hydrogen, CN, halogeno, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15-membered heterocycle, ether, -$CONR^{13}R^{14}$ or -$NR^{13}COR^{14}$;
R is hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15-membered heterocycle, ether, -$CONR^{13}R^{14}$ or -$NR^{13}COR^{14}$;
$R^{13}$ and $R^{14}$ are each independently hydrogen, $C_1$-$C_6$ alkyl, $C_3$-$C_8$ cycloalkyl, $C_6$-$C_{10}$ aryl, 4-15-membered heterocycle or ether;
T comprises a phosphoramidate alkylating agent comprising one or more $Z^5$-$X^5$-$Y^5$ moieties bonded to an -O-P($Z^1$) moiety, where $Z^5$ is a heteroatom such as nitrogen, sulfur or oxygen, $X^5$ is substituted or unsubstituted ethylene, $Y^5$ is halogeno or another leaving group, or $Z^5$-$X^5$-$Y^5$ together form an aziridinyl ($NCH_2CH_2$) moiety, and $Z^1$ is O or S; and wherein the alkyl, alkenyl, alkynyl, cycloalkyl, aryl, heterocyclyl, heteroaryl, ether groups are substituted or unsubstituted.

In

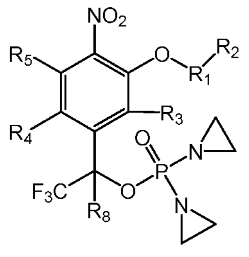 (2), 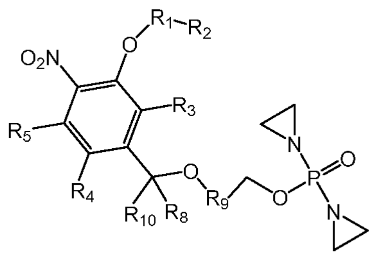 (3),

the definitions of $R_1$, $R_2$, $R_3$, $R_4$, $R_5$, $R_8$, $R_9$, and $R_{10}$ are described in the claims of Patent Application PCT/CN2020/089692 with Publication No. WO2020228685A1, which describes the synthesis and preparation methods of specific compounds as well and is incorporated herein by reference in its entirety, with detailed definitions being:

$R_1$ is $C_6$-$C_{10}$ aryl or Z-substituted aryl, 4-15-membered heterocycle or Z-substituted heterocycle, 5-15-membered heteroaryl or Z-substituted heteroaryl, a 7-15-membered fused ring or Z-substituted fused ring;

$R_2$ is hydrogen, a halogen atom, cyano or isocyano, hydroxy, sulfhydryl, amino, OTs, OMS, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, $C_6$-$C_{10}$ aryl or Z-substituted aryl, 4-15-membered heterocycle or Z-substituted heterocycle, 5-15-membered heteroaryl or Z-substituted heteroaryl, ether having from 1 to 6 carbon atoms or Z-substituted alkoxy having from 1 to 6 carbon atoms, $-CONR^6R^7$, $-SO_2NR^6R^7$, $- SO_2R^6$, $-OCOO-R^6$, $-COOR^6$, $-NR^6COR^7$, $-OCOR^6$, $-NR^6SO_2R^7$ or $-NR^6SO_2NR^6R^7$, or $R^2$ together with the atom in the group $R^1$ to which it is bonded to form a 7-15-membered fused ring or Z-substituted fused ring;

$R_3$ is hydrogen, halogen, cyano or isocyano, hydroxy, sulfhydryl, amino, OTs, OMS, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, $C_6$-$C_{10}$ aryl or Z-substituted aryl, 4-15-membered heterocycle or Z-substituted heterocycle, 5-15-membered heteroaryl or Z-substituted heteroaryl, $C_1$-$C_6$ alkoxy or Z-substituted $C_1$-$C_6$ alkoxy, $-CONR^6R^7$, $-SO_2NR^6R^7$, $-SO_2R^6$, $-OCO-R^6$, $-OCOO-R^6$, $-COOR^6$, $-NR^6COR^7$, $- OCOR^6$, or $-NR^6SO_2R^7$;

$R_4$ and $R_5$ are each independently hydrogen, a halogen atom, cyano or isocyano, hydroxy, sulfhydryl, amino, OTs, OMS, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, $C_6$-$C_{10}$ aryl or Z-substituted aryl, 4-15-membered heterocycle or Z-substituted heterocycle, 5-15-membered heteroaryl or Z-substituted heteroaryl, $C_1$-$C_6$ alkoxy or Z-substituted $C_1$-$C_6$ alkoxy, $-CONR^6R^7$, $-SO_2NR^6R^7$, $-SO_2R^6$, $-OCOO-R^6$, $- COOR^6$, $-NR^6COR^6$, $-OCOR^6$ or $-NR^6SO_2R^7$, or $R^4$ and $R^5$ together with the atom in the benzene ring to which they are bonded to form a 7-15-membered fused ring or Z-substituted fused ring;

$R_6$ and $R_7$ are each independently hydrogen, cyano or isocyano, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, $C_6$-$C_{10}$ aryl or Z-substituted aryl, 4-15-membered heterocycle or Z-substituted heterocycle, 5-15-membered heteroaryl or Z-substituted heteroaryl, $C_1$-$C_6$ alkoxy or Z-substituted $C_1$-$C_6$ alkoxy, or $R^6$ and $R^7$ together with the atom to which they are bonded to form 5-7-membered heterocyclyl or Z-substituted 5-7-membered heterocyclyl;

$R_8$ and $R_{10}$ are each independently hydrogen, deuterium, aryl or Z-substituted aryl, $C_1$-$C_6$ alkyl or Z-substituted alkyl, $C_2$-$C_6$ alkenyl or Z-substituted alkenyl, $C_2$-$C_6$ alkynyl or Z-substituted alkynyl, $C_3$-$C_8$ cycloalkyl or Z-substituted cycloalkyl, and at least one of $R^8$ and $R^{10}$ must be hydrogen or deuterium;

$R_9$ is substituted $C_6$-$C_{10}$ aryl which is substituted with at least one fluorine atom or nitro group, substituted 4-15-membered heterocycle which is substituted with at least one fluorine atom or nitro group, or substituted 5-15-membered heteroaryl which is substituted with at least one fluorine atom or nitro group; the substituent Z is a halogen atom, cyano or isocyano, hydroxy, sulfhydryl, amino, OTs, OMS, $C_1$-$C_3$ alkyl or substituted alkyl, $C_1$-$C_3$ alkoxy or substituted alkoxy, $C_2$-$C_3$ alkenyl or substituted alkenyl, $C_2$-$C_3$ alkynyl or substituted alkynyl, $C_3$-$C_8$ cycloalkyl or substituted cycloalkyl, an aromatic ring, heterocycle, heteroaromatic ring and fused ring or a substituted aromatic ring, heterocycle, heteroaromatic ring and fused ring, the pattern of substitution being mono- or gem-di-substitution;

in $R_9$, the substitution in the substituted $C_6$-$C_{10}$ aryl, substituted 4-15-membered heterocycle or substituted 5-15-membered heteroaryl is a halogen atom, nitro, cyano or isocyano, hydroxy, amino, $C_1$-$C_3$ alkyl or alkoxy, alkenyl, alkynyl, cycloalkyl or benzene ring, substituted benzene ring, $C_1$-$C_3$ alkoxy or halogen atom-substituted alkoxy.

The compound of formula (4) is an AKR1C3 enzyme-activated alkylating agent prodrug compound, and more specifically, it is an AKR1C3 enzyme-activated DNA alkylating agent prodrug compound;

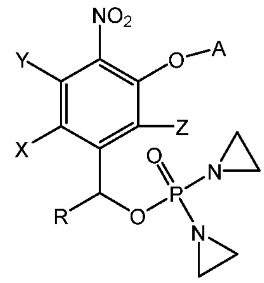

(4),

wherein:

A is substituted or unsubstituted $C_6$-$C_{10}$ aryl, biaryl or substituted biaryl, 5-15-membered heteroaryl, or -N=$CR^1R^2$; wherein the substituent is selected from the group consisting of halogeno, -CN, -$NO_2$, -O-($CH_2$)-O-, -$CO_2$H and salt thereof, -$OR^{100}$, -$CO_2R^{100}$, -$CONR^{101}R^{102}$, -$NR^{101}R^{102}$, -$NR^{100}SO_2R^{100}$, -$SO_2R^{100}$, -$SO_2NR^{101}R^{102}$, $C_1$-$C_6$ alkyl, and $C_3$-$C_{10}$ heterocyclyl;

wherein $R^{100}$, $R^{101}$ and $R^{102}$ are each independently hydrogen, $C_1$-$C_8$ alkyl, or $C_6$-$C_{12}$ aryl; or $R^{101}$ and $R^{102}$ together with the nitrogen atom to which they are attached form a 5-7-membered heterocycle;

wherein the alkyl group and the aryl group are each substituted by 1-3 halogeno groups or 1-3 $C_1$-$C_6$ alkyl groups;

$R^1$ and $R^2$ are each independently phenyl or methyl;

X, Y and Z are each independently hydrogen or halogeno; and

R is hydrogen or $C_1$-$C_6$ alkyl or halogen-substituted alkyl.

In

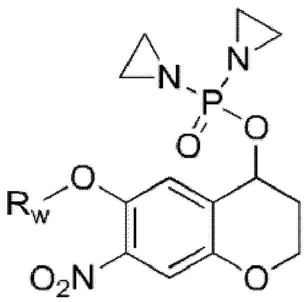

(5),

the definition of Rw has been described in the claims of Patent Application PCT/CN2020/120281 with Publication No. WO2021068952A1, which describes the synthesis and preparation methods of specific compounds as well and is incorporated herein by reference in its entirety, with detailed definitions being:

Rw is

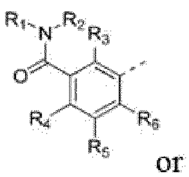

or

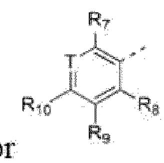

;

$R_1$ is H, $C_{1-6}$ alkyl, $C_{3-6}$ cycloalkyl, 4-6-membered heterocycloalkyl, 5-6-membered heteroaryl or phenyl, wherein the $C_{1-6}$ alkyl, $C_{3-6}$ cycloalkyl, 4-6-membered heterocycloalkyl, 5-6-membered heteroaryl and phenyl are optionally substituted with 1, 2 or 3 $R^a$ groups;

each $R^a$ is independently H, F, Cl, Br, I, -CN, -OH, $C_{1-3}$ alkoxy or $C_{1-3}$ alkyl;

$R_2$ is H or $C_{1-6}$ alkyl;

or $R_1$ and $R_2$, together with the N atom to which they are attached, to form a 4-6-membered heterocycloalkyl, wherein the 4-6-membered heterocycloalkyl is optionally substituted with 1, 2 or 3 $R^b$ groups;

each $R^b$ is independently H, F, Cl, Br, I, -CN, -OH, -$NH_2$, -$OCH_3$, -$OCH_2CH_3$, -$CH_3$ or -$CH_2CH_3$;

$R_3$ is H, F, Cl, Br, I, -OH, -$NH_2$, $C_{1-3}$ alkoxy or $C_{1-3}$ alkyl;
or $R_2$ and $R_3$ are attached together to make the structural unit

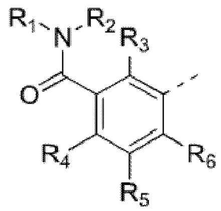

to be

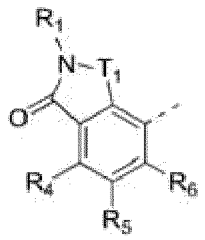 , 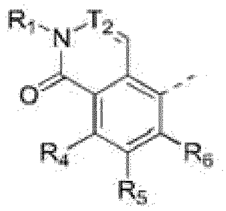 or 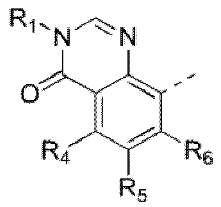 ;

$T_1$ is -$(CR^cR^d)_m$- or -$(CR^cR^d)_n$-O-;
m is 1, 2 or 3;
n is 1 or 2;
$T_2$ is N or CH;
$R^c$ and $R^d$ are each independently H, F, $C_{1-3}$ alkyl or $C_{1-3}$ alkoxy;
$R_4$, $R_5$ and $R_6$ are each independently H, F, Cl, Br, I, $C_{1-3}$ alkyl or $C_{1-3}$ alkoxy;
T is N or CH;
$R_7$ and $R_8$ are each independently H, F, Cl, Br or I;
$R_9$ and $R_{10}$ are each independently H, F, Cl, Br, I, -CN; or
the 4-6-membered heterocycloalkyl and 5-6-membered heteroaryl each contain 1, 2, 3 or 4 heteroatoms independently selected from N, -O- and -S-.

In

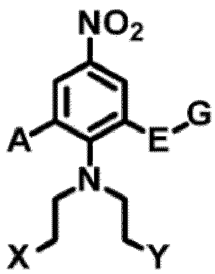 (6)

, the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A), which describes the synthesis and preparation methods of specific compounds as well and is incorporated herein by reference in its entirety, with detailed definitions being:

A is H, $C_1$-$C_6$ alkyl, $C_1$-$C_6$ alkenyl, $C_1$-$C_6$ alkynyl, $CFH_2$, $CF_2H$, $CF_3$, F, Cl, Br, I, $OCF_3$, COR or $CON(R)_2$;
E is SO or $SO_2$;
X is Cl, Br, I or $OSO_2R$;
Y is Cl, Br, I or $OSO_2R$;
each R is independently H or $C_1$-$C_6$ alkyl;
G is a radical group selected from the group consisting of Formulae (B)-(AA):

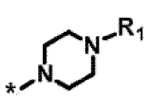
(B)
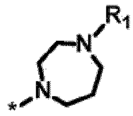
(C)
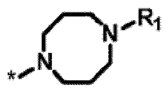
(D)
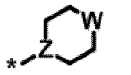
(F)
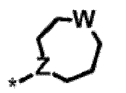
(H)
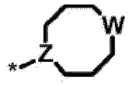
(J)

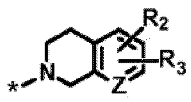
(K)
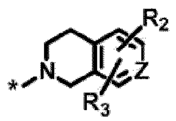
(L)
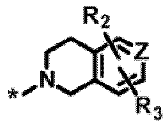
(M)
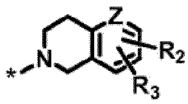
(N)
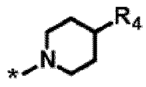
(O)

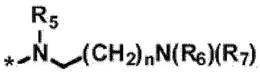
(P)
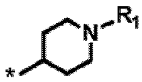
(Q)
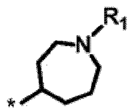
(S)
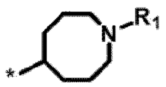
(T)
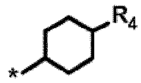
(U)
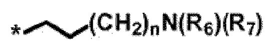
(AA)

wherein:

$R_1$ is H, $C_1$-$C_6$ alkyl, $CH_2(CH_2)_nOH$, $CH_2CH(OH)CH_2OH$, phenyl, pyridyl, benzyl, or pyridylmethyl, provided that when $R_1$ is phenyl, pyridyl, benzyl or pyridylmethyl, $R_1$ is optionally substituted at any available position with $C_1$-$C_6$ alkyl, $C_1$-$C_6$ alkenyl, $C_1$-$C_6$ alkynyl, $OR_6$, $N(R_6)(R_7)$, $CFH_2$, $CF_2H$, $CF_3$, F, Cl, Br, I, $OCF_3$, $COR_6$, $CON(R_6)(R_7)$, $SOR_6$, $SON(R_6)(R_7)$, $SO_2R_6$, $SO_2N(R_6)(R_7)$, CN, or $NO_2$;
$R_2$ and $R_3$ are each independently H, $C_1$-$C_6$ alkyl, $C_1$-$C_6$ alkenyl, $C_1$-$C_6$ alkynyl, $OR_6$, $N(R_6)(R_7)$, $CFH_2$, $CF_2H$, $CF_3$, F, Cl, Br, I, $OCF_3$, $COR_6$, $CON(R_6)(R_7)$, $SOR_6$, $SON(R_6)(R_7)$, $SO_2R_6$, $SO_2N(R_6)(R_7)$, CN or $NO_2$;
$R_4$ is $N(R_6)(R_7)$, OH, $OCH_2(CH_2)_nN(R_6)(R_7)$ or $CH_2(CH_2)_nN(R_6)(R_7)$;
$R_5$ is H or a $C_1$-$C_6$ alkyl group;
$R_6$ and $R_7$ are each independently H or $C_{1\text{-}6}$ alkyl, or $R_6$ and $R_7$ together form a substituted or unsubstituted 5-membered or 6-membered heterocycle;
Z is CH or N;
W is $CH_2$, O, S, SO or $SO_2$;
n is 0 to 6;
* represents a point of attachment to Formula (I).

In

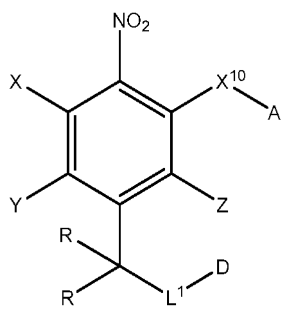
(7)

, the definitions of X, Y, Z, R, D, $L^1$, A and $X^{10}$ are described in the claims of Patent Application PCT/US2016/025665 with Publication No. WO2016161342A3 (corresponding to Chinese Patent Application No. 2016800200132 with Publication No. CN108136214A), which describes the synthesis and preparation methods of specific compounds as well and is incorporated herein by reference in its entirety, with detailed definitions being:

$X^{10}$ is O, S, SO, or $SO_2$;

A is $C_6$-$C_{10}$ aryl or substituted aryl, 5-15-membered heteroaryl or substituted heteroaryl, or $-N=CR^1R^2$; wherein $R^1$

and $R^2$ are each independently hydrogen, $C_1$-$C_6$ alkyl, $C_3$-$C_8$ cycloalkyl, $C_6$-$C_{10}$ aryl, 4-15-membered heterocycle, 5-15-membered heteroaryl, ether, $-CONR^{13}R^{14}$, or $-NR^{13}COR^{14}$;

X, Y, and Z are each independently hydrogen, CN, a halogen group, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15-membered heterocycle, 5-15-membered heteroaryl, ether, - $CONR^{13}R^{14}$, or $-NR^{13}COR^{14}$;

each R is independently hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15-membered heterocycle, 5-15-membered heteroaryl, ether, $-CONR^{13}R^{14}$, or $-NR^{13}COR^{14}$;

$R^{13}$ and $R^{14}$ are each independently hydrogen, $C_1$-$C_6$ alkyl, $C_3$-$C_8$ cycloalkyl, $C_6$-$C_{10}$ aryl, 4-15-membered heterocycle, 5-15-membered heteroaryl, or ether, or $R^{13}$ and $R^{14}$ together with the nitrogen atom to which they are bonded to form a 5-7-membered heterocyclyl;

$L^1$ and D are as defined in the specification, with detailed definitions as follows

$L^1$ is selected from the group consisting of:

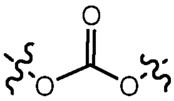, 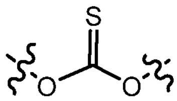, 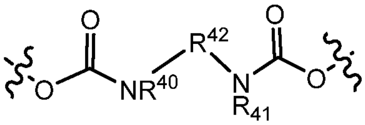, 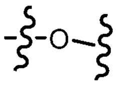,

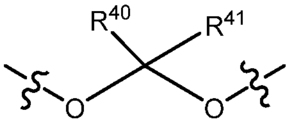, and 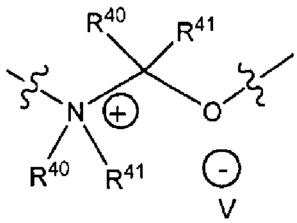;

wherein $R^{40}$ and $R^{41}$ are independently hydrogen, $C_1$-$C_6$ alkyl, $C_2$-$C_6$ alkenyl, $C_2$-$C_6$ alkynyl, $C_3$-$C_8$ cycloalkyl, $C_6$-$C_{10}$ aryl, 4-15-membered heterocycle, or 5-15-membered heteroaryl;

$R^{42}$ is $C_2$-$C_3$ alkane-diyl or heteroalkane-diyl optionally substituted with 1-3 $C_1$-$C_6$ alkyl groups; V (-) is any anion, preferably a pharmaceutically acceptable anion; and

D is a moiety that makes D-OH an anticancer drug, wherein OH is aliphatic hydroxyl or phenolic hydroxyl, or is an OH moiety attached to a phosphorus atom as provided herein; in other words, D is the remaining group after removing hydroxyl from the anticancer drug D-OH;
or

$L^1$ is:

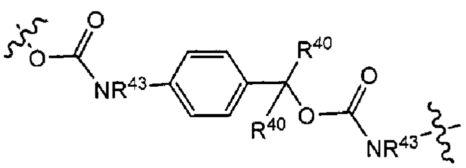, 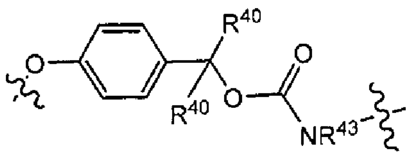, or 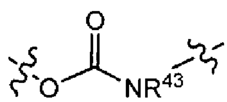;

wherein $R^{40}$ is as defined above, $R^{43}$ is hydrogen or together with D forms a heterocycle, and the phenyl moiety is optionally substituted; and

D is a moiety that makes $D-NR^{43}H$ an anticancer drug; in other words, D is the remaining group after removing amino or amine from the anticancer drug D- $NR^{43}H$;

or

$L^1$ is a bond, -O-C($R^{40}R^{41}$)-, -O-C($R^{40}R^{41}$)-N$R^{40}R^{41}$(+)-C($R^{40}R^{41}$)- or

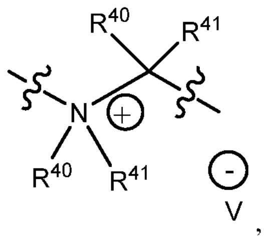

wherein $R^{40}$, $R^{41}$, and V are as defined above; and

D is an anticancer drug containing primary amine or secondary amine, wherein the primary amine or the secondary amine is bonded to $L^1$; and

wherein the alkyl, alkenyl, alkynyl, cycloalkyl, aryl, heterocycle, heteroaryl, and ether are optionally substituted.

In

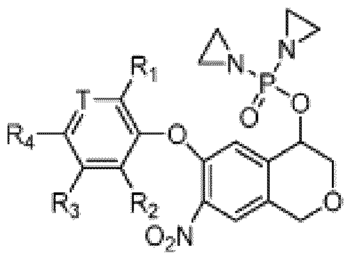
(8)

, the definitions of $R_1$, $R_2$, $R_3$, $R_4$, and T are described in the claims of Patent Application PCT/CN2021/118597 with Publication No. WO2022057838A1, which describes the synthesis and preparation methods of specific compounds as well and is incorporated herein by reference in its entirety, with detailed definitions being:

T is N or CH;

$R_1$ and $R_2$ are each independently H, F, Cl, Br, I, or $C_{1-3}$ alkyl, wherein the $C_{1-3}$ alkyl is optionally substituted with 1, 2, or 3 $R_a$ groups;

each $R_a$ is independently F, Cl, Br, I, -CN, -OH, or -$NH_2$;

$R_3$ and $R_4$ are each independently H, F, Cl, Br, I, CN, $C_{1-3}$ alkyl, $C_{1-3}$ alkoxy,

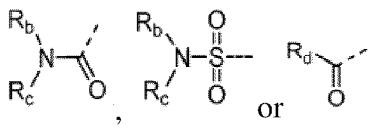

, wherein the $C_{1-3}$ alkyl is optionally substituted with 1, 2, or 3 $R_e$ groups; $R_b$ and $R_c$ are each independently H, -$CH_3$, -$CH_2CH_3$, -$(CH_2)_2CH_3$, or -$CH(CH_3)_2$;

$R_d$ is -$CH_3$, -$CH_2CH_3$, -$(CH_2)_2CH_3$, or -$CH(CH_3)_2$;

each $R_e$ is independently F, Cl, Br, I, -CN, -OH, or -$NH_2$.

In

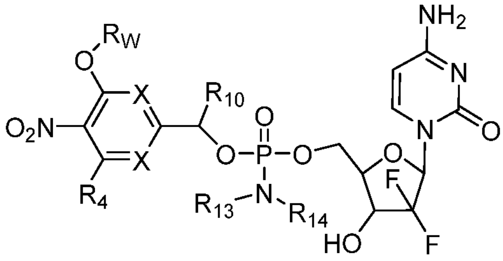

(9)

, or a pharmaceutically acceptable salt thereof, the definitions of $R_w$, X, $R_4$, $R_{10}$, $R_{13}$, and $R_{14}$ are described in the claims of Patent Application PCT/CN2022/098082 with Publication No. WO2022258043A1, which describes the synthesis and preparation methods of specific compounds as well and is incorporated herein by reference in its entirety, with detailed definitions being:

two X groups are each independently $CR^{15}$ or N;

$R_{13}$ and $R_{14}$ are each independently hydrogen; $C_1$-$C_6$ alkyl; cycloalkyl; alkenyl; alkynyl; $C_6$-$C_{20}$ aryl; 5-20-membered heterocyclyl; halogenated $C_1$-$C_6$ alkyl, cycloalkyl, alkenyl, or alkynyl; halogenated $C_6$-$C_{20}$ aryl; or halogenated 5-20-membered heterocyclyl; and $R_{13}$ and $R_{14}$ are not hydrogen at the same time;

$R_{10}$ is hydrogen; $C_1$-$C_6$ alkyl; cycloalkyl; alkenyl; alkynyl; $C_6$-$C_{20}$ aryl; 5-20-membered heterocyclyl; halogenated $C_1$-$C_6$ alkyl, cycloalkyl, alkenyl, or alkynyl; halogenated $C_6$-$C_{20}$ aryl; or halogenated 5-20-membered heterocyclyl;

or $R_{10}$ may be linked to $R_{13}$ or $R_{14}$ in accordance with the definitions of $R_{10}$, $R_{13}$, and $R_{14}$ above to form a 5-9-membered ring;

$R_4$ and $R^{15}$ are each independently hydrogen; halogen; $C_1$-$C_6$ alkyl; cycloalkyl; alkenyl; alkynyl; alkoxy; cyano; 5-20-membered heterocyclyl; $C_6$-$C_{20}$ aryl; or halogenated $C_1$-$C_6$ alkyl, cycloalkyl, alkenyl, alkynyl, alkoxy, 5-20-membered heterocyclyl, or $C_6$-$C_{20}$ aryl;

or $R_{10}$ and $R^{15}$ in accordance with the definitions of $R_{10}$ and $R^{15}$ above may form a 4-12-membered cyclic hydrocarbon or heterocycle;

$R_w$ is

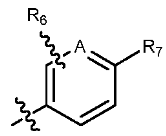

A is $CR^{16}$ or N, wherein the position of A is variable on the ring;

$R^{16}$ is hydrogen; $C_1$-$C_6$ alkyl; cycloalkyl; alkenyl; alkynyl; $C_6$-$C_{20}$ aryl; 5-20-membered heterocyclyl; halogenated $C_1$-$C_6$ alkyl, cycloalkyl, alkenyl, or alkynyl; halogenated $C_6$-$C_{20}$ aryl; or halogenated 5-20-membered heterocyclyl;

$R_6$ and $R_7$ meet the following conditions:

$R_6$ and $R_7$ are each independently hydrogen; halogen; cyano; hydroxyl; $C_1$-$C_6$ alkyl, cycloalkyl, alkenyl, alkynyl, alkoxy, 5-20-membered heterocyclyl, or $C_6$-$C_{20}$ aryl; or halogenated $C_1$-$C_6$ alkyl, cycloalkyl, alkenyl, alkynyl, alkoxy, 5-20-membered heterocyclyl, or $C_6$-$C_{20}$ aryl; or cyano-substituted $C_1$-$C_6$ alkyl, cycloalkyl, alkenyl, alkynyl, alkoxy, 5-20-membered heterocyclyl, or $C_6$-$C_{20}$ aryl; or hydroxyl-substituted $C_1$-$C_6$ alkyl, cycloalkyl, alkoxy, 5-20-membered heterocyclyl, or $C_6$-$C_{20}$ aryl; or $-CONR^{11}R^{12}$; or - $CH_2NR^{11}R^{12}$;

or $R_6$ and $R_7$ are linked to form

a 5-8-membered single heterocycle or fused heterocycle containing at least one N or S or O or containing two or three of N, S, and O simultaneously;

or a 5-8-membered single heterocycle or fused heterocycle containing at least one N or S or O or containing two or three of N, S, and O simultaneously, which is substituted with $C_1$-$C_6$ alkyl;

$R_6$ may be linked to $CR^{16}$ to form a 5-9-membered ring, heterocycle, or aromatic heterocycle;

$R^{11}$ and $R^{12}$ meet the following conditions:
$R^{11}$ and $R^{12}$ are each independently $C_1$-$C_6$ alkyl or halogenated $C_1$-$C_6$ alkyl, or $R^{11}$ and $R^{12}$ in accordance with the definitions above together with N in $-CONR^{11}R^{12}$ form a 5-7-membered ring, or together with N in $-CH_2NR^{11}R^{12}$ form a 5-7-membered ring.

In

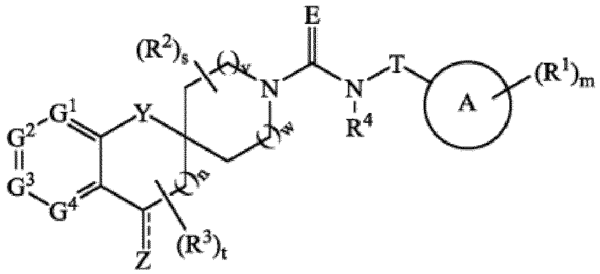

(10)

, the definitions of $R^1$, $R^3$, $R^4$, $G^1$, $G^2$, $G^3$, $G^4$, E, T, Y, Z, m, n, s, t, v, w, and Ring A are described in the claims of Patent Application CN202210585771.6 with Publication No. CN115403579A, which describes the synthesis and preparation methods of specific compounds as well and is incorporated herein by reference in its entirety, with detailed definitions being:

$G^1$, $G^2$, $G^3$, and $G^4$ are the same or different, and are each independently $CR^5$ or an N atom;

each $R^5$ is the same or different, and each is independently selected from the group consisting of: a hydrogen atom, halogen, alkyl, alkenyl, alkynyl, alkoxy, hydroxyl, cyano, amino, nitro, $-NR^aR^b$, $-C(O)NR^aR^b$, cycloalkyl, heterocyclyl, aryl, and heteroaryl, wherein the alkyl, alkenyl, alkynyl, alkoxy, cycloalkyl, heterocyclyl, aryl, and heteroaryl are each independently optionally substituted with one or more substituents selected from halogen, alkyl, haloalkyl, alkoxy, haloalkoxy, hydroxyalkyl, hydroxyl, oxo, cyano, amino, nitro, cycloalkyl, heterocyclyl, aryl, and heteroaryl;

Y is selected from the group consisting of: $-(C(R^{y2}R^{y3}))_f-NR^{y1}-$, $-(C(R^{y2}R^{y3}))_g-O-$, $-(C(R^{y2}R^{y3}))_h-S-$, $-(C(R^{y2}R^{y3}))_h-S(O)-$, $-(C(R^{y2}R^{y3}))_h-S(O)_2-$, $-C(R^{y2}R^{y3})-$, $-NR^{y1}-(C(R^{y2}R^{y3}))_f-$, $-O-(C(R^{y2}R^{y3}))_g-$, $-S-(C(R^{y2}R^{y3}))_h-$, $-S(O)-(C(R^{y2}R^{y3}))_h-$, and $-S(O)_2-(C(R^{y2}R^{y3}))_h-$;

$R^{y1}$ is selected from the group consisting of: a hydrogen atom, alkyl, haloalkyl, hydroxyalkyl, cycloalkyl, and heterocyclyl;

$R^{y2}$ and $R^{y3}$ are the same or different, and are each independently selected from the group consisting of: a hydrogen atom, halogen, alkyl, haloalkyl, hydroxyalkyl, cycloalkyl, and heterocyclyl;

or $R^{y2}$ and $R^{y3}$ together form =O;

Z is O or OH;

$\overline{\text{-----}}$ is a single bond or double bond, and Z is OH when $\overline{\text{-----}}$ is a single bond, and Z is O when $\overline{\text{-----}}$ is a double bond;

E is selected from NH, an O atom, and an S atom;

T is selected from $-C(R^{T1}R^{T2})-$, $-NR^{T3}-$, or -O-;

$R^{T1}$ and $R^{T2}$ are the same or different, and are each independently selected from a hydrogen atom, a deuterium atom, halogen, alkyl, haloalkyl, hydroxyalkyl, cycloalkyl, and heterocyclyl;

or $R^{T1}$ and $R^{T2}$ together with the carbon atom to which they are attached form a cycloalkyl or heterocyclyl, and each cycloalkyl or heterocyclyl is independently optionally substituted with one or more substituents selected from halogen, alkyl, and hydroxyl;

$R^{T3}$ is selected from a hydrogen atom, alkyl, haloalkyl, hydroxyalkyl, cycloalkyl, and heterocyclyl;

Ring A is 6-10-membered aryl or 5-10-membered heteroaryl;

each $R^1$ is the same or different, and each is independently selected from the group consisting of: a hydrogen atom, a deuterium atom, halogen, alkyl, alkenyl, alkynyl, alkoxy, hydroxyl, cyano, $-NR^aR^b$, - $C(O)NR^aR^b$, $-S(O)NR^aR^b$, $-S(O)_2NR^aR^b$, $-S(O)R^c$, $-S(O)_2R^c$, $-B(OR^d)_2$, nitro, cycloalkyl, heterocyclyl, aryl, and heteroaryl, wherein the alkyl, alkenyl, alkynyl, alkoxy, cycloalkyl, heterocyclyl, aryl, and heteroaryl are each independently optionally substituted with one or more substituents selected from halogen, alkyl, haloalkyl, alkoxy, haloalkoxy, hydroxyalkyl, hydroxyl, oxo, cyano, $-NR^aR^b$, $-C(O)NR^aR^b$, $-S(O)NR^aR^b$, $-S(O)_2NR^aR^b$, $-S(O)R^c$. $-S(O)_2R^c$, $-B(OR^d)_2$, nitro, cycloalkyl, heterocyclyl, aryl, and heteroaryl;

$R^a$ and $R^b$ are the same or different, and are each independently selected from the group consisting of: a hydrogen atom, alkyl, haloalkyl, hydroxyl, hydroxyalkyl, $-C(O)R^e$, cycloalkyl, and heterocyclyl; or $R^a$ and $R^b$ together with the nitrogen atom to which they are attached form a cycloalkyl or heterocyclyl, wherein the cycloalkyl or heterocyclyl is optionally substituted with one or more substituents selected from halogen, alkyl, haloalkyl, alkoxy, haloalkoxy, hydroxyalkyl, hydroxyl, cyano, amino, nitro, cycloalkyl, heterocyclyl, aryl, and heteroaryl;

$R^c$ is selected from alkyl, cycloalkyl, heterocyclyl, aryl, and heteroaryl, which are optionally substituted with one or more substituents selected from halogen, alkyl, haloalkyl, alkoxy, haloalkoxy, hydroxyalkyl, hydroxyl, cyano, amino, nitro, cycloalkyl, heterocyclyl, aryl, and heteroaryl;

$R^d$ is a hydrogen atom or $C_{1-6}$ alkyl;

$R^e$ is selected from alkyl, alkoxy, cycloalkyl, heterocyclyl, aryl, and heteroaryl, which are optionally substituted with one or more substituents selected from halogen, alkyl, haloalkyl, alkoxy, haloalkoxy, hydroxyalkyl, hydroxyl, cyano, amino, nitro, cycloalkyl, heterocyclyl, aryl, and heteroaryl;

each $R^2$ is the same or different, and each is independently selected from a hydrogen atom, a deuterium atom, halogen, alkyl, haloalkyl, alkoxy, haloalkoxy, hydroxyalkyl, oxo, hydroxyl, cyano, amino, nitro, cycloalkyl, heterocyclyl, aryl, and heteroaryl;

each $R^3$ is the same or different, and each is independently selected from a hydrogen atom, a deuterium atom, halogen, alkyl, haloalkyl, alkoxy, haloalkoxy, hydroxyalkyl, oxo, hydroxyl, cyano, amino, nitro, cycloalkyl, heterocyclyl, aryl, and heteroaryl;

$R^4$ is selected from a hydrogen atom, alkyl, haloalkyl, hydroxyl, and hydroxyalkyl;

n is 0, 1, 2, or 3;

v is 0, 1, or 2;

w is 0, 1, or 2;

f is 0, 1, or 2;

g is 0, 1, or 2;

h is 0, 1, or 2;

m is 0, 1, 2, 3, 4, or 5;

s is 0, 1, 2, 3, 4, 5, 6, 7, or 8;

t is 0, 1, 2, 3, 4, 5, or 6;

provided that

when Y is an -O- atom and E is an O atom, Ring A is phenyl or 5-6-membered heteroaryl, and $G^3$ is $CR^5$ or an N atom, and $R^5$ is not a hydrogen atom;

when Y is an -O- atom and E is an S atom, Ring A is phenyl or 5-6-membered heteroaryl;

when $G^1$, $G^2$, $G^3$, and $G^4$ are all $CR^5$, and Y is $NR^{y1}$, and n, v, and w are all 1, and E is an O atom, 1) T is not $CH_2$ or $CD_2$; 2) at least one $R^2$ or $R^3$ is a deuterium atom; 3) $R^4$ is selected from alkyl, haloalkyl, hydroxyl, and hydroxyalkyl; 4) one $R^1$ is 3-8-membered cycloalkyl or 5-8-membered heterocyclyl, wherein 3-8-membered cycloalkyl or 5-8-membered heterocyclyl is optionally substituted with one or more substituents selected from halogen, alkyl, haloalkyl, alkoxy, haloalkoxy, hydroxyalkyl, hydroxyl, oxo, cyano, amino, nitro, cycloalkyl, heterocycle, aryl, and heteroaryl; 5) Ring A is $R^d$, which is a hydrogen atom or $C_{1-6}$ alkyl.

In

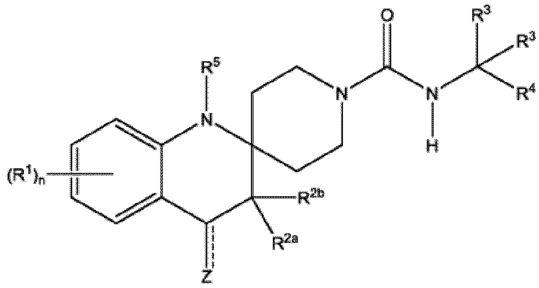

(11),

or a pharmaceutically acceptable salt thereof, the definitions of $R^1$, $R^{2a}$, $R^{2b}$, $R^3$, $R^4$, $R^5$, n, and Z are described in the claims of Patent Application PCT/IB2020/057285 with Publication No. WO2021005586A1 (corresponding to Chinese Patent Application No. CN202080053804.1 with Publication No. CN114206870A), which describes the synthesis and preparation methods of specific compounds as well and is incorporated herein by reference in its entirety, with detailed definitions being:

- - - - - is a single bond or double bond;

Z is OH when - - - - - is a single bond; or is O when - - - - - is a double bond;

each $R^1$ is independently selected from the group consisting of: $(C_1\text{-}C_6)$-alkyl, $(C_1\text{-}C_6)$-alkoxy, $(C_0\text{-}C_4)$-alkyl-$N(R^8)_2$, and a halogen group;

$R^{2a}$ and $R^{2b}$ are each independently selected from the group consisting of: H, $(C_1\text{-}C_6)$-alkyl, and a halogen group;

each $R^3$ is independently selected from the group consisting of: H and a halogen group;

$R^4$ is selected from the group consisting of: aryl; 5-6-membered heteroaryl containing 1, 2, 3, or 4 heteroatoms independently selected from N, O, and S; and 9-10-membered fused bicyclic heteroaryl containing 1, 2, 3, or 4 heteroatoms independently selected from N, O, and S; wherein any one of the above is optionally substituted with one or more $R^6$ groups;

$R^5$ is selected from the group consisting of: H, $(C_1\text{-}C_6)$-alkyl, $(C_2\text{-}C_6)$-alkenyl, $(C_0\text{-}C_4)$-alkyl-$OR^8$, $(C_1\text{-}C_4)$-alkyl-$(C_3\text{-}C_{10})$-cycloalkyl, halo-$(C_1\text{-}C_6)$-alkyl, $(C_2\text{-}C_3)$-alkynyl, and $(C_1\text{-}C_4)$-alkyl-$N(R^{10})_2$;

each $R^6$ is independently selected from the group consisting of: a halogen group, $(C_1\text{-}C_6)$-alkyl, $(C_1\text{-}C_6)$-alkoxy, halo-$(C_1\text{-}C_6)$-alkyl, OH, aryl, 3-6-membered heterocycle, 5-6-membered heteroaryl, $(C_0\text{-}C_4)$-alkyl-$S(O)_m$-$(C_1\text{-}C_6)$-alkyl, halo-$(C_1\text{-}C_6)$-alkoxy, $(C_0\text{-}C_4)$-alkyl-$S(O)_mN(R^8)_2$, $(C_0\text{-}C_4)$-alkyl-$N(R^8)_2$, (Co-$C_4$)-alkyl-(CO)$OR^7$, $N(R^8)S(O)_m$-$(C_1\text{-}C_6)$-alkyl, $N(R^8)S(O)_m$-$(C_3\text{-}C_6)$-cycloalkyl, $OP(O)(OH)_2$, $(C_0\text{-}C_3)$-alkyl-(CO)$NHR^{11}$, $(C_0\text{-}C_3)$-alkyl-

$OR^7$, and ($C_3$-$C_{10}$)-cycloalkyl; wherein each $R^6$ is optionally substituted with one to three $R^9$ groups when not being a halogen group, OH, or $OP(O)(OH)_2$; or two adjacent $R^6$ groups together with the atoms to which they are attached form a 5-7-membered heterocycle or ($C_5$-$C_8$)-cycloalkyl;

each $R^7$ and $R^8$ are independently selected from the group consisting of: H and ($C_1$-$C_6$)-alkyl optionally substituted with one to three $R^9$ groups;

each $R^9$ is independently selected from the group consisting of: a halogen group; -OH; amino, ($C_1$-$C_4$)-alkylamino, di-($C_1$-$C_4$)-alkylamino, $OP(O)(OH)_2$; ($C_1$-$C_6$)-alkyl; ($C_1$-$C_3$)-alkynyl; ($C_1$-$C_6$)-alkoxy; halo-($C_1$-$C_6$)-alkyl; ($C_0$-$C_4$)-alkyl-$S(O)_m$-($C_1$-$C_6$)-alkyl; halo-($C_1$-$C_6$)-alkoxy; 3-6-membered heterocycle optionally substituted with oxo (=O); ($C_0$-$C_4$)-alkyl-$S(O)_mN(R^{10})_2$; ($C_0$-$C_4$)-alkyl-(CO)$R^{10}$; ($C_0$-$C_4$)-alkyl-(CO)$OR^{10}$; ($C_0$-$C_4$)-alkyl-$NR^{10}S(O)_m$-($C_1$-$C_6$)-alkyl; ($C_0$-$C_4$)-alkyl-$OR^{10}$; ($C_0$-$C_4$)-alkyl-$N(R^{10})_2$; ($C_0$-$C_4$)-alkyl-CN; ($C_0$-$C_4$)-alkyl-$N(R^{10})_2$; and ($C_0$-$C_4$)-alkyl-(CO)$N(R^{10})_2$;

each $R^{10}$ is independently selected from the group consisting of: H; ($C_1$-$C_6$)-alkyl; and 3-6-membered heterocycle, which is optionally substituted with one or more of: ($C_1$-$C_6$)-alkyl and oxo (=O);

each $R^{11}$ is selected from the group consisting of: H; 4-6-membered heterocycle optionally substituted with one to four $R^{12}$ groups; ($C_3$-$C_6$)-cycloalkyl optionally substituted with one to four $R^{12}$ groups; ($C_0$-$C_3$)-alkyl-($C_3$-$C_6$)-cycloalkyl-($C_1$-$C_3$)-alkyl optionally substituted with a halogen group; $CH_2$-aryl optionally substituted with one to three $R^{12}$ groups; ($C_1$-$C_6$)-alkyl, ($C_2$-$C_6$)-alkenyl, and ($C_2$-$C_6$)-alkynyl, each one of which is optionally substituted with one or more $R^{13}$ groups;

each $R^{12}$ is independently selected from the group consisting of: OH; ($C_1$-$C_3$)-alkoxy; $NH_2$; and ($C_1$-$C_3$)-alkyl optionally substituted with one or more OH groups;

each $R^{13}$ is independently selected from the group consisting of: a halogen group, OH, amino, ($C_1$-$C_4$)-alkylamino, di-($C_1$-$C_4$)-alkylamino, ($C_1$-$C_3$)-alkoxy, and C(O)-($C_3$-$C_8$)-cycloalkyl;

m is 0, 1, or 2; and

n is 0, 1, or 2.

Particularly, chemical structures of specific compounds of Formulae (1)-(11) are described in claim 5, thus unnecessary details are not given here.

Therein specific compounds of Formula (1) are referenced to compounds in Patent Application PCT/US2016/021581 with Publication No. WO2016145092A1 (corresponding to Chinese Patent Application No. 2016800150788 with Publication No. CN107530556A), which is incorporated herein by reference in its entirety;

specific compounds of Formulae (2) and (3) are referenced to compounds in Patent Application PCT/CN2020/089692 with Publication No. WO2020228685A1, which is incorporated herein by reference in its entirety;

specific compounds of Formula (4) are referenced to compounds in Patent Application PCT/US2016/021581 with Publication No. WO2016145092A1 (corresponding to Chinese Patent Application No. 2016800150788 with Publication No. CN107530556A) and PCT/CN2020/089692 with Publication No. WO2020228685A1, both of which are incorporated herein by reference in their entirety;

specific compounds of Formula (5) are referenced to compounds in Patent Application PCT/CN2020/120281 with Publication No. WO2021068952A1, which is incorporated herein by reference in its entirety;

specific compounds of Formula (6) are referenced to compounds in Patent Application PCT/NZ2019/050030 with Publication No. WO2019190331A1 (corresponding to Chinese Patent Application No. 2019800234236 with Publication No. CN111918864A), which is incorporated herein by reference in its entirety;

specific compounds of Formula (7) are referenced to compounds in Patent Application PCT/US2016/025665 with Publication No. WO2016161342A3 (corresponding to Chinese Patent Application No. 2016800200132 with Publication No. CN108136214A), which is incorporated herein by reference in its entirety;

specific compounds of Formula (8) are referenced to compounds in Patent Application PCT/CN2021/118597 with Publication No. WO2022057838A1, which is incorporated herein by reference in its entirety;

specific compounds of Formula (9) are referenced to compounds in Patent Application PCT/CN2022/098082 with Publication No. WO2022258043A1, which is incorporated herein by reference in its entirety;

specific compounds of Formula (10) are referenced to compounds in Patent Application CN202210585771.6 with Publication No. CN115403579A, which is incorporated herein by reference in its entirety;

specific compounds of Formula (11) are referenced to compounds in Patent Application PCT/IB2020/057285 with Publication No. WO2021005586A1 (corresponding to Chinese Patent Application No. CN202080053804.1 with Publication No. CN114206870A), which is incorporated herein by reference in its entirety.

For the preparation methods and spectral data of specific compounds of Formulae (1)-(11), see the patent applications above.

Evidently, the compounds of structural Formula (1), similar to AST-3424, are prodrugs of a phosphoramidate alkylating agent, and can be activated by the AKR1C3 enzyme to form T (a phosphoramidate alkylating agent, AST-2660 is a phosphoramidate alkylating agent) to exert anticancer efficacy:

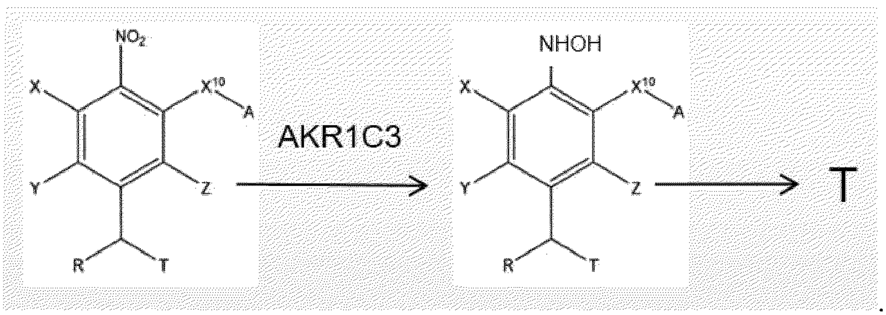


Particularly, taking AST-3424 as an example, these compounds as aldo-keto reductase AKR1C3 specific substrates, can be quickly and effectively reduced only in cancer cells with high expression of AKR1C3, thereby releasing the cytotoxin DNA alkylating agent AST-2660, which is cross-linked with the DNA to cause cancer cell death:

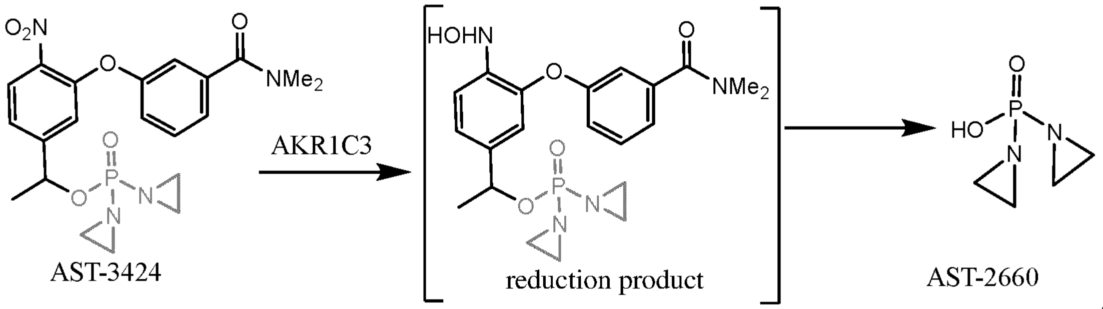

AST-3424 reduction product AST-2660

Evidently, the compounds of structural Formulae (2) and (3) (especially the compound AST therein), similar to AST-342, are prodrugs of AST-2660, and can be activated by the AKR1C3 enzyme to form AST-2660 (a DNA alkylating agent) to exert anticancer efficacy:

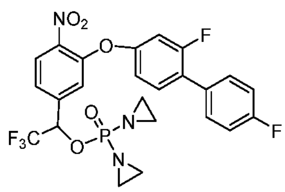
(AST)

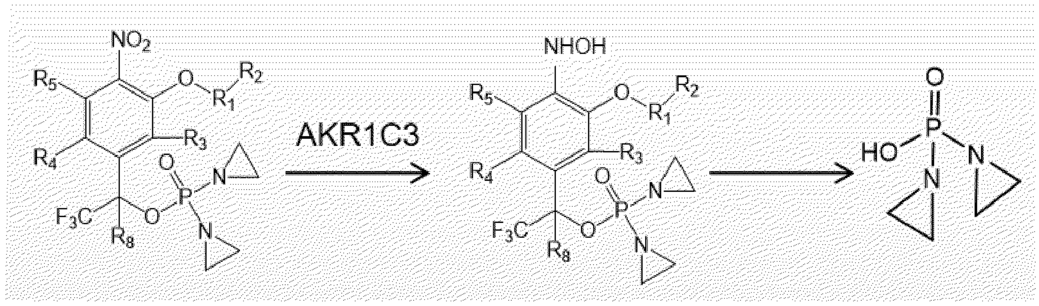


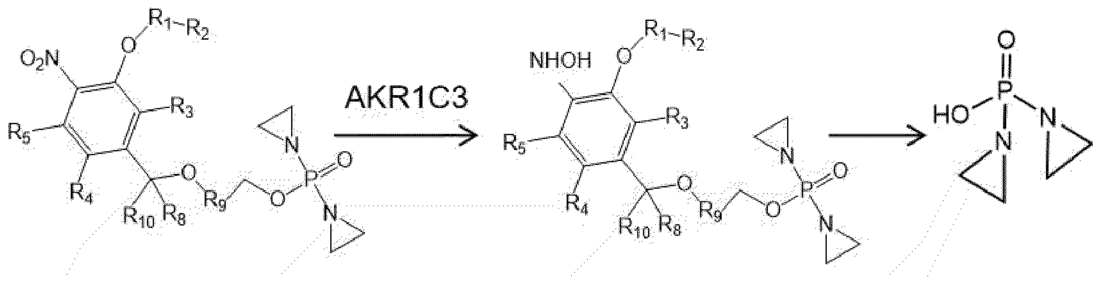


Evidently, the compounds of structural Formula (4), similar to AST-342 and AST, are prodrugs of AST-2660, and can be activated by the AKR1C3 enzyme to form AST-2660 to exert anticancer efficacy:

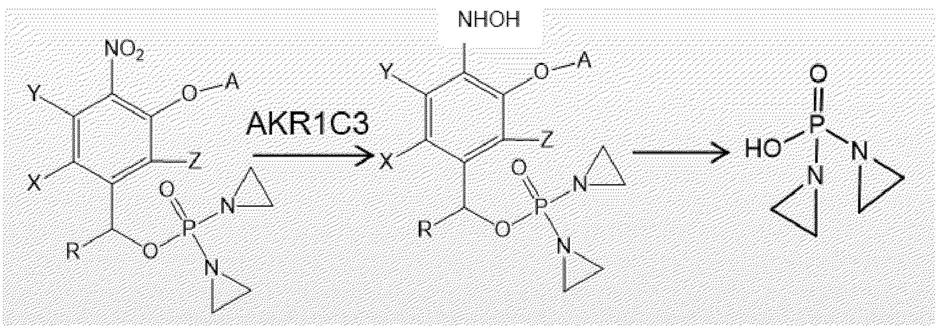


.

Evidently, the compounds of structural Formula (5), similar to AST-342 and AST, are prodrugs of AST-2660, and can be activated by the AKR1C3 enzyme to form AST-2660 (a DNA alkylating agent) to exert anticancer efficacy:

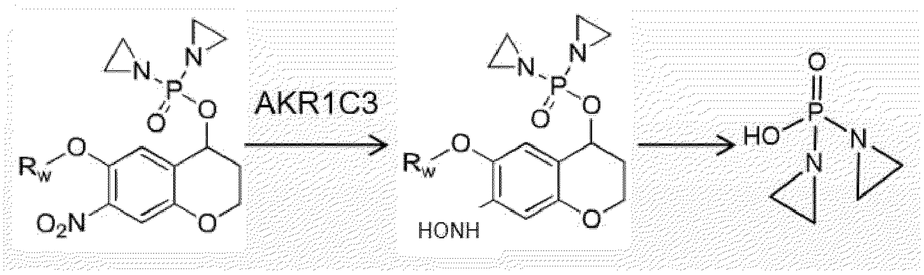


Evidently, the compounds of structural Formula (6), similar to AST-3424 and AST in the mechanism, are prodrugs of the nitrogen mustard analog

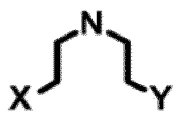

, and can be activated by the AKR1C3 enzyme to form the nitrogen mustard analog

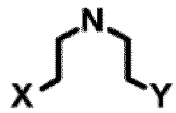

(a DNA alkylating agent) to exert anticancer efficacy:

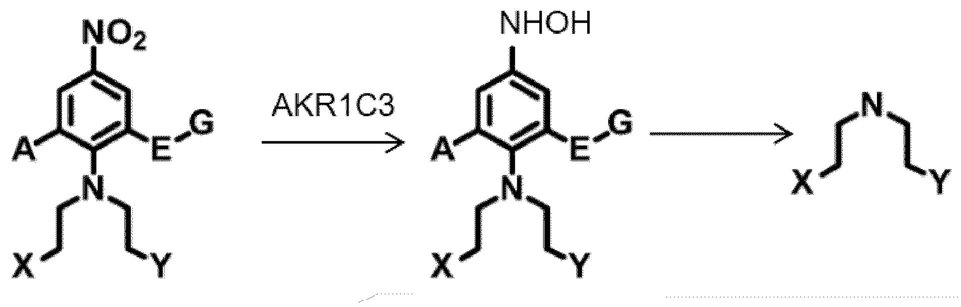

Evidently, the compounds of structural Formula (7) can be activated by the AKR1C3 enzyme to form the drug D capable of inhibiting cancer cell proliferation or tumor growth to exert anticancer efficacy:

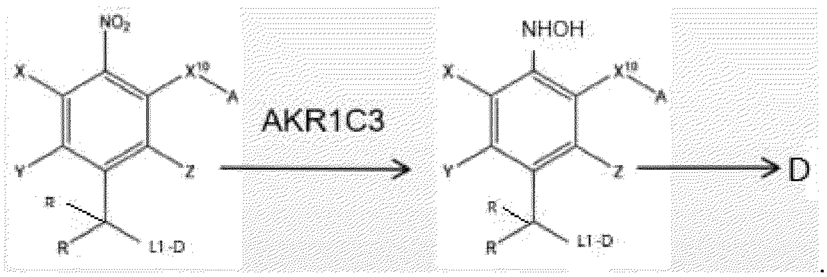

.

D may be an anticancer active substance such as paclitaxel, SN-38, or gemcitabine.

Paclitaxel can upset the dynamic equilibrium between tubulin and microtubule-forming tubulin dimers, induce and promote tubulin polymerization, microtubule assembly, and prevention of depolymerization, thereby stabilizing microtubules and inhibiting mitosis of cancer cells and triggering apoptosis, and thus effectively preventing proliferation of cancer cells to exert anticancer effect.

SN-38 can inhibit topoisomerase I essential for DNA replication in human cells, induce DNA single-strand damage and block DNA replication to exert cytotoxicity.

Gemcitabine (dFdC), as a cytidine nucleoside (pyrimidine) analog, can be incorporated into replicative DNA, thus inhibiting DNA replication. Deoxycytidine kinase (dCK) activates the phosphate of gemcitabine after it enters the body, thus converting it into gemcitabine diphosphate (dFdCDP) and triphosphate (dFdCTP). These active drug metabolites have various inhibitory effects on DNA replication.

Similarly, the compounds of structural Formulae (8), (9), (10), and (11) can also be activated by the AKR1C3 enzyme to form a drug capable of inhibiting cancer cell proliferation or tumor growth, such as AST-2660, gemcitabine, or a KARS inhibitor, to exert anticancer efficacy.

A KARS inhibitor is a lysyl-t-RNA synthetase inhibitor. A lysyl-t-RNA synthetase, as part of the multi-t-RNA synthetase complex, is a ubiquitous enzyme essential for protein synthesis,

Further, prodrug compounds are preferably selected from the group consisting of:

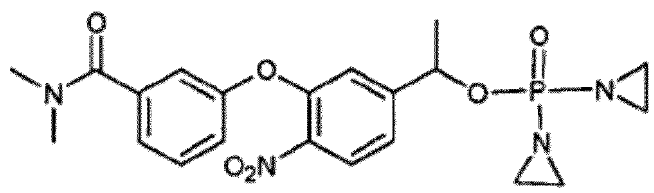

, ,

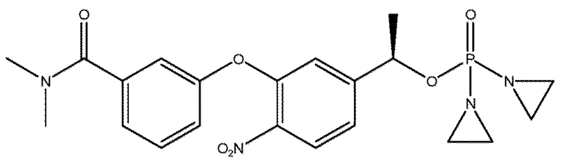,
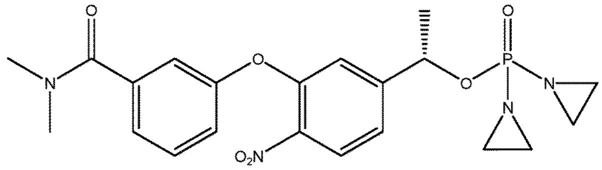,

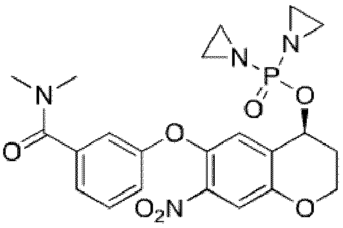,
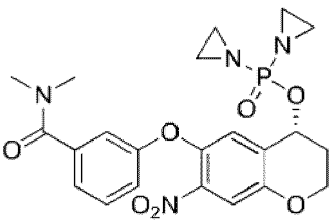,
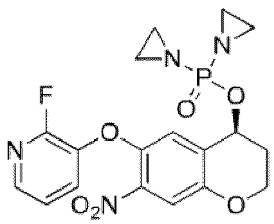

, and

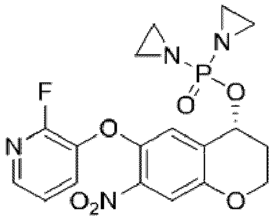.

Regarding the medicament described herein, it refers to a drug or preparation. The prepared medicament contains a specific dosage range of the active ingredients AKR1C3-enzyme-activated compounds of Formulae (1)-(11) or salts or solvates thereof, and/or the prepared medicament is in a specific dosage form and is administered using a specific mode of administration.

In addition to the AKR1C3-enzyme-activated prodrug compounds of Formulae (1)-(11), the above-mentioned medicament should also be supplemented with pharmaceutically acceptable auxiliaries or excipients based on the specific characteristics of the drug, medicament, or preparation. The medicament may be in any dosage form for clinical administration, such as tablets, suppositories, dispersible tablets, enteric tablets, chewable tablets, orally disintegrating tablets, capsules, sugar-coated preparations, granules, dry powders, oral solutions, small dose injections, lyophilized powder for injection, or infusion solutions. According to the specific dosage form and the mode of administration, the pharmaceutically acceptable auxiliaries or excipients in the medicament may include one or more of the following: diluents, solubilizers, disintegrants, suspending agents, lubricants, binding agents, fillers, flavoring agents, sweeteners, antioxidants, surfactants, preservatives, wrapping agents, and pigments, etc.

"Cancer" refers to leukemia, lymphoma, carcinoma, and other malignant tumors (including solid tumors) with potentially unrestrained growth that can expand locally by invasion and systemically by metastasis.

Listed here are examples of cancer treatable with such AKR1C3-enzyme-activated prodrugs as AST-3424, AST, etc., including, but not limited to, cancers of the adrenal gland, bone, brain, breast, bronchus, colon and/or rectum, gallbladder, head and neck, kidney, larynx, liver, lung, nervous tissue, pancreas, prostate, accessory thyroid gland, skin, stomach, and thyroid gland. Certain other examples of cancer include acute and chronic lymphocytic and granulocytic neoplasms, adenocarcinoma, adenoma, basal cell carcinoma, uterine cervical epithelial dysplasia and carcinoma *in situ,* Ewing's sarcoma, epidermoid carcinoma, giant cell tumor, glioblastoma multiforme, pilocytic tumor, intestinal ganglioneuroma, hyperplastic corneal nerve tumor, islet cell carcinoma, Kaposi's sarcoma, leiomyoma, leukemia, lymphoma, malignant carcinoid, malignant melanoma, malignant hypercalcemia, marfanoid habitus tumor, medullary epithelial carcinoma, metastatic skin cancer, mucosal neuroma, myeloma, mycosis fungoides, neuroblastoma, osteosarcoma, osteogenic and other sarcomas, ovarian tumor, pheochromocytoma, polycythemia vera, primary brain tumor, small cell lung cancer, ulcerative and papillary squamous cell carcinoma, hyperplasia, seminoma, soft tissue sarcoma, retinoblastoma, rhabdomyosarcoma, renal cell tumor, local skin lesion, reticulum cell sarcoma, and Wilm's tumor.

For the recommended dosage and administration dosage form of AST-3424 or such AKR1C3-enzyme-activated DNA alkylating agent prodrugs for treating cancer, see patent applications submitted by Threshold Pharmaceuticals, Inc., Ascentawits Pharmaceuticals, LTD., OBI Pharma Inc., etc. (e.g. WO2017087428A1, WO2019062919A1, and WO2021008520A1) and clinical trials registered with FDA and NMPA (CTR20201915, CTR20201908,

CTR20191399, CTR20191371, and NCT04315324, NCT03592264).

Treatment described in the present application includes single drug therapy and therapy in combination with other drugs.

Monotherapy refers to single drug therapy. Combination therapy refers to combined drug therapy. Single drug therapy refers to the use of only one anticancer drug in a course of treatment. Combination therapy refers to the simultaneous or sequential use of two or more anticancer drugs in a course of treatment.

Generally speaking, for combination therapy, different administration dosages and administration cycles need to be explored according to the characteristics of the disease and the types of drugs to be used in combination; and only according to the above conditions, the combined drug therapy regimen obtained from the exploration may achieve better therapeutic effect as compared with single drug therapy.

The administration dosages and administration cycles of drugs in both monotherapy and combination therapy regimens need to be explored through clinical trials with the reference to the dosages and administration regimens of the above AST-3424 and analogs thereof and drugs combined.

The preparation methods and spectral data of AST-3424 (OBI-3424),

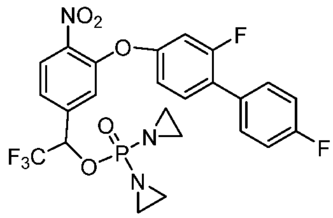

(hereinafter referred to as AST) and

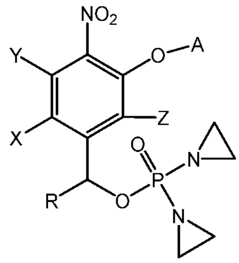

have been disclosed in the following patent applications: PCT/US2016/021581 with Publication No. WO2016145092A1 (corresponding to Chinese Patent Application No. 2016800150788 with Publication No. CN107530556A); PCT/US2016/062114 with Publication No. WO2017087428A1 (corresponding to Chinese Patent Application No. 2016800446081 with Publication No. CN108290911A); and PCT/CN2020/089692 with Publication No. WO2020228685A9; the relevant preparations concentrated injections, and the relevant formulations, preparation methods and clinical compatibility, and modes of administration have been described in detail and disclosed by the relevant patents: WO2021008520A1 and WO2021043275A1; the above patent applications are incorporated herein by reference in their entirety.

## Brief Description of the Drawings

Figure 1 shows the picture of the growth curve of tumor volume of each group of mice in the HuPrime® gastric cancer GA6201 model.

Figure 2 shows the picture of the relative tumor growth inhibition rate curve of each group of mice in the HuPrime® gastric cancer GA6201 model.

Figure 3 shows the picture of the body weight curve of each group of mice in the HuPrime® gastric cancer GA6201 model.

Figure 4 shows the picture of the percentage change curve of body weight of each group of mice in the HuPrime®

gastric cancer GA6201 model.

Figure 5 shows the picture of the growth curve of tumor volume of each group of mice in the HuPrime® pancreatic cancer PA1222 model.

Figure 6 shows the picture of the relative tumor growth inhibition rate curve of each group of mice in the HuPrime® pancreatic cancer PA1222 model.

Figure 7 shows the picture of the body weight curve of each group of mice in the HuPrime® pancreatic cancer PA1222 model.

Figure 8 shows the picture of the percentage change curve of body weight of each group of mice in the HuPrime® pancreatic cancer PA1222 model.

Figure 9 shows the picture of the growth curve of tumor volume of each group of mice in the HuPrime® lung cancer LU11693 model.

Figure 10 shows the picture of the relative tumor growth inhibition rate curve of each group of mice in the HuPrime® lung cancer LU11693 model.

Figure 11 shows the picture of the body weight curve of each group of mice in the HuPrime® lung cancer LU11693 model.

Figure 12 shows the picture of the percentage change curve of body weight of each group of mice in the HuPrime® lung cancer LU11693 model.

Figure 13 shows the picture of the growth curve of tumor volume of each group of mice in the HuPrime® pancreatic cancer PA1383 model.

Figure 14 shows the picture of the relative tumor growth inhibition rate curve of each group of mice in the HuPrime® pancreatic cancer PA1383 model.

Figure 15 shows the picture of the body weight curve of each group of mice in the HuPrime® pancreatic cancer PA1383 model.

Figure 16 shows the picture of the percentage change curve of body weight of each group of mice in the HuPrime® pancreatic cancer PA1383 model.

Figure 17 shows the photographs of IHC staining results of tumor tissue in three PDX models, gastric cancer GA6201, lung cancer LU11693, and pancreatic cancer PA1222, and control groups.

Figure 18 shows the correlation graph between the RNA expression level of AKR1C3 and the RNA expression level of NRF2 in the KRAS-G12D mutation model.

Figure 19 shows the correlation graph between the RNA expression level of AKR1C3 and the RNA expression level of NRF2 in the KRAS-G12C mutation model.

Figure 20 shows the correlation graph between the RNA expression level of AKR1C3 and the RNA expression level of NRF2 in the KRAS-G13D mutation model.

Figure 21 shows the photographs of immunoblotting of pancreatic cancer HPAF II and IHC staining results of lung cancer LU5161 and colorectal cancer CR3820.

Figure 22 shows the immunoblotting results of proteins of interest from lysed cells treated with different concentrations of SFN, wherein the upper graph is the immunoblotting photographs under treatment with different concentrations of SFN, and the lower graph is the protein band density analysis column chart of NRF2 and AKR1C3, wherein, under the concentration of each group, the left column represents NRF2 and the right column represents AKR1C3.

Figure 23 shows the immunoblotting results of proteins of interest from lysed cells treated with different concentrations of AST, wherein the upper graph is the immunoblotting photographs under treatment with different concentrations of AST, and the lower graph is the protein band density analysis column chart of p-ERK2 and ERK2 under treatment with different concentrations of AST, wherein, under the AST concentration of each group, the left column represents p-ERK2 and the right column represents ERK2.

Figure 24 shows the immunoblotting results of proteins of interest from lysed cells treated with 1 μM AST for different durations, wherein the upper graph is the immunoblotting photographs under treatment with 1 μM AST for different durations, and the lower graph is the protein band density analysis column chart of p-ERK2 and ERK2 under treatment with 1 μM AST for different durations, wherein, under the treatment duration of each group, the left column represents p-ERK2 and the right column represents ERK2.

Figure 25 shows the AKR1C3-dependent immunoblotting results of proteins of interest from lysed cells, wherein the upper graph is the AKR1C3-dependent immunoblotting photograph, and the lower graph is the AKR1C3-dependent protein band density analysis column chart of p-ERK2 and ERK2, wherein, in each treatment group, the left column represents p-ERK2 and the right column represents ERK2.

Figure 26 shows the results of immunoblotting of proteins of interest from lysed cells and apoptosis assay in monotherapy and combination groups of ERK2 inhibitor and AST, wherein the upper graph is the immunoblotting photographs with monotherapy or combination therapy in the ERK2 inhibitor group of PD98059 and suramin; the lower left graph is the protein band density analysis column chart with monotherapy or combination therapy thereof, wherein, in each treatment group, the left column represents p-ERK2 and the right column represents ERK2; and the lower right graph is the apoptosis column chart with monotherapy or combination therapy thereof, wherein Luminescence (RLU) represents apoptosis signal relative light units.

Figure 27 shows the results of immunoblotting of proteins of interest from lysed cells and apoptosis assay in monotherapy and combination groups of an ERK2 activator and AST, wherein the upper graph is the immunoblotting photographs with monotherapy or combination therapy in the ERK2 activator group of TPA; the lower left graph is the protein band density analysis column chart with monotherapy or combination therapy thereof, wherein, in each treatment group, the left column represents p-ERK2 and the right column represents ERK2; and the lower right graph is the apoptosis column chart with monotherapy or combination therapy thereof, wherein Luminescence (RLU) represents apoptosis signal relative light units.

Figure 28 shows the diagram of the process of apoptosis of AST inducing KRAS G12D pathogenic mutation cancer cell through the ERK2 signaling pathway, wherein (1), (3), and (4) represent relevant probative literature,

(1) Prior IA, Hood FE and Hartley JL. The Frequency of Ras Mutations in Cancer. Cancer Res 2020; 80: 2969-2974;

(3) Park HE, Yoo SY, Cho NY, Bae JM, Han SW, Lee HS, Park KJ, Kim TY and Kang GH. Tumor microenvironment-adjusted prognostic implications of the KRAS mutation subtype in patients with stage III colorectal cancer treated with adjuvant FOLFOX. Sci Rep 2021; 11: 14609; and

(4) Patricelli MP, Janes MR, Li LS, Hansen R, Peters U, Kessler LV, Chen Y, Kucharski JM, Feng J, Ely T, Chen JH, Firdaus SJ, Babbar A, Ren P and Liu Y. Selective Inhibition of Oncogenic KRAS Output with Small Molecules Targeting the Inactive State. Cancer Discov 2016; 6: 316-329;

"①" indicates that the compound AST acts on the MEK-ERK signaling pathway and activates it;

"②" indicates that the AKR1C3 enzyme activates the prodrug compound AST and eventually releases the DNA alkylating agent AST-2660; and

"③" indicates that cancer cell proliferation is inhibited under the action of AST-2660.

## Detailed Description of the Invention

The present invention will be described below with the reference to specific examples. Those skilled in the art will understand that these examples are only used to illustrate the present invention and do not limit the scope of the present

invention in any way.

"Administering" or "administration of" a medicament to patients (and the grammatical equivalents of this phrase) refers to direct administration, which means a medicament may be administered to patients by a medical professional or self-administration, and/or indirect administration, which may be the act of prescribing a medicament. For example, a physician who instructs patients to self-administer a medicament and/or provides patients with a prescription for a medicament is administering the medicament to the patient.

"Cancer" refers to leukemia, lymphoma, carcinoma, and other malignant tumors (including solid tumors) with potentially unrestrained growth that can expand locally by invasion and systemically by metastasis. Examples of cancer include (but are not limited to) cancers of the adrenal gland, bone, brain, breast, bronchus, colon and/or rectum, gallbladder, head and neck, kidney, larynx, liver, lung, nervous tissue, pancreas, prostate, accessory thyroid gland, skin, stomach, and thyroid gland. Certain other examples of cancer include acute and chronic lymphocytic and granulocytic neoplasms, adenocarcinoma, adenoma, basal cell carcinoma, uterine cervical epithelial dysplasia and carcinoma *in situ,* Ewing's sarcoma, epidermoid carcinoma, giant cell tumor, glioblastoma multiforme, pilocytic tumor, intestinal ganglioneuroma, hyperplastic corneal nerve tumor, islet cell carcinoma, Kaposi's sarcoma, leiomyoma, leukemia, lymphoma, malignant carcinoid, malignant melanoma, malignant hypercalcemia, marfanoid habitus tumor, medullary epithelial carcinoma, metastatic skin cancer, mucosal neuroma, myeloma, mycosis fungoides, neuroblastoma, osteosarcoma, osteogenic and other sarcomas, ovarian tumor, pheochromocytoma, polycythemia vera, primary brain tumor, small cell lung cancer, ulcerative and papillary squamous cell carcinoma, hyperplasia, seminoma, soft tissue sarcoma, retinoblastoma, rhabdomyosarcoma, renal cell tumor, local skin lesion, reticulum cell sarcoma, and Wilm's tumor.

"Patient" and "individual" may be used interchangeably and refer to a mammal in need of treatment for cancer. Typically, the patient is a human. Typically, the patient is a human diagnosed with cancer. In certain embodiments, a "patient" or "individual" may refer to a non-human mammal, such as a non-human primate, a dog, a cat, a rabbit, a pig, a mouse, or a rat used for screening, characterizing, and evaluating drugs and therapies.

"Solid tumors" refer to solid tumors including, but not limited to, metastatic tumors in the bone, brain, liver, lung, lymph node, pancreas, prostate, skin, and soft tissue (sarcoma).

"Therapeutically effective amount" of a drug refers to an amount of a drug that, when administered to patients with cancer, will have the intended therapeutic effect (e.g., alleviation, amelioration, palliation, or elimination of one or more clinical manifestations of cancer in the patient). A therapeutic effect does not necessarily occur by administration of one dose, and may occur only after administration of a series of doses. Thus, a therapeutically effective amount may be administered in one or more administrations.

"Treating", "treatment of" or "therapy of" conditions or patients refers to taking steps to obtain beneficial or desired results (including clinical results). For purposes of the present invention, beneficial or desired clinical results include (but are not limited to) alleviation or amelioration of one or more symptoms of cancer; diminishment of extent of disease; delay or slowing of disease progression; amelioration, remission, or stabilization of disease status; or other beneficial results. In some cases, treatment of cancer may result in partial response or stable disease.

"Tumor cells" refer to tumor cells of any appropriate species (e.g., a mammal, such as a murine, a canine, a feline, an equine, or a human).

"Patient" and "individual" may be used interchangeably and refer to a mammal in need of treatment for cancer. Typically, the patient is a human. Typically, the patient is a human diagnosed with cancer. In certain embodiments, a "patient" or "individual" may refer to a non-human mammal, such as a non-human primate, a dog, a cat, a rabbit, a pig, a mouse, or a rat used for screening, characterizing, and evaluating drugs and therapies.

"Treatment" or "treatment of patients" is to give, administer or use a therapeutically effective amount of a medicament in relation to the present invention to patients.

"Administering", "administration of" or "use of" a medicament to patients refers to give directly or direct administration, which means a medicament may be used or administered to patients by a medical professional or self-use or self-administration, and/or indirect use or administration, which may be the act of prescribing a medicament. For example, a physician who instructs patients to self-use or self-administer a medicament and/or provides patients with a prescription for a medicament is giving or administering the medicament to the patient.

"Treating", "treatment of" or "therapy of" conditions or patients refers to taking steps to obtain beneficial or desired results (including clinical results). For purposes of the present invention, beneficial or desired clinical results include (but are not limited to) alleviation or amelioration of one or more symptoms of cancer; diminishment of extent of disease; delay or slowing of disease progression; amelioration, remission, or stabilization of disease status; or other beneficial results. In some cases, treatment of cancer may result in partial response or stable disease.

The experimental methods in the following examples are conventional methods unless specified otherwise. The medicinal raw materials, reagent materials, etc. used are all commercially available products unless specified otherwise.

The above description of specific embodiments of the present invention does not limit the present invention. Those skilled in the art can make various modifications and changes according to the present invention, and any modification and change within the spirit of the present invention shall be covered in the scope of the claims appended to

the present invention.

The following provides specific experiments of the present invention.

1. Pharmacodynamics evaluation of the test substances AST, AST-3424, and Ifosfamide in **HuPrime®** gastric cancer GA6201 subcutaneous xenograft model

The HuPrime® gastric cancer GA6201 PDX model is a model of KRAS pathogenic mutation with G12D amino acid mutation (KRAS-G12D). The human gastric cancer subcutaneous graft tumor model is established by using BALB/c nude mice subcutaneously inoculated with HuPrime® model GA6201 tumor mass. The experiment was assigned to 5 groups, with 5 mice in each group: the test drug Ifosfamide 60 mg/kg group, the test drug AST-3424 5 mg/kg group, the AST 2.5 mg/kg and 5 mg/kg groups, and the physiological saline (pH 7.0-7.6) vehicle control group. Wherein, the physiological saline (pH 7.0-7.6) vehicle control group, the test drugs AST-3424 5 mg/kg, AST 2.5 mg/kg and 5 mg/kg groups were administered via tail vein injection once a week for a total of three weeks, and observed for four weeks. The Ifosfamide 60 mg/kg group was administered via intraperitoneal injection for five consecutive days per week, with two days off, for a total of two weeks, and observed for five weeks. Evaluation of therapeutic efficiency was based on the relative tumor growth inhibition TGI (%), and safety evaluation was based on weight changes and mortality of the animals.

The specific dosage regimen for each group is shown in Table 1.

Table 1: Experimental design of the anti-tumor effect of different doses of test drugs in HuPrime® gastric cancer GA6201 tumor model

| Group | Number of animals | Administration group | Dose (mg/kg ) | Mode of administration | Planned dosing cycle | Actual dosing cycle |
|---|---|---|---|---|---|---|
| 1 | 5 | Physiological saline, pH 7.0-7.6 | 0 | Tail vein | Q7D×3 | Q7D×3 |
| 2 | 5 | Ifosfamide | 60 | intraperitoneal | QD×5/week× 2 wks | QD×5/week× 2 wks |
| 3 | 5 | AST-3424 | 5 | Tail vein | Q7D×3 | Q7D×3 |
| 4 | 5 | AST | 5 | Tail vein | Q7D×3 | Q7D×3 |
| 5 | 5 | AST | 2.5 | Tail vein | Q7D×3 | Q7D×3 |

The tumor volumes of mice in different groups were measured respectively on different days and the average values were obtained. The results are as shown in Table 2.

Table 2: Changes in tumor volume of mice in each group over treatment time in HuPrime® gastric cancer GA6201 model

| Time | **Tumor volume(mm³)**($\overline{x\pm S}$) | | | | |
|---|---|---|---|---|---|
| | Group 1 physiological saline pH 7.0-7.6 | Group 2 Ifosfamide 60 mg/kg | Group 3 AST-3424 5 mg/kg | Group 4 AST 5 mg/kg | Group 5 AST 2.5 mg/kg |
| Day 0 after the start of administration | 94.22±8.5 | 94.15±6.68 | 94.19±12.46 | 94.36±14.61 | 94.61±11.1 |
| Day 3 after the start of administration | 123.26±13.74 | 122.22±12.32 | 114.98±21.76 | 118.42±20.83 | 120.19±15.57 |
| Day 7 after the start of administration | 161.08±22.55 | 129.39±13.47 | 84.33±17.54 | 101.91±19.72 | 118.33±27.54 |
| Day 10 after the start of administration | 182.31±28.05 | 175±22.09 | 77.04±15.37 | 97.17±25.14 | 118.65±31.45 |

(continued)

| Time | Tumor volume(mm$^3$)($\overline{x}\pm S$) | | | | |
|---|---|---|---|---|---|
| | Group 1 physiological saline pH 7.0-7.6 | Group 2 Ifosfamide 60 mg/kg | Group 3 AST-3424 5 mg/kg | Group 4 AST 5 mg/kg | Group 5 AST 2.5 mg/kg |
| Day 14 after the start of administration | 242.26±40.75 | 190.74±29.81 | 63.59±10.46 | 74.2±16.99 | 96.08±26.3 |
| Day 17 after the start of administration | 297.28±47.21 | 189.23±29.28 | 52.08±9.25 | 59.77±15.76 | 76.14±18.07 |
| Day 21 after the start of administration | 398.37±87.32 | 217.96±41.06 | 45.81±13.3 | 46.21±13.48 | 71.72±9.08 |
| Day 24 after the start of administration | 479.52±119.81 | 225.66±44.63 | 40.55±10.4 | 44.03±11.57 | 57.94±10.4 |
| Day 28 after the start of administration | 579.9±154.47 | 247.04±50.8 | 30.28±3.78 | 38.5±11 | 45.51±1.64 |
| Day 31 after the start of administration | 648.12±177.44 | 265.76±52.69 | 23.24±7.23 | 35.92±10.45 | 43.44±4.51 |
| Day 35 after the start of administration | 831.15±234.39 | 324.51±73.75 | 18.55±5.77 | 22.7±11.73 | 32.04±4.52 |
| Day 38 after the start of administration | 905.75±252.4 | 371.02±75.96 | 12.65±7.91 | 19.97±10.18 | 28.69±8.2 |
| Day 42 after the start of administration | 1026.01±294.0 5 | 403.47±72.29 | 13.42±8.22 | 5.05±5.05 | 23.49±6.18 |
| Day 45 after the start of administration | 1130.43±319.1 3 | 484.83±98.33 | 10.82±7.05 | 11.14±7.04 | 25±6.37 |
| Day 49 after the start of administration | 1283.11±366.6 6 | 578.6±99.79 | 8.15±5.03 | 9.96±7.12 | 17.72±4.6 |

Figure 2 was made to illustrate the tumor growth of each treatment group and control group according to the data in Table 2.

Analysis data of pharmacodynamic evaluation was made according to the data in Table 2, and the specific data are shown in Table 3.

Table 3: Pharmacodynamic analysis table of each group in HuPrime® gastric cancer GA6201 model

| Experimental group | Day 3 after the end of all administration (i.e. Day 38, 2/27/2020) | | | | |
|---|---|---|---|---|---|
| | Tumor volume ($\overline{x}\pm S$) | Relative tumor volume ($\overline{x}\pm S$) | TGI (%) | T/C (%) | P Value (Compared to the control group) |
| Group 1 physiological saline pH 7.0-7.6 | 905.75±252.4 | 9.34±2.42 | - | - | - |

(continued)

| Experimental group | Day 3 after the end of all administration (i.e. Day 38, 2/27/2020) | | | | |
|---|---|---|---|---|---|
| | Tumor volume ($\bar{x}$±S) | Relative tumor volume ($\bar{x}$ ±S) | TGI (%) | T/C (%) | P Value (Compared to the control group) |
| Group 2 Ifosfamide 60 mg/kg | 371.02±75.96 | 3.85±0.63 | 58.83 | 41.17 | 0.857 |
| Group 3 AST-3424 5 mg/kg | 12.65±7.91 | 0.11±0.07 | 98.79 | 1.21 | 0.00000265 |
| Group 4 AST 5 mg/kg | 19.97±10.18 | 0.19±0.08 | 97.96 | 2.04 | 0.0000182 |
| Group 5 AST 2.5 mg/kg | 28.69±8.2 | 0.29±0.08 | 96.85 | 3.15 | 0.000135 |

In Table 3, the relative tumor proliferation rate, T/C%, represents the percentage value of relative tumor volume or tumor weight in treatment groups and control groups at a certain time point. The calculation formula is as follows:

$$T/C\%=TRTV/CRTV \times 100\%$$

(TRTV: average RTV of treatment group; CRTV: average RTV of vehicle control group; RTV=Vt/V0, where V0 represents the tumor volume of the animal at the time of grouping and Vt represents the tumor volume of the animal after treatment); and

for the relative tumor growth inhibition, TGI (%), the calculation formula is as follows: TGI%=(1-T/C) × 100% (T and C are the average relative tumor volume (RTV) of the treatment groups and control groups at a certain time point, respectively).

Table 4: Relative tumor growth inhibition of tumor in each group in HuPrime® gastric cancer GA6201 model

| Group | 0 | 3 | 7 | 10 | 14 | 17 | 21 | 24 | 28 | 31 | 35 | 38 | 42 | 45 | 49 |
|---|---|---|---|---|---|---|---|---|---|---|---|---|---|---|---|
| Group 02 | 0.00% | 3.34% | 47.28% | 8.21% | 34.75% | 53.17% | 59.29% | 65.87% | 68.52% | 69.02% | 68.74% | 65.88% | 66.80% | 62.30% | 59.25% |
| Group 03 | 0.00% | 28.39% | 114.75% | 119.47% | 120.67% | 120.74% | 115.90% | 113.92% | 113.16% | 112.81% | 110.26% | 110.05% | 108.67% | 103.05% | 107.24% |
| Group 04 | 0.00% | 17.14% | 88.71% | 96.81% | 113.62% | 117.03% | 115.83% | 113.06% | 111.50% | 110.55% | 109.72% | 109.17% | 109.58% | 108.03% | 107.10% |
| Group 05 | 0.00% | 11.94% | 64.53% | 72.71% | 99.01% | 109.10% | 107.53% | 109.52% | 110.11% | 109.24% | 103.49% | 108.12% | 107.63% | 106.72% | 106.47% |

Figure 2 can be obtained by making a curve graph from the data in Table 4 above.

The body weights of mice in different groups were measured respectively on different days and the average values were obtained. The results are shown in Table 5.

Table 5: Body weights of mice with different inoculation days in HuPrime® gastric cancer GA6201 model

| Group | 0 | 1 | 2 | 3 | 4 | 7 | 8 | 9 | 10 | 11 | 14 | 15 | 16 | 17 | 18 | 21 | 24 | 28 | 31 | 35 | 38 |
|---|---|---|---|---|---|---|---|---|---|---|---|---|---|---|---|---|---|---|---|---|---|
| Group 01 | 25.3 | | | 25.4 | | 25.3 | | | 24.4 | | 25.4 | | | 24.6 | | 23.8 | 24.3 | 23.7 | 24.0 | 24.4 | 25.1 |
| Group 02 | 25.8 | 25.5 | 25.0 | 25.5 | 24.4 | 24.4 | 24.5 | 24.1 | 24.4 | 24.4 | 24.3 | | | 25.1 | | 26.3 | 26.1 | 25.5 | 25.2 | 26.0 | 26.2 |
| Group 03 | 24.8 | | | 25.1 | | 25.1 | | | 24.5 | | 25.4 | | | 25.0 | | 25.5 | 25.6 | 25.3 | 25.4 | 25.7 | 26.0 |
| Group 04 | 25.0 | | | 24.4 | | 24.8 | | | 24.5 | | 25.2 | | | 24.9 | | 25.4 | 25.1 | 24.6 | 25.2 | 25.6 | 25.7 |
| Group 05 | 25.3 | | | 25.1 | | 25.0 | | | 25.0 | | 25.2 | | | 24.9 | | 25.4 | 25.8 | 25.0 | 25.4 | 25.6 | 25.4 |
| | | | | | | | | | | | | | | | | | | | | | |

Figure 3, that is, the body weight curves of mice in each group in HuPrime® gastric cancer GA6201 model can be obtained by making a curve graph from the data in the table above.
Similarly, the following Table 6 can be obtained by processing the data in Table 5.

Table 6: The percentage of body weight changes of mice with different inoculation days in HuPrime® gastric cancer GA6201 model (% Group Mean Change=mean ((T-T0)/ T0) * 100, where T represents current value and T0 represents initial value)

| Group | 0 | 1 | 2 | 3 | 4 | 7 | 8 | 9 | 10 | 11 | 14 | 15 | 16 | 17 | 18 | 21 | 24 | 28 | 31 | 35 | 38 |
|---|---|---|---|---|---|---|---|---|---|---|---|---|---|---|---|---|---|---|---|---|---|
| Goup 01 | 0.00% | | | 0.72% | | 0.09% | | | -3.34% | | 0.31% | | | 2.58% | | -4.69% | -2.40% | -4.98% | 3.68% | -2.33% | 0.69% |
| Group 02 | 0.00% | -1.02% | -2.93% | -1.20% | -5.32% | -5.50% | -5.07% | -6.48% | -5.33% | -5.32% | -5.80% | | | -2.46% | | 1.96% | 1.12% | -0.90% | -2.41% | 0.86% | 1,53% |
| Group 03 | 0.00% | | | 1.21% | | 1.46% | | | -1.11% | | 2.52% | | | 0 87% | | 3.03% | 3.31% | 2.27% | 2.52% | 3.58% | 4.87% |
| Group 04 | 0.00% | | | -2.44% | | -0.77% | | | -2.14% | | 0.65% | | | -0.46% | | 1.57% | 0.53% | -1.72% | 0.85% | 2.51% | 2.73% |
| Group 05 | 0.00% | | | -0.70% | | -1.21% | | | -1.22% | | -0.28% | | | -1.77% | | 0.50% | 1.97% | -1.37% | 0.38% | 1.23% | 0.43% |

The Group01, Group02, Group03, Group04, and Group05 in Tables 4/5/6 above refer to above-mentioned Group 1, Group 2, Group 3, Group 4, Group 5 and Group 6. 0/1/2/3/4/7/8/9/10/11/14/15/16/17/18/21/24/28/31/35/38 are the days after inoculation.

Figure 4 can be obtained by making a curve graph from the data in the table above.

Analysis of experimental data shows that in terms of therapeutic effect:
Test drug AST-3424 at a dose of 5 mg/kg, test drug AST at doses of 2.5 mg/kg and 5 mg/kg have a significant inhibitory effect on tumor growth of HuPrime® gastric cancer GA6201, with a statistically significant difference compared to the control group. The test drug Ifosfamide at a dose of 60 mg/kg has a certain inhibitory effect on tumor growth of HuPrime® gastric cancer GA6201, however, without statistically significant difference compared to the control group.

Analysis of experimental data shows that tumor bearing mice have good tolerance to Ifosfamide, AST-3424, and AST at the test doses.

2. Pharmacodynamics and safety evaluation of test substances AST and Gemcitabine in **HuPrime®** pancreatic cancer PA1222 subcutaneous xenograft model

HuPrime® pancreatic cancer PA1222 PDX model is a model of KRAS pathogenic mutation with G12D amino acid mutation (KRAS-G12D). The human pancreatic cancer subcutaneous graft tumor model is established by using BALB/c nude mice subcutaneously inoculated with HuPrime® model PA1222 tumor mass. The experiment was assigned to 3 groups, with a total of 5 mice in each group: the test drug Gemcitabine 120mg/kg group, the test drug AST 10mg/kg group, and the 7.5% anhydrous ethanol + 7.5% polyoxyethylene (35) castor oil + 85% glucose injection D5W (pH 7.4) vehicle control group. Wherein, the 7.5% anhydrous ethanol + 7.5% polyoxyethylene (35) castor oil + 85% glucose injection D5W (pH 7.4) vehicle control group and the test drug AST 10 mg/kg group were administered via tail vein injection once a week for three consecutive weeks. The test drug Gemcitabine 120mg/kg group was administered via intraperitoneal injection once a week for 3 consecutive weeks. Evaluation of therapeutic efficiency was based on the relative tumor growth inhibition TGI (%), and safety evaluation was based on weight changes and mortality of the animals.

The specific dosage regimen for each group is shown in Table 7.

Table 7: Experimental design of the anti-tumor effect of different doses of test drugs in HuPrime® pancreatic cancer PA1222 PDX tumor model

| **Group** | **Number of animals** | **Administration group** | **Dose (mg/kg)** | **Mode of administration** | **Dosing cycle** |
|---|---|---|---|---|---|
| 1 | 5 | 7.5% anhydrous ethanol + 7.5% polyoxyethylene (35) castor oil + 85% glucose injection D5W (pH 7.4) | - | i.v. | QWx3 |
| 2 | 5 | Gemcitabine | 120 | i.p. | QW×3 |
| 3 | 5 | AST | 10 | i.v. | QW×3 |
| Note: 1. The administration volume is 10 μl/g; and<br>2. QW×3; administered once a week for three weeks. | | | | | |

The tumor volumes of mice in different groups were measured respectively on different days and the average values were obtained. The results are shown in Table 8.

Table 8: Changes in tumor volume of mice in each group over treatment time in HuPrime® pancreatic cancer PA1222 model

| | **Tumor volume(mm³)**($\bar{x}$±S) | | |
|---|---|---|---|
| **Time** | Group 1 7.5% anhydrous ethanol + 7.5% polyoxyethylene (35) castor oil + 85% glucose injection D5W (pH 7.4), 0 mg/kg, QW×3, i.v | Group 2 Gemcitabine 120 mg/kg, QW×3, i.p | Group 3 AST, 10 mg/kg, QW×3, i.v. |
| Day 0 after the start of administration | 112.01±7.53 | 111.91±7.78 | 111.50±8.63 |

(continued)

| Time | Tumor volume(mm³)($\bar{x}\pm S$) | | |
|---|---|---|---|
| | Group 1 7.5% anhydrous ethanol + 7.5% polyoxyethylene (35) castor oil + 85% glucose injection D5W (pH 7.4), 0 mg/kg, QW×3, i.v | Group 2 Gemcitabine 120 mg/kg, QW×3, i.p | Group 3 AST, 10 mg/kg, QW×3, i.v. |
| Day 3 after the start of administration | 139.75±12.85 | 136.17±12.64 | 117.62±6.91 |
| Day 7 after the start of administration | 177.43±24.82 | 153.75±10.18 | 113.91±10.61 |
| Day 10 after the start of administration | 199.20±31.63 | 157.55±9.68 | 107.17±11.77 |
| Day 14 after the start of administration | 249.21±43.56 | 147.44±16.31 | 66.08±4.54 |
| Day 17 after the start of administration | 306.79±53.54 | 136.71±13.80 | 42.94±2.17 |
| Day 21 after the start of administration | 339.57±63.97 | 138.01±14.81 | 23.22±2.75 |
| Day 24 after the start of administration | 362.58±69.17 | 153.69±22.96 | 21.20±2.38 |
| Day 28 after the start of administration | 395.45±76.89 | 166.11±24.40 | 12.51±5.25 |

The tumor growth of each treatment group and control group is shown in Table 8 and Figure 5, and the pharmacodynamic evaluation is shown in Table 9.

Table 9: Pharmacodynamic analysis table of each group in HuPrime® gastric cancer GA6201 model

| Experimental group | Day 28 after the first administration (i.e. Day 38, 2/27/2020) | | | | |
|---|---|---|---|---|---|
| | Tumor volume ($\bar{x}\pm S$) | Relative tumor volume($\bar{x}\pm S$) | TGI (%) | T/C (%) | P Value (Compared to the control group) |
| Group 1 7.5% anhydrous ethanol + 7.5% polyoxyethylene (35) castor oil + 85% glucose injection D5W (pH 7.4), 0 mg/kg, QW×3, i.v | 395.45±76.89 | 3.49±0.62 | - | - | - |
| Group 2 Gemcitabine, 120 mg/kg, QW×3, i.p | 166.11±24.40 | 1.49±0.19 | 57.17% | 42.83% | 0.000778 |
| Group 3 AST, 10 mg/kg, QW×3, i.v. | 12.51±5.25 | 0.10±0.04 | 97.14% | 2.86% | 0.000000914 |

In Table 9, the relative tumor proliferation rate, T/C%, represents the percentage value of relative tumor volume or tumor weight in treatment groups and control groups at a certain time point. The calculation formula is as follows:

$$T/C\% = TRTV/CRTV \times 100\%$$

(TRTV: average RTV of treatment group; CRTV: average RTV of vehicle control group; RTV=Vt/V0, where V0 represents the tumor volume of the animal at the time of grouping and Vt represents the tumor volume of the animal after treatment); and

for the relative tumor growth inhibition, TGI (%), the calculation formula is as follows: TGI% = (1-T/C) × 100% (T and C are the average relative tumor volume (RTV) of the treatment group and control group at a certain time point, respectively).

Table 10: Relative tumor growth inhibition in each tumor group in HuPrime® pancreatic cancer PA1222 model

| Group | Dates/Study Days | | | | | | | | |
|---|---|---|---|---|---|---|---|---|---|
| | 12/1/2020 | 12/4/2020 | 12/8/2020 | 12/11/2020 | 12/15/2020 | 12/18/2020 | 12/22/2020 | 12/25/2020 | 12/29/2020 |
| | 0 | 3 | 7 | 10 | 14 | 17 | 21 | 24 | 28 |
| Group 01 | | | | | | | | | |
| Group 02 | 0.09% | 2.56% | 13.35% | 20.91% | 40.84% | 55.44% | 59.36% | 57.61% | 57.99% |
| Group 03 | 0.45% | 15.84% | 35.80% | 46.20% | 73.48% | 86.00% | 93.16% | 94.15% | 96.84% |

Figure 6 can be obtained by making a curve graph from the data in the table above.

The body weights of mice in different groups were measured respectively on different days and the average values were obtained. The results are shown in Table 11.

Table 11: Body weights of mice with different inoculation days in HuPrime® pancreatic cancer PA1222 model

| Group | 0 | 3 | 7 | 10 | 14 | 17 | 21 | 24 | 28 |
|---|---|---|---|---|---|---|---|---|---|
| Group 01 | 24.0 | 24.1 | 24.6 | 24.6 | 24.4 | 24.3 | 25.1 | 24.8 | 25.0 |
| Group 02 | 24.1 | 23.9 | 24.4 | 24.3 | 24.6 | 24.2 | 25.1 | 25.1 | 25.4 |
| Group 03 | 24.0 | 23.5 | 24.0 | 23.6 | 23.6 | 23.6 | 24.0 | 24.0 | 24.1 |

Figure 7, that is, the body weight curves of mice in each group in HuPrime® pancreatic cancer PA1222 model can be obtained by making a curve graph from the data in the table above.

Similarly, the following Table 12 can be obtained by processing the data in Table 11.

Table 12: The percentage of body weight changes of mice with different inoculation days in HuPrime® pancreatic cancer PA1222 model (% Group mean Change=mean ((T-T0)/T0) * 100, where T represents current value and T0 represents initial value)

| Group | 0 | 3 | 7 | 10 | 14 | 17 | 21 | 24 | 28 |
|---|---|---|---|---|---|---|---|---|---|
| Group 01 | 0.00% | 0.67% | 2.49% | 2.75% | 1.91% | 1.52% | 4.70% | 3.60% | 4.52% |
| Group 02 | 0.00% | -0.63% | 1.18% | 0.82% | 2.12% | 0.38% | 4.19% | 4.36% | 5.38% |
| Group 03 | 0.00% | -1.99% | 0.18% | -1.50% | -1.50% | -1.51% | 0.24% | 0.09% | 0.66% |

Figure 8 can be obtained by making a curve graph from the data in the table above.

Analysis of experimental data shows that in terms of therapeutic effect:
Test drug Gemcitabine at a dose of 120mg/kg (Group 2) has a certain inhibitory effect on tumor growth of HuPrime® Pancreatic cancer PA1222, with a statistically significant difference compared to the control group. Test drug AST at a dose of 10 mg/kg (Group 3) has a significant tumor inhibitory effect on HuPrime® Pancreatic cancer PA1222, with a statistically significant difference compared to the control group; meanwhile, the tumors of two mice in the group were cured, with a cure rate of 40% for both. The tumor inhibitory effect of the test drug AST 10 mg/kg (Group 3) was significantly better than that of the test drug Gemcitabine (120 mg/kg, Group 2) ($p=0.000778$).

Analysis of experimental data shows that there was no significant weight loss observed in the mice of the test drug Gemcitabine (120 mg/kg, Group 2) treatment group, AST 10 mg/kg (Group 3) treatment group, and the control group (Group 1), indicating good tolerance during the treatment period.

3. Pharmacodynamics and safety evaluation of test substances AST and Cisplatin in **HuPrime®** lung cancer LU11693 subcutaneous xenograft model

HuPrime® lung cancer LU11693 PDX model is a model of KRAS pathogenic mutation with G12C amino acid mutation. The human lung cancer subcutaneous graft tumor model is established by using BALB/c Nude nude mice subcutaneously inoculated with HuPrime® model LU11693 tumor mass. The experiment was assigned to 3 groups, with a total of 6 mice in each group: the test drug Cisplatin 4 mg/kg group, the test drug AST 10 mg/kg group, and the 7.5% anhydrous ethanol + 7.5% polyoxyethylene (35) castor oil + 85% glucose injection D5W (pH 7.4) vehicle control group, administered via tail vein injection once a week for 3 consecutive weeks. Evaluation of therapeutic efficiency was based on the relative tumor growth inhibition TGI (%), and safety evaluation was based on weight changes and mortality of the animals.

The specific dosage regimen for each group is shown in Table 13.

Table 13: Experimental design of the anti-tumor effect of different doses of test drugs in HuPrime® lung cancer LU11693 PDX tumor model

| Group | Number of animals | Administration group | Dose (mg/kg) | Mode of administration | Dosing cycle |
|---|---|---|---|---|---|
| 1 | 6 | 7.5% anhydrous ethanol + 7.5% polyoxyethylene (35) castor oil + 85% glucose injection D5W (pH 7.4) | 0 | Tail vein | QWx3 |
| 2 | 6 | Cisplatin | 4 | Tail vein | QW×3 |
| 3 | 6 | AST | 10 | Tail vein | QW×3 |
| Note: 1. The administration volume is 10 μL/g; and<br>2. QW×3; administered once a week for three weeks. | | | | | |

The tumor volumes of mice in different groups were measured respectively on different days and the average values were obtained. The results are shown in Table 14.

Table 14: Changes in tumor volume of mice in each group over treatment time in HuPrime® lung cancer LU11693 model

| Time | Tumor volume($mm^3$)($\bar{x}\pm S$) | | |
|---|---|---|---|
| | Group 1 7.5% anhydrous ethanol + 7.5% polyoxyethylene (35) castor oil + 85% glucose injection D5W (pH 7.4) | Group 2 Cisplatin 4 mg/kg | Group 3 AST 10 mg/kg |
| Day 0 after the start of administration | 100.73±6.73 | 100.12±6.13 | 100.52±7.48 |
| Day 3 after the start of administration | 148.49±20.70 | 134.73±12.15 | 136.39±17.11 |
| Day 7 after the start of administration | 176.25±19.10 | 156.35±14.10 | 150.25±21.20 |
| Day 10 after the start of administration | 220.66±33.68 | 176.12±15.18 | 164.92±23.46 |
| Day 14 after the start of administration | 259.63±48.15 | 196.54±20.83 | 175.66±24.12 |
| Day 17 after the start of administration | 298.53±50.33 | 244.94±21.34 | 216.17±27.04 |
| Day 21 after the start of administration | 364.53±61.94 | 267.58±22.10 | 221.04±25.74 |
| Day 24 after the start of administration | 432.25±71.05 | 309.77±23.28 | 220.53±26.00 |
| Day 28 after the start of administration | 485.88±78.14 | 365.88±29.58 | 221.93±25.52 |

The tumor growth of each treatment group and control group is shown in Table 14 and Figure 9, and the pharmacodynamic evaluation is shown in Table 15.

Table 15: Pharmacodynamic analysis table of each group in HuPrime® lung cancer LU11693 model

| Experimental group | Day 28 after the first administration (i.e. Day28, 1/28/2021) | | | | |
|---|---|---|---|---|---|
| | Tumor volume ($\bar{x}\pm S$) | Relative tumor volume ($\bar{x}\pm S$) | TGI (%) | T/C (%) | P Value (Compared to the control group) |
| Group 1<br>7.5% anhydrous ethanol + 7.5% polyoxyethylene (35) castor oil + 85% glucose injection D5W (pH 7.4) | 485.88±78.14 | 4.79±0.58 | - | - | - |
| Group 2<br>Cisplatin 4 mg/kg | 365.88±29.58 | 3.64±0.14 | 23.98 | 76.02 | 0.0152 |

(continued)

| Experimental group | Day 28 after the first administration (i.e. Day28, 1/28/2021) | | | | |
|---|---|---|---|---|---|
| | **Tumor volume** ($\bar{x}$±S) | **Relative tumor volume** ($\bar{x}$ ±S) | **TGI (%)** | **T/C (%)** | **P Value (Compared to the control group)** |
| Group 3 AST 10 mg/kg | 221.93±25.52 | 2.17±0.11 | 54.64 | 45.36 | 0.00000337 |

In Table 15, the relative tumor proliferation rate, T/C%, represents the percentage value of relative tumor volume or tumor weight in treatment groups and control groups at a certain time point. The calculation formula is as follows:

$$T/C\%=TRTV/CRTV \times 100\%$$

(TRTV: average RTV of treatment group; CRTV: average RTV of vehicle control group; RTV=Vt/V0, where V0 represents the tumor volume of the animal at the time of grouping and Vt represents the tumor volume of the animal after treatment); and
for the relative tumor growth inhibition, TGI (%), the calculation formula is as follows: TGI%=(1-T/C) × 100% (T and C are the average relative tumor volume (RTV) of the treatment groups and control groups at a certain time point, respectively).

Table 14: Relative tumor growth inhibition in each tumor group in HuPrime® lung cancer LU11693 model

| | Dates/Study Days | | | | | | | | |
|---|---|---|---|---|---|---|---|---|---|
| Group | 12/31/2020 | 1/4/2021 | 1/7/2021 | 1/11/2021 | 1/14/2021 | 1/18/2021 | 1/21/2021 | 1/25/2021 | 1/28/2021 |
| | 0 | 4 | 7 | 11 | 14 | 18 | 21 | 25 | 28 |
| Group 01 | | | | | | | | | |
| Group 02 | 0.61% | 9.27% | 11.29% | 20.19% | 24.30% | 17.95% | 26.60% | 28.34% | 24.70% |
| Group 03 | 0.21% | 8.15% | 14.75% | 25.26% | 32.34% | 27.59% | 39.36% | 48.98% | 54.32% |

Figure 10 can be obtained by making a curve graph from the data in the table above.

The body weights of mice in different groups were measured respectively on different days and the average values were obtained. The results are shown in Table 15.

Table 15: Body weights of mice with different inoculation days in HuPrime® lung cancer LU11693 model

| Group | 0 | 4 | 7 | 11 | 14 | 18 | 21 | 25 | 28 |
|---|---|---|---|---|---|---|---|---|---|
| Group 01 | 24.5 | 24.6 | 24.5 | 24.5 | 24.9 | 24.9 | 24.6 | 24.3 | 24.4 |
| Group 02 | 24.3 | 23.6 | 23.9 | 22.8 | 24.0 | 22.4 | 23.0 | 23.4 | 23.6 |
| Group 03 | 24.3 | 24.3 | 23.9 | 23.3 | 23.6 | 23.3 | 23.0 | 22.4 | 22.8 |

Figure 11, that is, the body weight curves of mice in each group in HuPrime® lung cancer LU11693 model can be obtained by making a curve graph from the data in the table above.

Similarly, the following Table 16 can be obtained by processing the data in Table 15.

Table 16: The percentage of body weight changes of mice with different inoculation days in HuPrime® lung cancer LU11693 model (% Group Mean Change=mean ((T-T0)/T0) * 100, where T represents current value and T0 represents initial value)

| Group | 0 | 4 | 7 | 11 | 14 | 18 | 21 | 25 | 28 |
|---|---|---|---|---|---|---|---|---|---|
| Group 01 | 0.00% | 0.53% | 0.07% | 0.20% | 1.54% | 1.81% | 0.63% | -0.54% | -0.18% |
| Group 02 | 0.00% | -2.95% | -1.68% | -6.23% | -1.32% | -8.04% | -5.33% | -3.86% | -2.74% |
| Group 03 | 0.00% | 0.15% | -1.86% | -4.24% | -3.09% | -4.15% | -5.47% | -7.78% | -6.38% |

Figure 12 can be obtained by making a curve graph from the data in the table above.

Analysis of experimental data shows that in terms of therapeutic effect:

The test drug Cisplatin (4 mg/kg) treatment group showed a certain tumor inhibitory effect on Day 28 after the first administration, with a statistically significant difference compared to the control group (p=0.0152), and the relative tumor growth inhibition TGI (%) was 23.98%.

The test drug AST (10 mg/kg) treatment group showed a certain tumor inhibitory effect on Day 28 after the first administration, with a statistically significant difference compared to the control group (p<0.001). The relative tumor growth inhibition TGI (%) was 54.64%. TGI was less than 60%, and there was no significant tumor inhibitory effect.

Analysis of experimental data shows that there were individual mice who showed severe weight loss in the test drug AST (10 mg/kg) and Cisplatin (4 mg/kg) treatment group, which may be related to the potential toxicity of high-dose drugs.

4. Antitumor effect and safety evaluation of the test substances AST, AST-3424, and Ifosfamide monotherapy in **HuPrime®** pancreatic cancer PA1383 subcutaneous model

HuPrime® pancreatic cancer PA1383 PDX model is a model of KRAS pathogenic mutation with G12C amino acid mutation (KRAS-G12C). The human pancreatic cancer subcutaneous graft tumor model is established by using Balb/nude female mice subcutaneously inoculated with HuPrime® pancreatic cancer PA1383 tumor mass. The experiment was assigned to a test drug Ifosfamide 60 mg/kg monotherapy group (Group 2), administered once a day for 5 consecutive days, 2 days off, and then administered once a day again for 5 consecutive days; AST 4 mg/kg monotherapy group (Group 3) and AST 8 mg/kg monotherapy group (Group 4) were both administered once a week for a total of 3 weeks; AST 4 mg/kg monotherapy group (Group 5) and AST-3424 1 mg/kg monotherapy group (Group 6), administered once a day for 5 consecutive days, 2 days off, with another 2 weeks off, and then administered once a day again for 5 consecutive days; Glucose injection (pH 7.7-8.0) vehicle control group (Group 1), administered once a day for 5 consecutive days, 2 days off, with another 2 weeks off, and then administered once a day again for 5 consecutive days. This study consists of 6 groups, with 6 mice in each group. Wherein, the test drug Ifosfamide was administered intraperitoneally, while the vehicle control group, AST and AST-3424 were all administered via tail vein injection.

The specific dosage regimen for each group is shown in Table 17.

Table 17: The route of administration, dosage of administration and dosage regimen in HuPrime® pancreatic cancer PA1383 animal model

| Group | Number of animals | Administration group | Dose (mg/kg) | Mode of administration | Dosing cycle |
|---|---|---|---|---|---|
| 1 | 6 | Glucose Injection (pH 7.7-8.0) | - | *i.v.* | QD×5, 2 days off, 2 weeks off, QD×5 |
| 2 | 6 | Ifosfamide | 60 | *i.p.* | QD×5/week×2 weeks |
| 3 | 6 | AST | 4 | *i.v.* | QW×3 |
| 4 | 6 | AST | 8 | *i.v.* | QW×3 |
| 5 | 6 | AST | 4 | *i.v.* | QD×5, 2 days off, 2 weeks off, QD×5 |
| 6 | 6 | AST-3424 | 1 | *i.v.* | QD×5, 2 days off, 2 weeks off, QD×5 |
| Note: 1. The administration volume is 10 μL/g;<br>2. QD×5, 2 days off, 2 weeks off, QD×5: Administered once a day for 5 consecutive days, 2 days off, 2 weeks off, then administered once a day again for 5 consecutive days;<br>3. QDx5/week × 2 weeks: Administered once a day for 5 consecutive days, 2 days off, and then administered once a day again for 5 consecutive days; and<br>4. QW×3: Administered once a week for 3 consecutive weeks. | | | | | |

The tumor volumes of mice in different groups were measured respectively on different days and the average values were obtained. The results are shown in Table 18.

Table 18: Changes in tumor volume of mice in each group over treatment time in HuPrime® pancreatic cancer PA1222 model

| Group | 0 | 4 | 7 | 11 | 14 | 18 | 21 | 25 | 28 | 31 |
|---|---|---|---|---|---|---|---|---|---|---|
| Group 01 | 131.14 | 220.07 | 402.23 | 630.24 | 726.31 | 847.35 | 1077.74 | 1291.64 | 1417.06 | 1536.48 |
| Group 02 | 131.04 | 215.30 | 391.66 | 570.57 | 643.25 | 727.85 | 859.10 | 986.71 | 1064.57 | 1202.01 |
| Group 03 | 130.71 | 182.15 | 308.55 | 385.85 | 385.14 | 437.10 | 399.60 | 473.47 | 521.74 | 616.17 |
| Group 04 | 131.08 | 152.43 | 195.20 | 156.69 | 84.43 | 45.14 | 31.21 | 25.71 | 23.93 | 18.57 |
| Group 05 | 131.05 | 134.98 | 147.15 | 82.79 | 57.51 | 82.53 | 87.39 | 100.47 | 58.54 | 39.94 |
| Group 06 | 130.77 | 181.13 | 323.90 | 491.59 | 520.46 | 681.60 | 797.42 | 986.77 | 1119.52 | 1225.40 |

The tumor growth of each treatment group and control group is shown in Table 18 and Figure 13, and the pharmacodynamic evaluation is shown in Table 19.

Table 19: Pharmacodynamic analysis table of each group in HuPrime® pancreatic cancer PA1383 subcutaneous model

| Experimental group | Day 31 after the start of administration (January 6, 2022) | | | | |
|---|---|---|---|---|---|
| | Tumor volume ($\bar{x}$±S) | Relative tumor volume ($\bar{x}$±S) | TGI (%) | T/C (%) | P Value (Compared to the control group) |
| **Group 1**<br>Glucose injection (pH 7.7-8.0), 10 μL/g, i.v., QD×5, 2 days off, 2 weeks off, QD×5 | 1536.48±165.08 | 11.74±1.31 | - | - | - |

(continued)

| Experimental group | Day 31 after the start of administration (January 6, 2022) | | | | |
|---|---|---|---|---|---|
| | **Tumor volume** ($\bar{x}\pm$S) | **Relative tumor volume** ($\bar{x}\pm$S) | **TGI (%)** | **T/C (%)** | **P Value (Compared to the control group)** |
| **Group 2** Ifosfamide, 60 mg/kg, 10 μL/g, i.p., QD×5/week×2weeks | 1202.01±85.63 | 9.18±0.54 | 21.84 | 78.16 | >0.05 |
| **Group 3** AST, 4 mg/kg, 10 μL/g, i.v., QWx3 | 616.17±95.26 | 4.65±0.65 | 60.42 | 39.58 | <0.001*** |
| **Group 4** AST, 8 mg/kg, 10 μL/g, i.v., QW×3 | 18.57±7.43 | 0.15±0.06 | 98.72 | 1.28 | <0.001*** |
| **Group 5** AST, 4 mg/kg, 10 μL/g, i.v., QDx5, 2 days off, 2 weeks off, QDx5 | 39.94±20.41 | 0.30±0.15 | 97.46 | 2.54 | <0.001*** |
| **Group 6** AST-3424, 1 mg/kg, 10 μL/g, i.v., QDx5, 2 days off, 2 weeks off, QDx5 | 1225.40±150.50 | 9.34±1.02 | 20.45 | 79.55 | >0.05 |

The relative tumor proliferation rate, T/C%, represents the percentage value of relative tumor volume or tumor weight in treatment groups and control groups at a certain time point. The calculation formula is as follows:

$$T/C\% = TRTV/CRTV \times 100\%$$

(TRTV: average RTV of treatment group; CRTV: average RTV of vehicle control group; RTV=Vt/V0, where V0 represents the tumor volume of the animal at the time of grouping and Vt represents the tumor volume of the animal after treatment); and

for the relative tumor growth inhibition, TGI (%), the calculation formula is as follows: TGI%=(1-T/C) × 100% (T and C are the average relative tumor volume (RTV) of the treatment groups and control groups at a certain time point, respectively).

Table 20: Relative tumor growth inhibition in each tumor group in HuPrime® pancreatic cancer PA1383 model

| Group | 0 | 4 | 7 | 11 | 14 | 18 | 21 | 25 | 28 | 31 |
|---|---|---|---|---|---|---|---|---|---|---|
| Group 02 | 0.08% | 2.17% | 2.63% | 9.47% | 11.44% | 14.10% | 20.29% | 23.61% | 24.87% | 21.77% |
| Group 03 | 0.33% | 17.23% | 23.29% | 38.78% | 46.97% | 48.42% | 62.92% | 63.34% | 63.18% | 59.90% |
| Group 04 | 0.05% | 30.74% | 51.47% | 75.14% | 88.38% | 94.67% | 97.10% | 96.01% | 98.31% | 98.79% |
| Group 05 | 0.07% | 38.67% | 63.42% | 36.86% | 92.08% | 90.26% | 91.89% | 92.22% | 95.87% | 97.40% |
| Group 06 | 0.28% | 17.70% | 19.47% | 22.00% | 28.34% | 19.56% | 26.01% | 23.60% | 21.00% | 20.25% |

Figure 14 can be obtained by making a curve graph from the data in the table above.

The body weights of mice in different groups were measured respectively on different days and the average values were obtained. The results are as shown in Table 21.

Table 21: Body weights of mice with different inoculation days in HuPrime® pancreatic cancer PA1383 model

| Group | 0 | 1 | 2 | 3 | 4 | 7 | 8 | 9 | 10 | 11 | 14 | 18 | 21 | 22 | 23 | 24 | 25 | 28 | 31 |
|---|---|---|---|---|---|---|---|---|---|---|---|---|---|---|---|---|---|---|---|
| Group 01 | 22.5 | 22.7 | 22.5 | 22.5 | 22.3 | 23.1 | | | | 23.6 | 23.0 | 23.8 | 23.5 | 24.8 | 24.5 | 24.3 | 23.7 | 23.5 | 23.3 |
| Group 02 | 22.5 | 22.8 | 22.6 | 22.2 | 22.0 | 22.1 | 23.0 | 22.6 | 22.9 | 22.3 | 22.9 | 23.3 | 24.5 | | | | 24.5 | 23.6 | 23.5 |
| Group 03 | 22.8 | | | | 22.8 | 23.1 | | | | 24.0 | 23.9 | 24.4 | 24.5 | | | | 24.7 | 24.6 | 24.2 |
| Group 04 | 22.7 | | | | 22.9 | 23.9 | | | | 24.0 | 24.4 | 24.0 | 23.8 | | | | 24.3 | 24.3 | 24.3 |
| Group 05 | 22.7 | 23.1 | 23.2 | 22.8 | 22.8 | 23.3 | | | | 23.9 | 24.1 | 24.3 | 24.1 | 24.7 | 24.1 | 23.9 | 23.S | 24.5 | 24.7 |
| Group 06 | 23.2 | 23.6 | 23.4 | 23.0 | 22.8 | 23.2 | | | | 23.6 | 23.6 | 24.1 | 23.6 | 25.0 | 24.6 | 24.1 | 23.6 | 23.1 | 23.1 |

Figure 15, that is, the body weight curves of mice in each group in HuPrime® pancreatic cancer PA1383 model can be obtained by making a curve graph from the data in the table above.

Similarly, the following Table 22 can be obtained by processing the data in Table 21.

Table 22: The percentage of body weight changes of mice with different inoculation days in HuPrime® pancreatic cancer PA1383 model (% Group mean Change=mean ((T-T0)/T0) * 100, where T represents current value and T0 represents initial value)

| Group | 0 | 1 | 2 | 3 | 4 | 7 | 8 | 9 | 10 | 11 | 14 | 18 | 21 | 22 | 23 | 24 | 25 | 28 | 31 |
|---|---|---|---|---|---|---|---|---|---|---|---|---|---|---|---|---|---|---|---|
| Group 01 | 0.00% | 0.76% | 0.16% | 0.19% | -0.99% | 2.88% | | | | 5.00% | 2.54% | 5.77% | 4.49% | 10.28% | 8.94% | 8.20% | 5.41% | 4.37% | 3.46% |
| Group 02 | 0.00% | 1.45% | 0.57% | -1.33% | -2.25% | -1.43% | 2.27% | 0.79% | 1.76% | -0.70% | 2.01% | 3.59% | 9.06% | | | | 9.12% | 4.90% | 4.44% |
| Group 03 | 0.00% | | | | -0.10% | 1.15% | | | | 5.33% | 4.97% | 6.95% | 7.50% | | | | 8.62% | 7.99% | 6.31% |
| Group 04 | 0.00% | | | | 0.68% | 5.22% | | | | 5.83% | 7.37% | 5.55% | 4.97% | | | | 6.77% | 6.91% | 7.13% |
| Group 05 | 0.00% | 2.03% | 2.53% | 0.44% | 0.61% | 2.91% | | | | 5.66% | 6.39% | 7.35% | 6.31% | 9.09% | 6.44% | 5.39% | 5.27% | 8.10% | 8.93% |
| Group 06 | 0.00% | 1.72% | 0.74% | -1.03% | -1.82% | 0.01% | | | | 1.68% | 1.85% | 3.94% | 1.75% | 7.55% | 5.92% | 3.91% | 1.67% | -0.23% | -0.47% |

Figure 16 can be obtained by making a curve graph from the data in the table above.

Analysis of experimental data shows that in terms of therapeutic effect:

The average tumor volume of the mice in the vehicle control group on Day 31 after the start of administration was 1536.48 $mm^3$. For the test drug Ifosfamide in the 60 mg/kg treatment group (Group 2) and AST-3424 in the 1 mg/kg (QD×5, 2 days off, 2 weeks off, QD×5) dose group (Group 6), the average tumor volumes on Day 31 were 1202.01 $mm^3$, 1225.40 $mm^3$, respectively. The relative tumor growth inhibitions TGI (%) were 21.84%, 20.45%, and 7.55%, respectively, and there is no statistically significant difference compared to the control group (p>0.05).

For the test drug AST at doses of 4 mg/kg (QW × 3), 8 mg/kg (QW × 3), and 4 mg/kg (QD×5, 2 days off, 2 weeks off, QD×5) treatment groups (Group 3, Group 4, and Group 5) on Day 31, the average tumor volumes were 616.27 $mm^3$, 18.57 $mm^3$, and 39.94 $mm^3$, respectively, with statistically significant differences compared to the control group (p<0.05), and the relative tumor growth inhibitions (TGI) were 60.42%, 98.72%, and 97.46%, respectively.

The mice in each test drug treatment group did not experience a decrease in body weight and were well tolerated during the treatment period.

From the above four sets of experimental data, we can draw the following conclusions:

1. AST-3424 and AST have significant pharmacodynamics effects in the KRAS pathogenic mutation model with G12D amino acid mutation: gastric cancer GA6021 and pancreatic cancer PA1222 PDX models, with TGI% all greater than 90%;
2. AST shows no significant anti-tumor effect in the KRAS pathogenic mutation model with G12C amino acid mutation: lung cancer LU11693, with TGI% less than 60%; however, shows obvious anti-tumor effect in pancreatic cancer PA1383, and TGI% can reach 98%; and
3. AST-3424 and AST have good tolerance in all models.

5. Detection of AKR1C3 RNA expression level and enzyme content in gastric cancer GA6021, pancreatic cancer PA1222 and lung cancer LU11693 tissues

A. FPKM detection of AKR1C3 RNA expression level

According to the method described in the literature (Meng, F., Li, W. F., Jung, D., Wang, C. C., Qi, T., Shia, C. S., Hsu, R. Y., Hsieh, Y. C., & Duan, J. (2021). A novel selective AKR1C3-activated prodrug AST-3424/OBI-3424 exhibits broad anti-tumor activity. American journal of cancer research, 11(7), 3645-3659.), RNA Seq was used to analyze the AKR1C3 RNA expression level (AKR1C3 RNA expression level, and quantification was performed by using Log2 FPKM) in the above-mentioned gastric cancer GA6021, pancreatic cancer PA1222, and lung cancer LU11693 tissues. The results are as follows: AKR1C3 LOG2 (FPKM) detected in GA6201 was 6.78, detected in LU11693 was 11.14, and detected in PA1222 was 7.39, see Table 23.

According to the above-mentioned literatures, it is known that AKR1C3 RNA is highly expressed in all three tumor tissues.

B. IHC method detecting and H-SCORE scoring for AKR1C3 protein content

The AKR1C3 protein content of these three tissues was determined according to the commonly used IHC (immunohistochemistry) staining method (using commercial IHC reagents, the primary antibody was Abcam's rabbit antibody Rabbit IgG mAb, and the secondary antibody was Leica's polymer optimized detection system Bond Polymer Refine Detection, staining conditions: antigen repair at 100 °C, pH 9.0 EDTA buffer for 20 minutes, dilution ratio: 1:800), and the staining results were scored with H-SCORE: The immunohistochemical staining intensity will be assigned to 0 (negative), 1+ (weak staining), 2+ (medium staining), and 3+ (strong staining). The threshold values for weak staining, medium staining, and strong staining will be manually set on the scoring instrument, and then color recognition will be performed on the stained sample photos by image processing software. According to a unified standard, the staining photos of all samples will be scored by the scoring software for the corresponding staining of a certain cell: 0/1/2/3. And the percentage of the number of positive cells with different staining intensities to the total number of cells in the section is counted. The H-Score is calculated by the following formula as the IHC result score for each sample. The H-score will be between 0 and 300, with higher scores indicating higher expression levels of the corresponding target (AKR1C3 enzyme protein) of the antibody in the sample. The calculation formula is as follows:

$$\text{H-Score} = (\% \text{ at } 0)\times 0 + (\% \text{ at } 1)\times 1 + (\% \text{ at } 2)\times 2 + (\% \text{ at } 3)\times 3.$$

The staining results are shown in Figure 17, and the scoring results are shown in Table 17:

Table 23: Test results of IHC and RNA analysis of the three models and control groups

| Model ID | Cancer type | AKR1C3 H-Score | AKR1C3 LOG2 (FPKM) |
|---|---|---|---|
| GA6201 | Gastric Cancer | 248.75 | 6.78 |
| LU11693 | Lung Cancer | 269.10 | 11.14 |
| PA1222 | Pancreatic Cancer | 204.28 | 7.39 |
| BL9214 (positive control) | Bladder Cancer | 265.86 | 10.86 |
| LI5129 (negative control) | Liver Cancer | 0.00 | -2.00 |

The specific staining statistical results are shown in Table 24:

Table 24: IHC results and H-SCORE scoring of the three models and control groups

| Model ID | Cancer type | AKR1C3 | | | | |
|---|---|---|---|---|---|---|
| | | 0 | 1 | 2 | 3 | H-Score |
| GA6201 | Gastric Cancer | 1.07 | 8.75 | 30.54 | 59.63 | 248.75 |
| LU11693 | Lung Cancer | 1.70 | 5.51 | 14.79 | 78.00 | 269.10 |
| PA1222 | Pancreatic Cancer | 6.82 | 24.13 | 27.01 | 42.04 | 204.28 |
| BL9214 (positive control) | Bladder Cancer | 1.47 | 7.23 | 15.26 | 76.04 | 265.86 |
| LI5129 (negative control) | Liver Cancer | 100.00 | 0.00 | 0.00 | 0.00 | 0.00 |

The positive and negative control results in the above staining results are within the control range, indicating that the H-SCORE scoring results of the IHC staining agent are reliable.

It can be seen from the above results that AKR1C3 protein was highly expressed in the tissues corresponding to the three PDX models with G12D or G12C mutation.

6. Distribution of AKR1C3 in KRAS G12D PDXs and correlation statistics with NRF2

A total of 179 model data with KRAS-G12D mutation were exported from the publicly available tumor PDX model gene database of CrownBio (https://www.crownbio.cn/model-systems/in-vivo/pdx-models/), and the RNA expression of AKR1C3 in these models was statistically analyzed. The results are shown in Table 25:

Table 25: Statistical results of the correspondence between AKR1C3 RNA expression level grading represented by $LOG_2$ (FPKM) and KRAS-G12D

| AKR1C3 RNA Expression | AKR1C3 RNA Expression Level | % Distribution in KRAS G12D mutant PDXs |
|---|---|---|
| ≥ 8 | H | 8.9% |
| 6-7.9 | M-H | 57.5% |
| 4-5.9 | M | 24.0% |
| 2-3.9 | M-L | 7.8% |
| <2 | L | 1.7% |
| Total #PDX | | ADC #PDX 179 |

The NRF2 expression of these KRAS-G12D mutant models were further statistical analyzed. The RNA expression level of AKR1C3 and the RNA expression level of NRF2 in these 179 PDX models were projected onto an X-Y coordinate graph, resulting in Figure 18.

The distribution trend of AKR1C3 expression level in the KRAS G12D PDX model is mainly concentrated in medium to high expression range (LOG2 (FPKM) ≥ 4), accounting for 90.4%.

In the KRAS G12D PDX model, the distribution trend of NRF2 expression level is also concentrated in the

medium to high expression range, which is consistent with the protein expression trend of AKR1C3 and there is a certain correlation.

7. Distribution of AKR1C3 in KRAS G12C PDXs and correlation statistics with NRF2

A total of 51 model data with KRAS-G12C mutation were exported from the publicly available tumor PDX model gene database of CrownBio (https://www.crownbio.cn/model-systems/in-vivo/pdx-models/), and the RNA expression of AKR1C3 in these models was statistically analyzed. The results are shown in Table 26:

Table 26: Statistical results of the correspondence between the expression levels grading of AKR1C3 RNA expressed as LOG2 (FPKM) and KRAS-G12C

| AKR1C3 RNA Expression | AKR1C3 RNA Expression Level | % Distribution in KRAS G12C mutant PDXs |
|---|---|---|
| ≥ 8 | H | 23.5% |
| 6-7.9 | M-H | 21.6% |
| 4-5.9 | M | 21.6% |
| 2-3.9 | M-L | 19.6% |
| <2 | L | 13.7% |
| Total #PDX | | ADC #PDX 51 |

The NRF2 expression of these KRAS-G12C mutant models were further statistical analyzed. The RNA expression level of AKR1C3 and the RNA expression level of NRF2 in these 51 PDX models were projected onto an X-Y coordinate graph, resulting in Figure 19.

The average distribution of low, medium, and high expression levels of AKR1C3 in the KRAS G12C PDX model was observed, wherein the proportion of medium or above expression levels (LOG2 (FPKM) ≥ 4) was 66.7%.

In the KRAS G12C PDX model, the distribution trend of NRF2 expression is weakly correlated with the distribution of protein expression level of AKR1C3.

8. Distribution of AKR1C3 in KRAS G13D PDXs and correlation statistics with NRF2

A total of 48 model data with KRAS-G13D mutation were exported from the publicly available tumor PDX model gene database of CrownBio (https://www.crownbio.cn/model-systems/in-vivo/pdx-models/), and the RNA expression of AKR1C3 in these models was statistically analyzed. The results are shown in Table 27:

Table 27: Statistical results of the correspondence between the expression levels grading of AKR1C3 RNA expressed as LOG2 (FPKM) and KRAS-G13D

| AKR1C3 RNA expression | AKR1 C3 RNA Expression Level | % Distribution in KRAS G12C mutant PDXs |
|---|---|---|
| ≥8 | H | 83% |
| 6-7.9 | M-H | 39.6% |
| 4-5.9 | M | 35.4% |
| 2-3.9 | M-L | 10.4% |
| <2 | L | 6.3% |
| Total #PDX | | ADC #PDX 48 |

The NRF2 expression of these KRAS-G13D mutant models were further statistical analyzed. The RNA expression level of AKR1C3 and the RNA expression level of NRF2 in 48 PDX models were projected onto an X-Y coordinate graph, resulting in Figure 20.

The average distribution of low, medium, and high expression levels of AKR1C3 in the KRAS G13D PDX model was observed, wherein the proportion of medium or above expression levels was 83.3%.

In the KRAS G13D PDX model, there is a certain correlation between the distribution trend of NRF2 expression level and the distribution of protein expression level of AKR1C3.

The inventor further studied the tumor inhibitory effects of 3424 and AST in KRAS G12D-mutated tumor models

by using KRAS G12D-mutated HPAF II, LU5161 and CR3820 mouse tumor in vivo pharmacodynamic models, and detected their protein expression levels and enzyme content by using IHC assay and Western Blot assay. HPAF II (KRAS G12D) tumor cells were used to verify that AKR1C3 enzyme activated DNA alkylating agent prodrugs can activate the MEK/ERK signaling pathway. In the meantime, MEK/ERK modulators were used to investigate that the apoptosis of KRAS G12D cells is induced by AKR1C3 enzyme activated DNA alkylating agent prodrugs through the ERK2 pathway. NRF2 inhibitors and activators were used to investigate that AKR1C3 expression in KRAS G12D mutant cells is regulated by NRF2.

The in vivo pharmacodynamic studies and experimental results of AST-3424 and AST in HPAF II, LU5161, and CR3820 mouse tumor models with KRAS G12D mutation are disclosed in another patent of the applicant (application number PCT/CN2022/120817, publication number WO2023046060A1). The experimental results show that AST-3424 and AST have statistically significant anti-tumor effects and are well tolerated in the models with KRAS G12D pathogenic mutation at various test doses and administration frequencies. The patent WO2023046060A1 is incorporated by reference herein in its entirety.

In the present application and patent WO2023046060A1, gastric cancer GA6201 PDX model, pancreatic cancer PA1222 PDX model, pancreatic cancer HPAF-II CDX model, lung cancer LU5161 PDX model, colorectal cancer CR3820 PDX model are tumor models with KRAS G12D mutation, lung cancer LU11693 PDX model, pancreatic cancer PA1383 PDX model are tumor models with KRAS G12C mutation.

9. Detection of RNA expression levels and enzyme content in pancreatic cancer HPAF II cells, lung cancer LU5161 and colorectal cancer CR3820 tumor tissues

The FPKM detection method for AKR1C3 RNA expression level, IHC detection method and H-SCORE scoring for AKR1C3 protein content, which were the same as in Example 5, were used to obtain the RNA expression levels and enzyme content data in lung cancer LU5161 and colorectal cancer CR3820 tissues, as shown in Table 28. The FPKM detection method for AKR1C3 RNA expression level was used to evaluate the RNA expression level data of AKR1C3 in pancreatic cancer HPAF II cells, as shown in Table 28. The corresponding IHC staining results and Western Blot images are as shown in Figure 21.

Table 28: Results of IHC scoring and RNA expression level in HPAF II, LU5161 and CR3820 tissues

| **Tissue ID** | **Cancer type** | **AKR1C3 H-Score** | **AKR1C3 LOG2 (FPKM)** |
|---|---|---|---|
| HPAF II | Pancreatic cancer | NA | 8.31 |
| LU5161 | Lung Cancer | 272.78 | 11.56 |
| CR3820 | Colorectal cancer | 274.89 | 8.34 |

According to the above results, it is known that the AKR1C3 protein is highly expressed in the tumor tissues corresponding to these three KRAS G12D mutated mouse in vivo pharmacodynamic models.

10. The expression of AKR1C3 in KRAS G12D mutated HPAF II tumor cells is regulated by NRF2

The solution of SFN compound (Sulforaphane, NRF2 activator) was added to HPAF II cell suspension, cultured overnight in a 37 °C, 5% $CO_2$ incubator, and the cell protein lysate was collected for Western blot (WB) detection.

The experiment was assigned to 5 groups: 0.5% DMSO, 0.875 μM SFN, 1.75 μM SFN, 3.5 μM SFN, and 7 μM SFN.

HPAF II cells were treated with different concentrations of NRF2 activator SFN, and then the expression of NRF2 and AKR1C3 was detected by Western blot. The results of Western blot and analysis of NRF2 and AKR1C3 protein band density when treated with different concentrations of SFN are shown in Figure 22.

The experimental results show that with the increase of SFN treatment concentrations, the expressions of NRF2 were significantly increased, and the expressions of AKR1C3 were also increased simultaneously.

The experimental results indicate that the NRF2 activating factor SFN upregulates the expression of NRF2 in a concentration dependent manner. The expression of AKR1C3 is upregulated simultaneously with the increase of SFN concentration under the same experimental conditions. This result clearly indicates that the expression of AKR1C3 is regulated by NRF2. In addition, combined with the significant correlation between mRNA of NRF2 and AKR1C3 in the medium to high expression regions as shown in Example 6, it indicates that AKR1C3 expression is positively correlated with NRF2 expression in the KRAS G12D mutated PDX model.

According to the in vivo pharmacodynamic experiments 1-4 and the HPAF II, LU5161, and CR3820 mice tumor in vivo pharmacodynamic model studies as stated above, it is known that the AKR1C3 enzyme activated prodrugs AST-3424 and AST have significant anti-tumor effects on PDX and CDX models with high expression of AKR1C3 enzyme, and are well tolerated.

Meanwhile, combined with the experimental conclusion that AKR1C3 expression is positively correlated with NRF2 expression in Example 10, those skilled in the art can reasonably speculate that upregulating or activating NRF2 can promote high expression of AKR1C3 and thereby AKR1C3 enzyme activates prodrugs AST-3424 and AST to produce significant anti-tumor effects.

The compounds of general formulas (1) - (11) in this application are all AKR1C3 enzyme activated anticancer prodrugs. Under the effect of AKR1C3 enzyme, they are cleaved to produce anticancer active drugs such as AST-2660, paclitaxel, SN-38, gemcitabine, KARS inhibitors, etc. Therefore, those skilled in the art can reasonably speculate that upregulating or activating NRF2 will result in high expression of AKR1C3 enzyme, thereby activating compounds of general formulas (1) - (11) to metabolic produce anticancer drugs. That is, patients detected with a gene mutation that can upregulate or activate NRF2 can benefit from treatment with compounds of general formulas (1) - (11), especially from treatment with AST-3424 or AST.

The following takes AKR1C3 enzyme activated prodrug AST as an example, exploring its mechanism of action in tumor cells with KRAS mutation, especially with KRAS G12D mutation.

11. AST activates MEK/ERK signaling pathway

AST induced ERK phosphorylation experiment. Three sets of experiments were conducted with corresponding three sets of variables: AST concentration, AST action time, and AKRAC3 enzyme dependent. Wherein, the AKRAC3 enzyme dependent variables were tested under the condition of AKR1C3 enzyme specific inhibitor AST-3021, with or without the presence of specific AKR1C3 enzyme inhibitor AST-3021. The inhibitor used is AST-3021 (also known as TH-3021, which is compound 36 reported by Flanagan et al. Bioorganic and Medicinal Chemistry (2014) pages 962-977, and its structural formula is

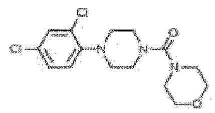

The experiment was assigned to three groups, conducted by treatment with different concentrations of AST (Group 1), 1 μM AST for different times (Group 2), with or without the AKR1C3 inhibitor AST-3021 (Group 3), respectively.

The three specific groups of compounds to be treated are as follows:

Group 1 (concentration group): 0.5% DMSO treatment group (24h), 0.5 μM AST treatment group (24h), 1 μM AST treatment group (24h), and 10 μM AST treatment group (24h);
Group 2 (time group): 0.5% DMSO treatment group (24h), 1 μM AST treatment group (2h), 1 μM AST treatment group (8h), and1 μM AST treatment group (24h); and
Group 3 (AKR1C3 dependent group): 0.5% DMSO treatment group (24h), 1 μM AST treatment group (24h), 3 μM AST-3021 treatment group (24h), and 1 μM AST + 3 μM AST-3021 treatment group (24h).

Each group of compound solution was separately added to HPAF II cell suspension, cultured overnight in a 37 °C, 5% $CO_2$ incubator, and the cell protein lysate was collected for Western blot (WB) detection according to the experimental group.

The expressions of NRF2 and AKR1C3 were detected by Western blot. The results of the Western blot images and p-NRF2 and NRF2 protein band density analysis obtained by treating with different concentrations of AST in Group 1 are shown in Figure 23; the results of the Western blot images and p-NRF2 and NRF2 protein band density analysis obtained by treating with 1 μM AST at different times in Group 2 are shown in Figure 24; and the results of the Western blot images and p-NRF2 and NRF2 protein band density analysis obtained by adding or not adding AKR1C3 inhibitor AST-3021 in Group 3 are shown in Figure 25.

The experimental results show that, in Figure 23, as the concentration of AST treatment increases, the expression level of p-NRF2 increased correspondingly and the expression of p-NRF2 increased significantly when treated with 10 μM AST for 24 hours, while the expression level of NRF2 was not affected by various AST treatment concentrations. In Figure 24, the expression level of p-NRF2 remained basically unchanged when treated with 1 μM AST for 2 hours and 8 hours, but increased significantly compared to the DMSO control group when treated for 24 hours. In Figure 25, only the single drug 1 μM AST treatment group (24h) showed a significant increase in p-NRF2 expression, while

the p-NRF2 expression levels of the other 0.5% DMSO treatment group (24h), 3 μM AST-3021 treatment group (24h), and 1 μM AST + 3 μM AST-3021 treatment group (24h) remained basically unchanged. In the above three experiments, the total ERK2 expression in each treatment group did not change.

The above experiments indicate that AST induces phosphorylation of ERK2 in a concentration dependent manner without affecting the expression level of ERK2. After treatment with 1 μ mol/L AST for 24 hours, the phosphorylation level of ERK2 significantly increased compared to the DMSO control group. As shown in Figure 25, HPAF II cells were pretreated with AKR1C3 specific inhibitor AST-3021 2 hours before the addition of 1 μmol/L AST, and 3 μmol/L AST-3021 strongly inhibited the increase of AST-mediated phosphorylation of ERK2. Using AST-3021 alone did not affect the level of p-ERK2. The expression of total ERK2 remained unchanged under all testing conditions. It was conformed that all samples were added in the same amount by actin Western blot. AST activates the MEK/ERK signaling pathway in a concentration, time, and AKR1C3 dependent manner.

12. AST induces apoptosis in KRAS G12D cells through the ERK2 signaling pathway

The experiment used three types of MEK/ERK modulators, including two inhibitors and one activator. The inhibitors were PD98059, a MEK1/2 specific inhibitor, and Suramin, a broad-spectrum growth factor receptor inhibitor; while the activator of the ERK signaling pathway is Phorbol Ester (TPA). The chemical structures of inhibitor PD98059, inhibitor Suramin, and activator TPA are as follows:

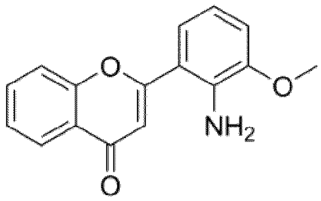

(PD98059),

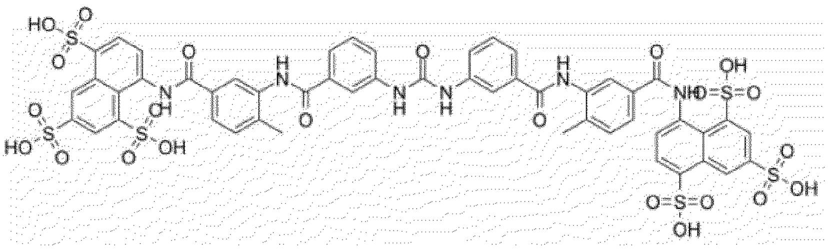

(Suramin),

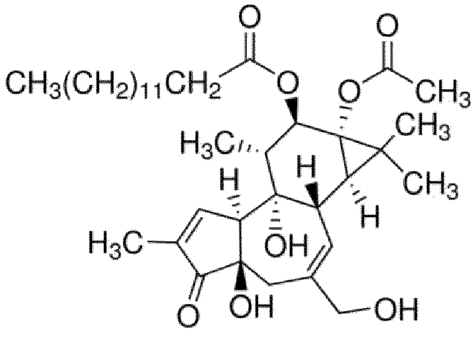

(TPA).

The experiment was assigned to inhibitor group and activator group, both of which were performed by Western Blot and cell apoptosis experiments.

In the Western blot experiment, HPAF II cell suspensions were treated with each group of compounds, and cultured overnight in a 37 °C, 5% $CO_2$ incubator, and the cell protein lysate was collected for Western blot (WB) detection.

The specific compounds to be treated in the Western blot inhibitor group and activator group are as follows: Inhibitor group: 1% DMSO treatment group, 1 μM AST treatment group, 30 μM PD98059 treatment group, 1 mM Suramin treatment group, 1 μM AST + 30 μM PD98059 treatment group, and 1 μM AST + 1 mM Suramin treatment group; and Activator group: 1% DMSO treatment group, 0.5 μM AST treatment group, 25 nM TPA treatment group, and 0.5 μM AST + 25 nM TPA treatment group.

Additionally, cell apoptosis detection was performed as follows:

1) Caspase Glo® 3/7 reagents were prepared and equilibrated to room temperature. The buffer of the reagents and the substrate were mixed homogeneously at 1:1 to prepare Caspase Glo® 3/7 reagents. The configured reagents can be stored at 4 °C for 3 days.

2) 100 μL Caspase Glo® 3/7 reagents were added to each well of culture medium containing 100 μL blank (wells without cells), negative control cells, or treated cells. Due to the sensitivity of the analysis, be careful not to make the tip of the pipette to touch the holes that contained samples to avoid cross contamination. The plates were covered with a plate sealant or lid.

3) The reagent solutions were mixed gently using a shaker at a speed of 300-500 rpm for 30 seconds, and left in the dark at room temperature for 1 hour.

4) The luminescence of each sample was measured in a plate reading photometer. And the luminescence value of each group was calculated, and analyze by making a column diagram.

The specific compounds to be treated in the apoptosis inhibitor group and activator group are as follows: Inhibition group: 1% DMSO treatment group, 1 μM AST treatment group, 30 μM PD98059 treatment group, 1 mM Suramin treatment group, 1 μM AST + 30 μM PD98059 treatment group, and 1 μM AST + 1 mM Suramin treatment group; and Activator group: 1% DMSO treatment group, 1 μM AST treatment group, 50 nM TPA treatment group, and 1 μM AST + 50 nM TPA treatment group.

The experimental results of Western Blot, protein band density analysis, and cell apoptosis of the ERK2 inhibitor and AST monotherapy and combination therapy groups are as shown in Figure 26. The experimental results of Western Blot, protein band density analysis, and cell apoptosis of the ERK2 activator and AST monotherapy and combination therapy groups are as shown in Figure 27.

The experimental results show that in the inhibitor group of Figure 26, the upper and lower left figures show that PD98059 and suramin alone slightly reduced the expression level of p-ERK2, while the combination therapy group of PD98059 and AST significantly reduced the expression level of p-ERK2. The expression level of p-ERK2 slightly increased in the combination therapy group of Suramin and AST, but significantly increased in the AST monotherapy group. The lower right figure show that the AST monotherapy group significantly affected cell apoptosis, while the PD98059, suramin monotherapy group, and their combination therapy groups with AST respectively had almost no effect on cell apoptosis. In the activator group of Figure 27, as shown in the upper and lower left figures, the p-ERK2 expression levels of the AST and TPA monotherapy treatment groups increased compared to the DMSO control group, while the p-ERK2 expression levels in the combination therapy group of AST and TPA significantly increased. As shown in the lower right figure, the AST monotherapy group promoted cell apoptosis, while the combination therapy group of AST and TPA had a more significant effect on cell apoptosis.

The experimental results show that treatment with two ERK inhibitors, PD98059 and suramin alone, slightly reduced the phosphorylation level of endogenous ERK, while treatment with AST alone significantly increased the level of p-ERK2 in cells. Meanwhile, the combined use of AST and two ERK inhibitors almost completely counteracted the increase in p-ERK2 induced by AST. The Caspase 3/7 kit was used to detect the effects of AST or ERK2 inhibitor monotherapy, and combined use of AST and ERK2 inhibitor on cell apoptosis. The results show that ERK2 inhibitor significantly inhibited AST induced cell apoptosis. In comparison, treatment with ERK2 activator TPA alone can also promote the level of p-ERK2 in cells, while the combined treatment with TPA and AST further enhanced the phosphorylation of ERK2 induced by AST and cell apoptosis. Treating the cells using either monotherapy with any of the above-mentioned various drugs or combination therapy did not affect the expression of total ERK2. These results clearly indicate that AST induces apoptosis in KRAS G12D mutant cells through the ERK2 signaling pathway.

By combining the results of Examples 10, 11, and 12, the mechanism diagram in Figure 28 can be inferred. That is, AST induces apoptosis of KRAS G12D pathogenic mutant cancer cells through the ERK2 signaling pathway. Further combining the research of relevant literature: the MEK/ERK signaling pathway can be activated by DNA alkylating agents (Wang X, Martindale JL and Holbrook NJ. Requirement for ERK activation in cisplatin-induced apoptosis. J Biol Chem 2000; 275: 39435-39443); Examples 11 and 12 confirmed that AST, a DNA alkylating agent prodrug, has the effect of activating the MEK/ERK signaling pathway, which can induce apoptosis of KRAS G12D mutant cancer cells through the ERK2 signaling pathway. AST compound is a DNA alkylating agent prodrug activated by AKR1C3 enzyme, which can be activated by AKR1C3 enzyme to produce AST-2660 and exert anti-cancer effects.

Considering the AKR1C3 and ERK2 related regulatory pathways revealed in Figure 28, the inventors speculate that AST compounds can activate ERK2 to phosphorylate it, and then activating the MEK-ERK pathway to upregulate NRF2, ultimately leading to high expression of AKR1C3 enzyme, and then resulting in cancer cell apoptosis. That is, by

activating the RAF/MEK/ERK signaling pathway, the KRAS-G12D mutation upregulates the protein expression of NRF2, and then upregulates the protein expression of AKR1C3, which can activate an AST drug more effectively to make it release the DNA alkylating agent AST-2660.

In fact, research has shown that many gene mutations can upregulate the protein expression of NRF2, and then upregulate the protein expression of AKR1C3. These gene mutations include KRAS mutation, KEAP1 mutation, CUL3 mutation, NRF2 mutation, p21 mutation, p62 mutation, FTL1 mutation, FTH1 mutation, HMOX1 mutation, GSR mutation, SLC7A11 mutation, GCLM mutation, GCLC mutation, GPX2 mutation, TXN1 mutation, TXNRD1 mutation, PRDX1 mutation, SRXN1 mutation, ABCC1 mutation, ABCC2 mutation, G6PD mutation, PGD mutation, ME1 mutation, IDH1 mutation, EGFR mutation, etc.

The mechanism of the AST compounds revealed in Figure 28 was further extended to other similar AKR1C3 enzyme activated DNA alkylating agent prodrugs: compounds of general formulas (1), (2), (3), (4), (5), (6), and (8) and similar compounds such as AST, AST-3424. These compounds and the investigated drug AST in Figure 28 have a same mechanism of action and are all AKR1C3 enzyme activated DNA alkylating agent prodrugs, which will be activated by the AKR1C3 enzyme to produce AST-2660 and exert anti-cancer effects. Therefore, by combining the mechanism shown in Figure 28, it can be confirmed that compounds of general formulas (1), (2), (3), (4), (5), (6), and (8) should be similar to AST and can also activate the MEK/ERK signaling pathway, inducing apoptosis in cells with a gene mutation that can upregulate or activate NRF2 through the ERK2 signaling pathway. In other words, a gene mutation that can upregulate or activate NRF2, by activating the RAF/MEK/ERK signaling pathway, upregulating the protein expression of NRF2, and then upregulating the protein expression of AKR1C3, are able to activate the AKR1C3 enzyme activated DNA alkylating agent prodrug more effectively.

Based on this, combined with Figure 28, it can be inferred that compounds of general formulas (1), (2), (3), (4), (5), (6), and (8) should be similar to AST, and can also activate the MEK/ERK signaling pathway, and ultimately induce apoptosis of cells with a gene mutation that can upregulate or activate NRF2 through the ERK2 signaling pathway.

The compounds of general formulas (7), (9), (10), and (11) in the application are similar to AST-3424 and AST, and are also anticancer prodrugs activated by AKR1C3 enzyme. They are activated by AKR1C3 enzyme to produce anticancer active drugs such as paclitaxel, SN-38, gemcitabine, or KARS inhibitors. Therefore, those skilled in the art should be aware that compounds of general formulas (7), (9), (10), and (11) have significant tumor inhibitory effect similar to AST-3424 and AST.

## Claims

**1.** A treatment method for cancer patients with an AKR1C3 enzyme-activated prodrug, **characterized in that**:

the tumor or cancer tissue of the patients is detected to have a gene mutation capable of up-regulating or activating NRF2; or
the patients are detected to have a gene mutation capable of up-regulating or activating NRF2.

**2.** The treatment method according to claim 1, wherein:
the gene mutation capable of up-regulating or activating NRF2 is selected from the group consisting of KRAS mutation, KEAP1 mutation, CUL3 mutation, NRF2 mutation, p21 mutation, p62 mutation, FTL1 mutation, FTH1 mutation, HMOX1 mutation, GSR mutation, SLC7A11 mutation, GCLM mutation, GCLC mutation, GPX2 mutation, TXN1 mutation, TXNRD1 mutation, PRDX1 mutation, SRXN1 mutation, ABCC1 mutation, ABCC2 mutation, G6PD mutation, PGD mutation, ME1 mutation, IDH1 mutation, and EGFR mutation.

**3.** The treatment method according to claim 2, wherein the KRAS mutation is selected from the following six subtypes: G12D, G12V, G12R, G12C, G12A and G13D.

**4.** The treatment method according to claim 1, wherein the AKR1 C3 enzyme-activated prodrug is selected from the group consisting of the compounds with formula (1), (2), (3), (4), (5), (6), (7), (8), (9), (10), and (11), and salt, ester, solvate, isotope isomer thereof:

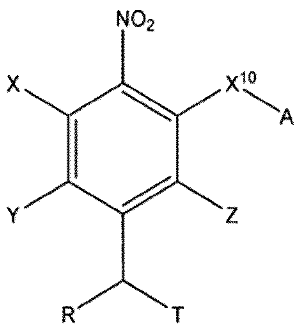

(1),

wherein the definitions of X, Y, Z, R, T, A and $X^{10}$ are as described in the claims of Patent Application PCT/US2016/021581 with Publication No. WO2016145092A1 (corresponding to the Chinese Application No. 2016800150788 with Publication No. CN107530556A);

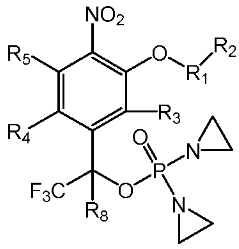

(2),

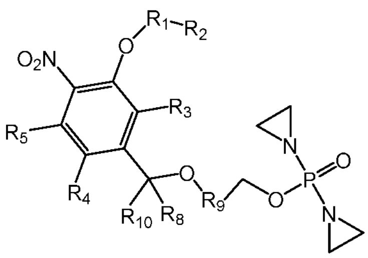

(3),

wherein the definitions of $R_1$, $R_2$, $R_3$, $R_4$, $R_5$, $R_8$, $R_9$, $R_{10}$ are as described in the claims of Patent Application PCT/CN2020/089692 with Publication No. WO2020228685A9 (corresponding to the Chinese Application No. 2020800358890 with Publication No. CN113853379A);

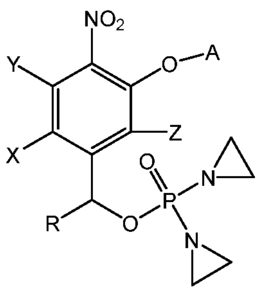

(4),

wherein:

A is a substituted or unsubstituted $C_6$-$C_{10}$ aryl, a biaryl or substituted biaryl, a 5-15-membered heteroaryl, or $-N=CR^1R^2$; wherein the substituent is selected from the group consisting of halogeno, -CN, $-NO_2$, $-O-(CH_2)-O-$, $-CO_2H$ and salt thereof, $-OR^{100}$, $-CO_2R^{100}$, $-CONR^{101}R^{102}$, $-NR^{101}R^{102}$, $-NR^{100}SO_2R^{100}$, $-SO_2R^{100}$, $-SO_2NR^{101}R^{102}$, $C_1$-$C_6$ alkyl, and $C_3$-$C_{10}$ heterocyclyl;
wherein $R^{100}$, $R^{101}$ and $R^{102}$ are each independently hydrogen, $C_1$-$C_8$ alkyl, or $C_6$-$C_{12}$ aryl; or $R^{101}$ and $R^{102}$ together with the nitrogen atom to which they are attached form a 5-7-membered heterocycle;
wherein the alkyl group and the aryl group are each substituted by 1-3 halogeno groups or 1-3 $C_1$-$C_6$ alkyl groups;
$R^1$ and $R^2$ are each independently phenyl or methyl;
X, Y and Z are each independently hydrogen or halogeno; and
R is hydrogen or $C_1$-$C_6$ alkyl or halogen-substituted alkyl;

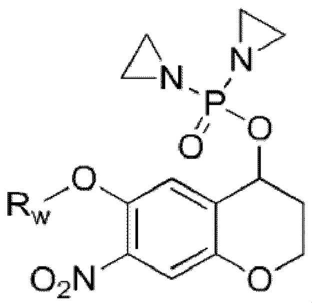

(5),

wherein the definitions of Rw are as described in the claims of Patent Application PCT/CN2020/120281 with Publication No. WO2021068952A1 (corresponding to the Chinese Application No. 202080071652.8 with Publication No. CN114555574A);

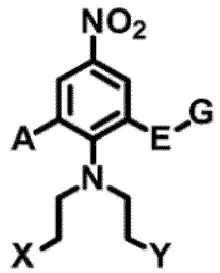

(6),

wherein the definitions of A, E, G, X and Y are as described in the claims of Patent Application PCT/NZ2019/050030 with Publication No. WO2019190331A1 (corresponding to Chinese Patent Application No. 2019800234236 with Publication No. CN111918864A);

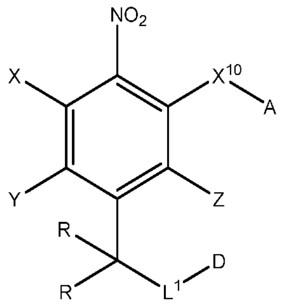

(7),

wherein the definitions of X, Y, Z, R, D, $L^1$, A and $X^{10}$ are as described in the claims of Patent Application PCT/US2016/025665 with Publication No. WO2016161342A3 (corresponding to Chinese Patent Application No. 2016800200132 with Publication No. CN108136214A);

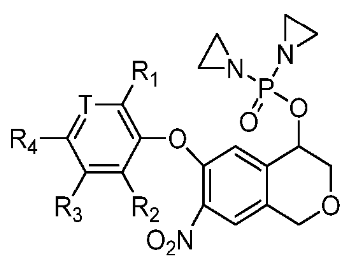

(8),

wherein the definitions of $R_1$, $R_2$, $R_3$, $R_4$ and T are as described in the claims of Patent Application PCT/CN2021/118597 with Publication No.WO2022057838A1;

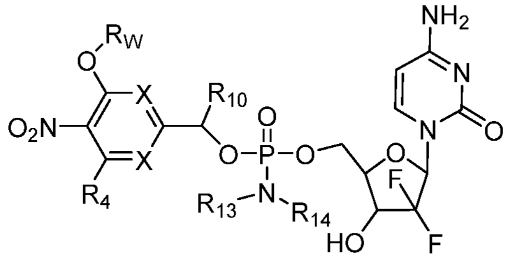

(9),

or pharmaceutically acceptable salt thereof,
wherein the definitions of $R_w$, X, $R_4$, $R_{10}$, $R_{13}$ and $R_{14}$ are as described in the claims of Patent Application PCT/CN2022/098082 with Publication No. WO2022258043A1;

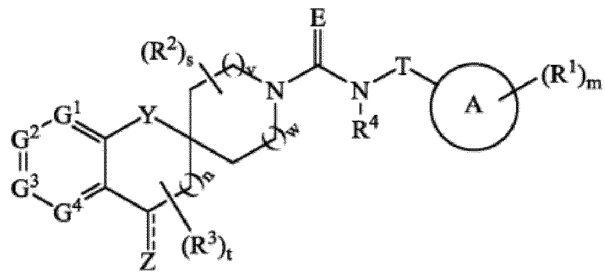

(10),

wherein the definitions of $R_1$, $R_2$, $R_3$, $R_4$, $G_1$, $G_2$, $G_3$, $G_4$, E, T, Y, Z, m, n, s, t, v, w and ring A are as described in the claims of Patent Application CN202210585771.6 with Publication No. CN115403579A;

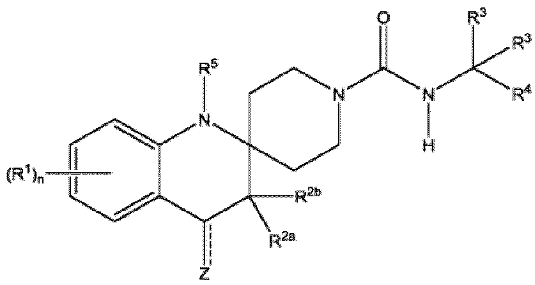

(11),

or pharmaceutically acceptable salt thereof,
wherein the definitions of $R^1$, $R^{2a}$, $R^{2b}$, $R^3$, $R^4$, $R^5$, n and Z are as described in the claims of Patent Application PCT/IB2020/057285 with Publication No. WO2021005586A1 (corresponding to Chinese Patent Application No. CN202080053804.1 with Publication No. CN114206870A).

**5.** The treatment method according to any one of claims 1-4, wherein:

the AKR1C3 enzyme-activated prodrug compound with formula (1) is selected from the compounds with the following structural formulae:

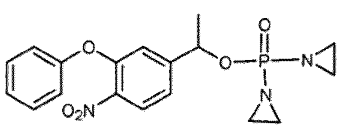
,
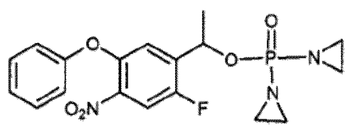
,
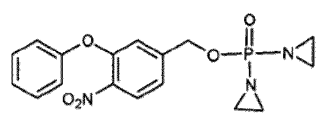
,

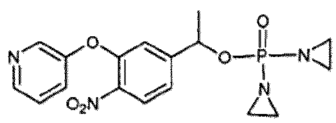
,
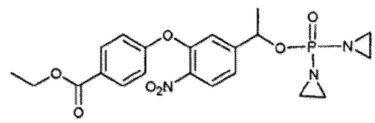
,
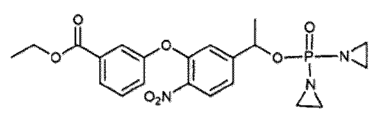
,

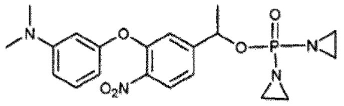 , 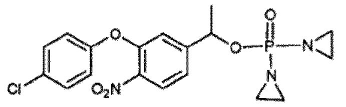 , 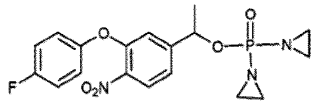 ,

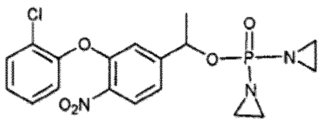 , 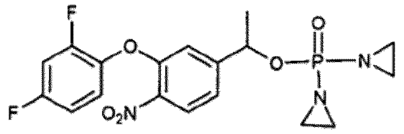 , 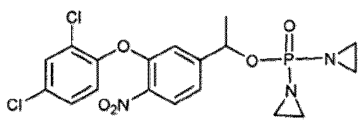 ,

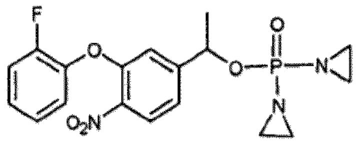 , 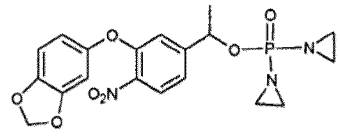 , 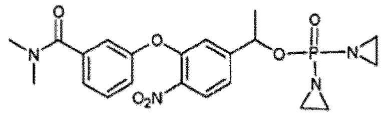 ,

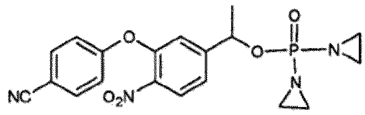 , 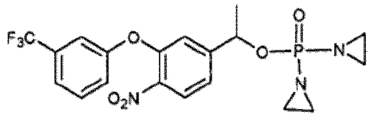 , 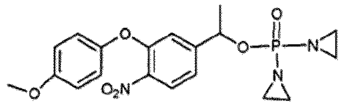 ,

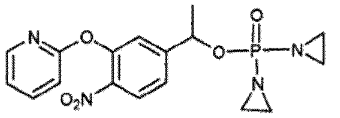 , 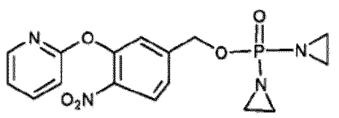 , 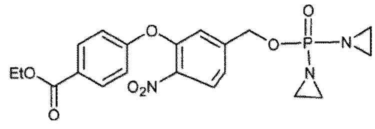 ,

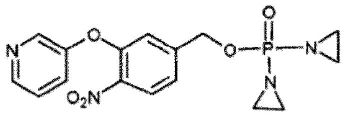 , 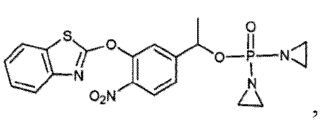 , 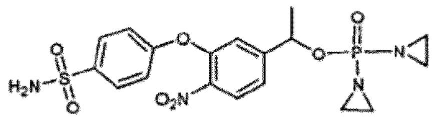 ,

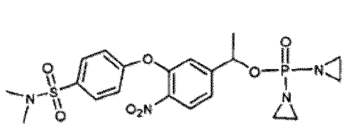 , 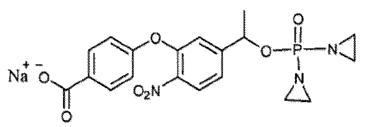 , 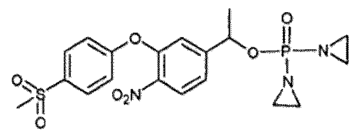 ,

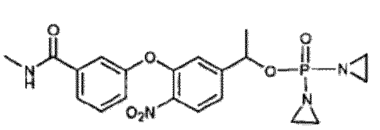 , 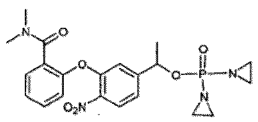 , 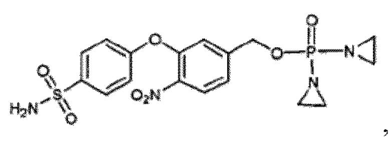 ,

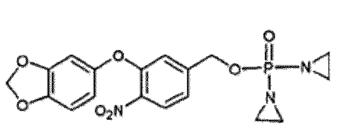 , 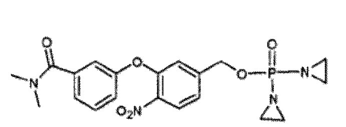 , 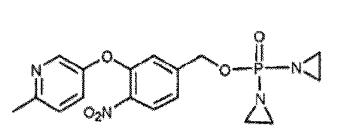 ,

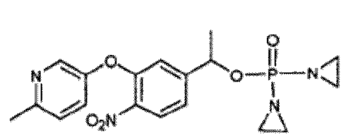 , 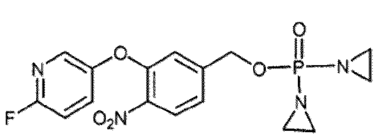 , 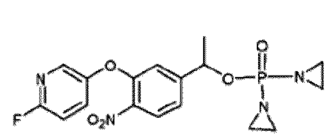 ,

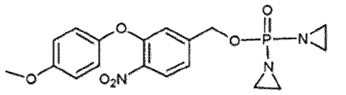, 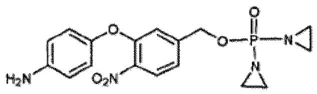, 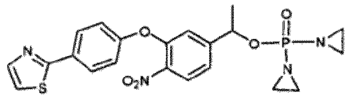,

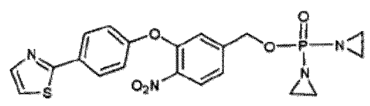, 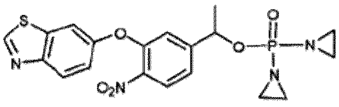, 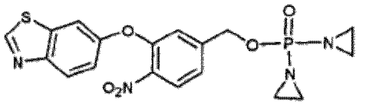

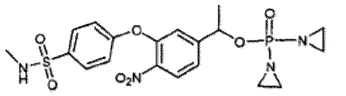, 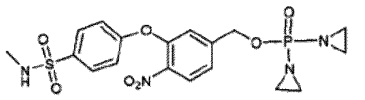, 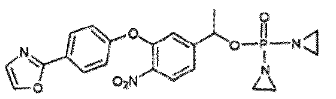,

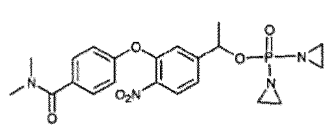, 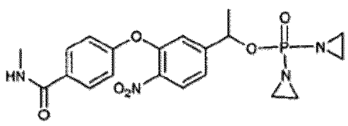, 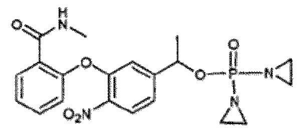,

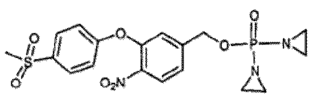, 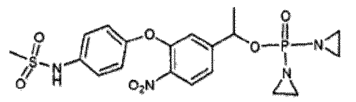, 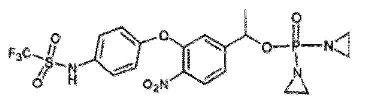,

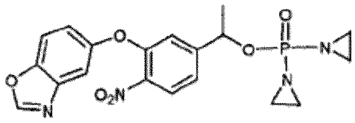, 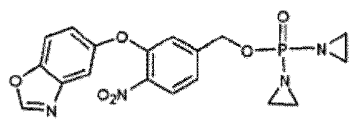, 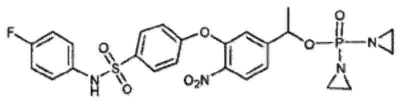

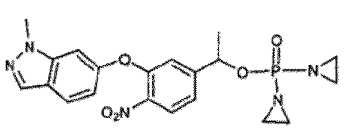, 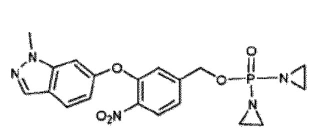, 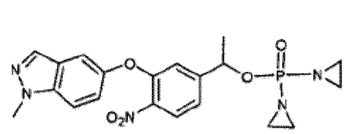,

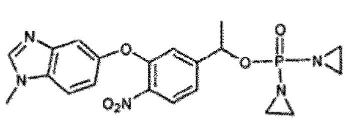, 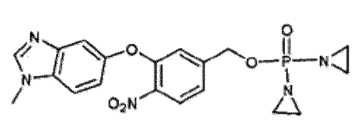, 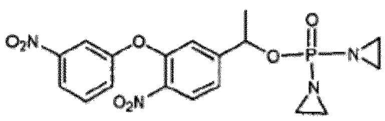,

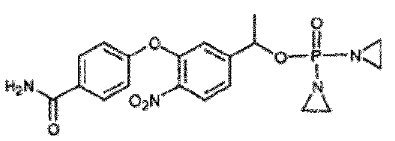, 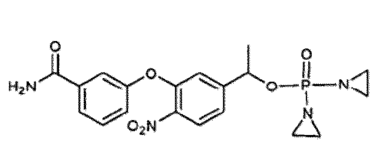, 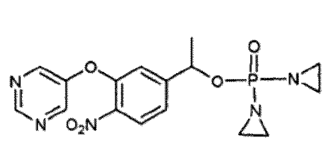,

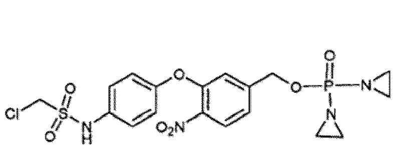, 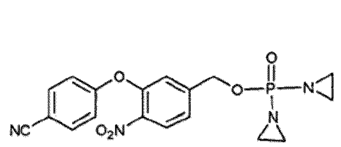, 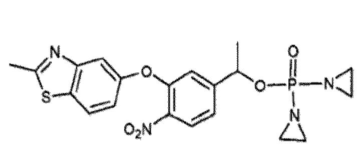,

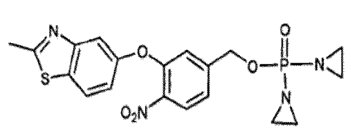,
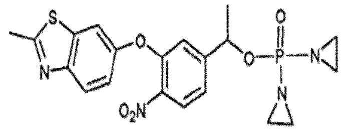,
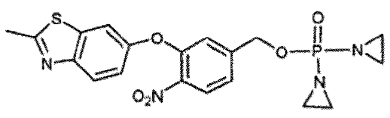,

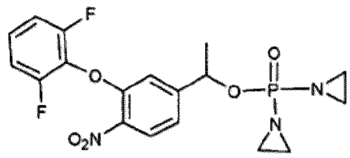,
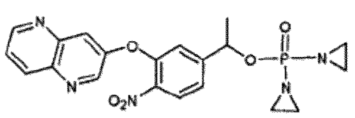,
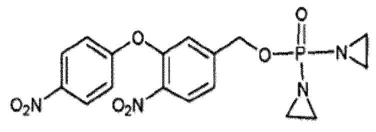,

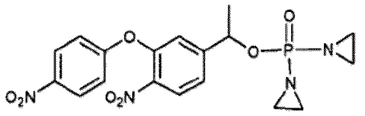,
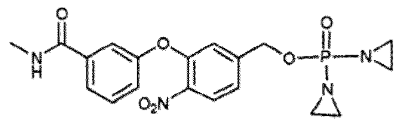,
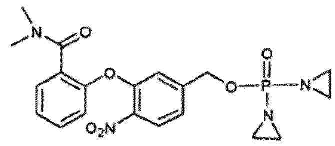,

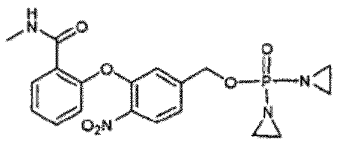,
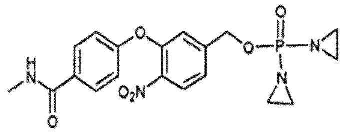,
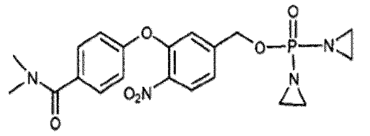,

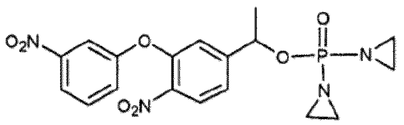,
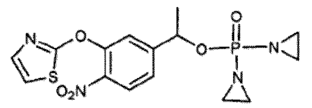,
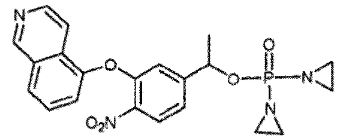,

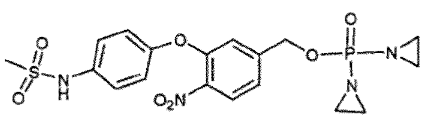,
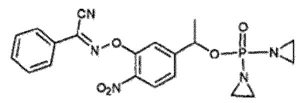,
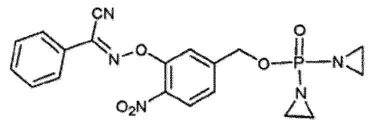,

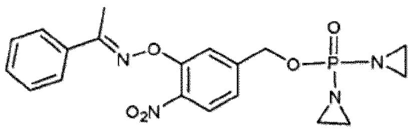,
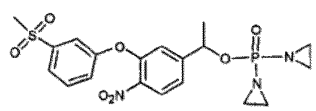,
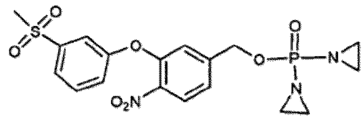,

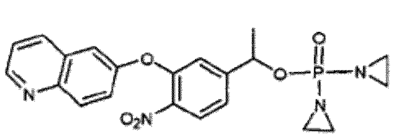,
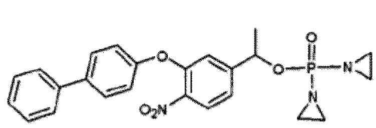,
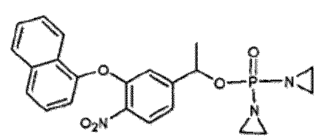,

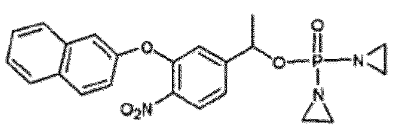,
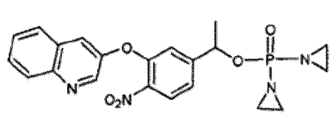,
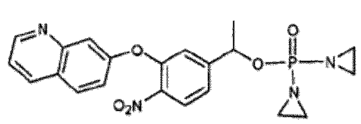,

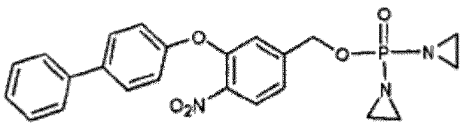 , 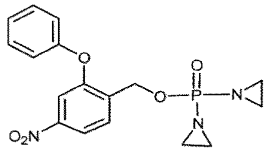 , 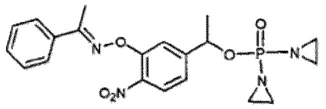 ,

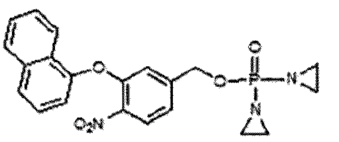 , 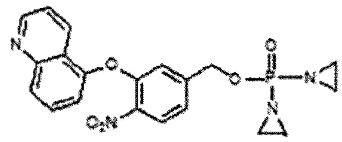 , 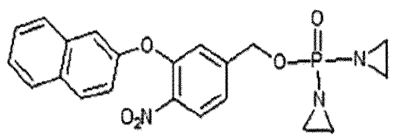 ,

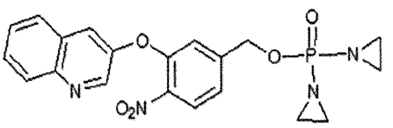 , 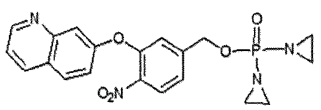 , 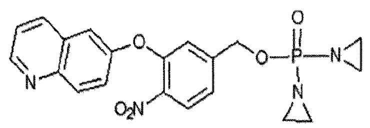 ,

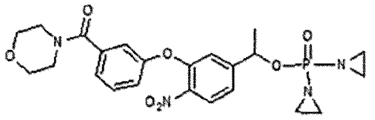 , 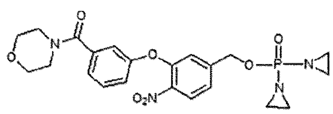 , 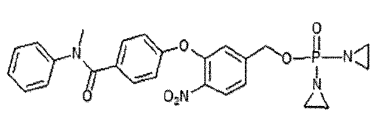 ,

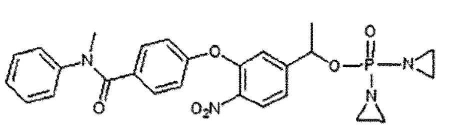 , 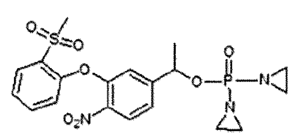 , 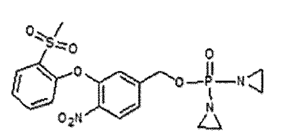 ,

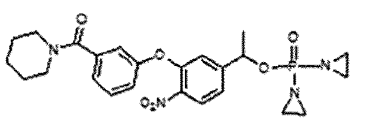 , 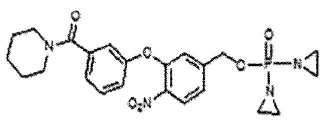 , 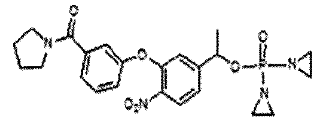 ,

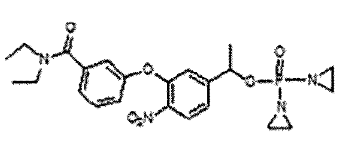 , 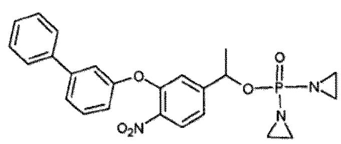 , 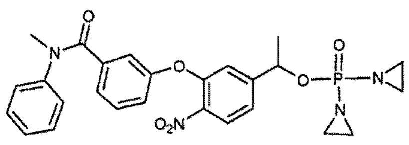 ,

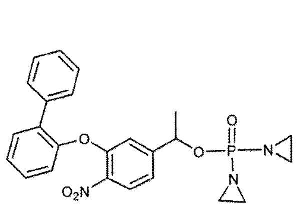 , 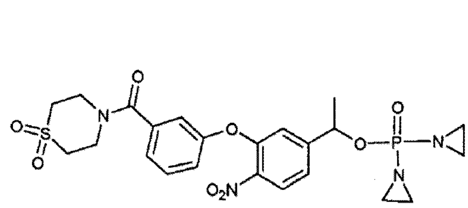 , 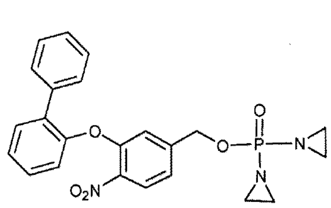 ,

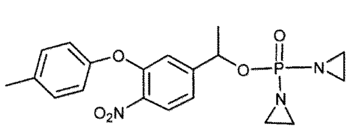 , 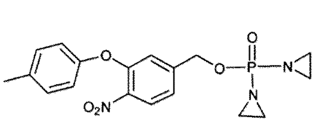 , 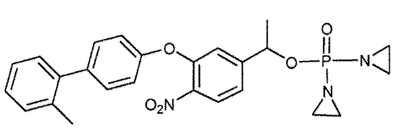 ,

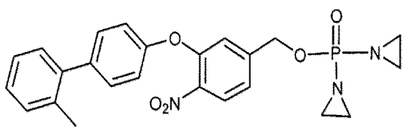 , 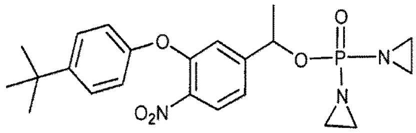 ,

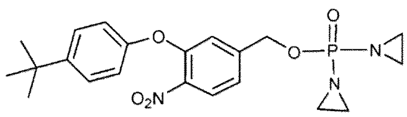 , 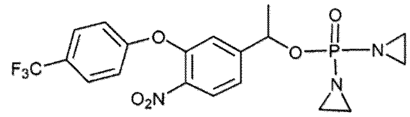 ,

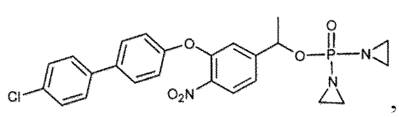 , 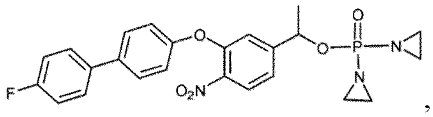 ,

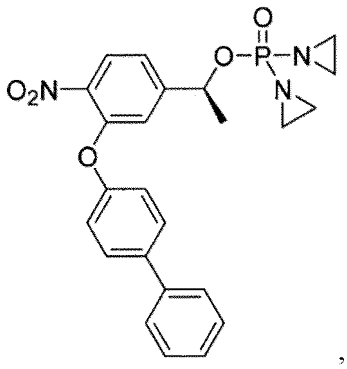 , 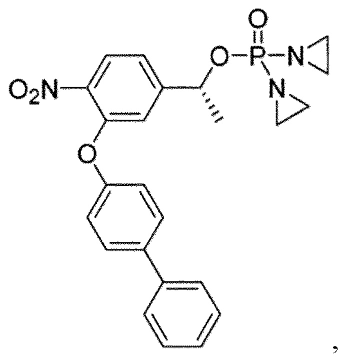 ,

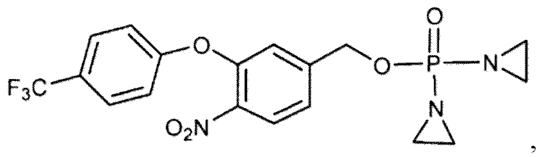 , 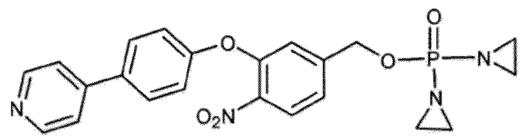 ;

the AKR1C3 enzyme-activated prodrug compound with formula (2) is selected from the compounds with the following structural formulae:

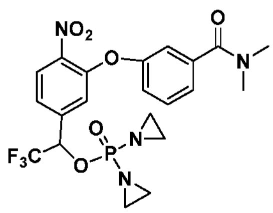 , 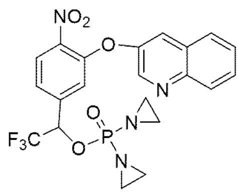 , 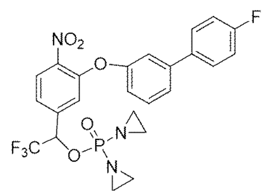 , 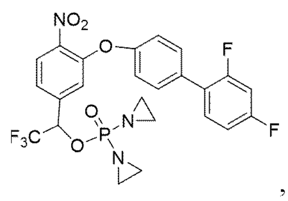 ,

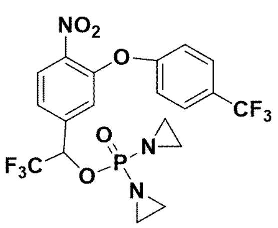 , 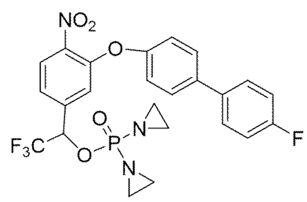 , 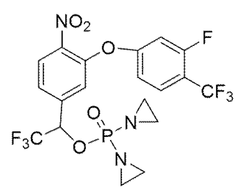 , 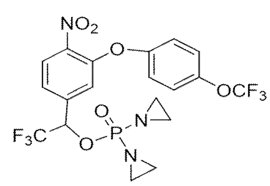 ,

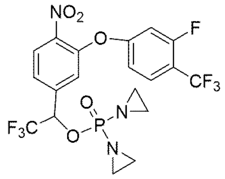,
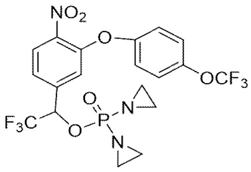,
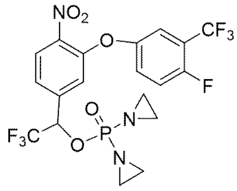,
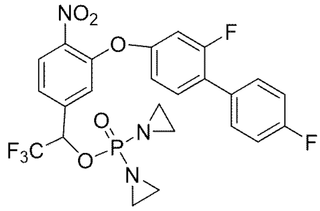,

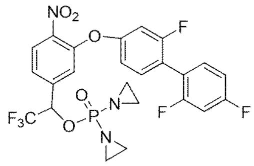,
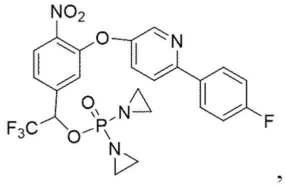,
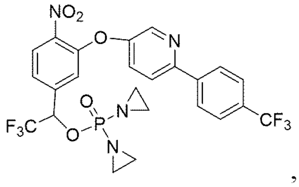,
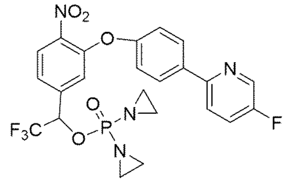,

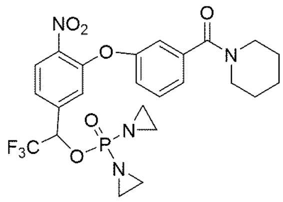,
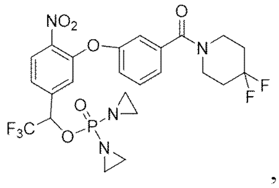,
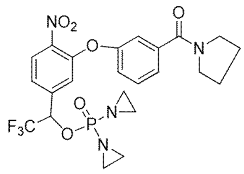,
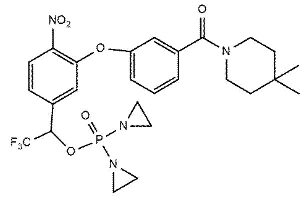,

;
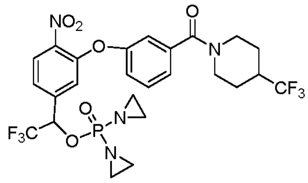,
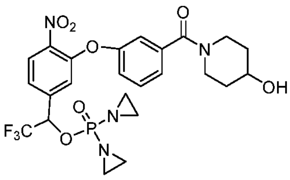,
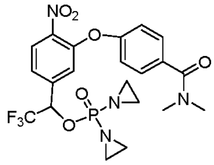,
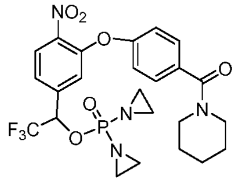,

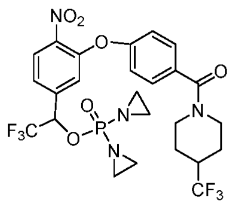,
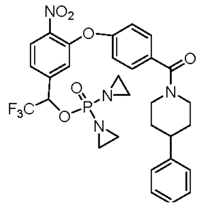,
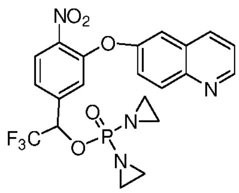,
AST-A-143
Bellen00036076
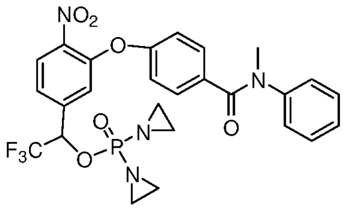,
AST-A-144
Bellen00036077

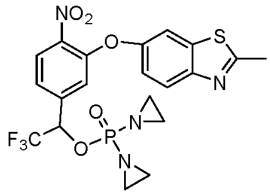,
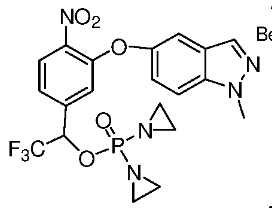,
Bellen00036079
AST-A-146
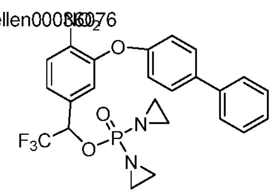,
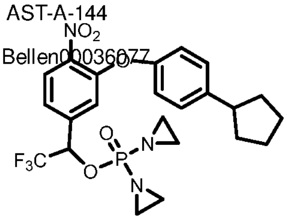,

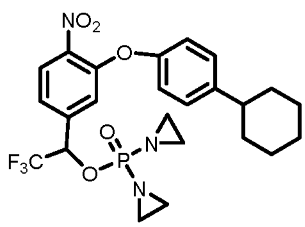,
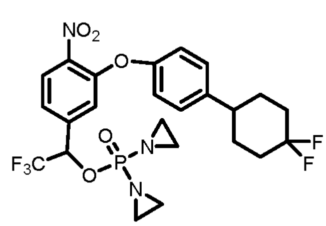;

the AKR1C3 enzyme-activated prodrug compound with formula (3) is selected from the compounds with the following structural formulae:

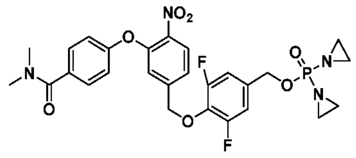, 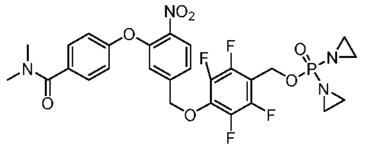, 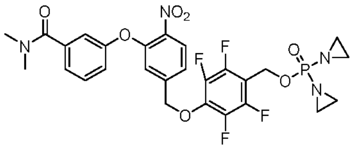,

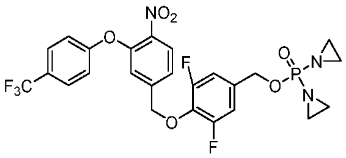, 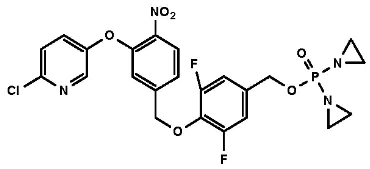, 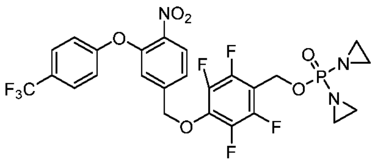,

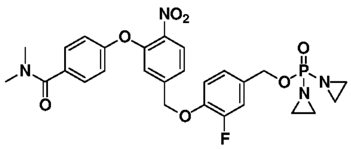, 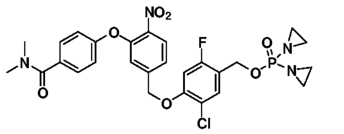, 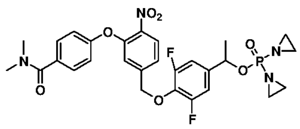,

AST-A-017 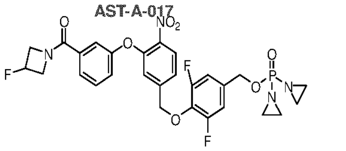, AST-A-018 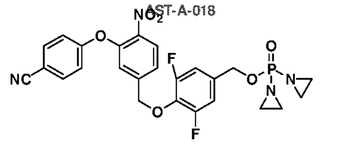, MW: 602.53 AST-A-021 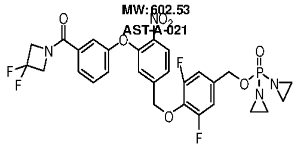,

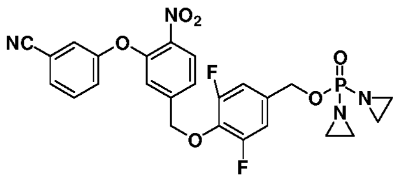;

the AKR1C3 enzyme-activated prodrug compound with formula (4) is selected from the compounds with the following structural formulae:

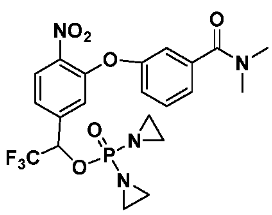, 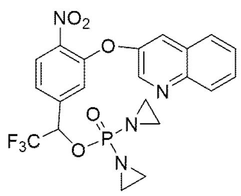, 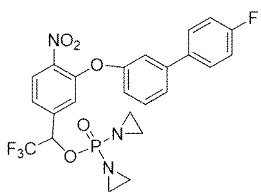, 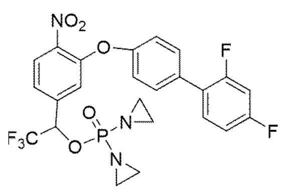

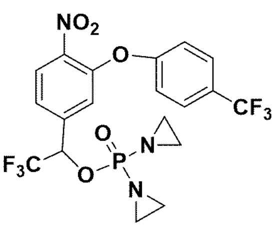, 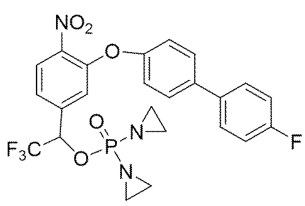, 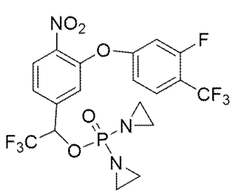, 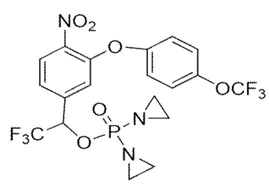

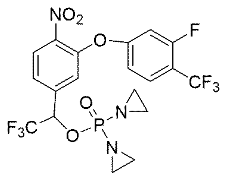 , 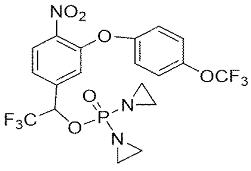 , 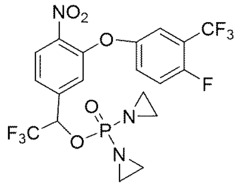 , 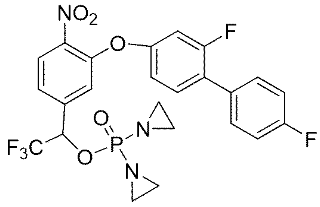 ,

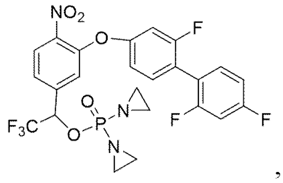 , 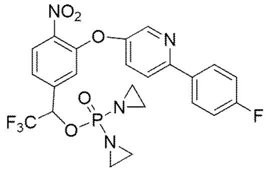 , 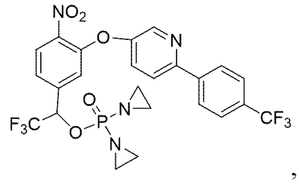 , 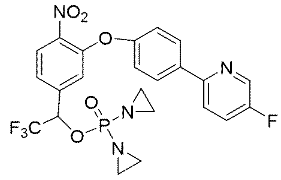

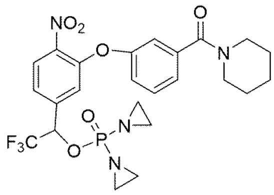 , 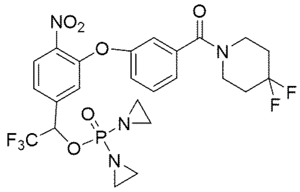 , 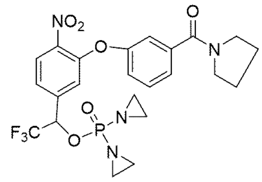 , 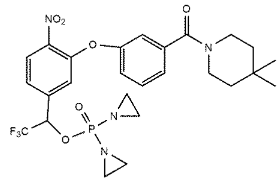

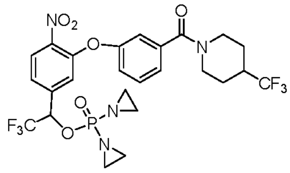 , 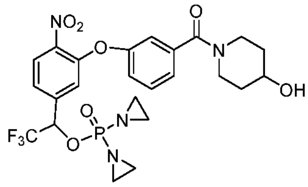 , 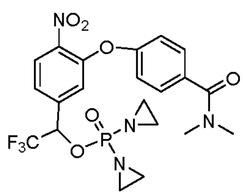 , 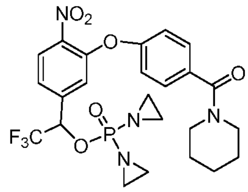 ,

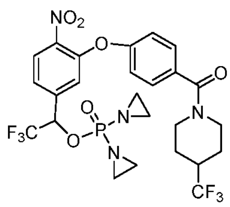 , 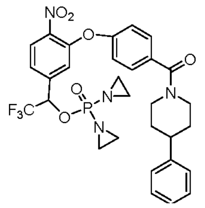 ,

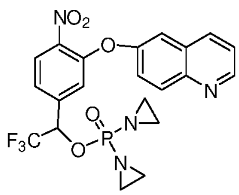

AST-A-143

Bellen00036076

,

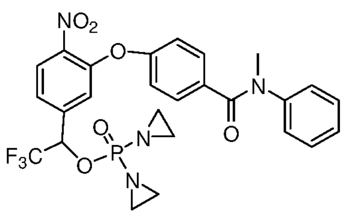

AST-A-144

Bellen00036077

,

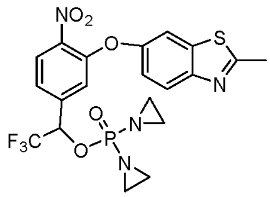 ,

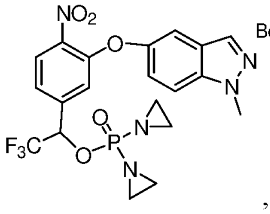

Bellen00036079

AST-A-146

, 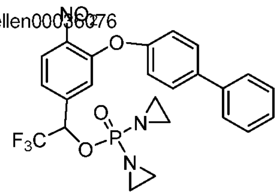 , 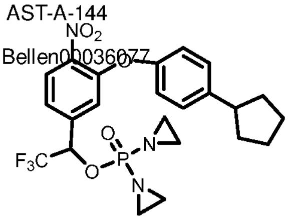 ,

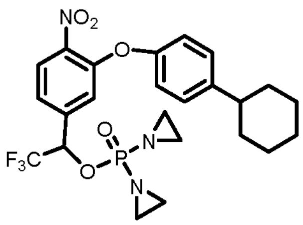 , 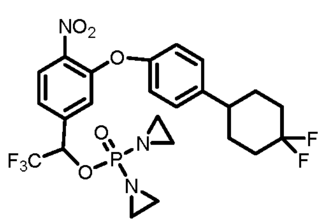

; and

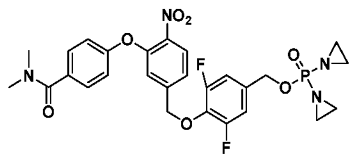,
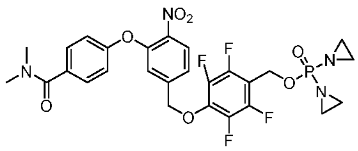,
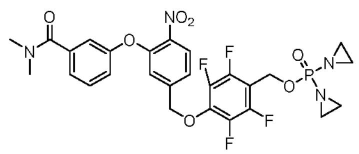

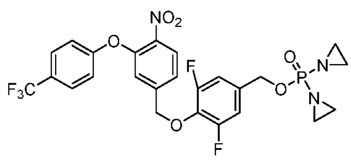,
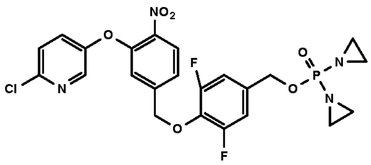,
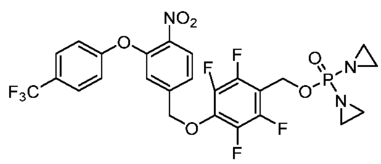

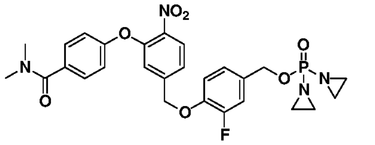,
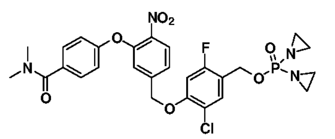,
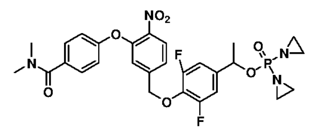,

AST-A-017
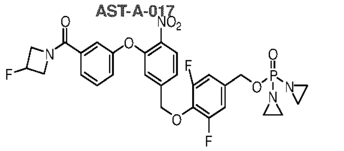,
AST-A-018
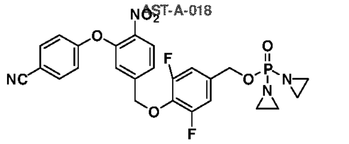,
MW: 602.53
AST-A-021
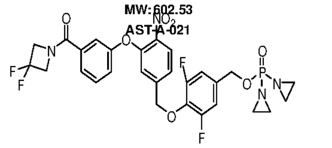,

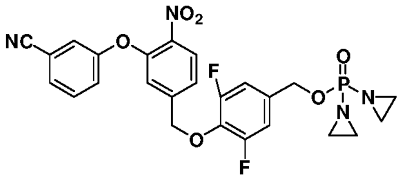

; and

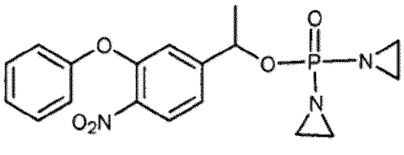,
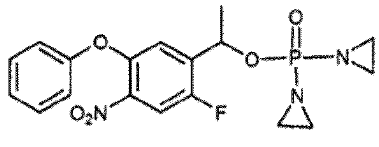,

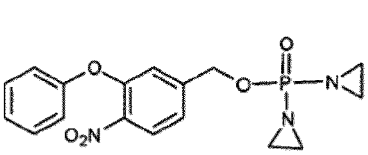,
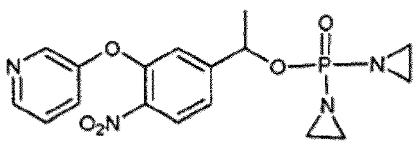,

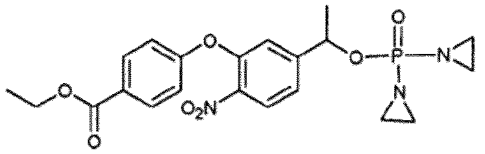,
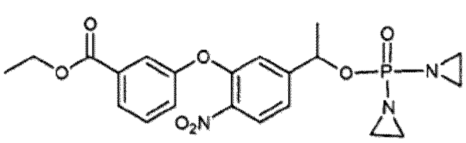,

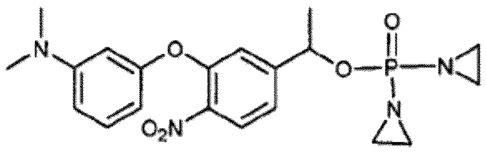,
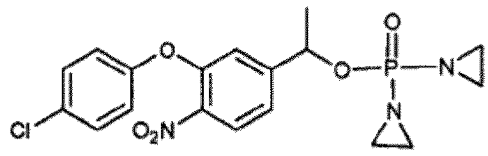,
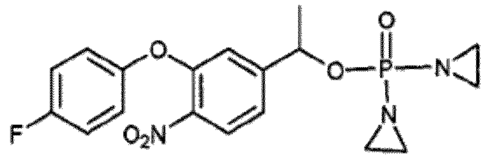,
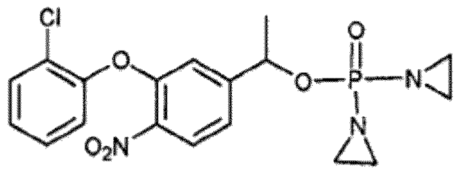,
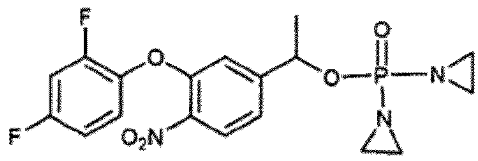,
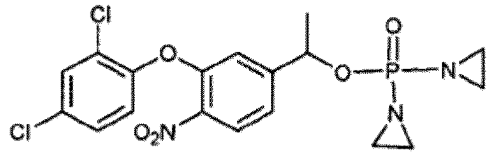,
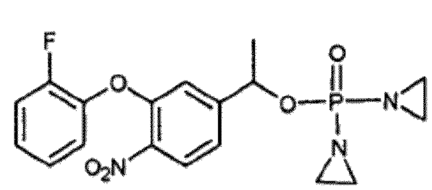,
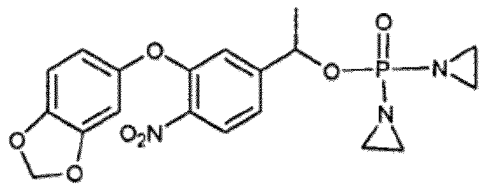,
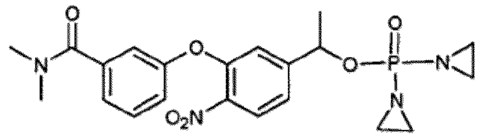,
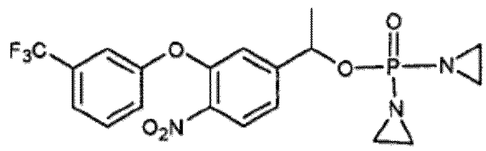,
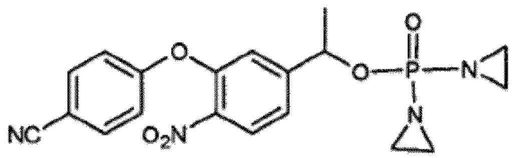,
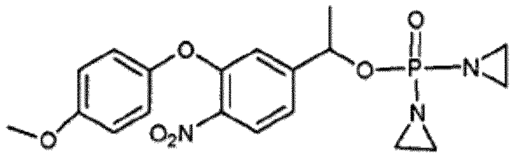,
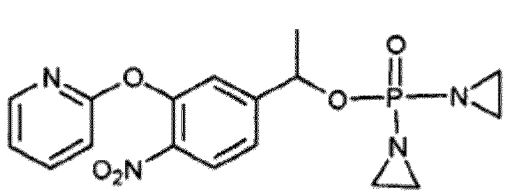,
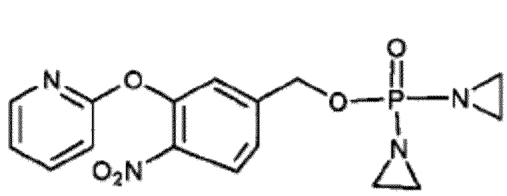,
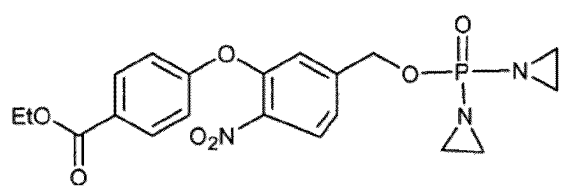,
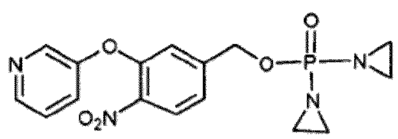,

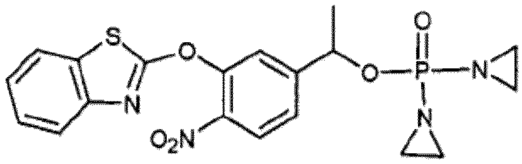 , 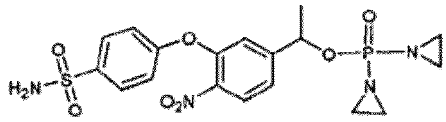 ,

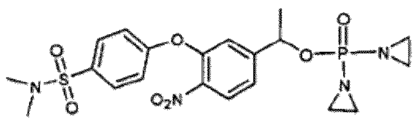 , 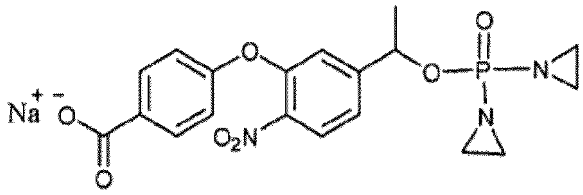 ,

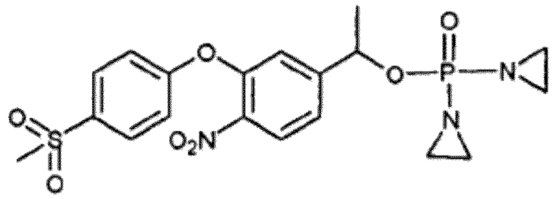 , 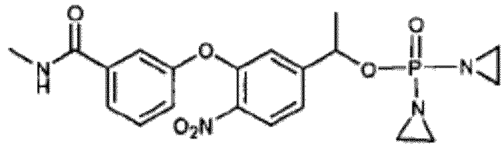 ,

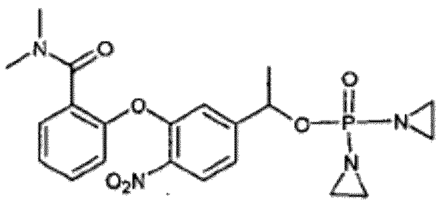 , 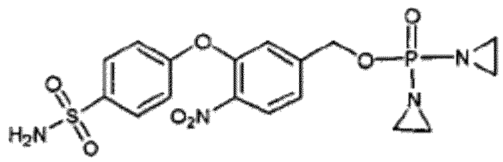 ,

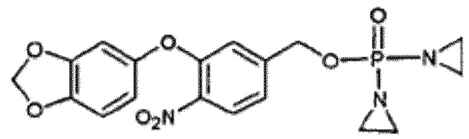 , 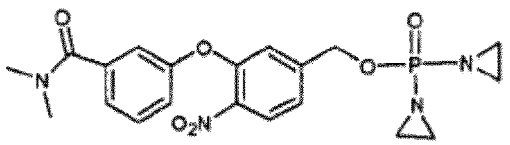 ,

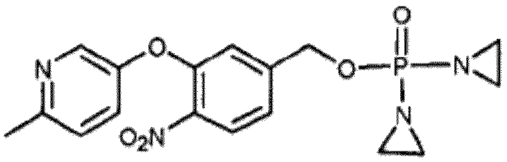 , 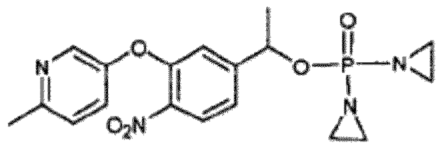 ,

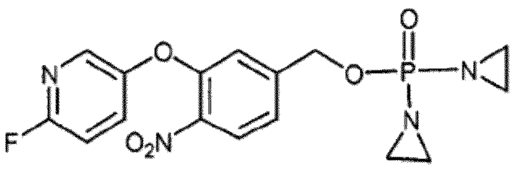 , 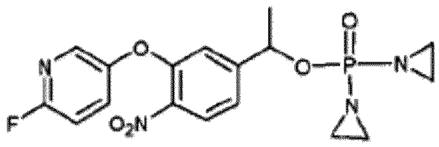 ,

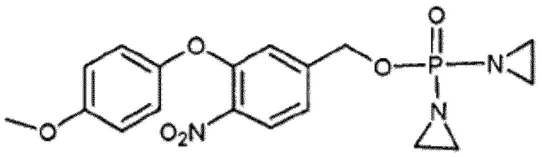 , 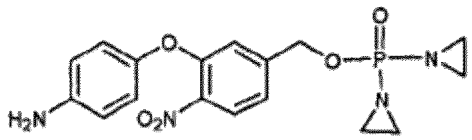 ,

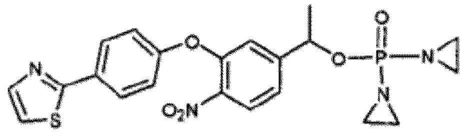,
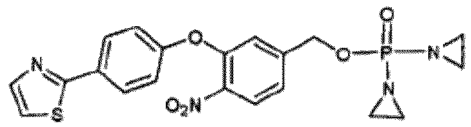,

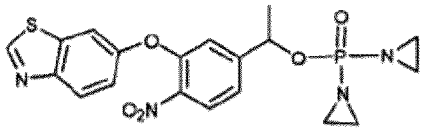,
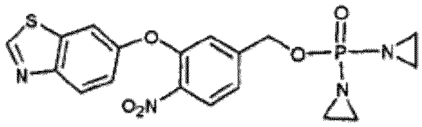,

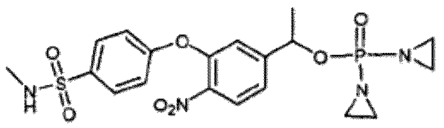,
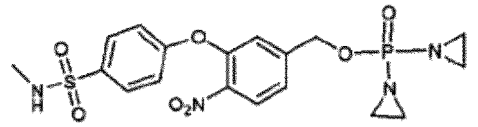,

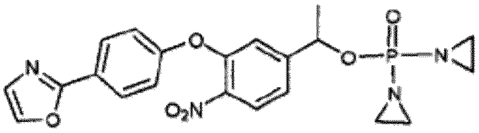,
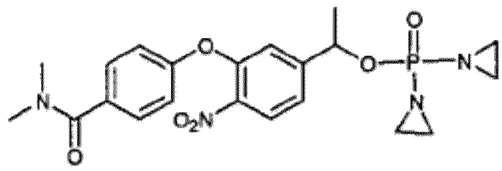,

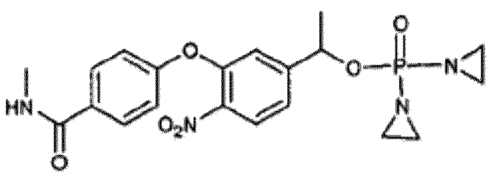,
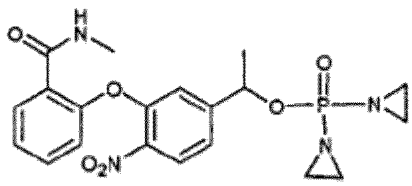,

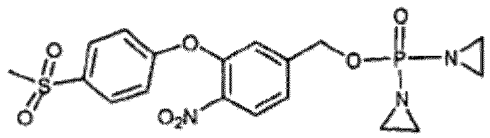,
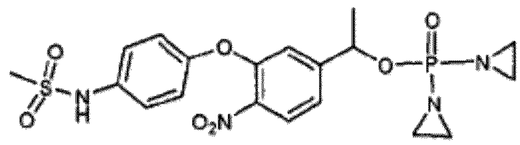,

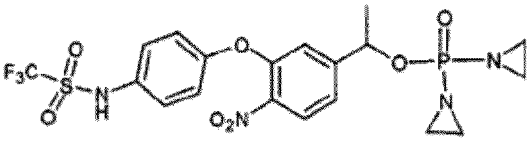,
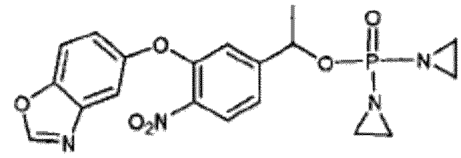,

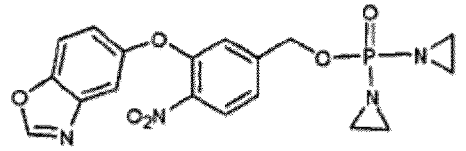,
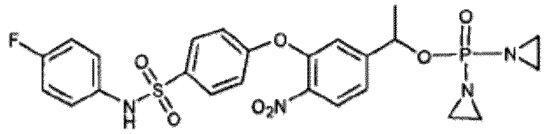,

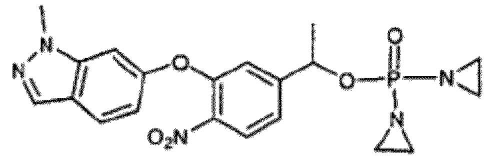 , 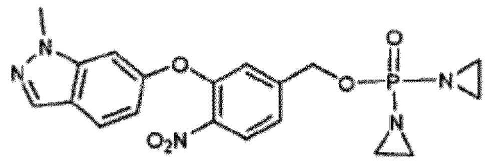 ,

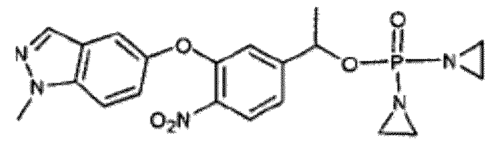 , 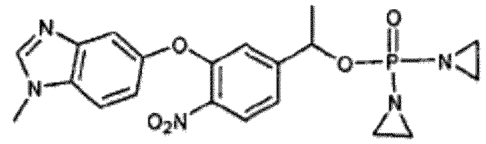 ,

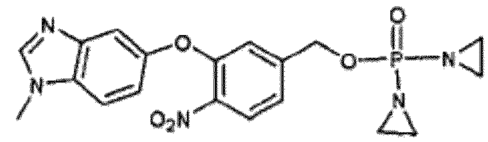 , 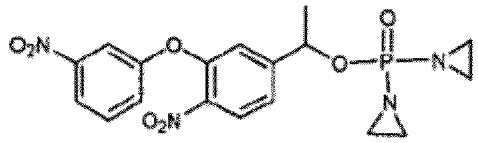 ,

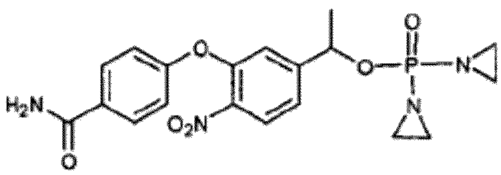 , 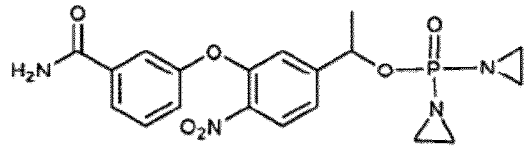 ,

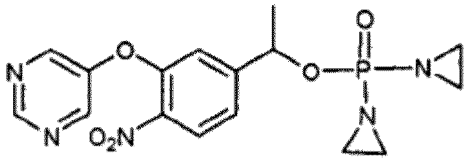 , 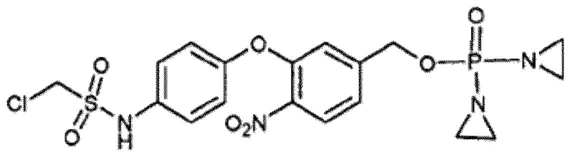 ,

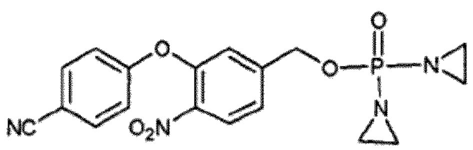 , 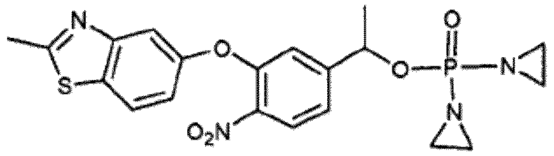 ,

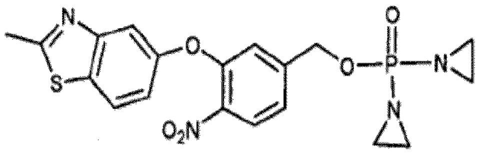 , 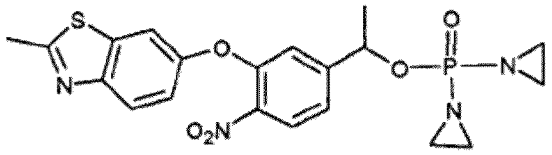 ,

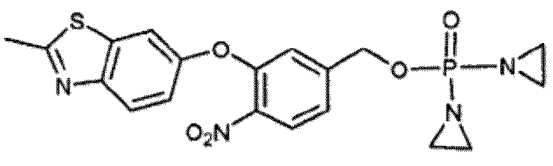 , 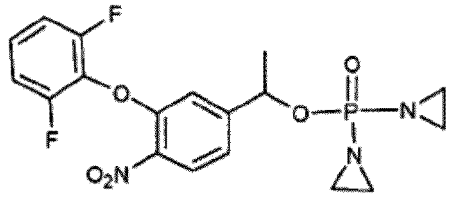 ,

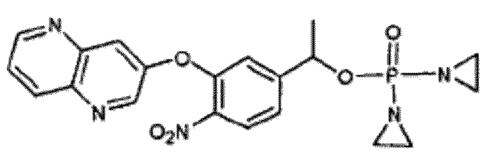 ,
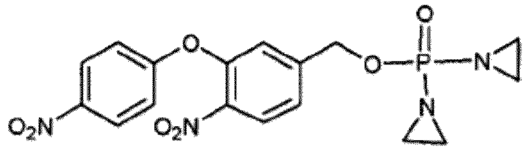 ,

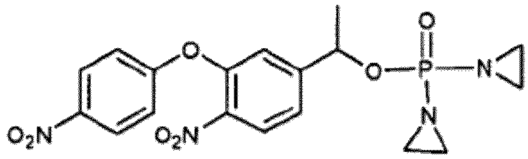 ,
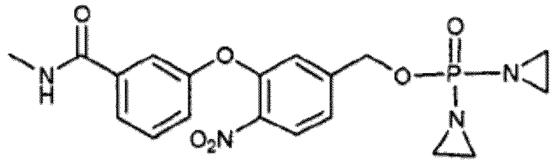 ,

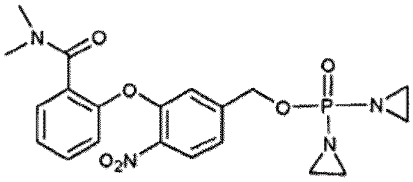 ,
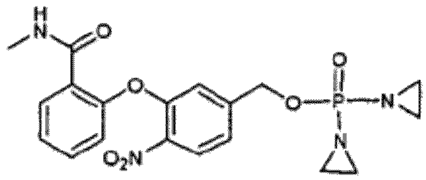 ,

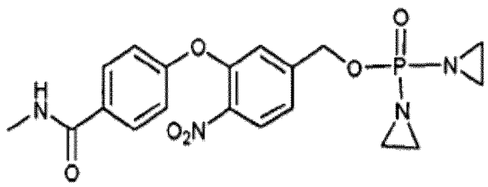 ,
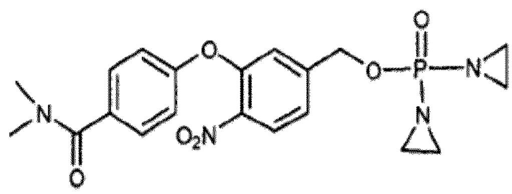 ,

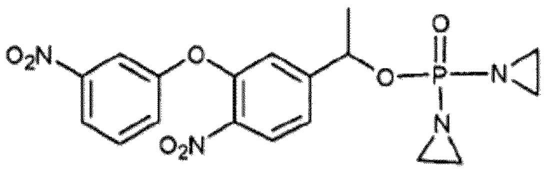 ,
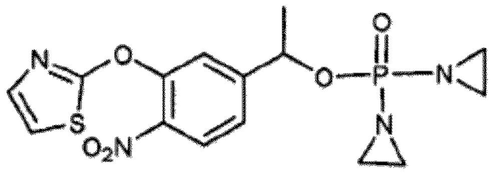 ,

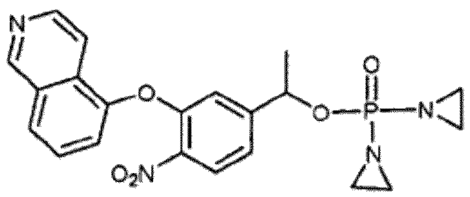 ,
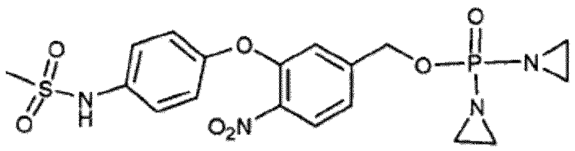 ,

, 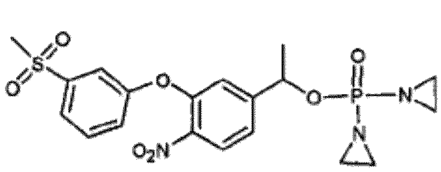 ,
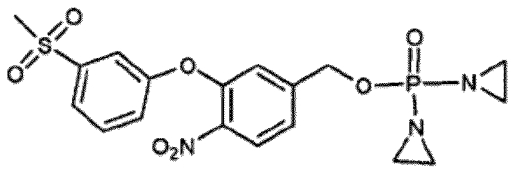 ,

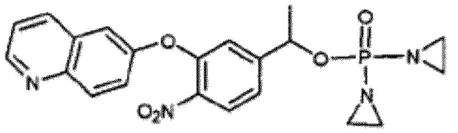 ,
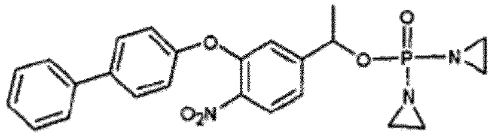 ,

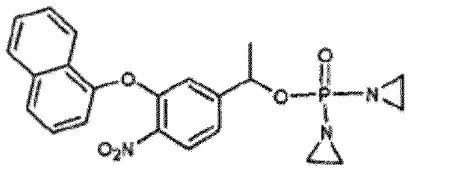
,
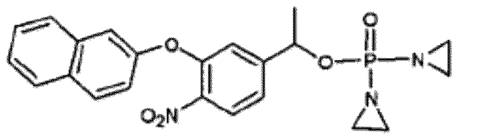
,

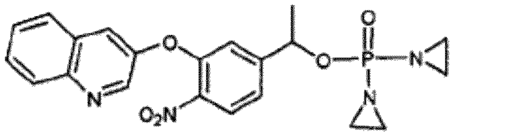
,
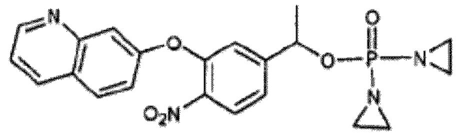
,

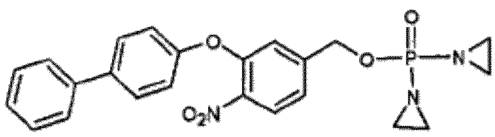
,
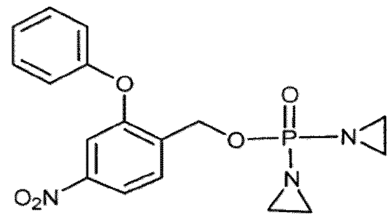
, ,

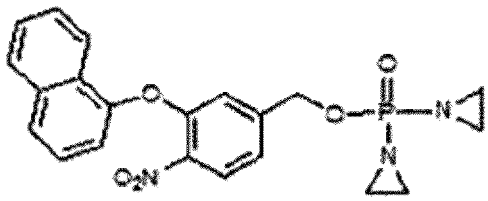
,
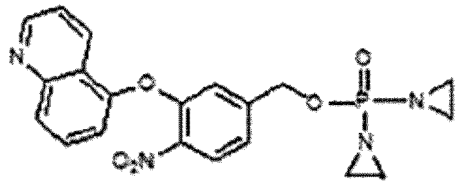
,

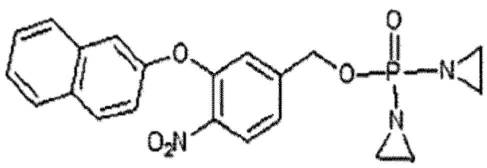
,
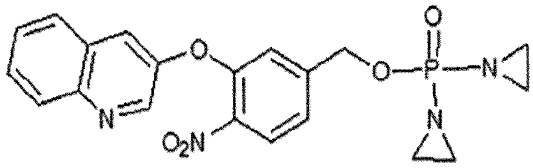
,

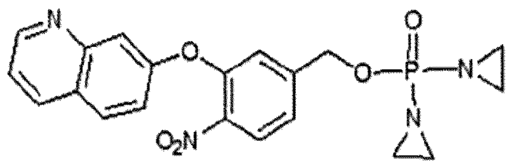
,
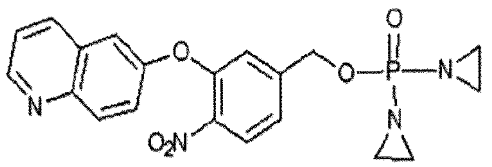
,

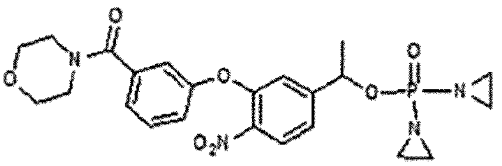
,
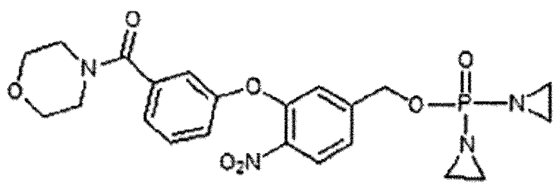
,

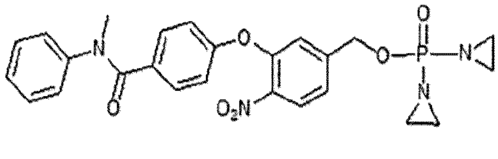
,
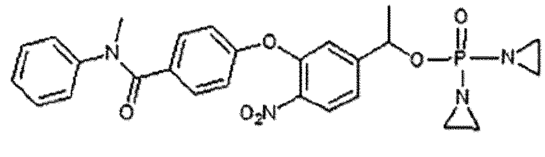
,

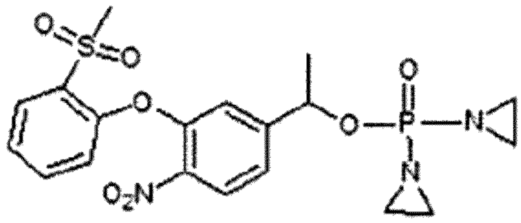 ,

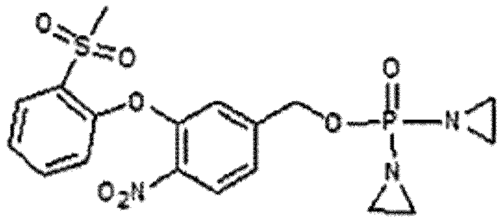 ,

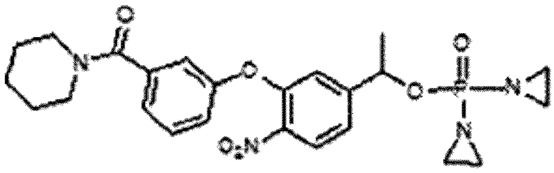 ,

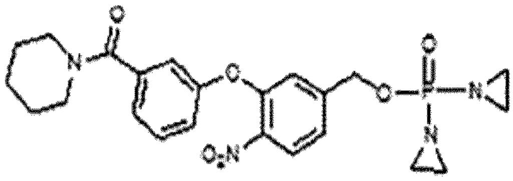 ,

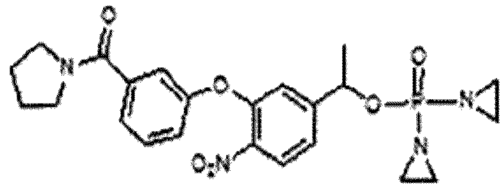 ,

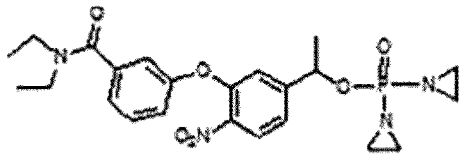 ,

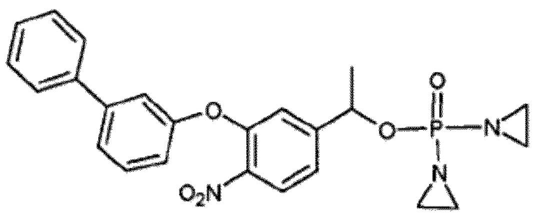 ,

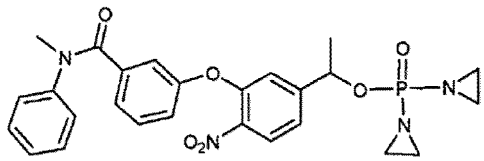 ,

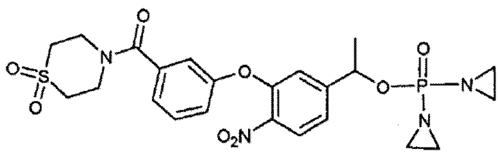 ,

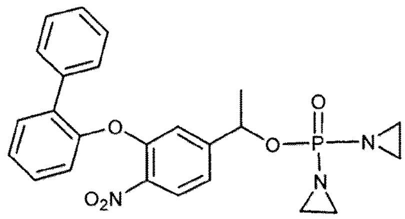 ,

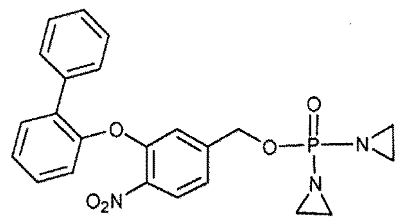 ,

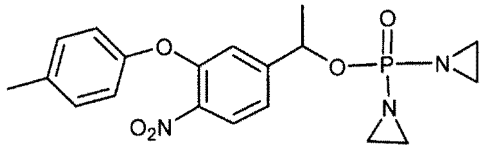 ,

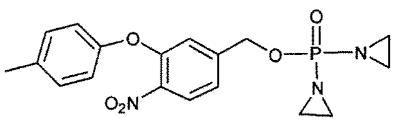 ,

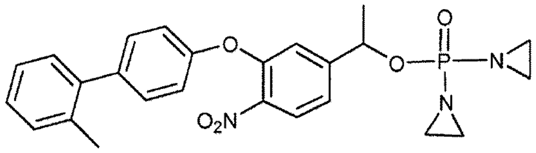 ,

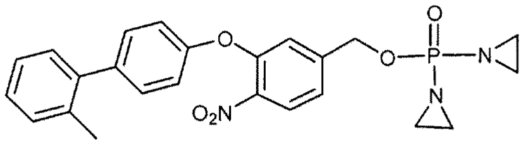 , 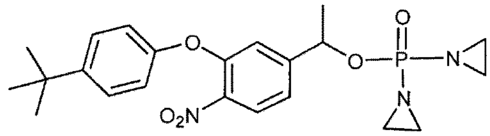 ,

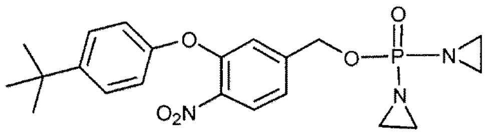 , 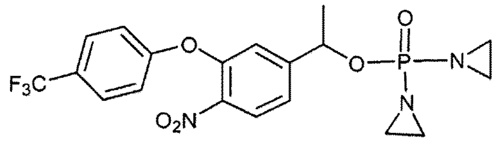 ,

, 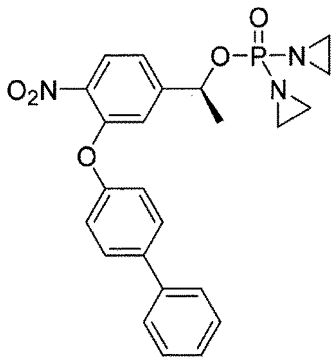 , 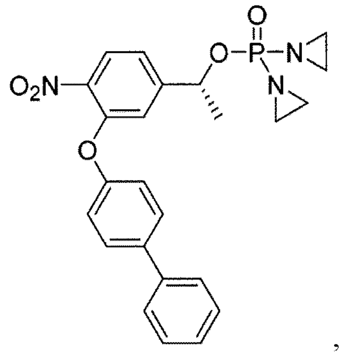 ,

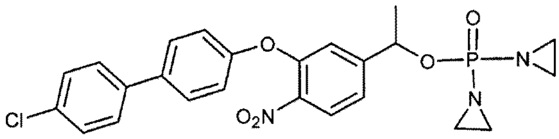 , 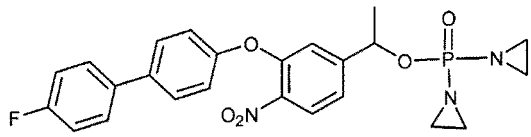 ,

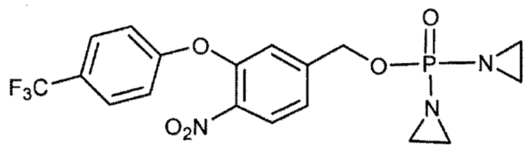 and 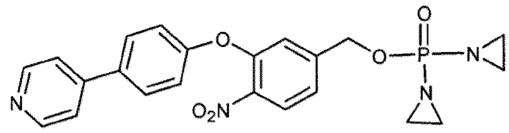 ;

the AKR1C3 enzyme-activated prodrug compound with formula (5) is selected from the compounds with the following structural formulae:

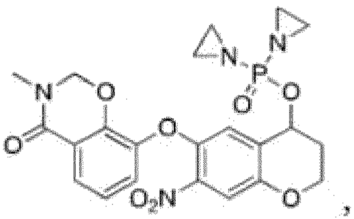 , 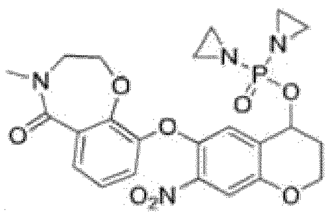 , 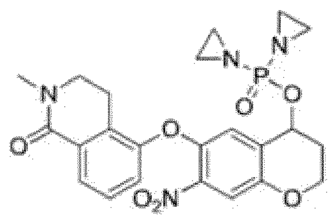 ,

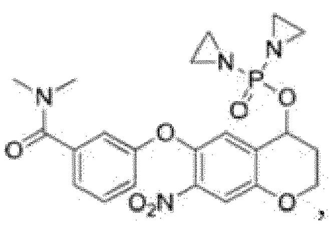 , 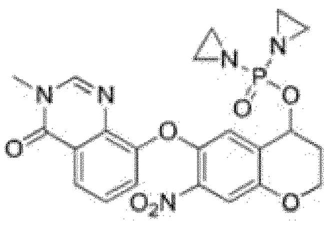 , 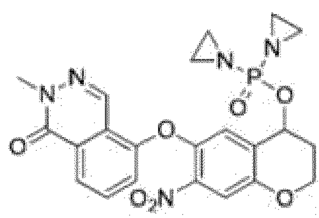 ,

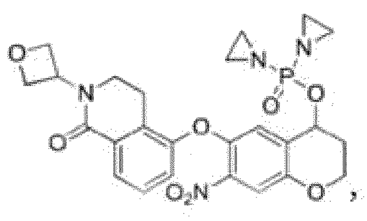, 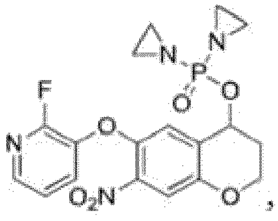, 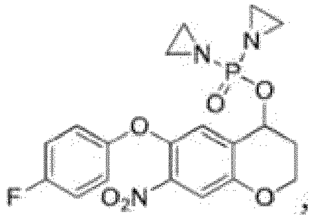,

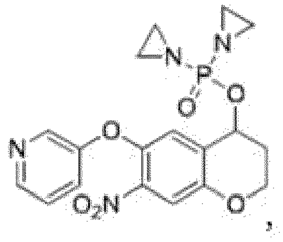, 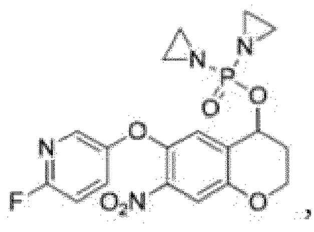, 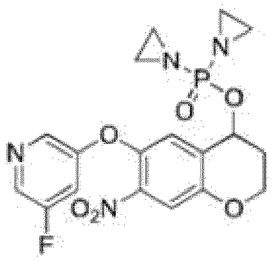,

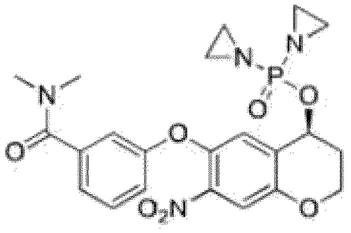, 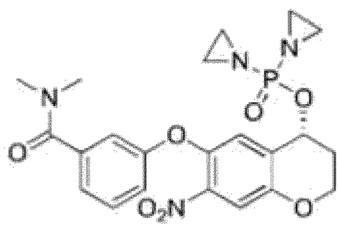, 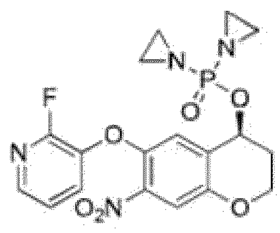 or

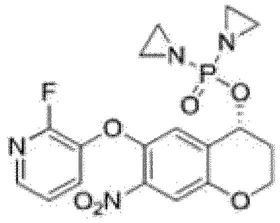

;

the AKR1C3 enzyme-activated prodrug compound with formula (6) is selected from the compounds with the following structural formulae:

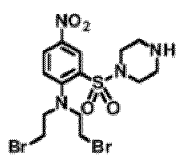
562

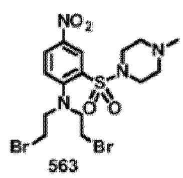
563

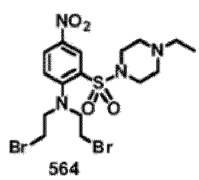
564

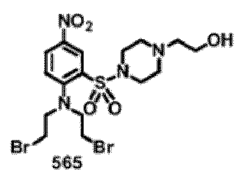
565

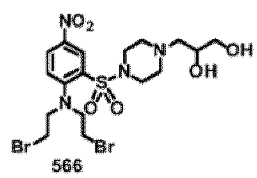
566

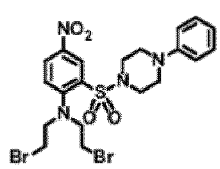
567

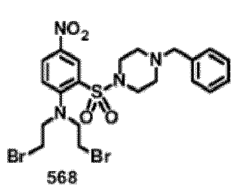
568

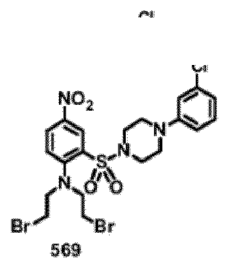
569

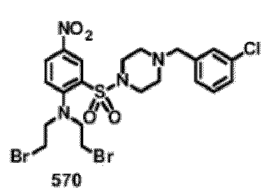
570

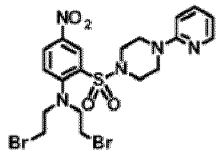
571
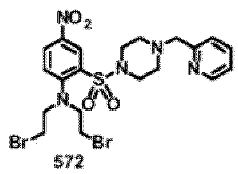
572
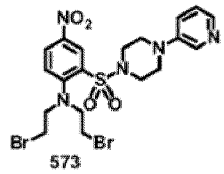
573
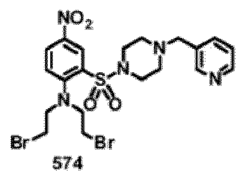
574

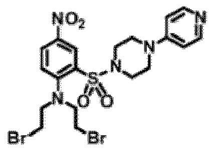
575
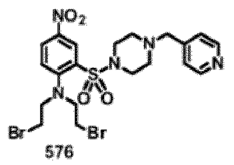
576
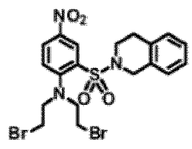
577
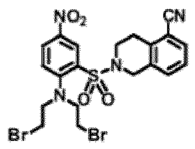
578
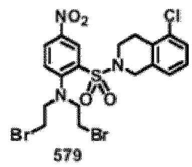
579

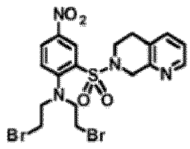
580
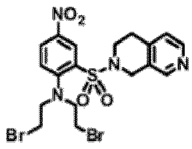
581
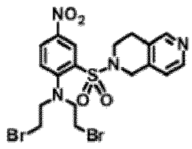
582
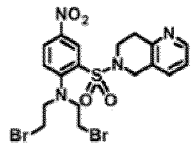
583
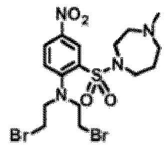
584
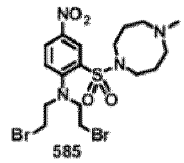
585

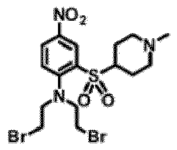
586
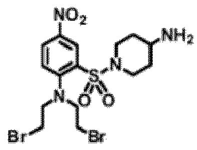
587
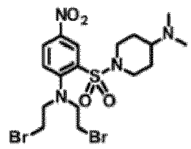
588
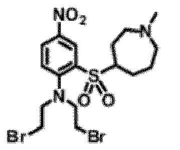
589
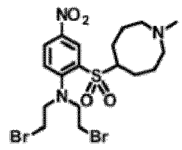
590
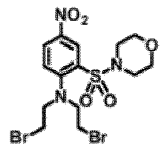
591

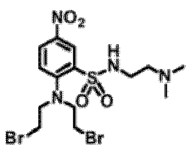
592
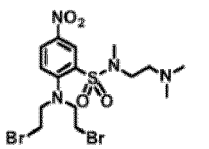
593
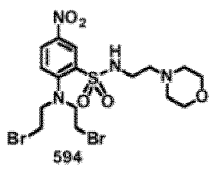
594
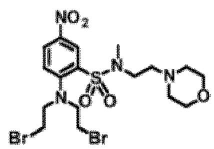
595
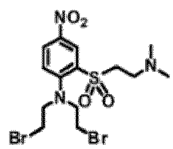
596

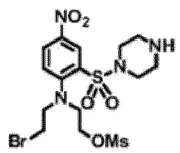
601
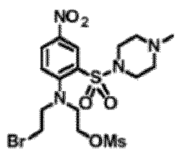
602
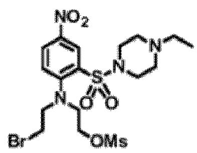
603
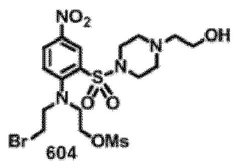
604
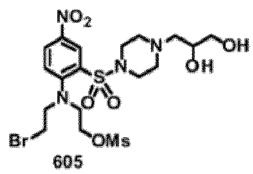
605

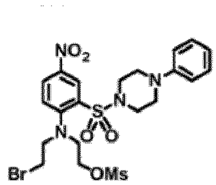
606
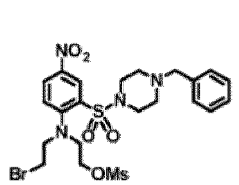
607
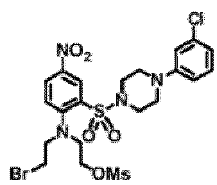
608
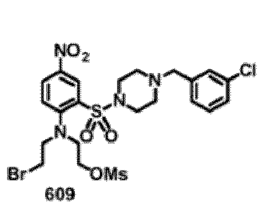
609

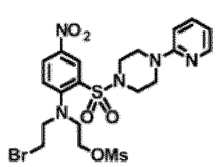
610
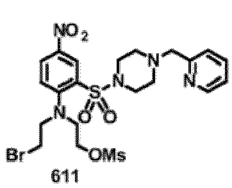
611
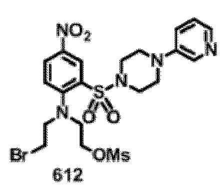
612
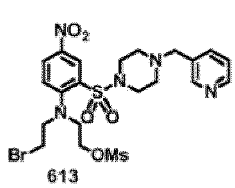
613

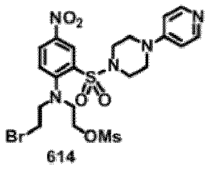
614

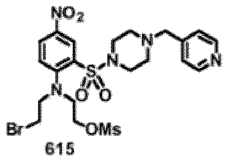
615

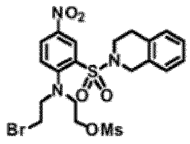
616

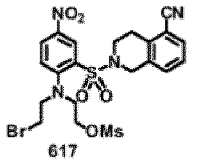
617

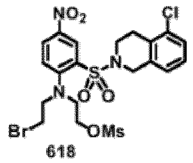
618

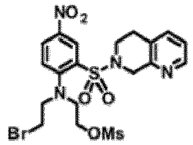
619

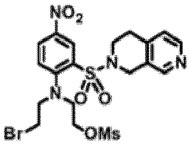
620

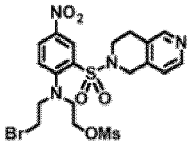
621

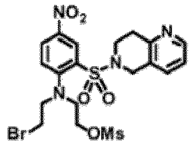
622

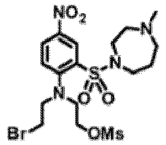
623

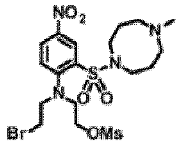
624

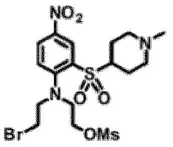
625

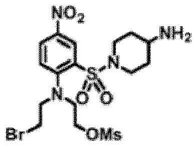
626

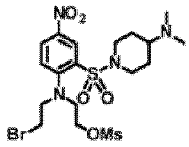
627

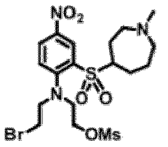
628

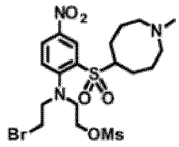
629

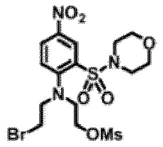
630

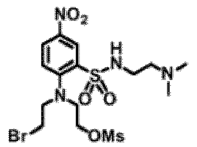
631

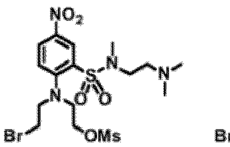
632

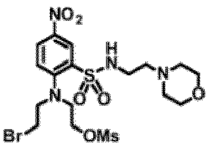
633

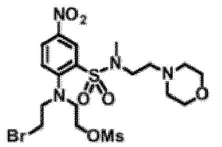
634

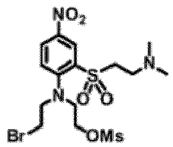
635

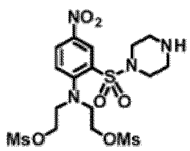
640

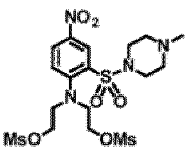
641

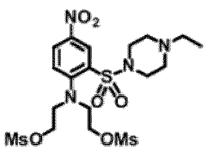
642

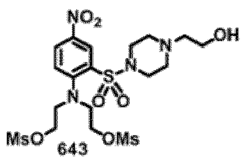
643

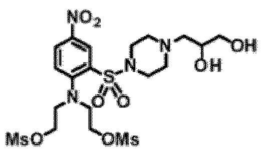
644

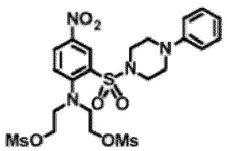
645

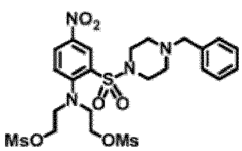
646

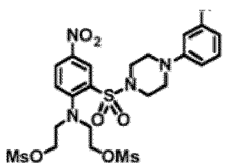
647

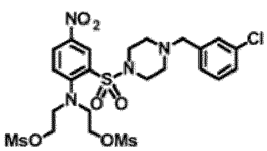
648

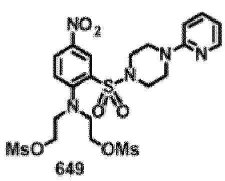
649

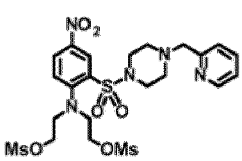
650

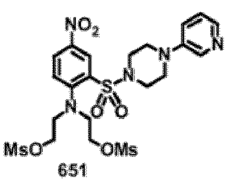
651

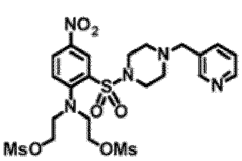
652

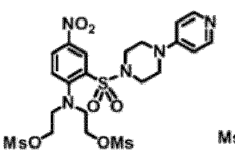
653

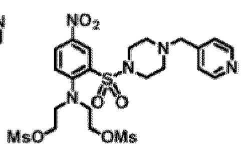
654

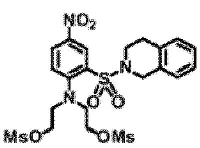
655

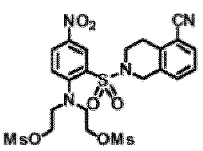
656

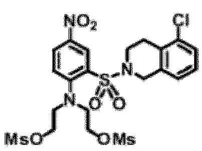
657

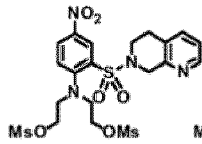
658
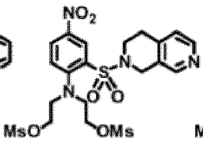
659
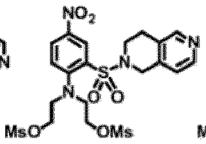
660
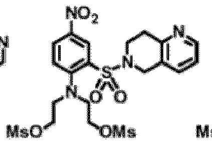
661
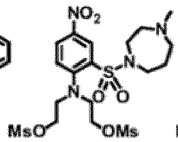
662
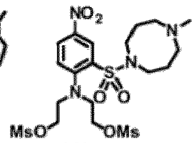
663

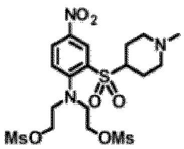
664
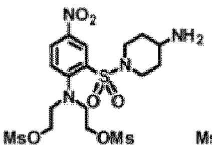
665
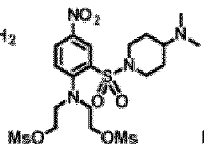
666
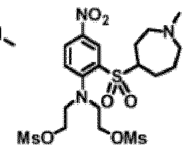
667
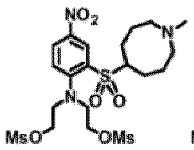
668
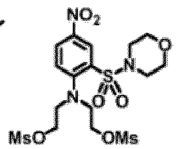
669

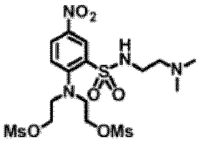
670
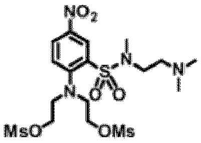
671
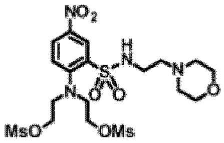
672
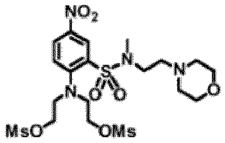
673
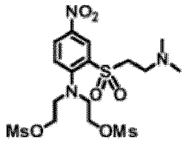
674

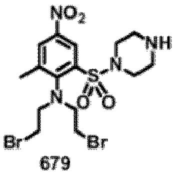
679
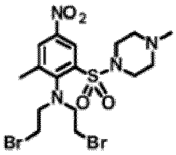
680
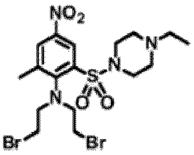
681
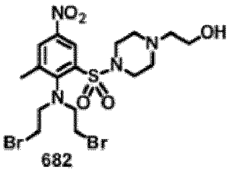
682
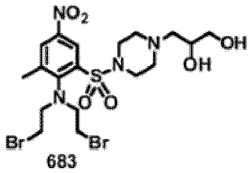
683

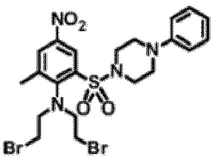
684
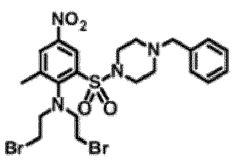
685
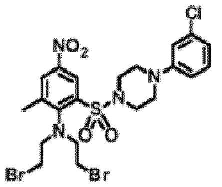
686
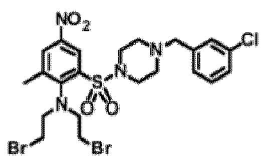
687

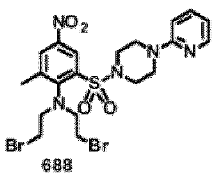
688
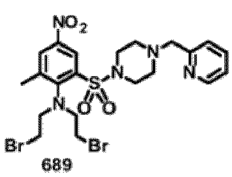
689
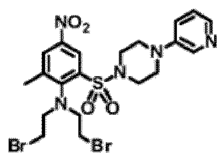
690
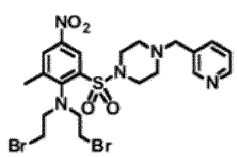
691

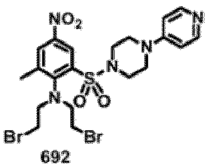
692
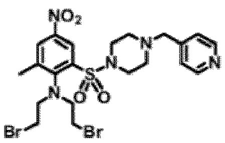
693
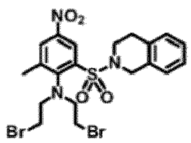
694
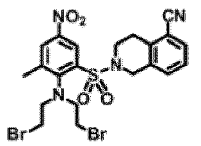
695
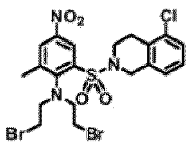
696

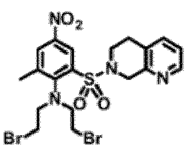
697
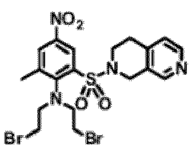
698
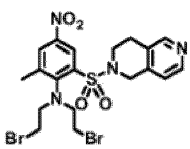
699
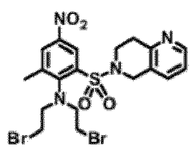
700
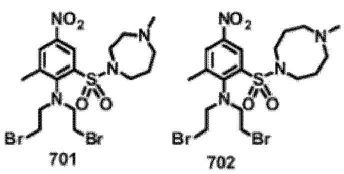
701
702

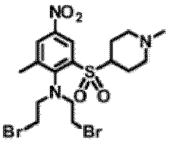
703
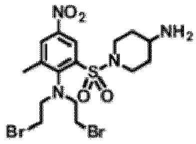
704
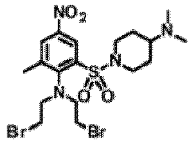
705
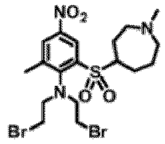
706
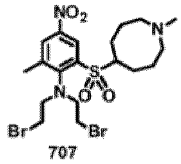
707
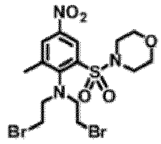
708

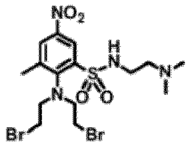
709
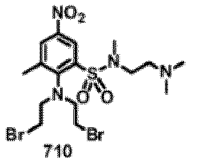
710
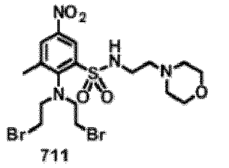
711
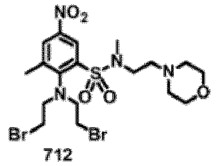
712
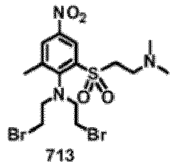
713

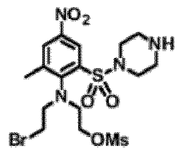
718
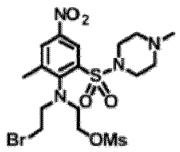
719
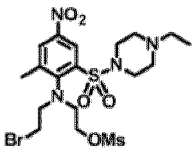
720
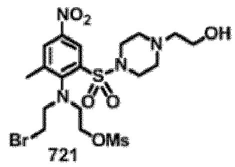
721
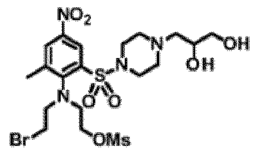
722

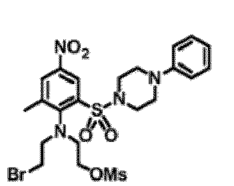
723
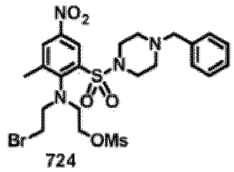
724
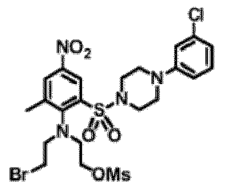
725
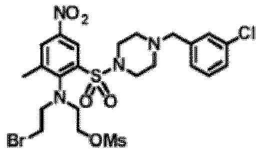
726

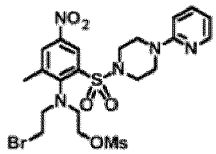
727
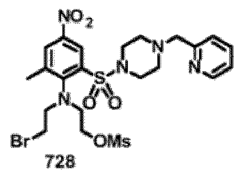
728
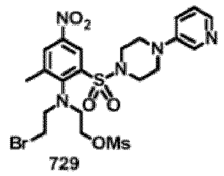
729
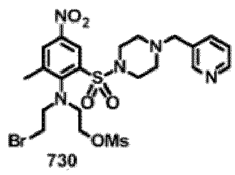
730

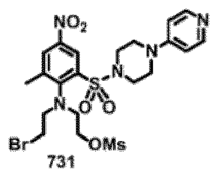
731
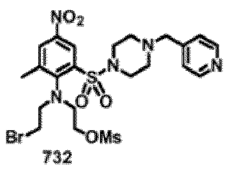
732
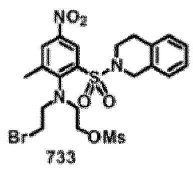
733
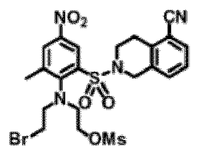
734
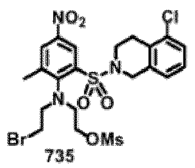
735

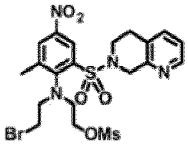
736
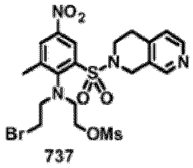
737
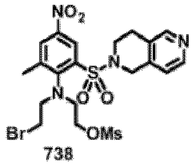
738
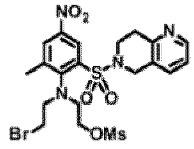
739
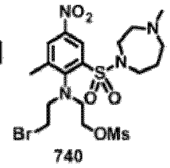
740
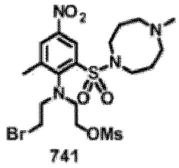
741

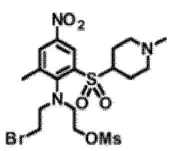
742
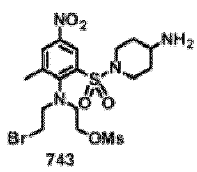
743
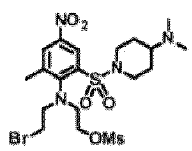
744
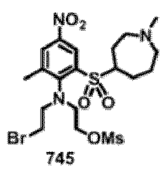
745
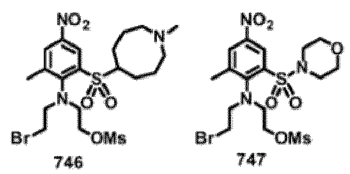
746
747

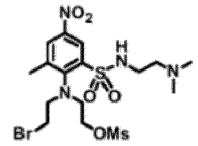
748

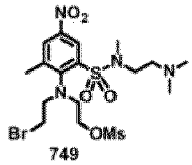
749

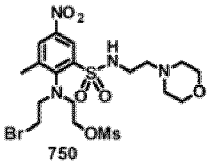
750

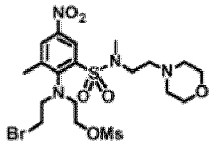
751

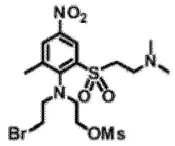
752

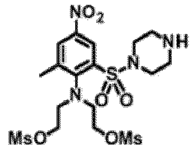
757

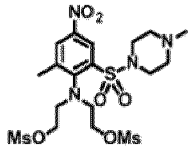
758

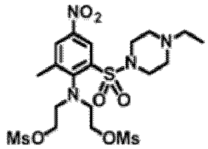
759

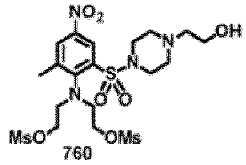
760

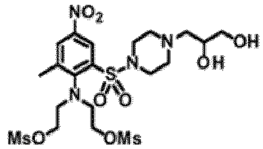
761

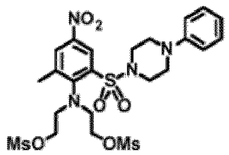
762

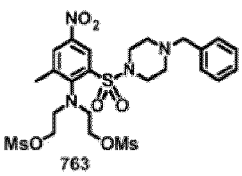
763

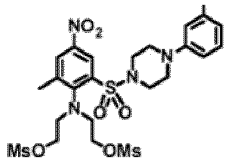
764

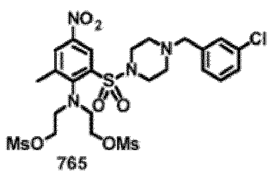
765

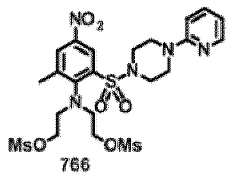
766

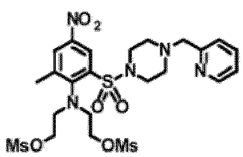
767

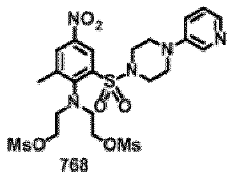
768

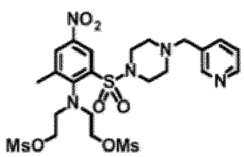
769

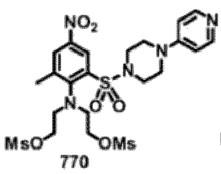
770

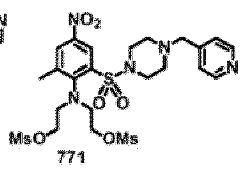
771

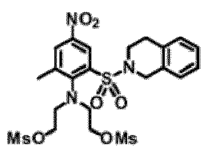
772

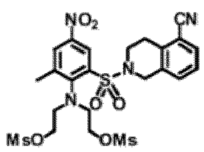
773

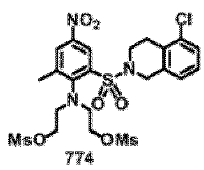
774

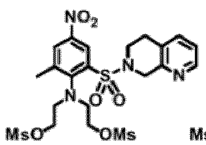
775

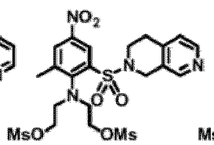
776

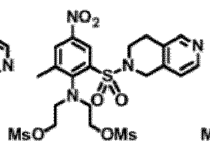
777

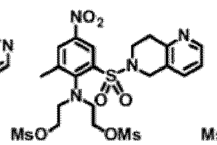
778

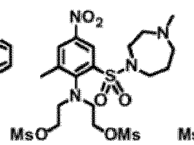
779

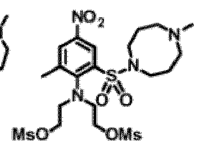
780

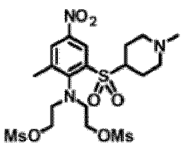
781

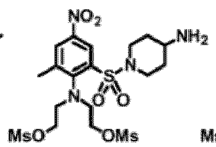
782

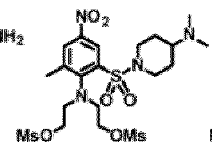
783

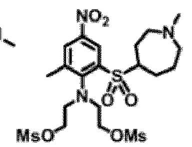
784

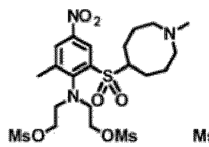
785

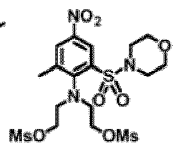
786

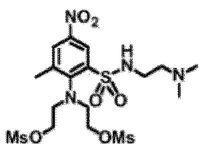
787

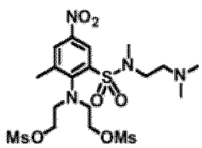
788

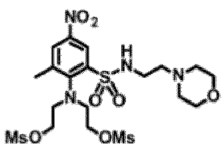
789

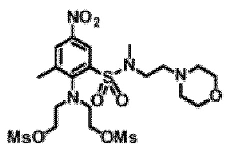
790

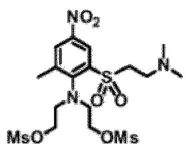
791

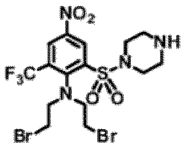
913
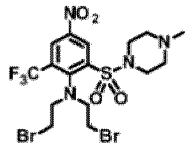
914
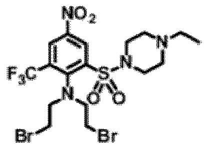
915
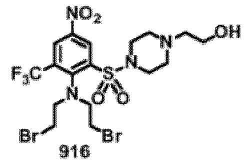
916
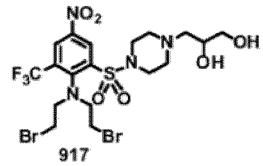
917

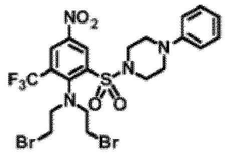
918
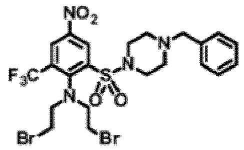
919
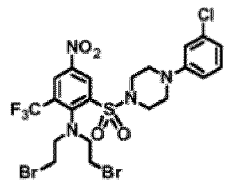
920
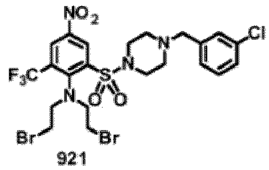
921

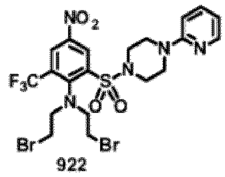
922
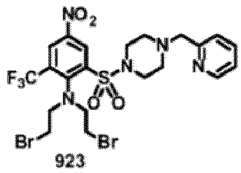
923
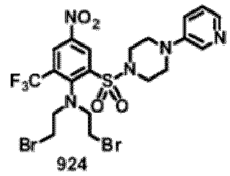
924
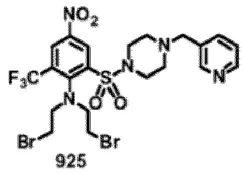
925

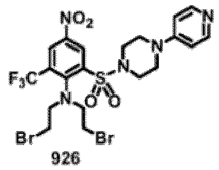
926
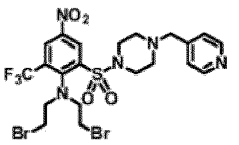
927
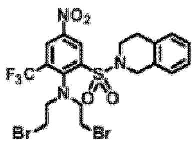
928
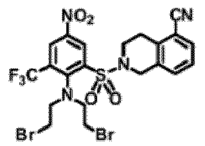
929
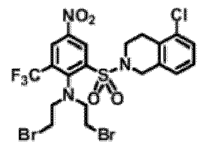
930

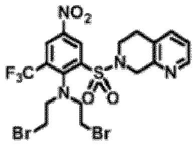
931
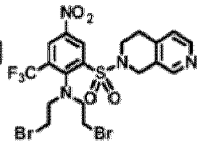
932
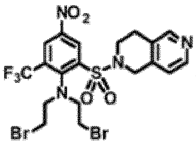
933
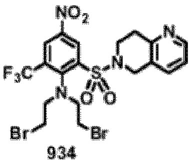
934
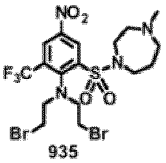
935
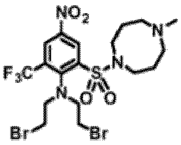
936

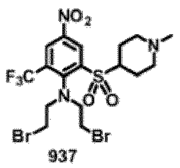
937
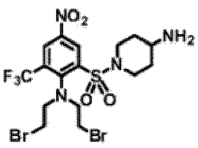
938
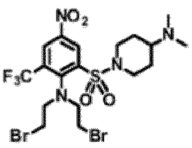
939
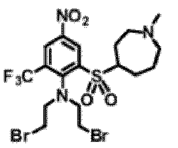
940
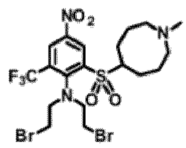
941
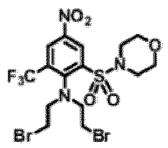
942

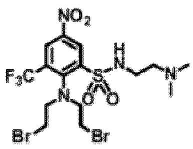
943
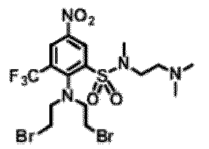
944
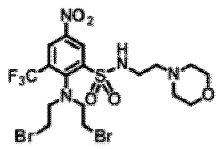
945
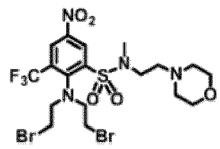
946
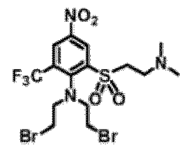
947

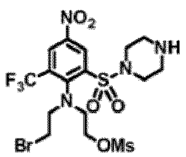
952
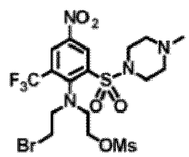
953
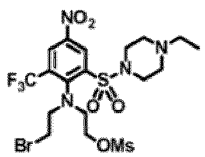
954
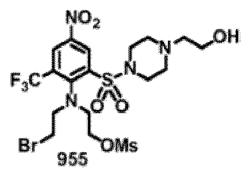
955
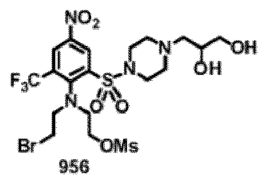
956

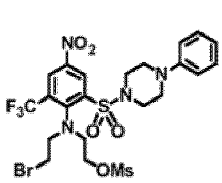
957
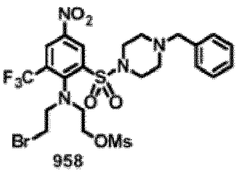
958
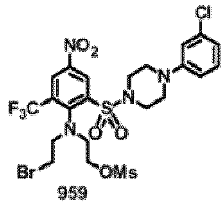
959
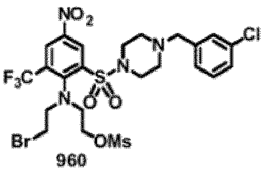
960

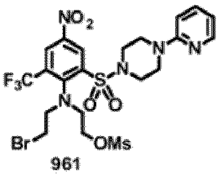
961
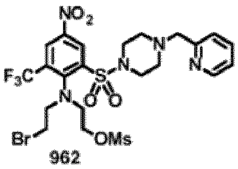
962
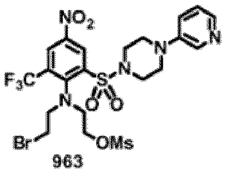
963
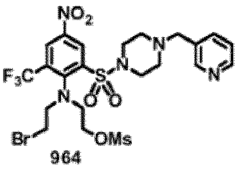
964

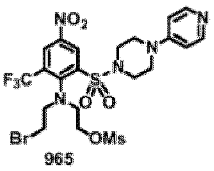
965
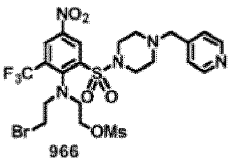
966
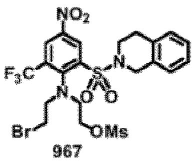
967
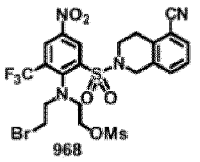
968
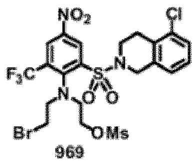
969

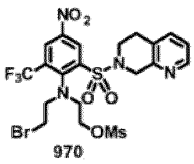
970
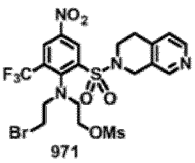
971
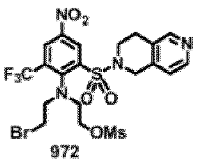
972
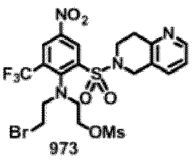
973
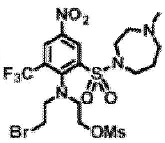
974
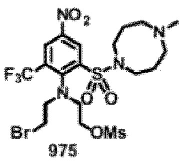
975

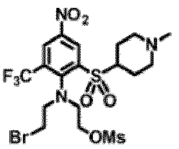
976
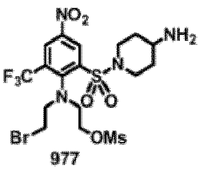
977
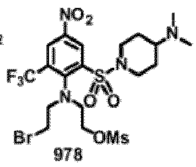
978
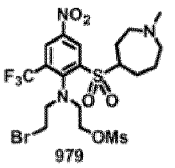
979
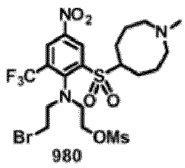
980
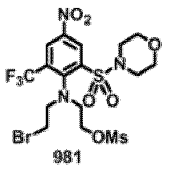
981

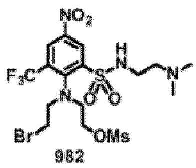
982
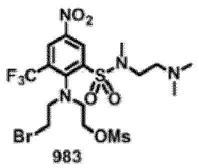
983
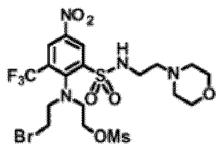
984
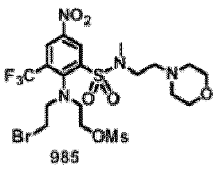
985
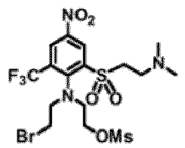
986

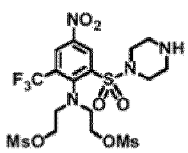
991
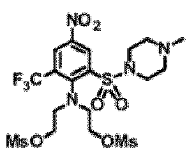
992
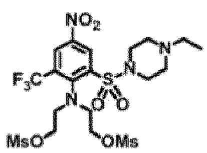
993
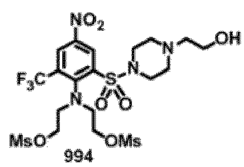
994
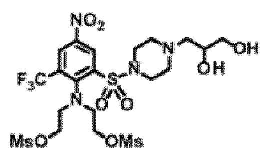
995

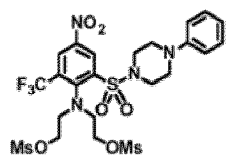
996
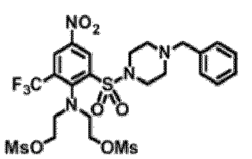
997
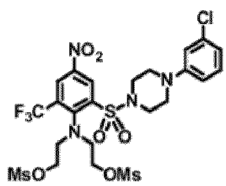
998
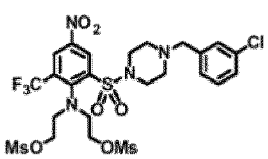
999

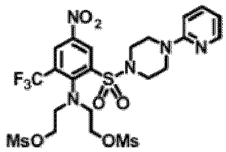
1000

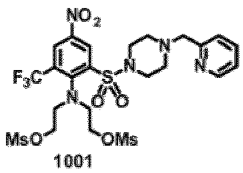
1001

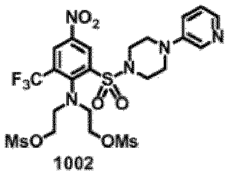
1002

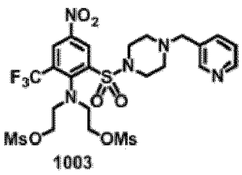
1003

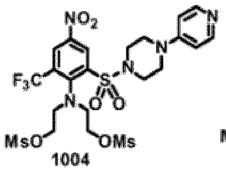
1004

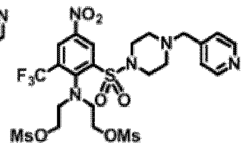
1005

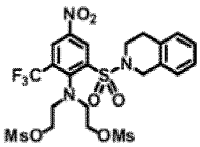
1006

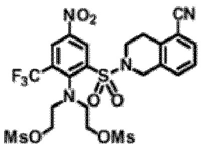
1007

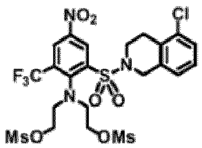
1008

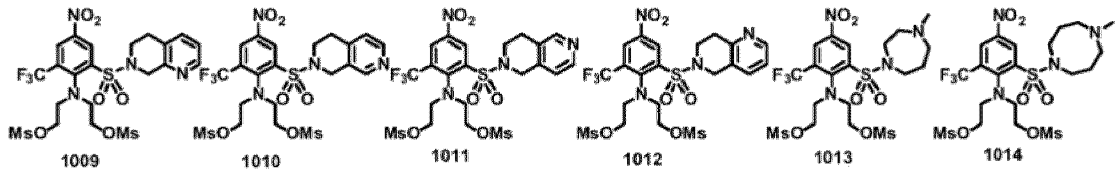
1009 1010 1011 1012 1013 1014

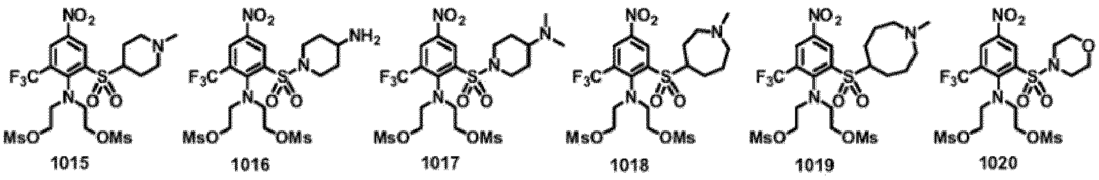
1015 1016 1017 1018 1019 1020

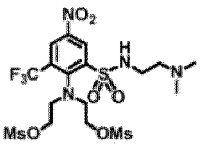
1021

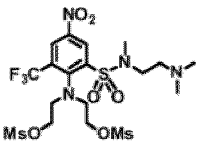
1022

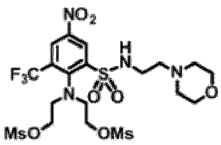
1023

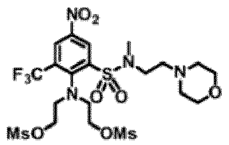
1024

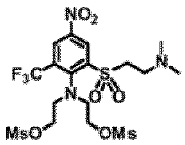
1025

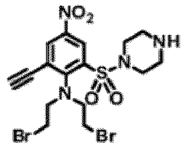
1030

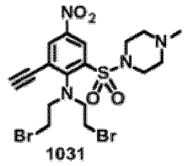
1031

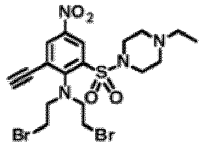
1032

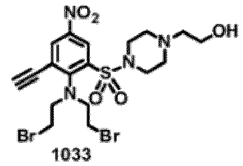
1033

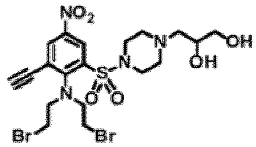
1034

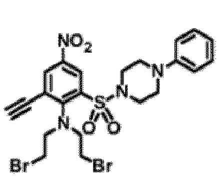
1035

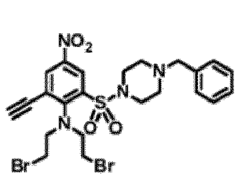
1036

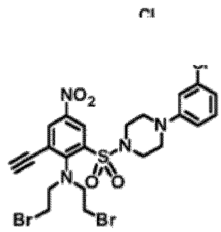
1037

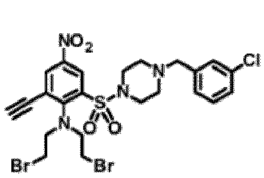
1038

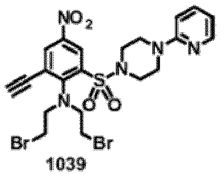
1039

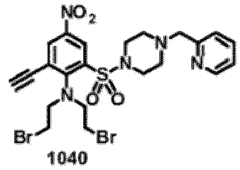
1040

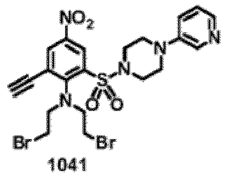
1041

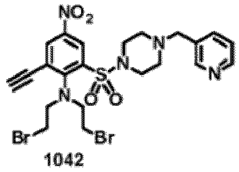
1042

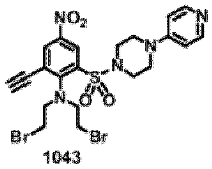
1043

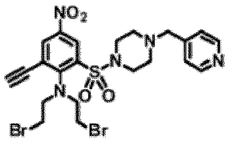
1044

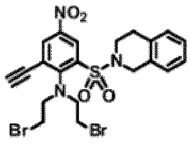
1045

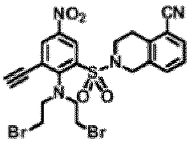
1046

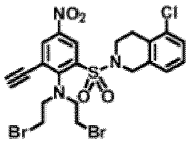
1047

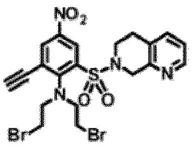
1048

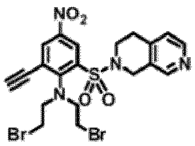
1049

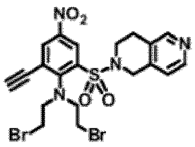
1050

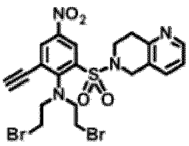
1051

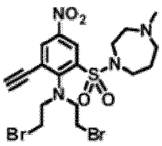
1052

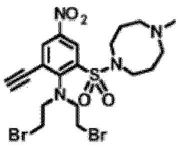
1053

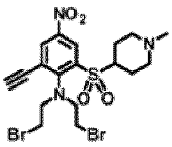
1054

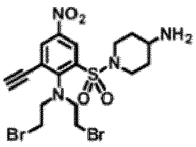
1055

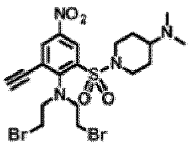
1056

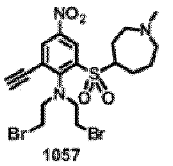
1057

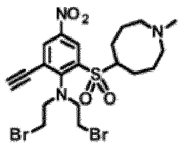
1058

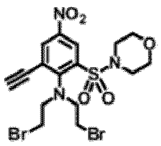
1059

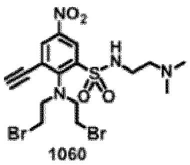
1060

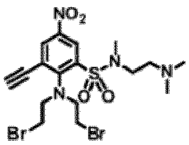
1061

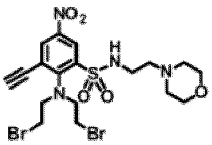
1062

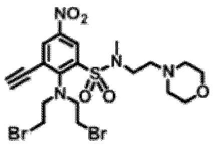
1063

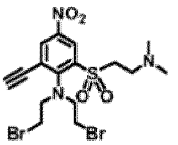
1064

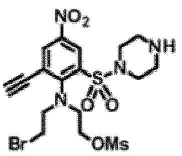
1069

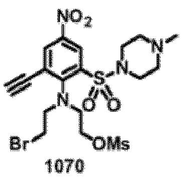
1070

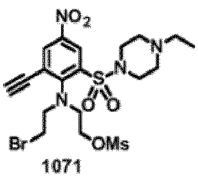
1071

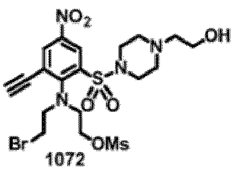
1072

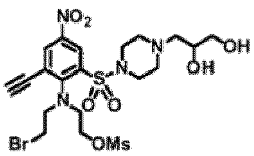
1073

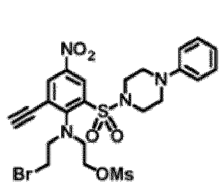
1074

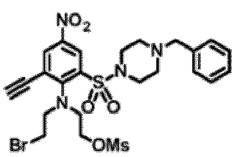
1075

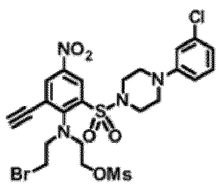
1076

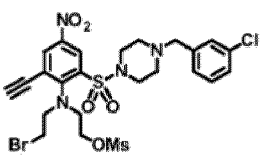
1077

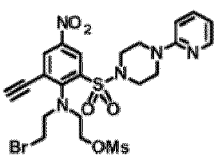
1078

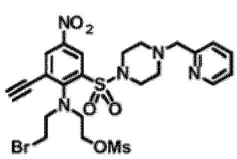
1079

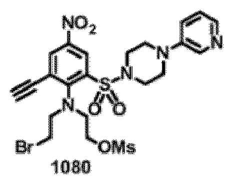
1080

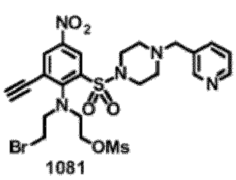
1081

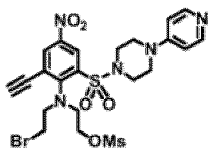
1082

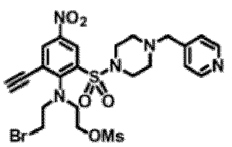
1083

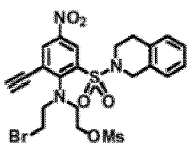
1084

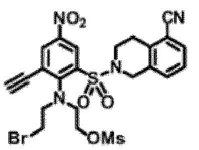
1085

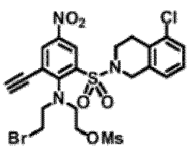
1086

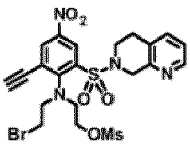
1087
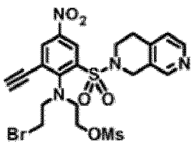
1088
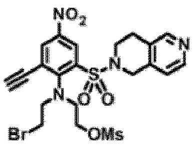
1089
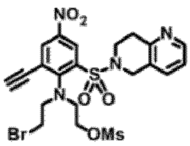
1090
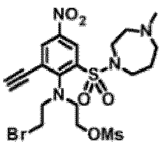
1091
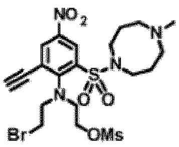
1092

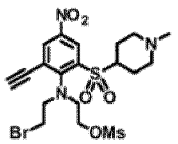
1093
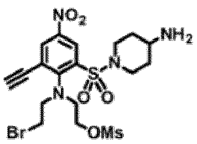
1094
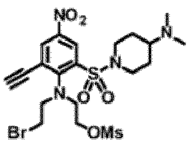
1095
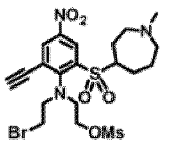
1096
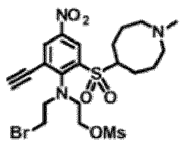
1097
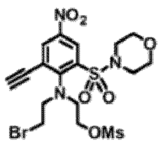
1098

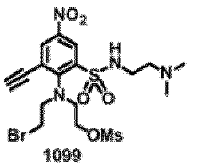
1099
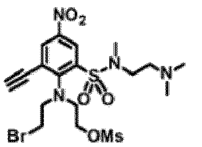
1100
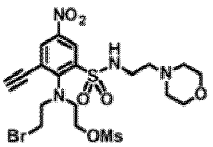
1101
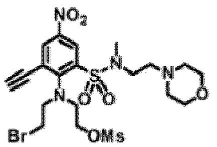
1102
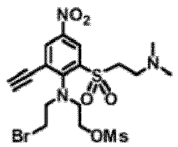
1103

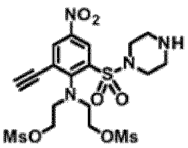
1108
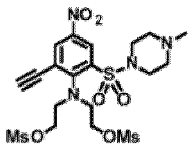
1109
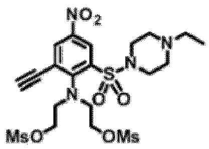
1110
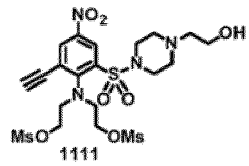
1111
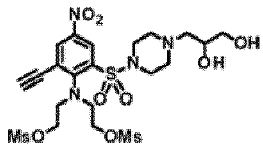
1112

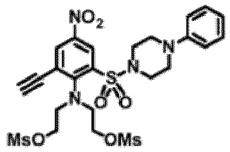
1113
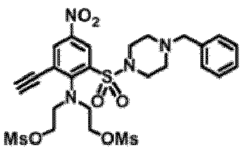
1114
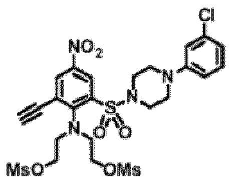
1115
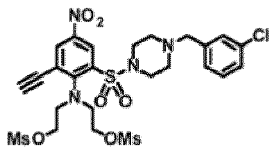
1116

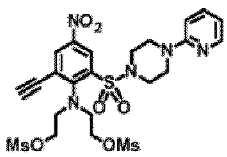
1117
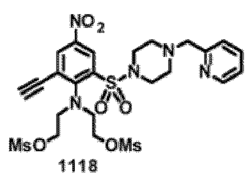
1118
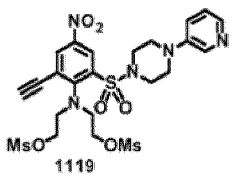
1119
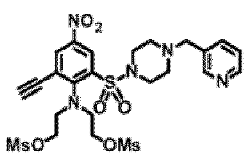
1120

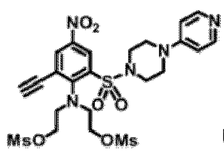
1121
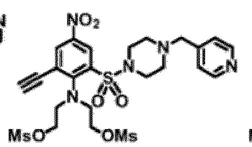
1122
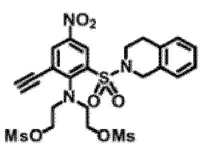
1123
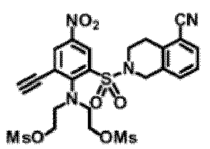
1124
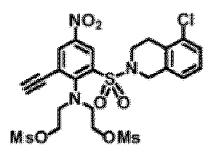
1125

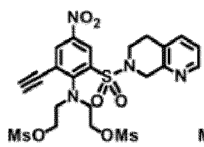
1126
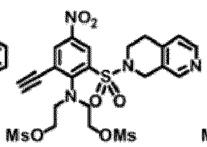
1127
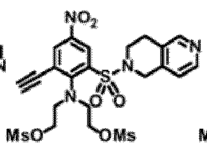
1128
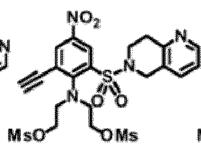
1129
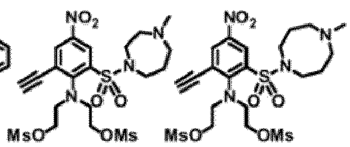
1130
1131

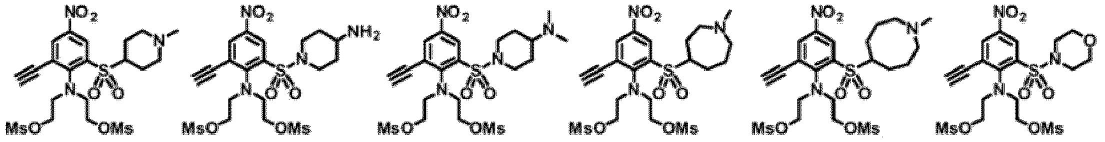

1132 1133 1134 1135 1136 1137

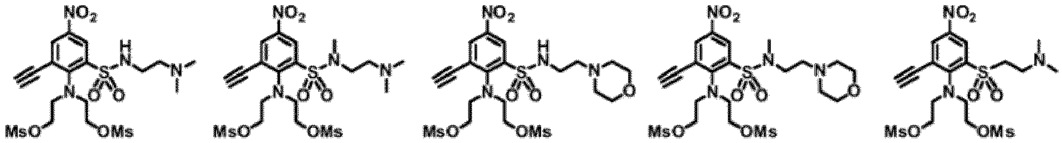

1138 1139 1140 1141 1142

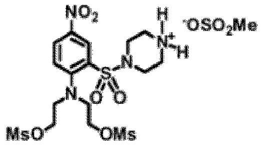

640.Ms

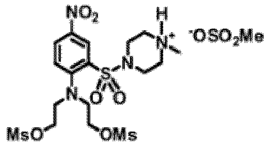

641.Ms

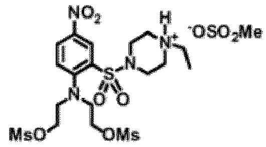

642.Ms

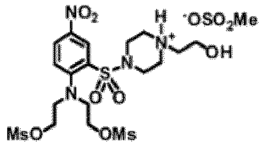

643.Ms

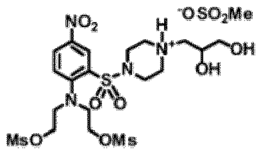

644.Ms

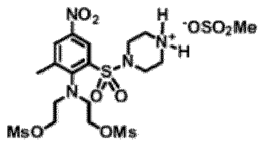

757.Ms

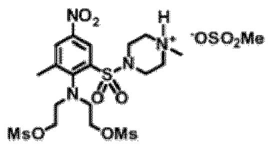

758.Ms

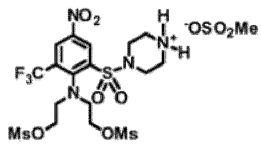

991.Ms

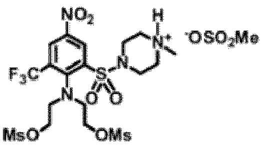

992.Ms

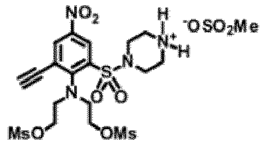

1108.Ms

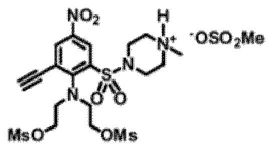

1109.Ms

;

the AKR1C3 enzyme-activated prodrug compound with formula (7) is selected from the compounds with the following structural formulae:

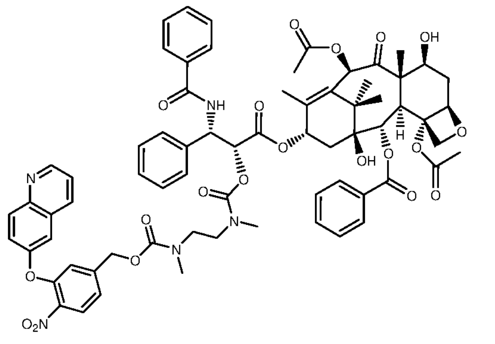

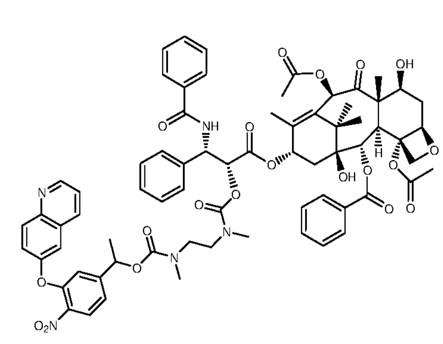

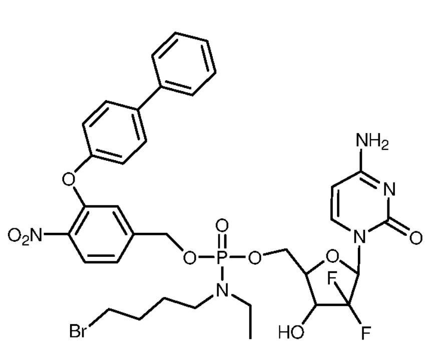

,

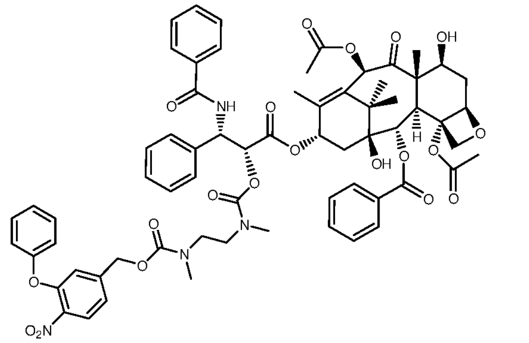

,

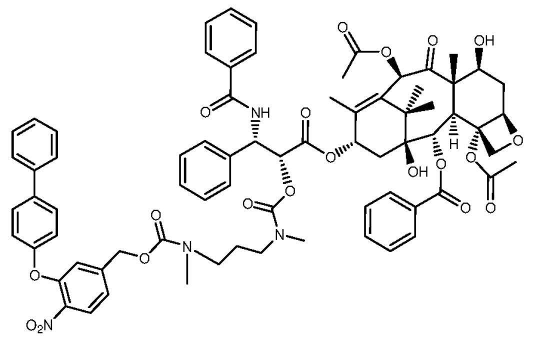

,

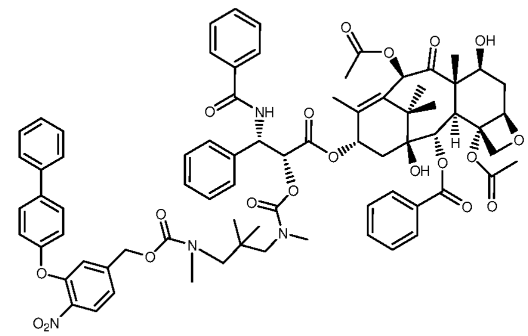

,

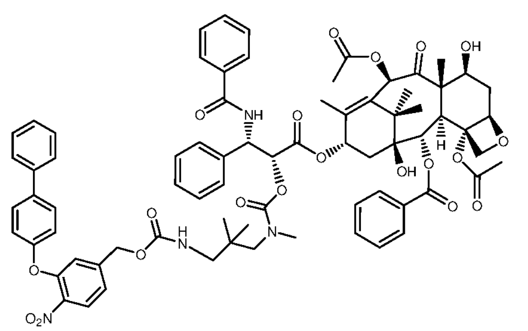

,

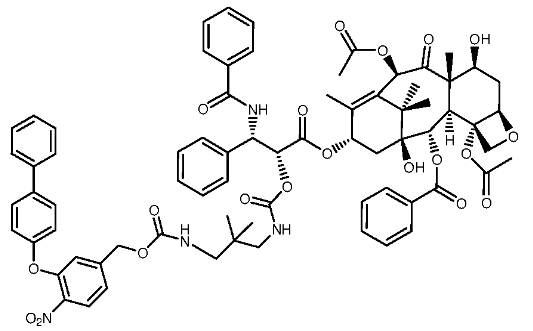

,

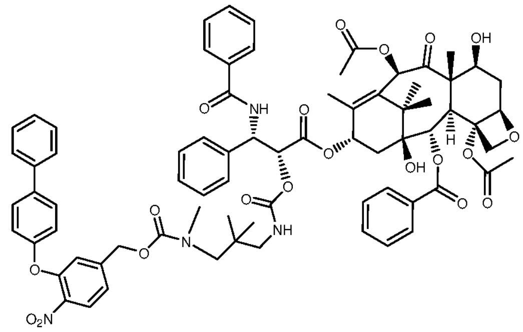

,

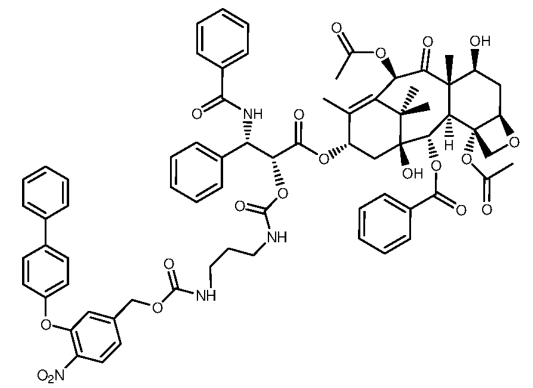

,

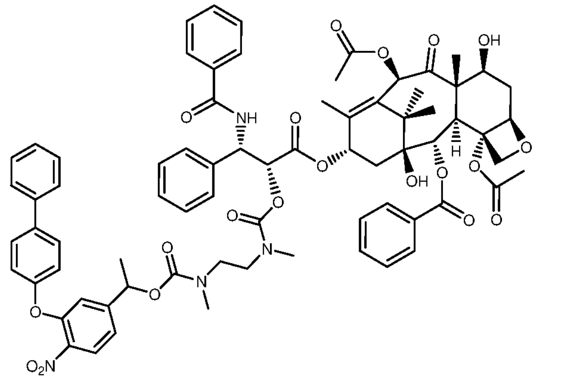
,
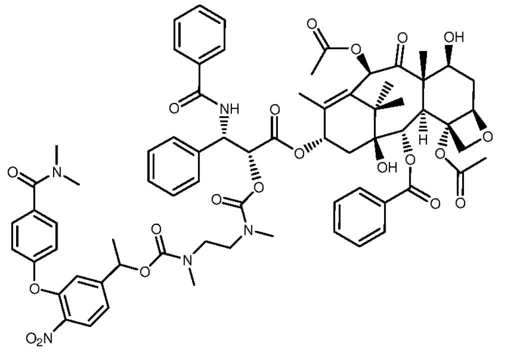
,

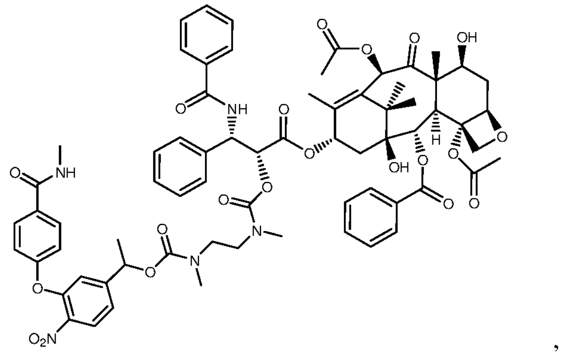
,
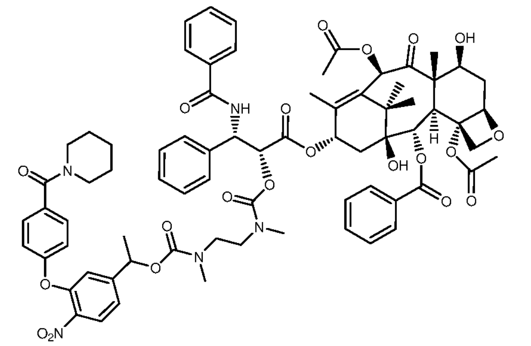
,

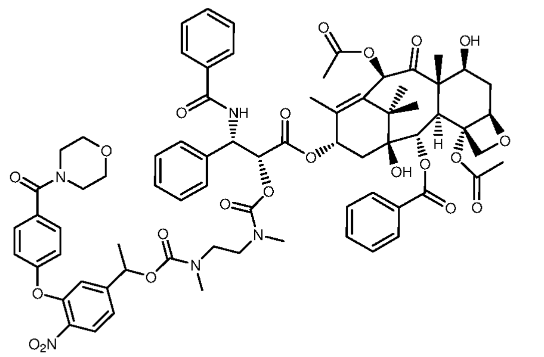
,
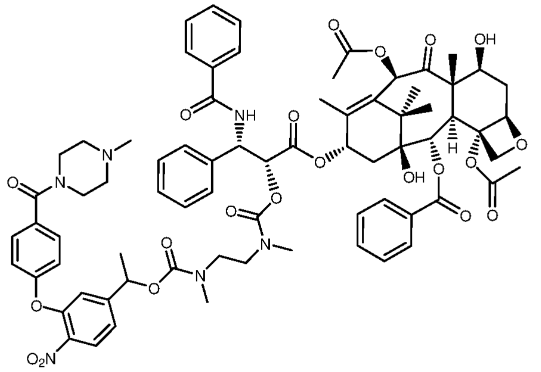
,

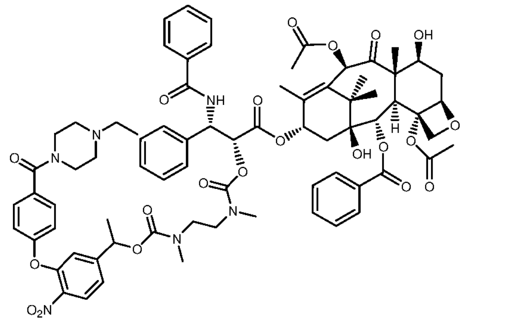
,
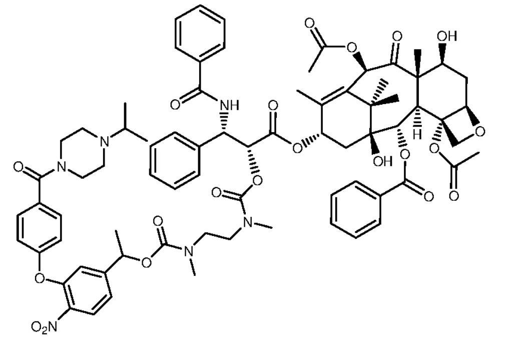
,

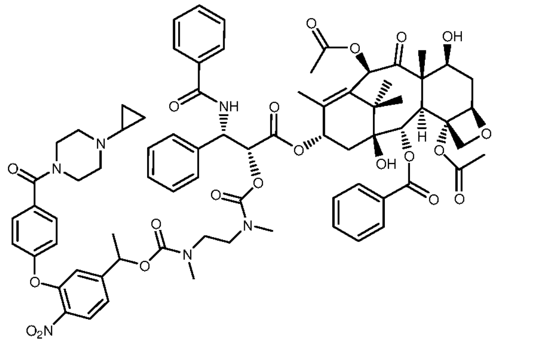,
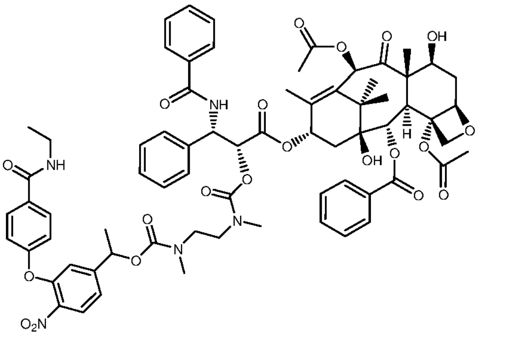,

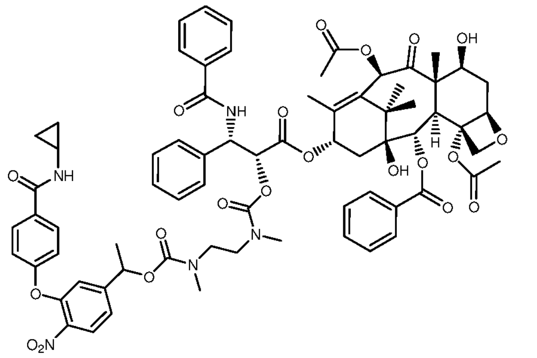,
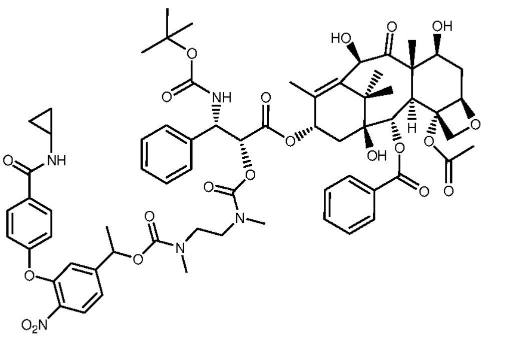,

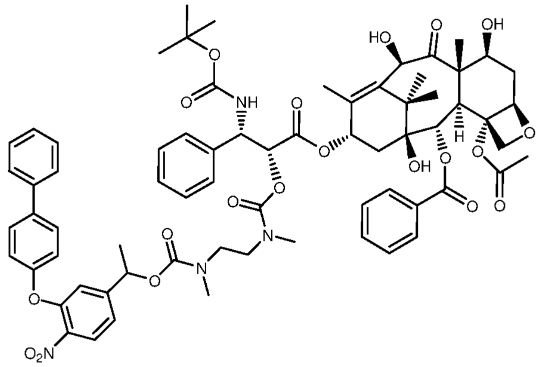,
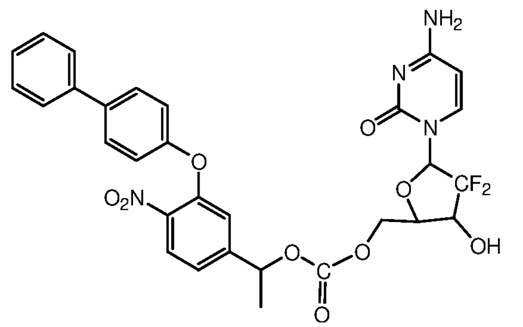,

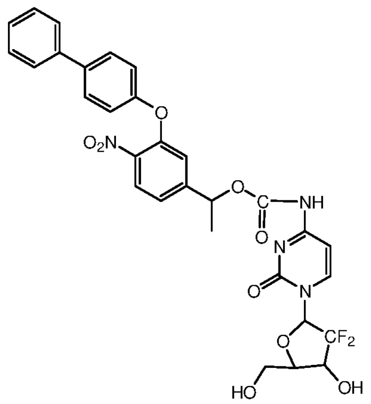,
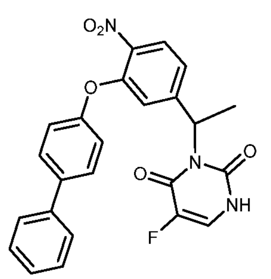,
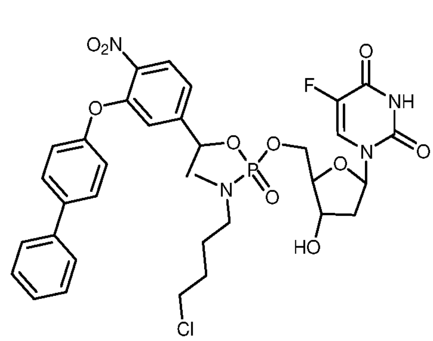,

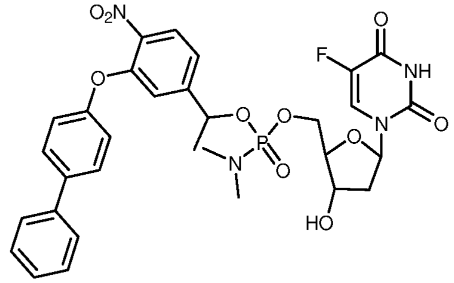

,

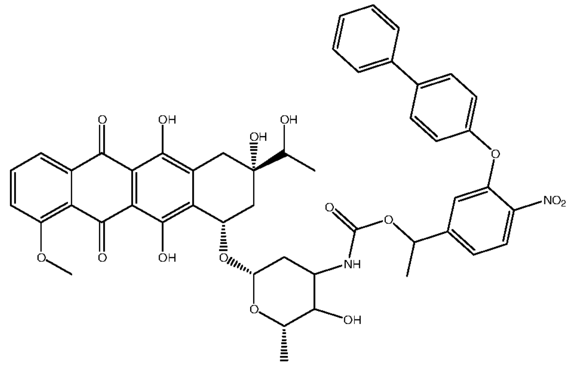

,

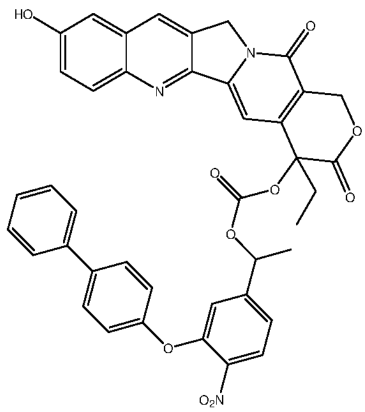

,

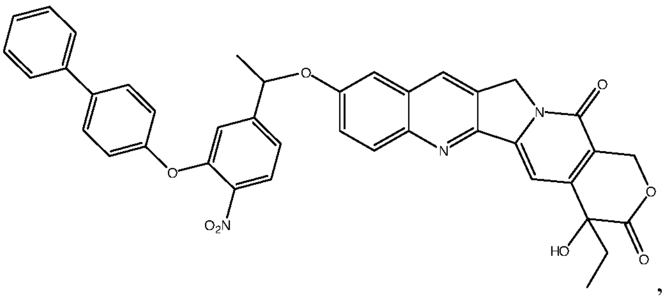

,

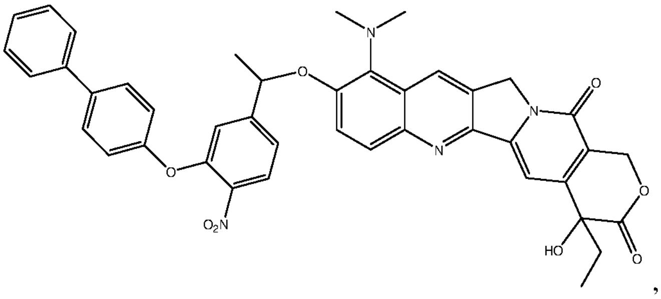

,

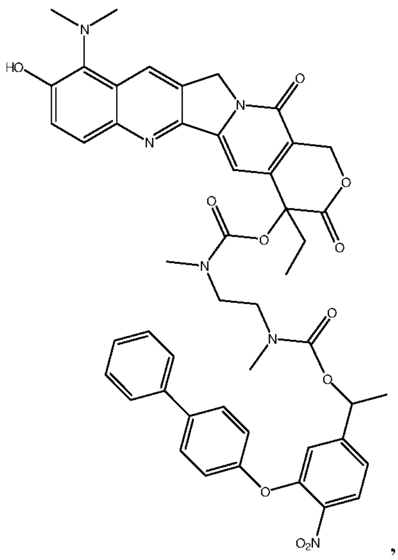

,

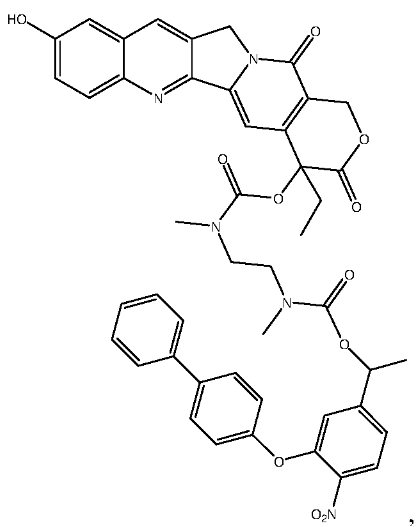

,

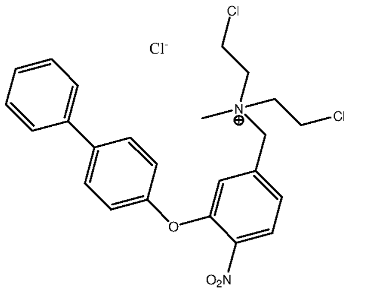, 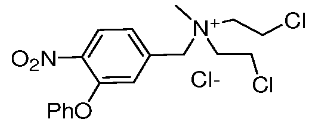, 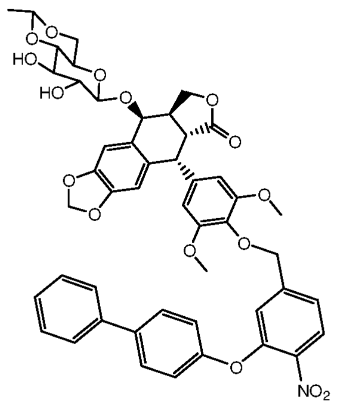,

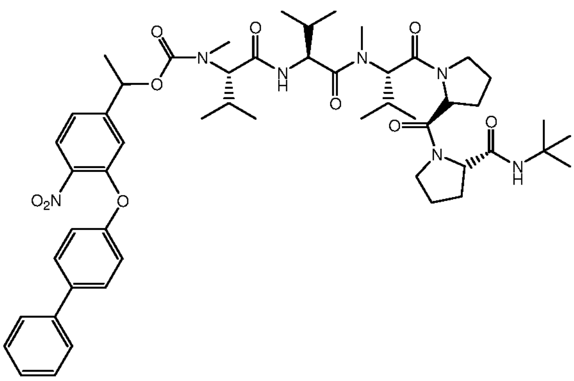, 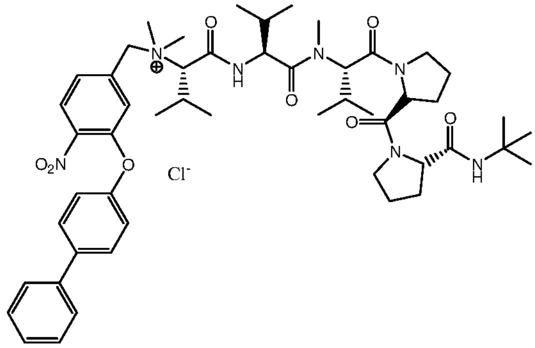,

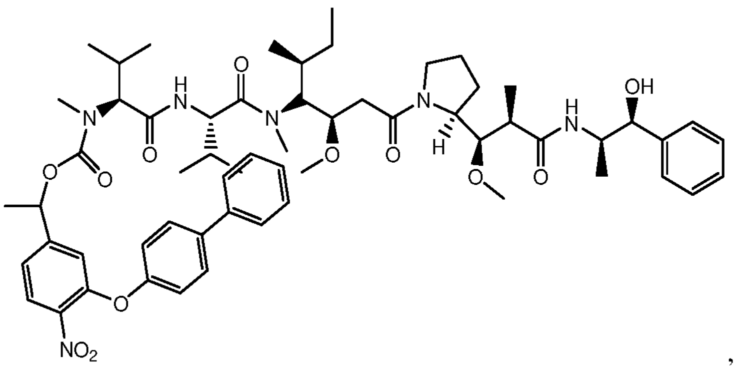,

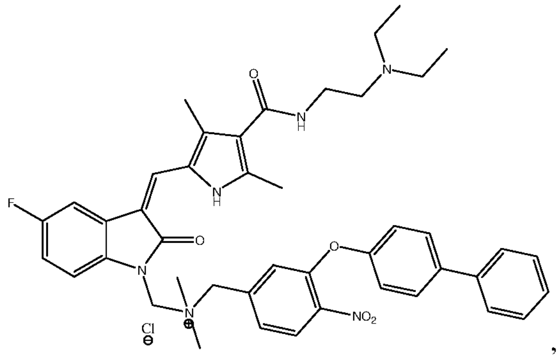, 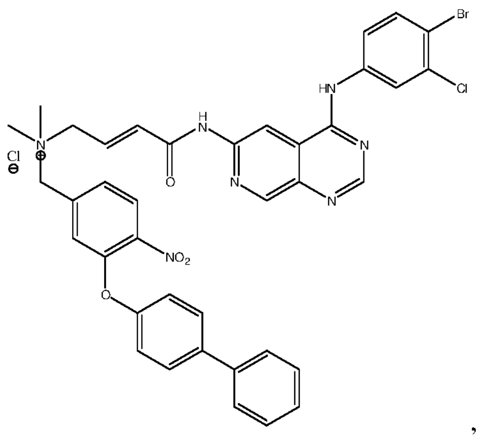,

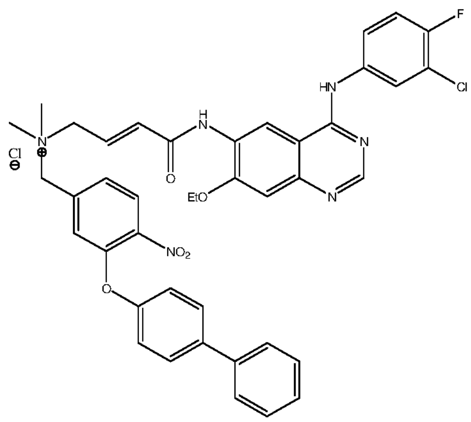,
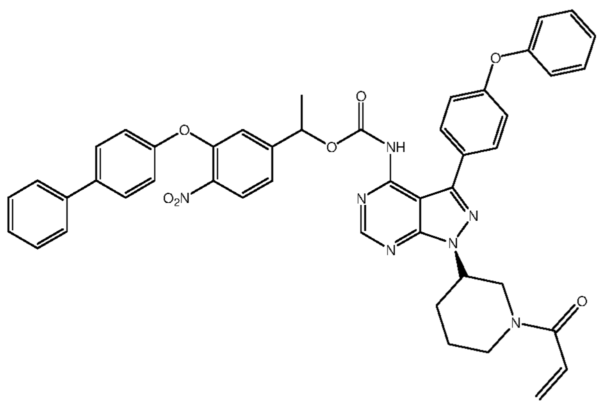;

the AKR1C3 enzyme-activated prodrug compound with formula (8) is selected from the compounds with the following structural formulae:

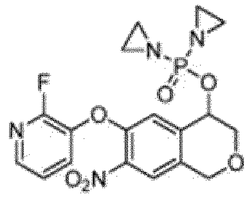,
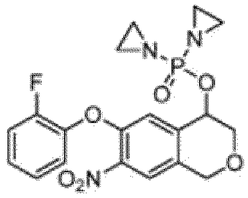,

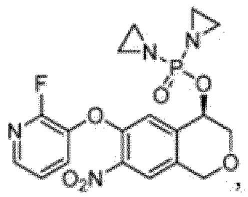,
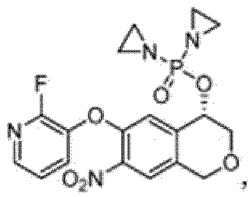,
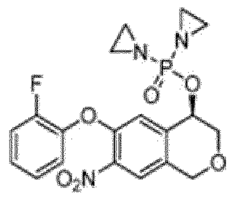 or
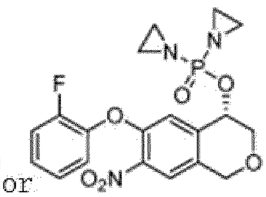;

the AKR1C3 enzyme-activated prodrug compound with formula (9) is selected from the compounds with the following structural formulae:

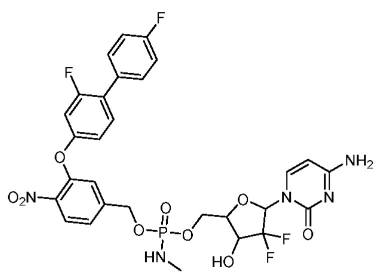,
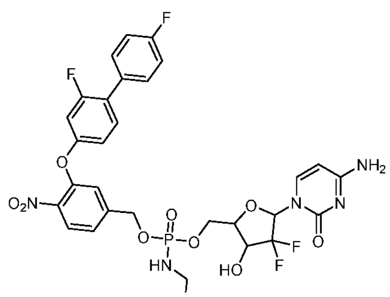,
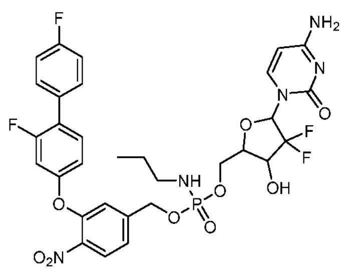,

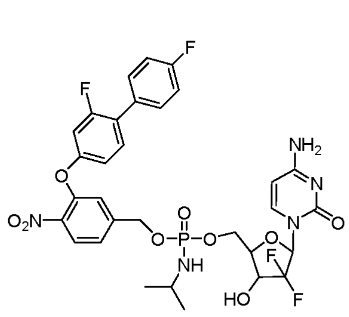,
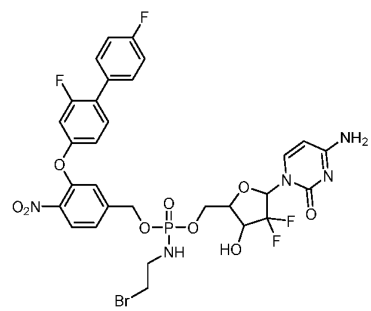,
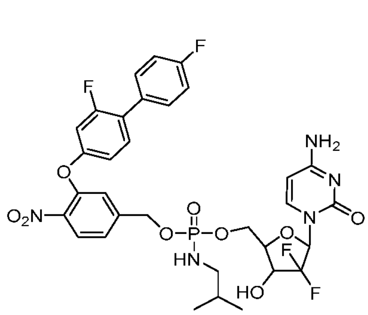,

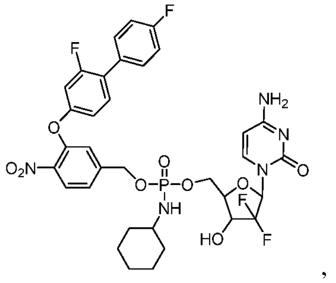,
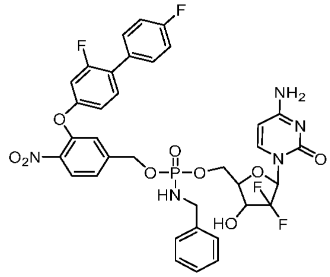,
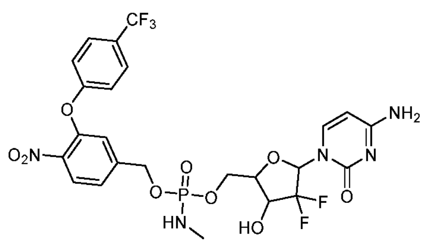,

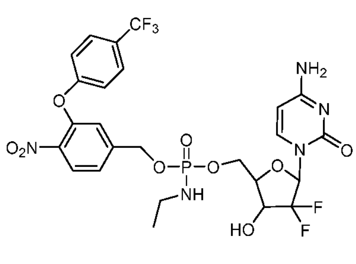,
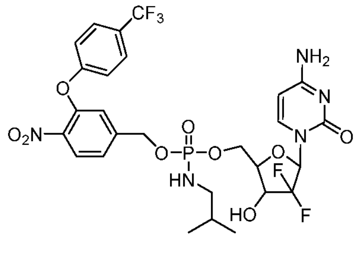,
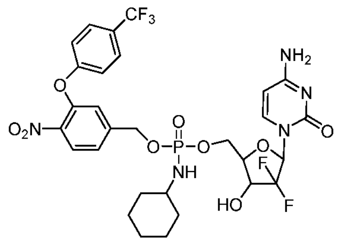,

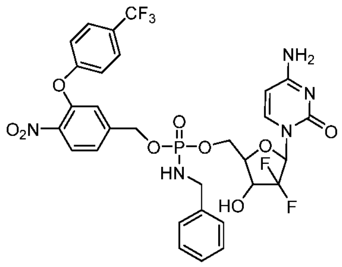,
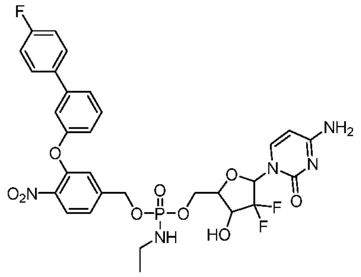,
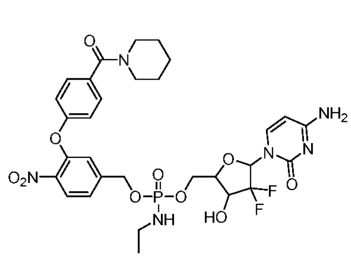,

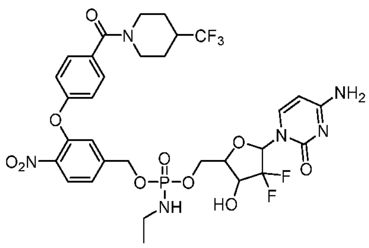,
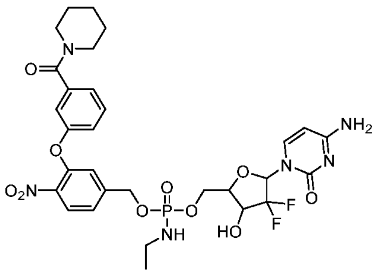,
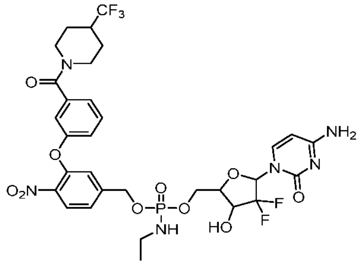,

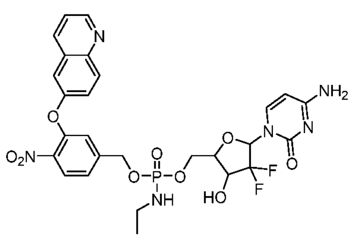,
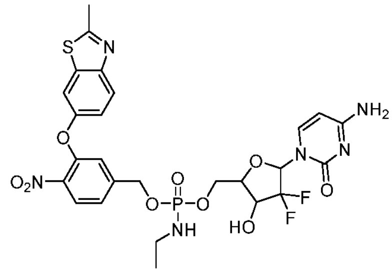,
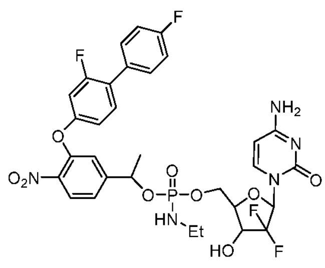,

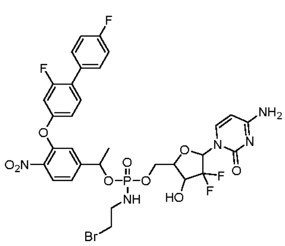,
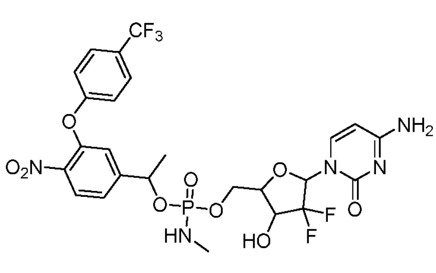,
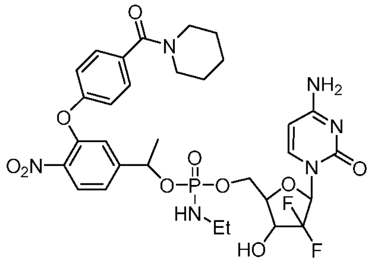,

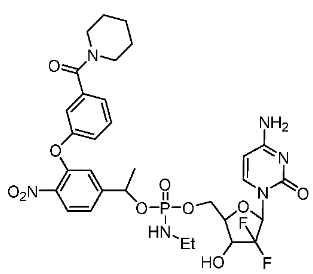,
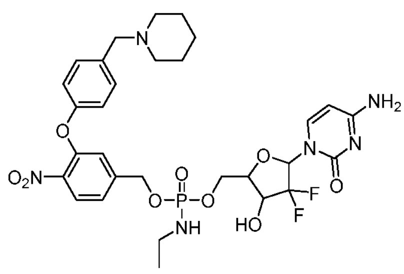,
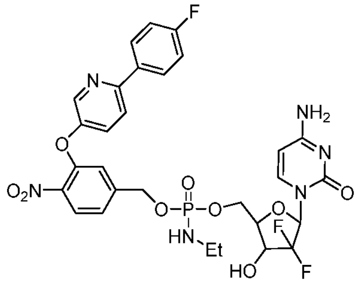,

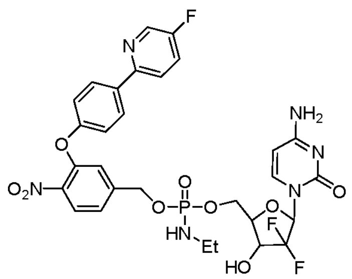,
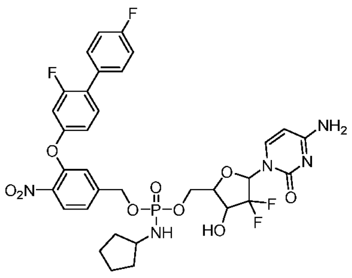,
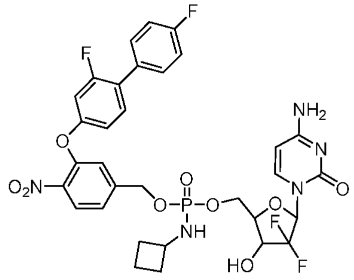,

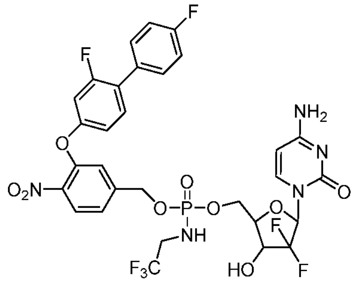,
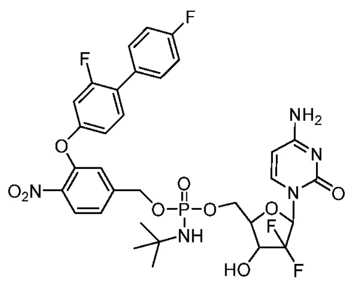,
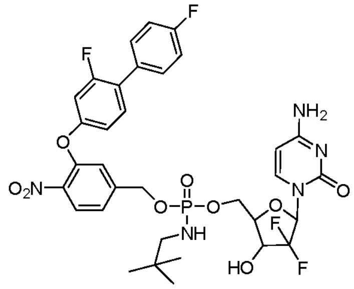,

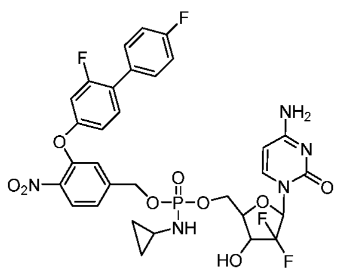,
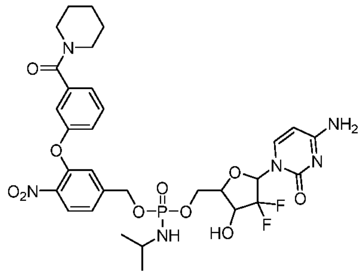,
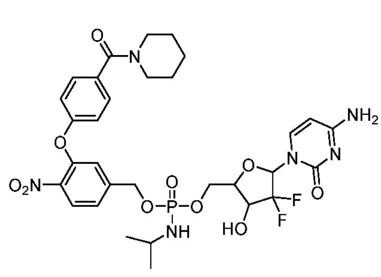,

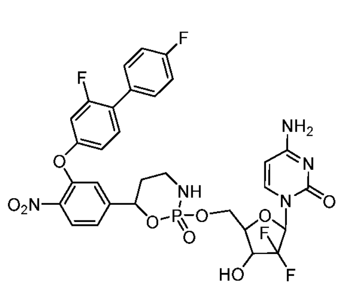,
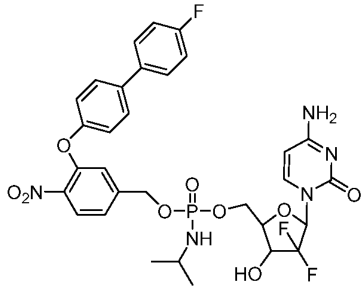,
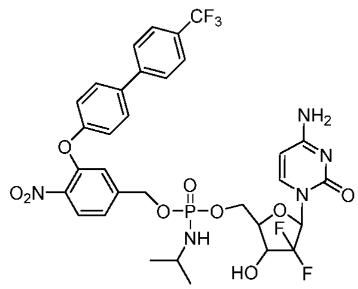,

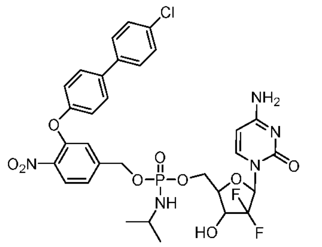,
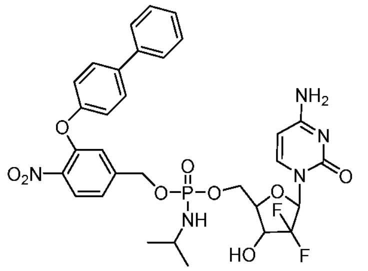,
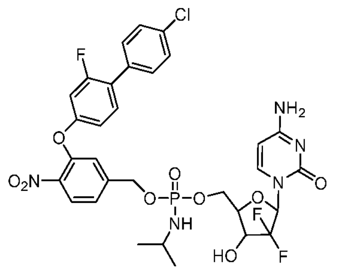,

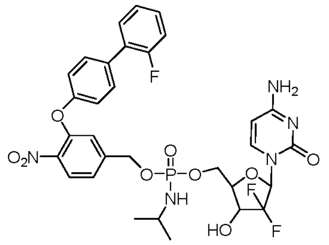,
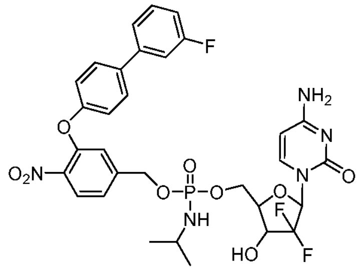,
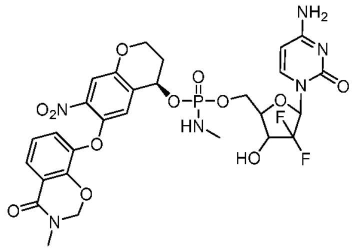,

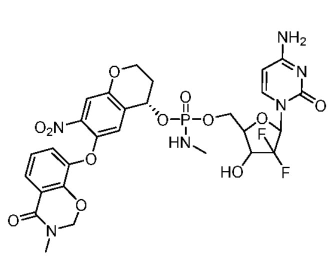,
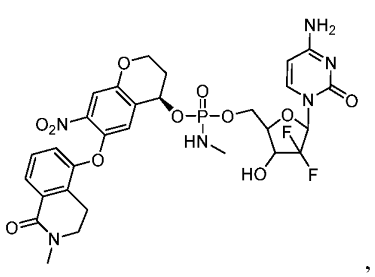,
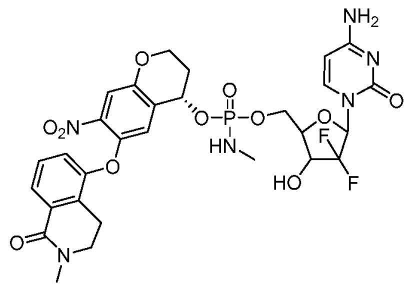,

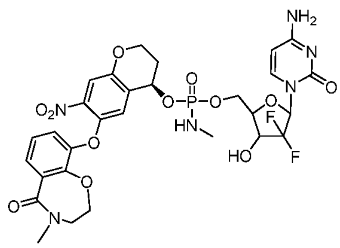,
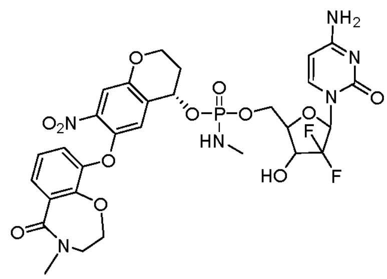,
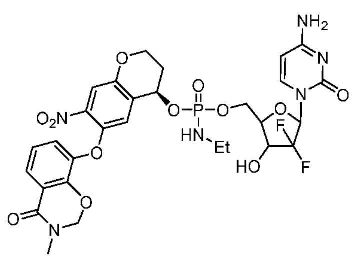,

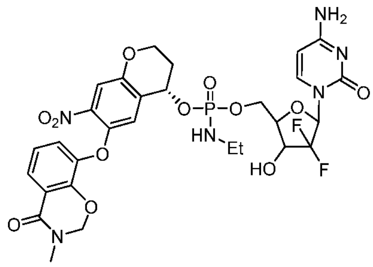,
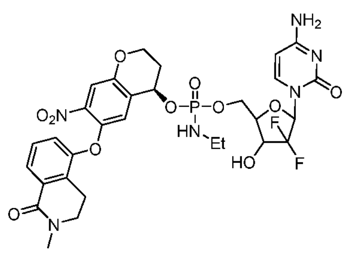,
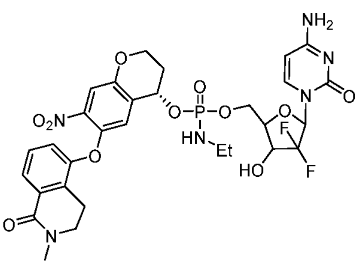,

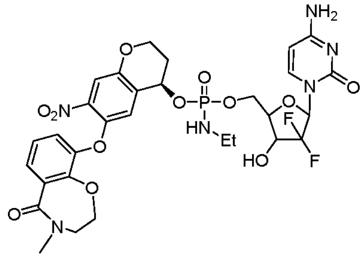,
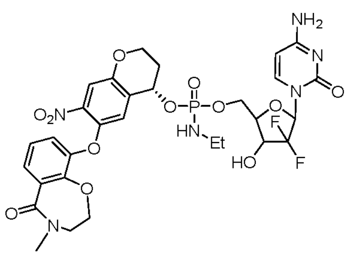,
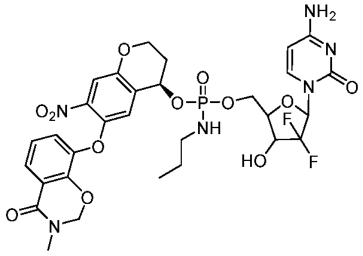,

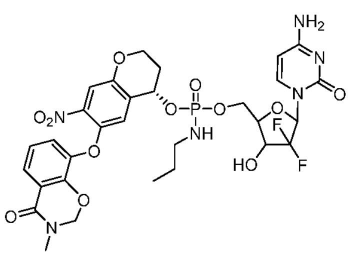,
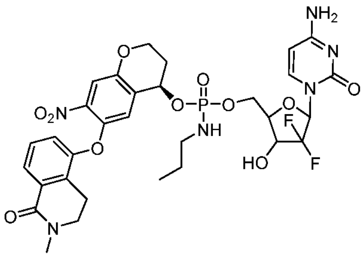,
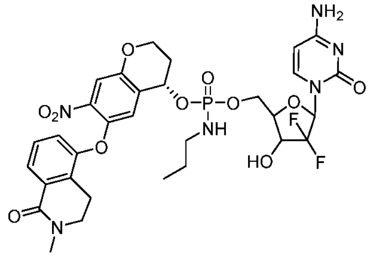,

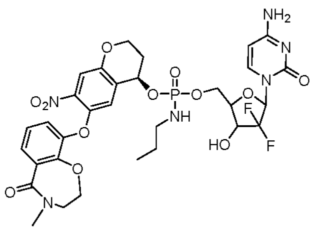,
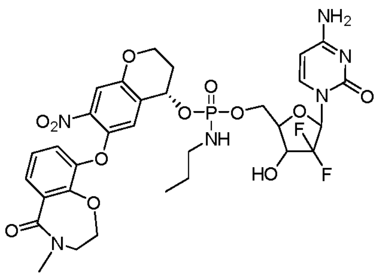,
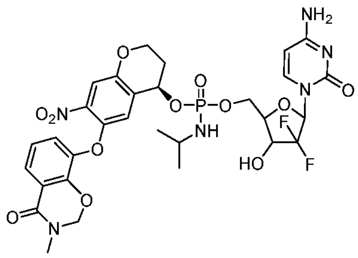,

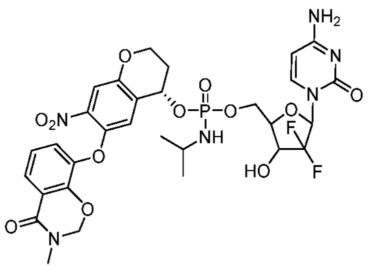,
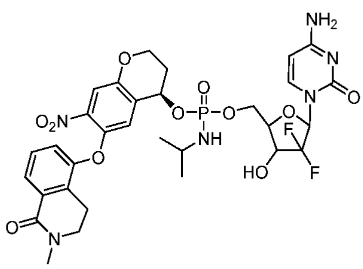,
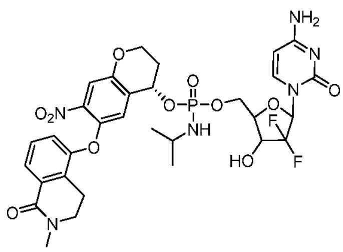,

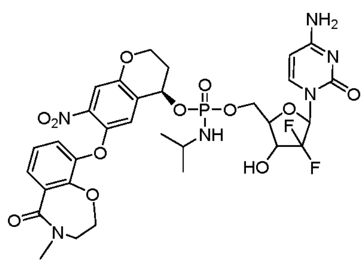,
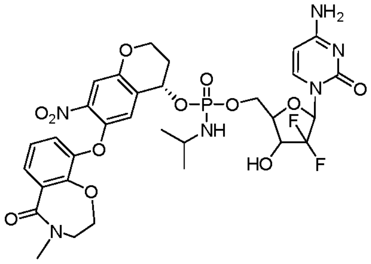,
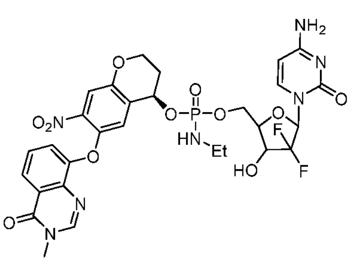,

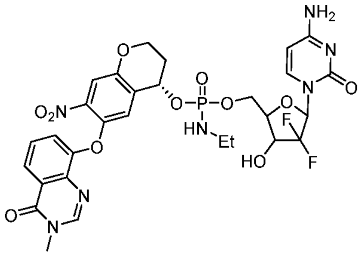 , 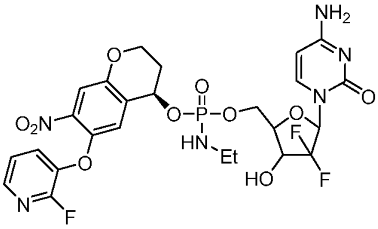 , 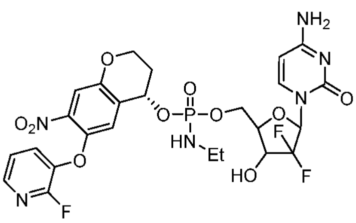 ,

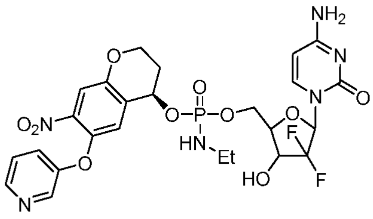 , 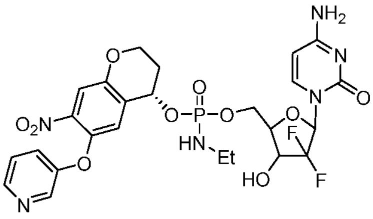 , 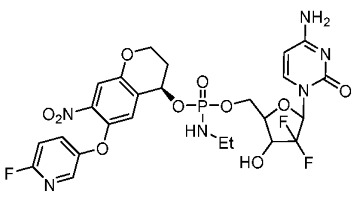 ,

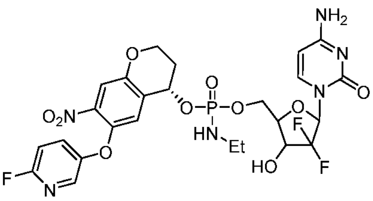 , 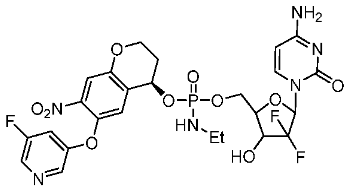 , 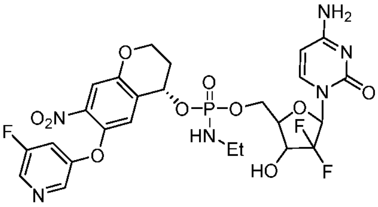 ,

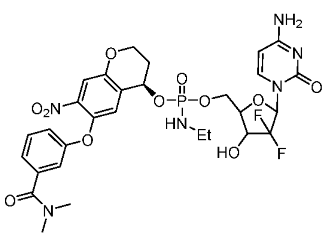 , 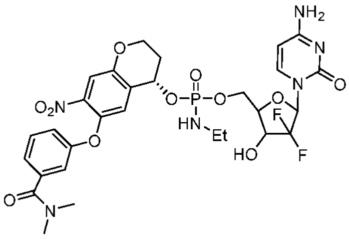 , 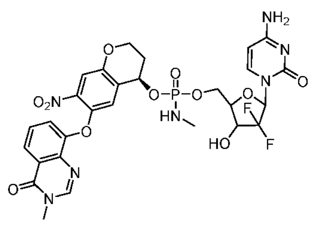 ,

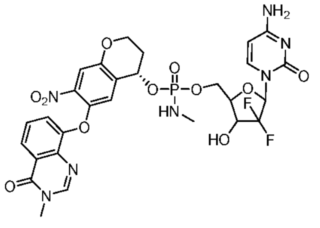 , 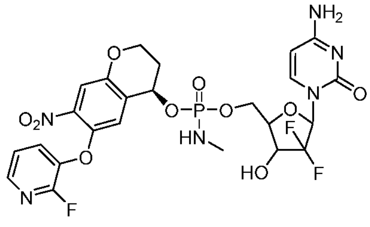 , 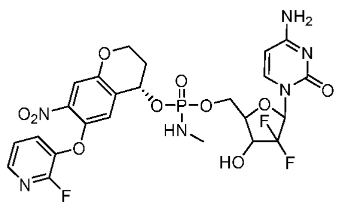 ,

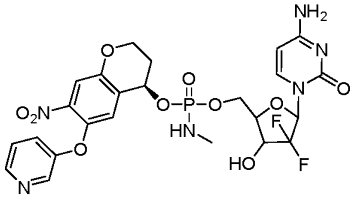 , 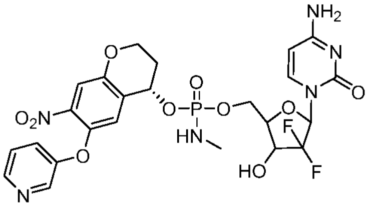 , 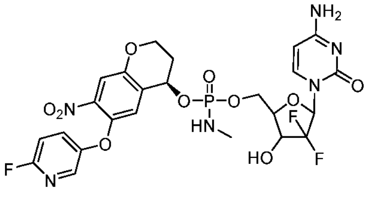 ,

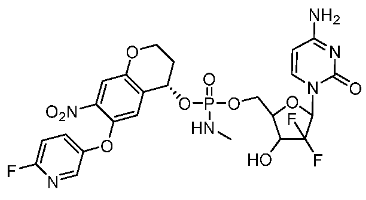,
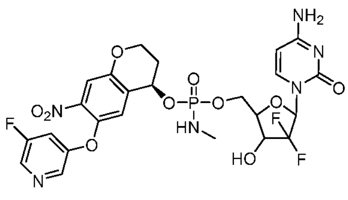,
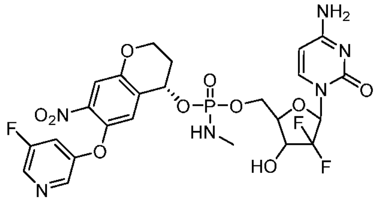,

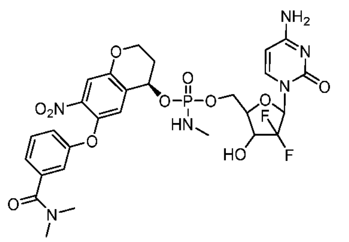,
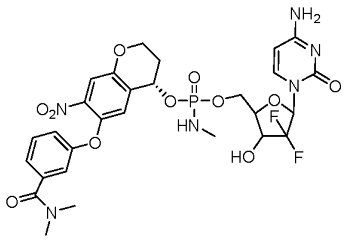,
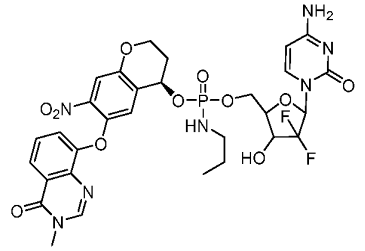,

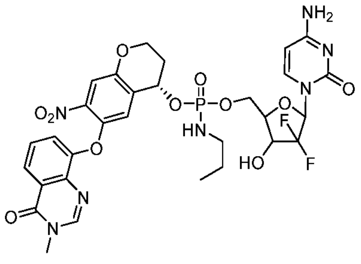,
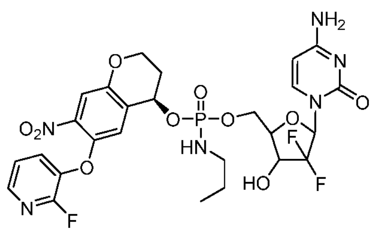,
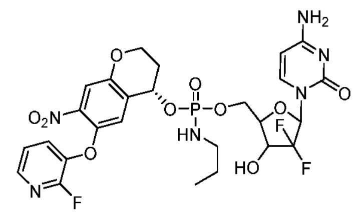,

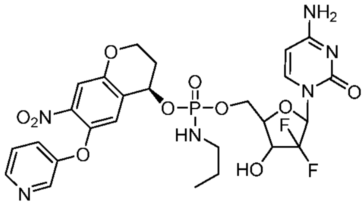,
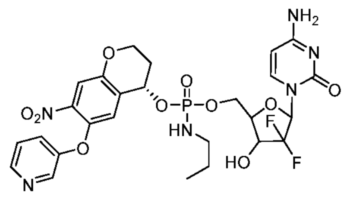,
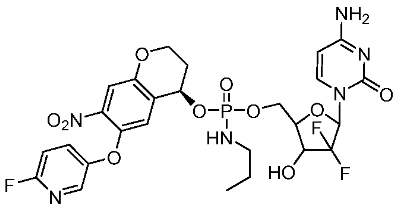,

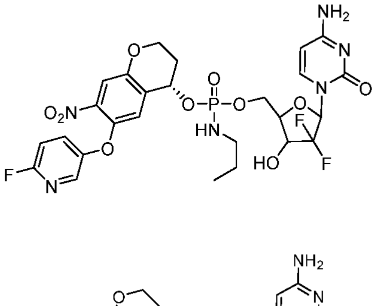,
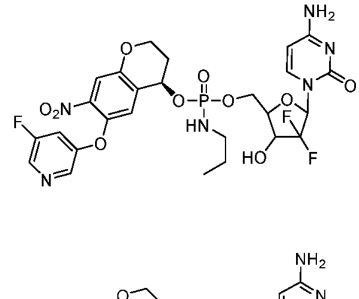,
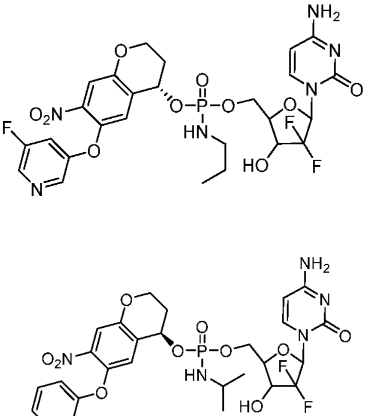,

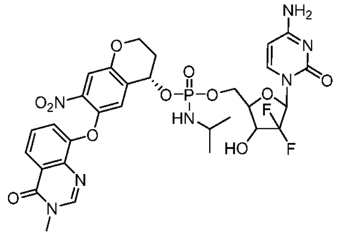, 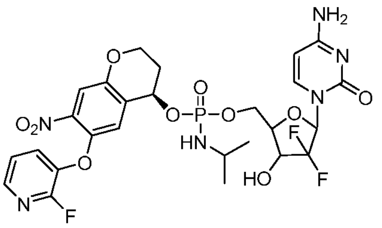, 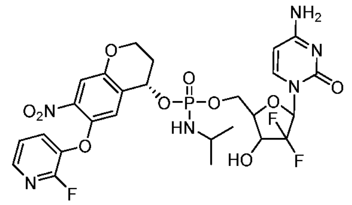,

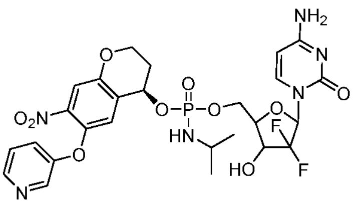, 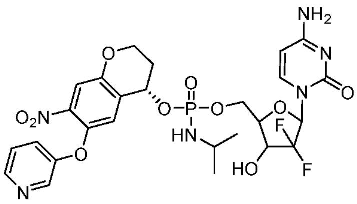, 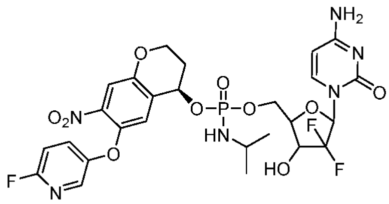,

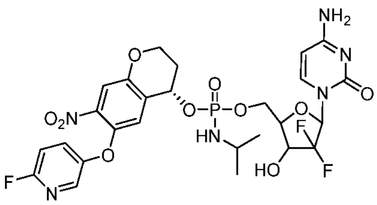, 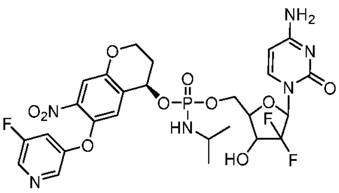, 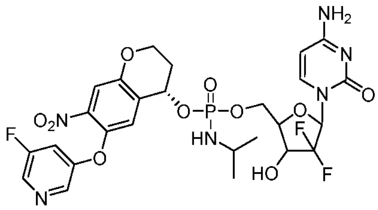,

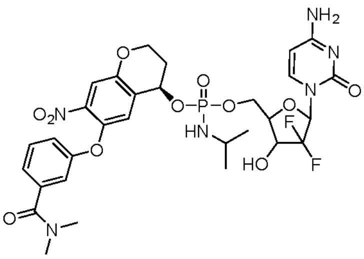, 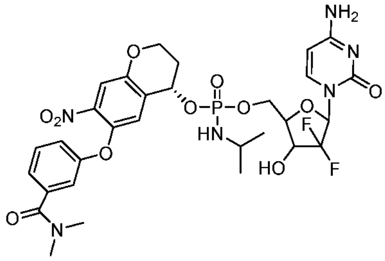;

the AKR1C3 enzyme-activated prodrug compound with formula (10) is selected from the compounds with the following structural formulae:

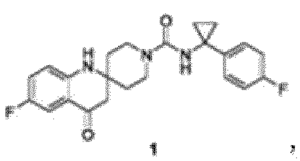
1 ,
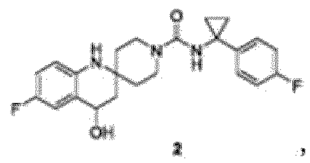
2 ,
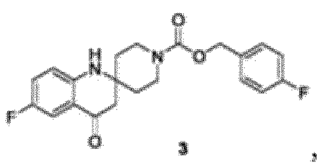
3 ,

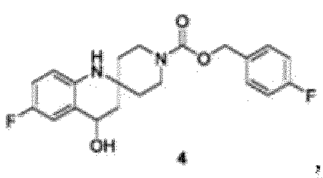
4 ,
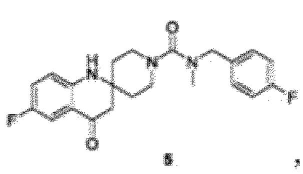
5 ,
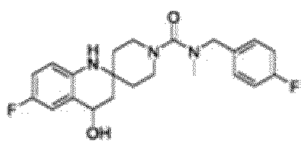
6 ,

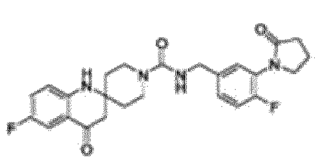, 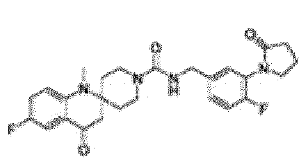, 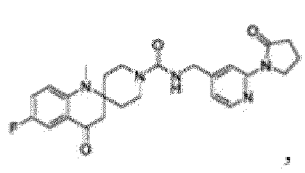,

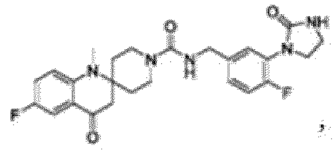,

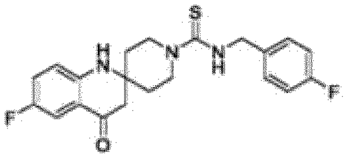
7,

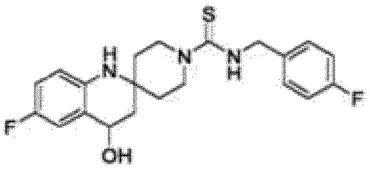
8,

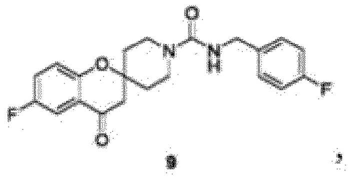
9,

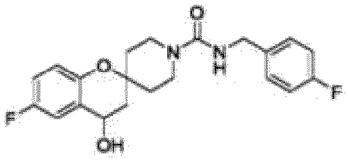
10,

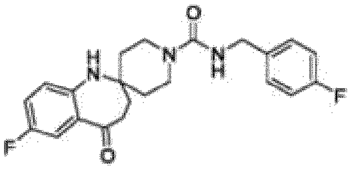
11,

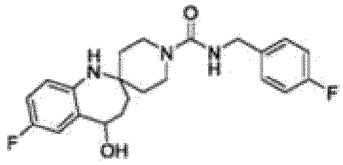
12,

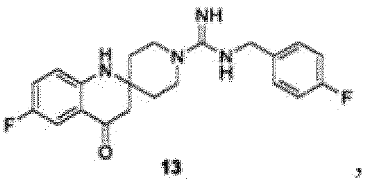
13,

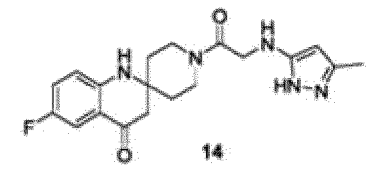
14,

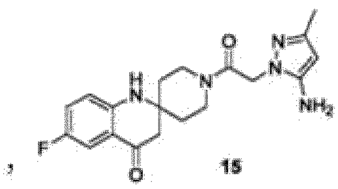
15,

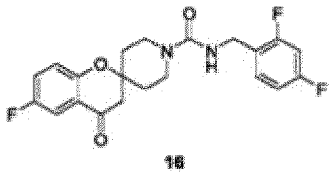
16,

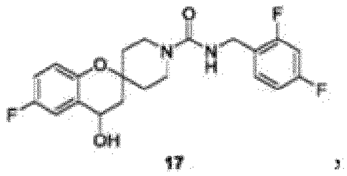
17,

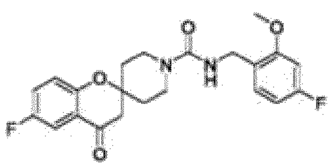
18,

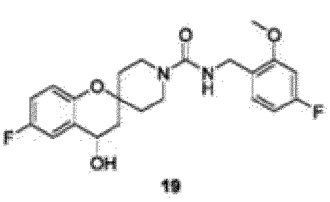
19,

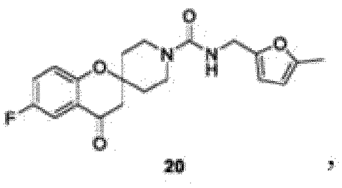
20,

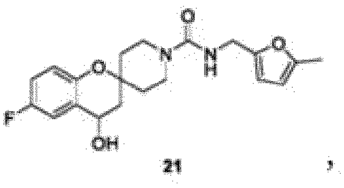
21,

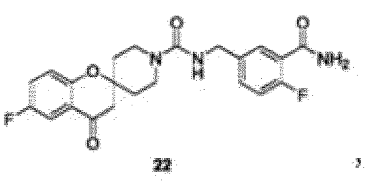
22,

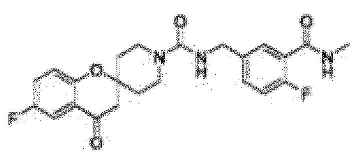
23,

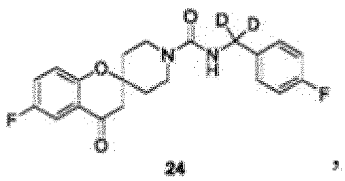
24,

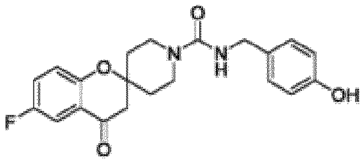
25
,
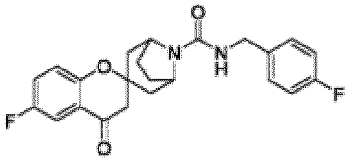
26
,
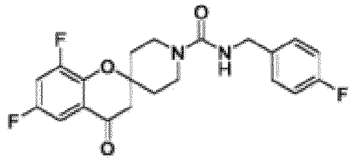
27
,
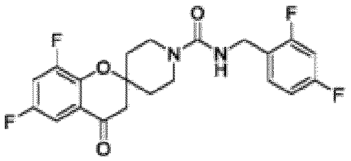
28
,
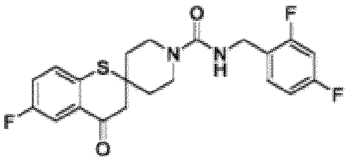
29
,
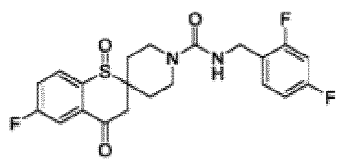
30
,
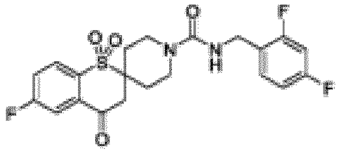
31
,
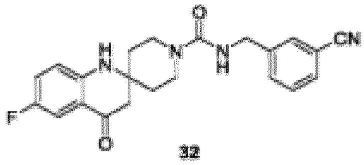
32
,
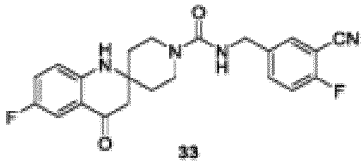
33
,
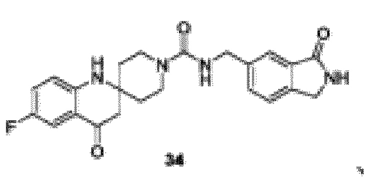
34
,
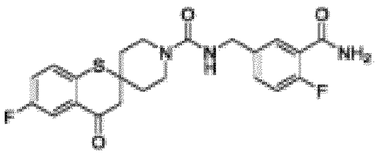
35
,
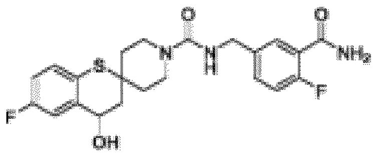
36
,
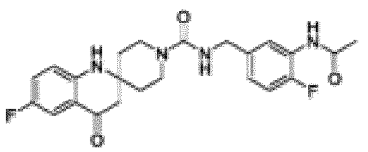
37
,
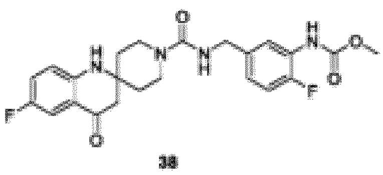
38
,
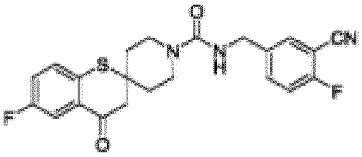
39
,
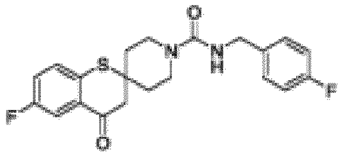
40
,
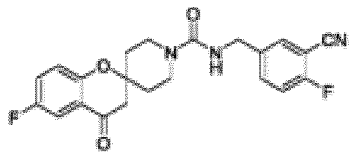
41
,
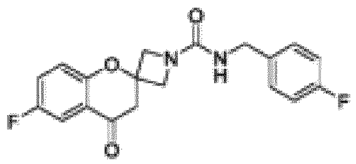
42
,
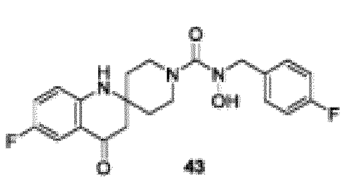
43
,
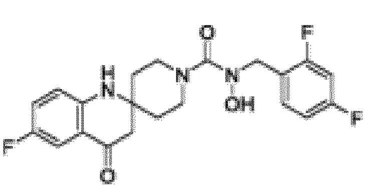
,
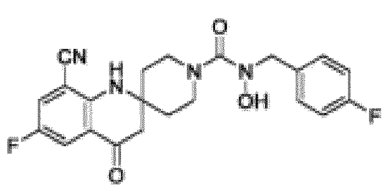
,
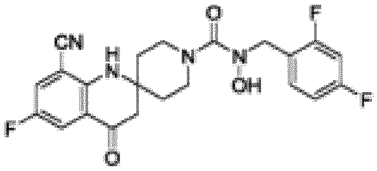
,
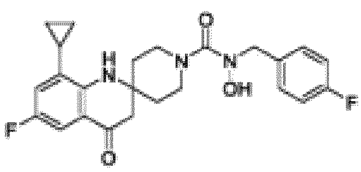
,
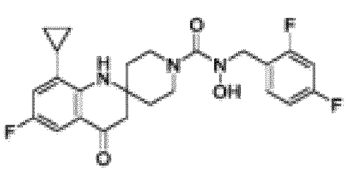
,

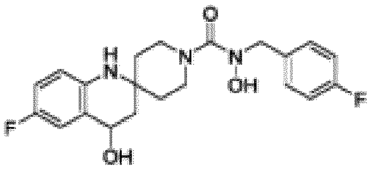,
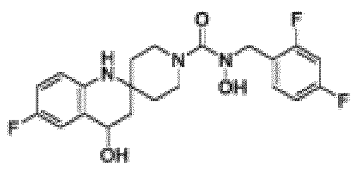,
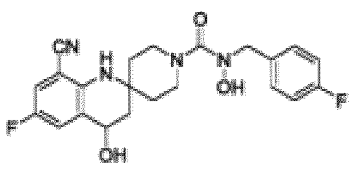,

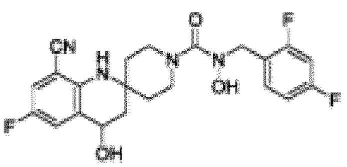,
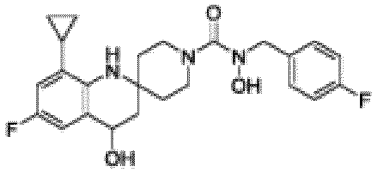,
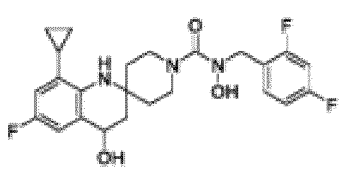,

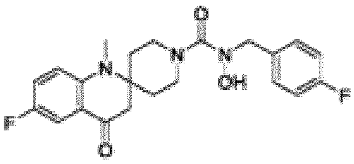,
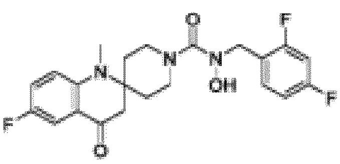,
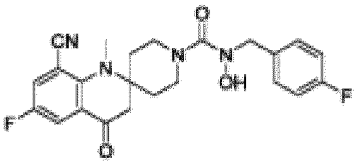,

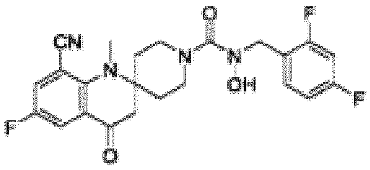,
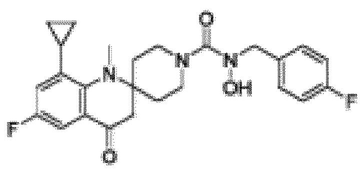,
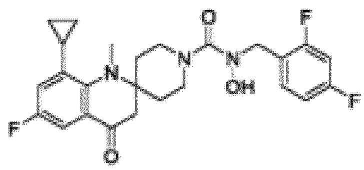,

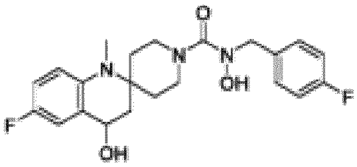,
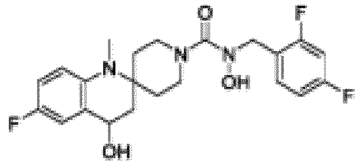,
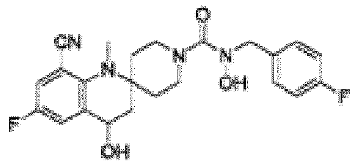,

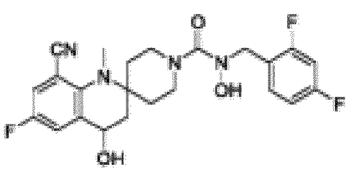,
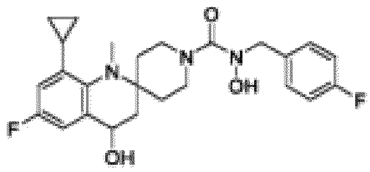,
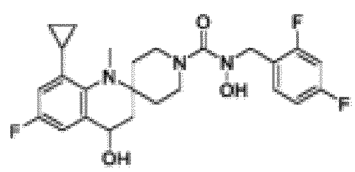,

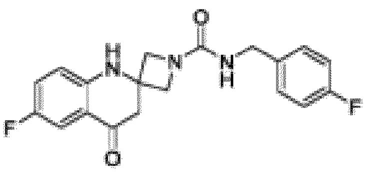,
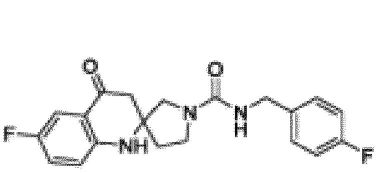,
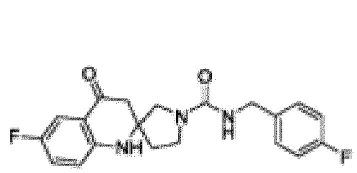,

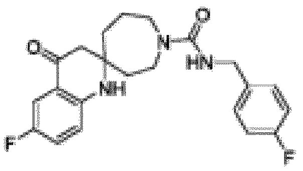,
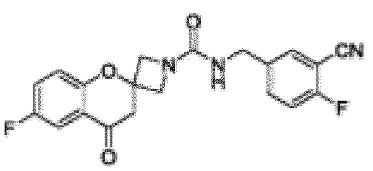,
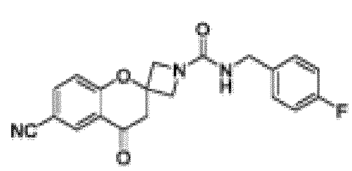,

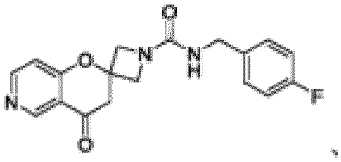,
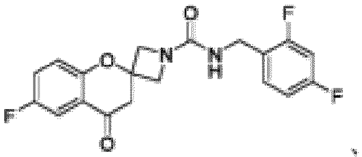,
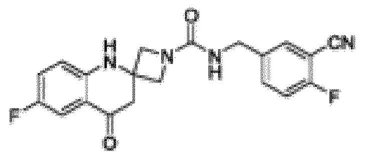,

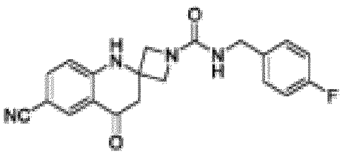,
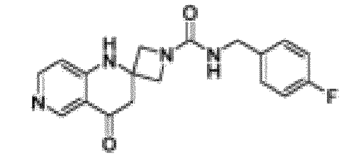,
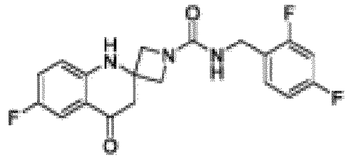,

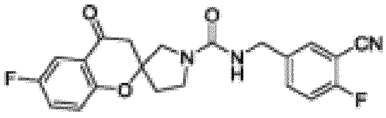,
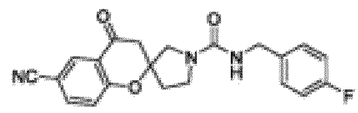,
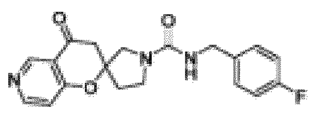,

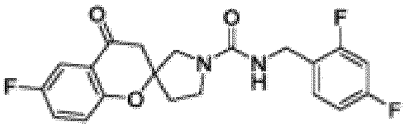,
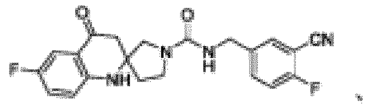,
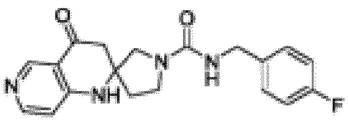,

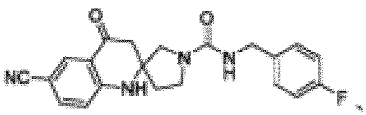,
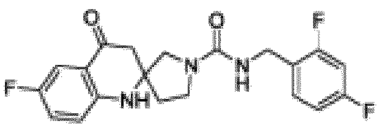,
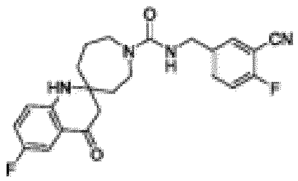,

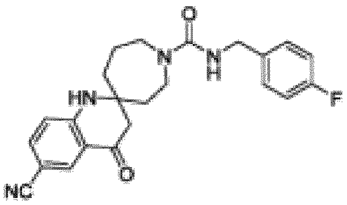,
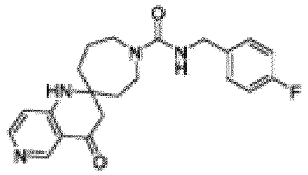,
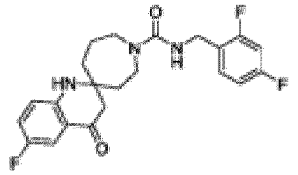,

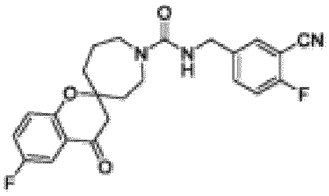,
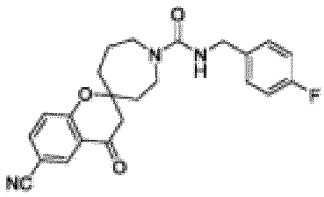,
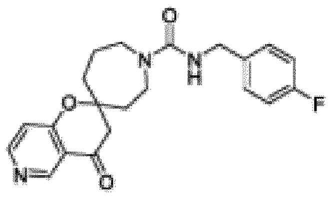,

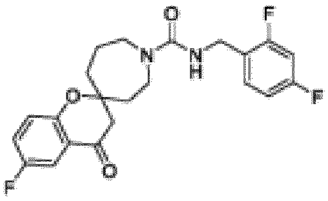,
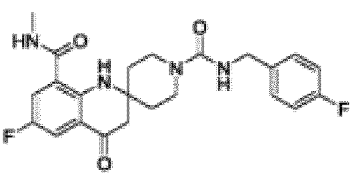,
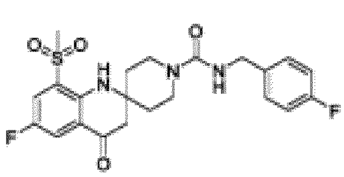,

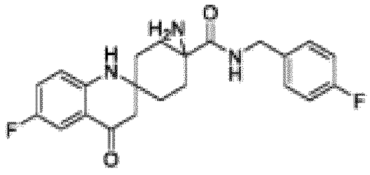 , 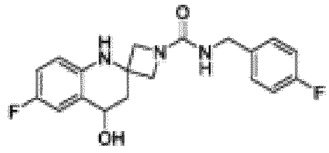 , 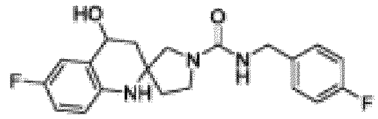 ,

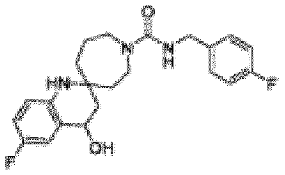 , 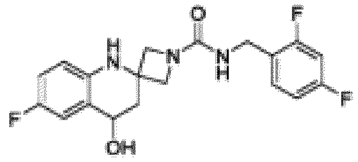 , 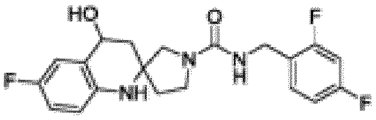 ,

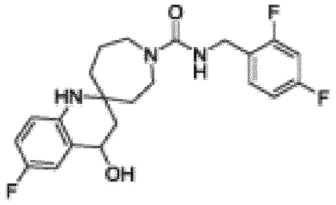 , 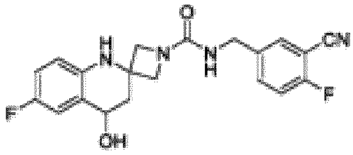 , 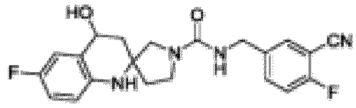 ,

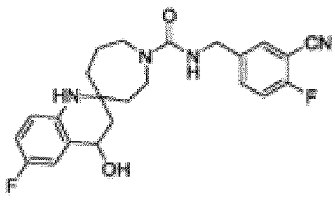 , 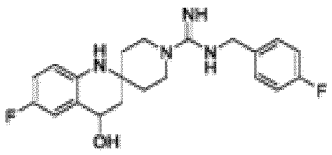 , 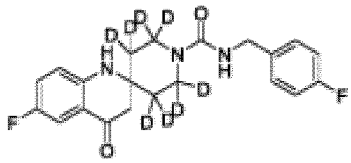 ,

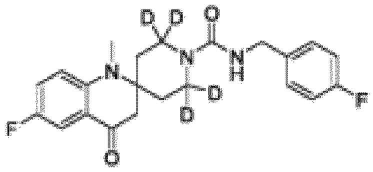 , 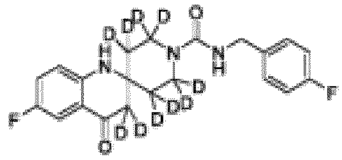 , 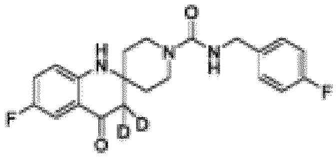 ,

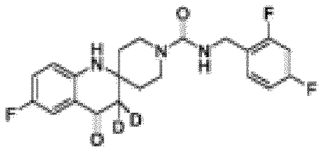 , 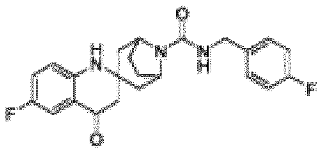 , 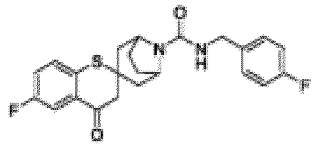 ,

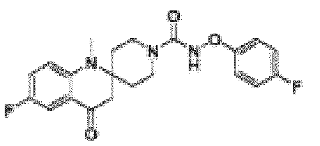 , 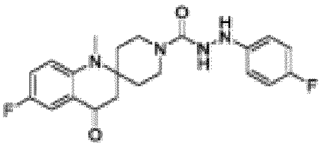 , 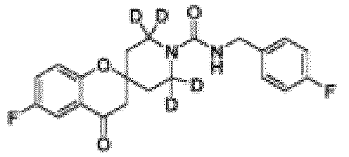 ,

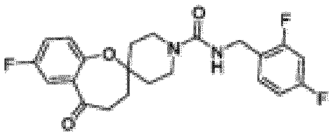 , 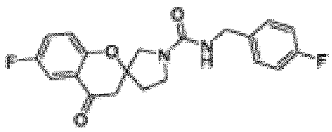 , 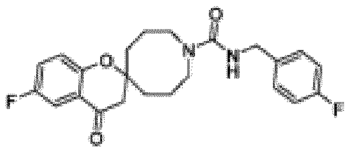 ,

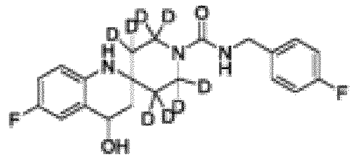 、 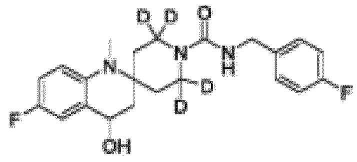 、 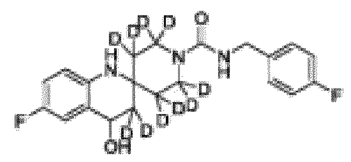 、

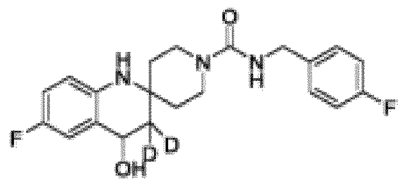 、 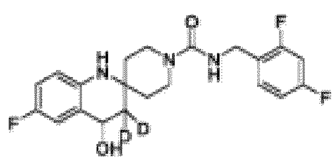 、 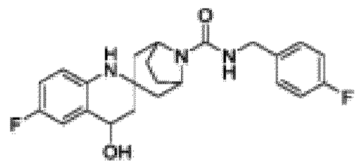 、

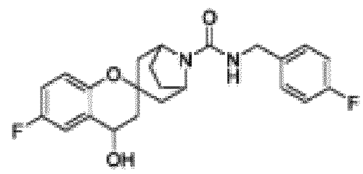 、 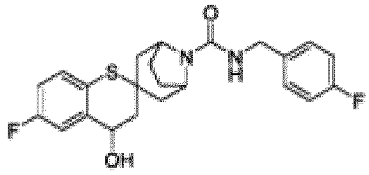 、 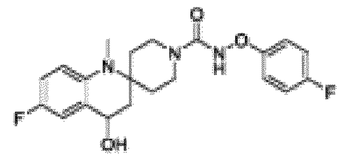 、

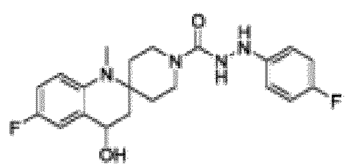 、 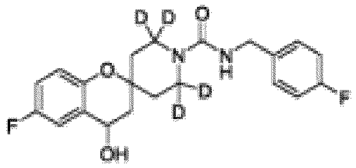 、 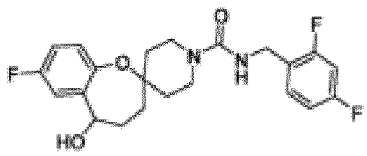 、

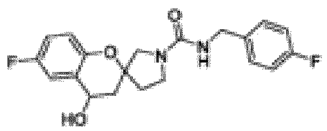 、 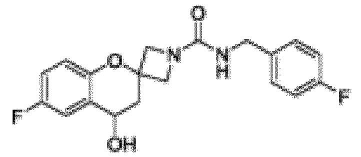 、 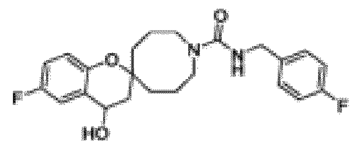 、

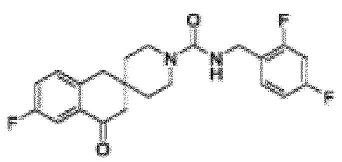 、 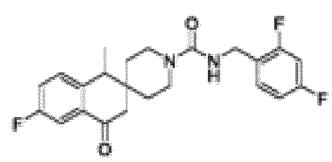 、 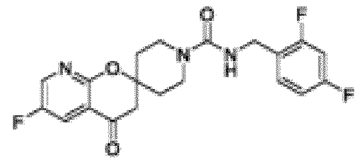 、

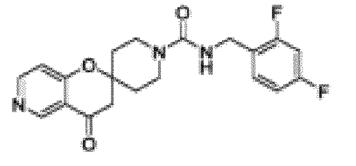 、 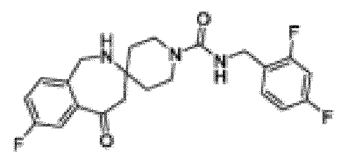 、 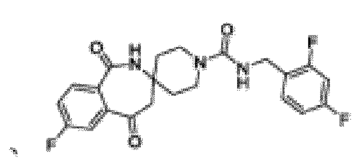 、

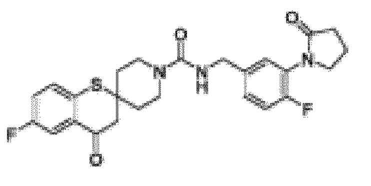 、 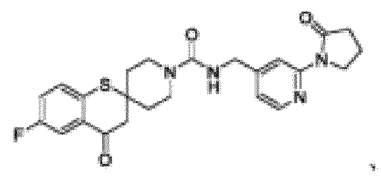 、 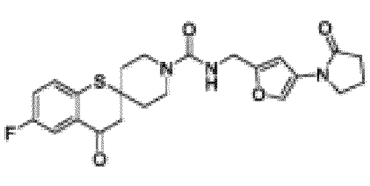 ;

the AKR1C3 enzyme-activated prodrug compound with formula (11) is selected from the compounds with the following structural formulae:

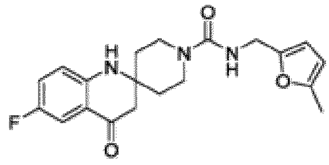,

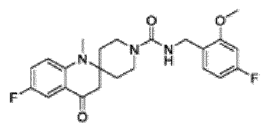, 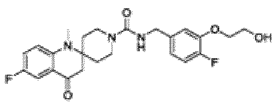, 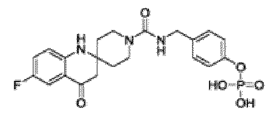, 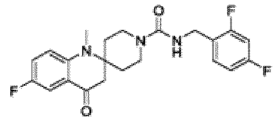,

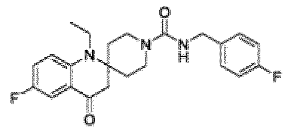, 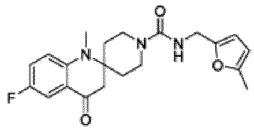, 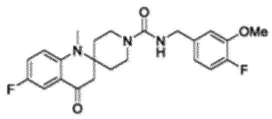, 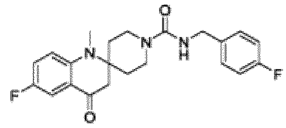,

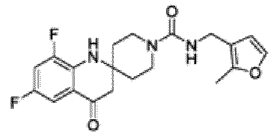, 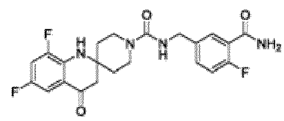, 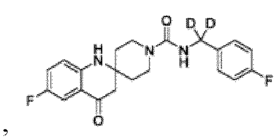, 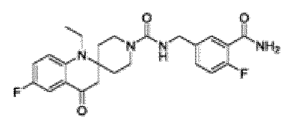,

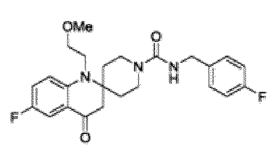, 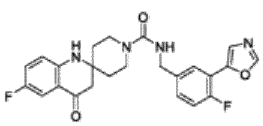, 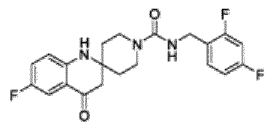, 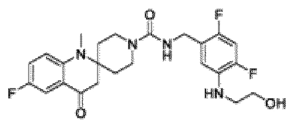,

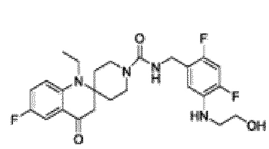, 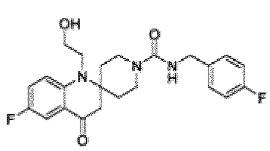, 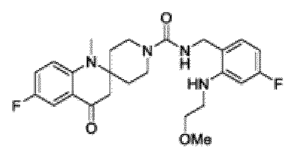, 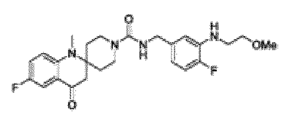,

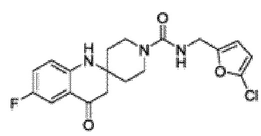, 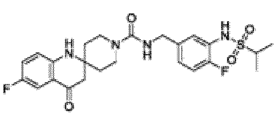, 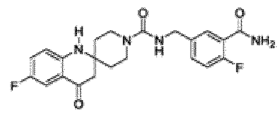, 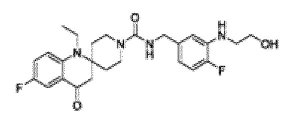,

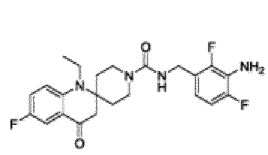, 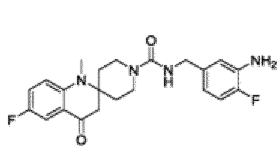, 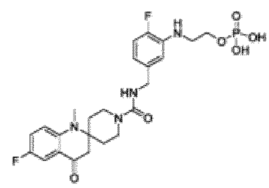, 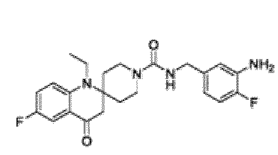,

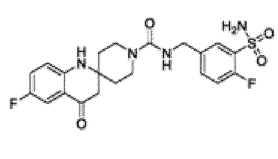, 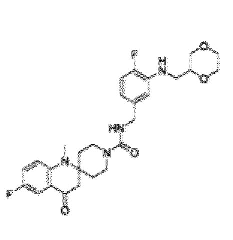, 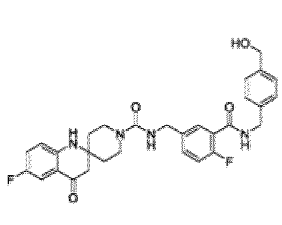, 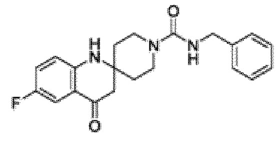,

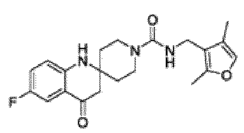 , 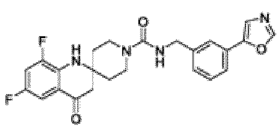 , 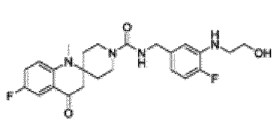 , 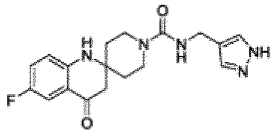 ,

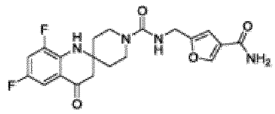 , 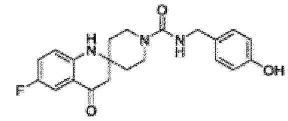 , 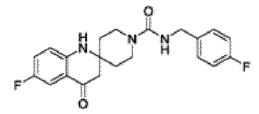 , 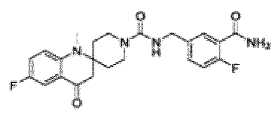 ,

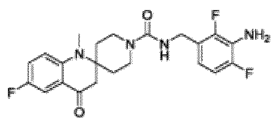 , 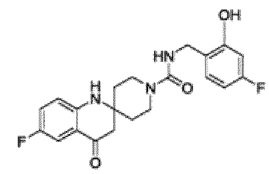 , 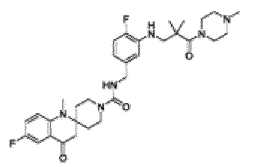 , 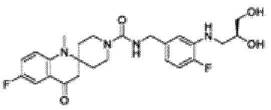 ,

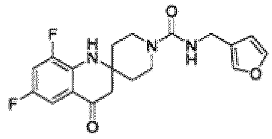 , 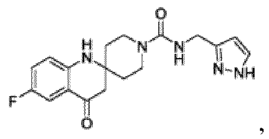 , 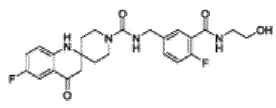 , 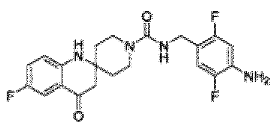 ,

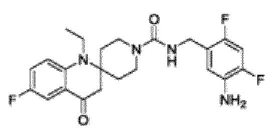 , 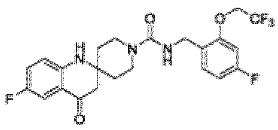 , 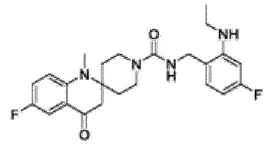 , 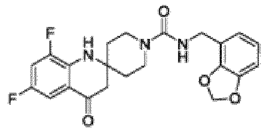 ,

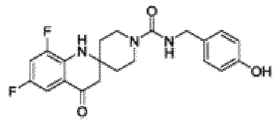 , 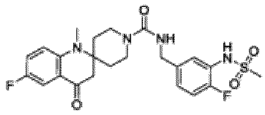 , 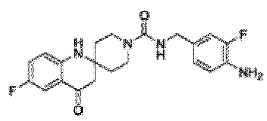 , 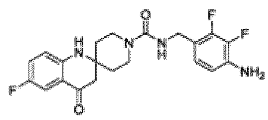 ,

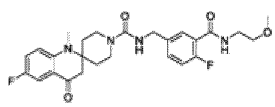 , 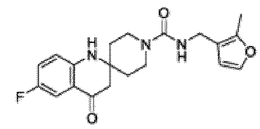 , 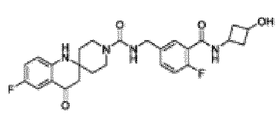 , 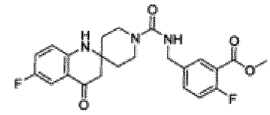 ,

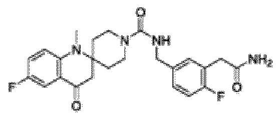 , 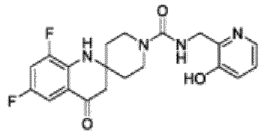 , 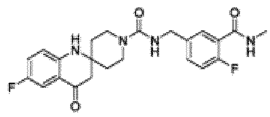 , 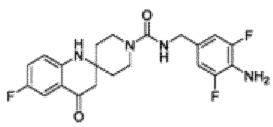 ,

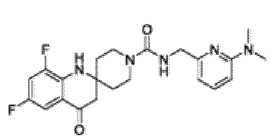 , 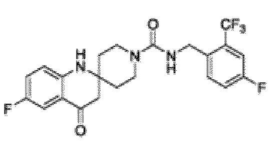 , 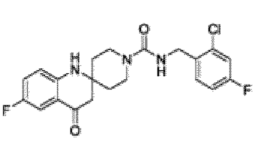 , 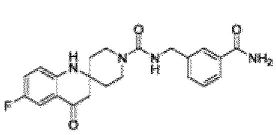 ,

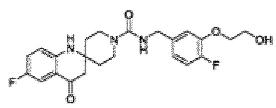 , 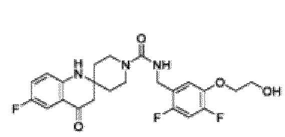 , 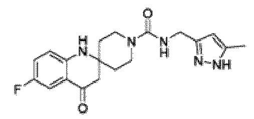 , 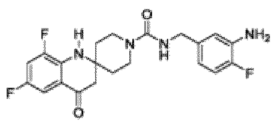 ,

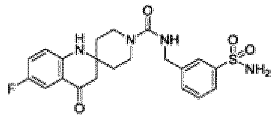, 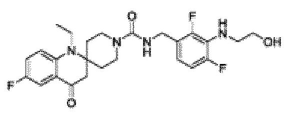, 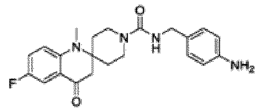, 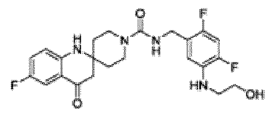,

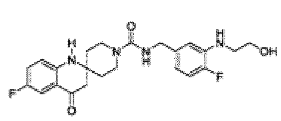, 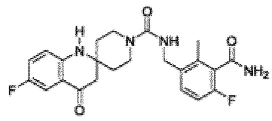, 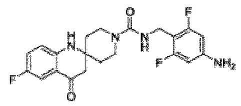, 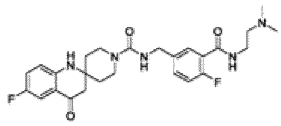,

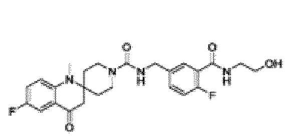, 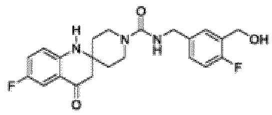, 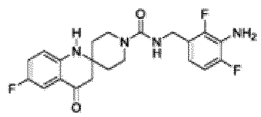, 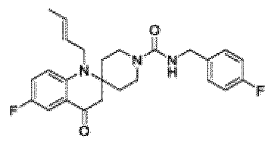,

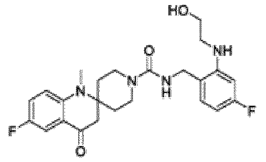, 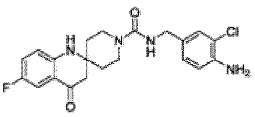, 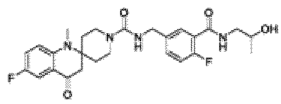, 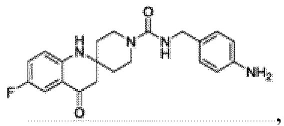,

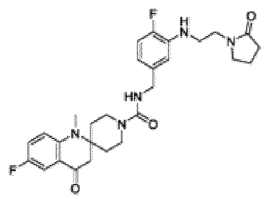, 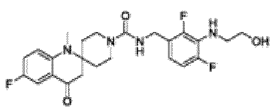, 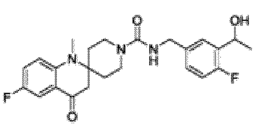, 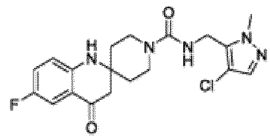,

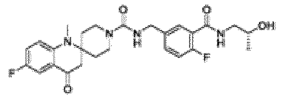, 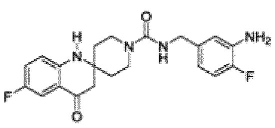, 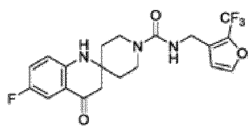, 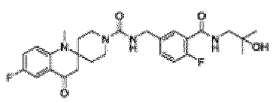,

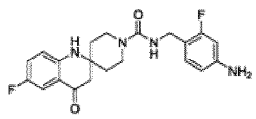, 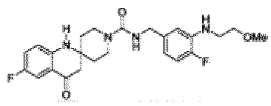, 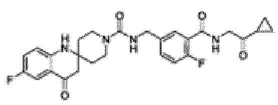, 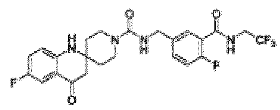,

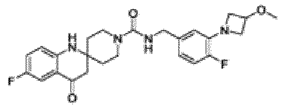, 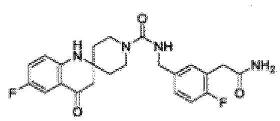, 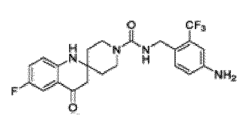, 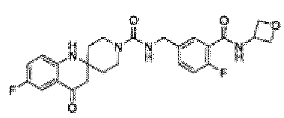,

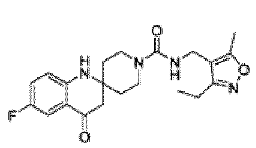, 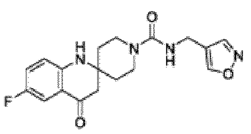, 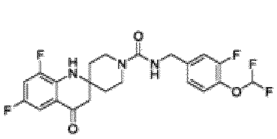, 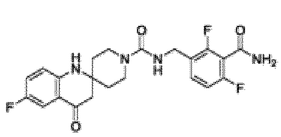,

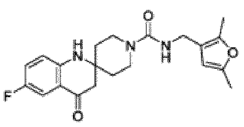, 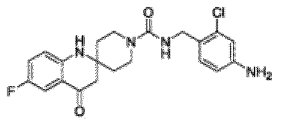, 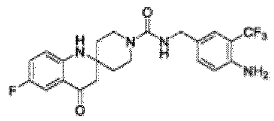, 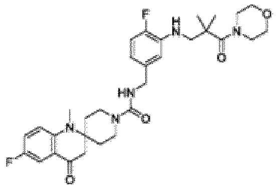,

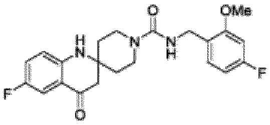, 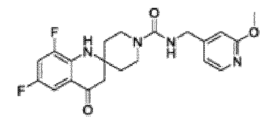, 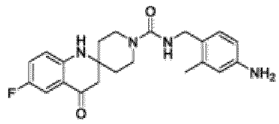, 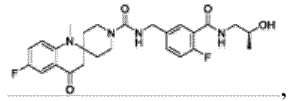,

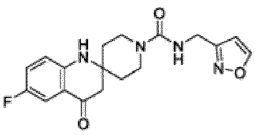, 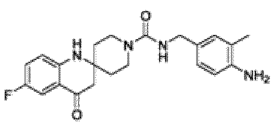, 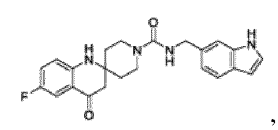, 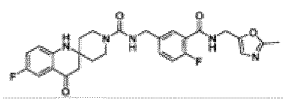,

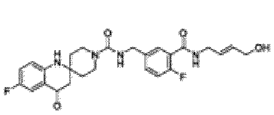, 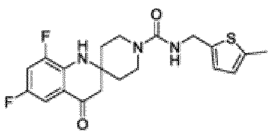, 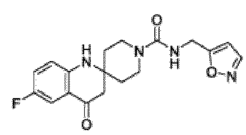, 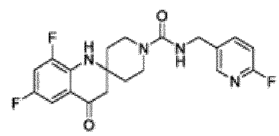,

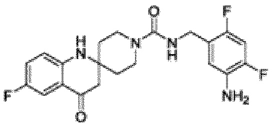, 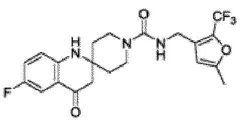, 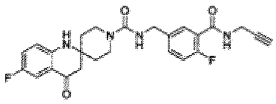, 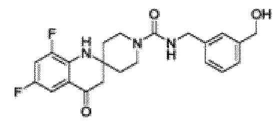,

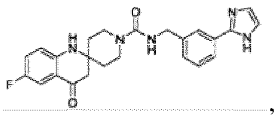, 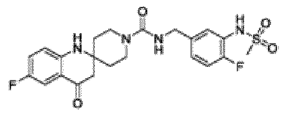, 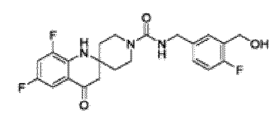, 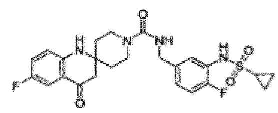,

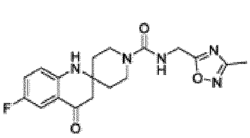, 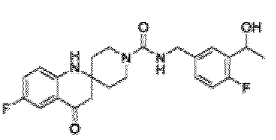, 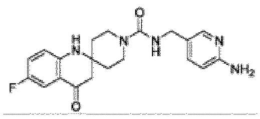, 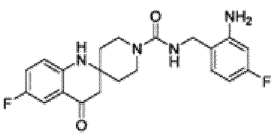,

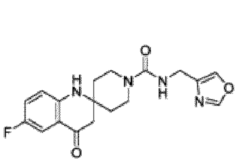, 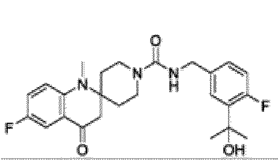, 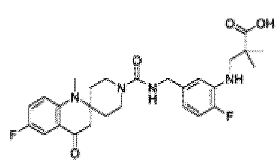, 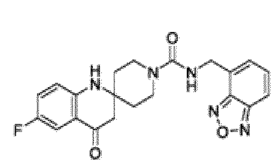,

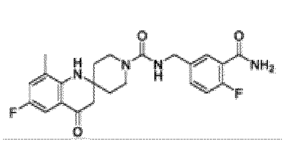, 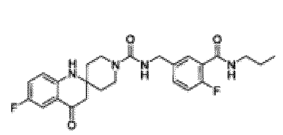, 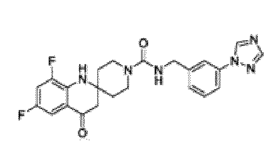, 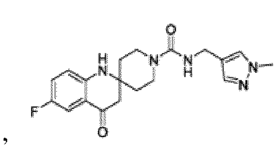,

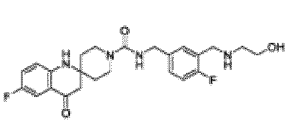, 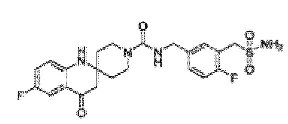, 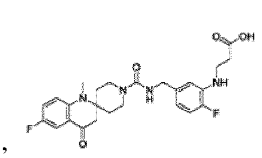, 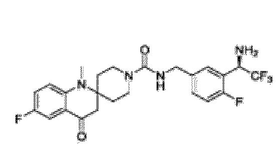,

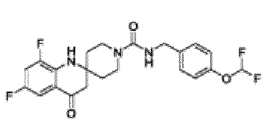, 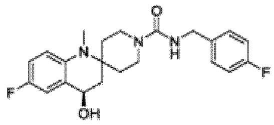, 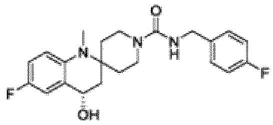, 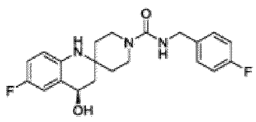,

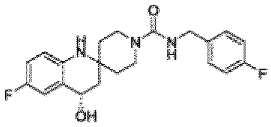, 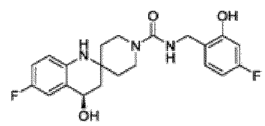, 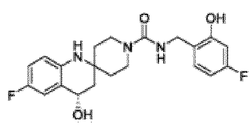, 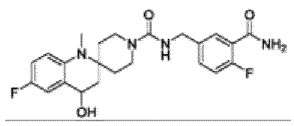,

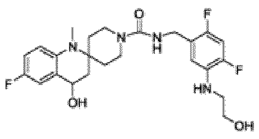, 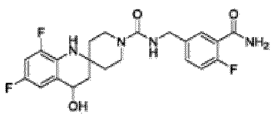, 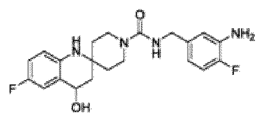, 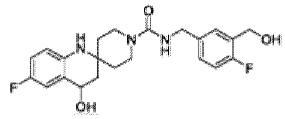,

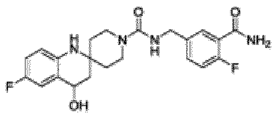, 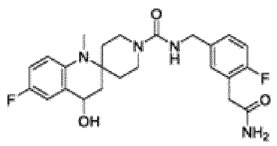, 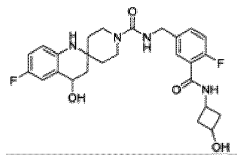, 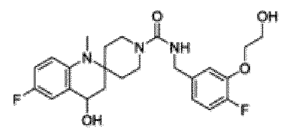,

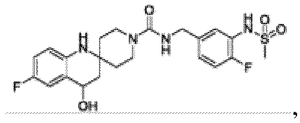, 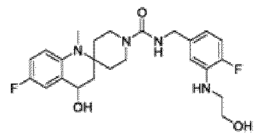, 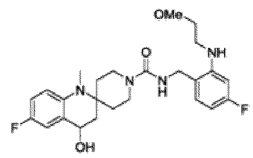, 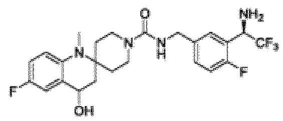,

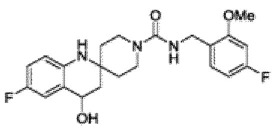, 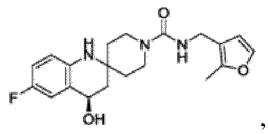, 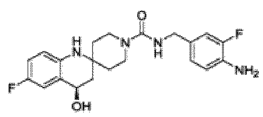, 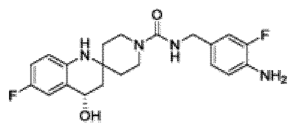.

6. Pharmaceutical use of AKR1C3 enzyme-activated prodrug in the manufacture of a medicament for treating cancer, **characterized in that**:

   the tumor or cancer tissue of the patient is detected to have a gene mutation capable of up-regulating or activating NRF2; or
   the patient is detected to have a gene mutation capable of up-regulating or activating NRF2.

7. A medicament comprising AKR1C3 enzyme-activated prodrug compound, which is used to treat cancer patients,

   wherein the tumor or cancer tissue of the patients is detected to have a gene mutation capable of up-regulating or activating NRF2; or
   the patients are detected to have a gene mutation capable of up-regulating or activating NRF2.

8. A method for treating cancer patients with an AKR1C3 enzyme-activated prodrug, which comprises the step of administrating a medicament or preparation containing AKR1C3 enzyme-activated prodrug; and the step of detecting NRF2 content or expression level in a cancer cell or tissue of the patients,
   where the NRF2 content or expression level is measured to be equal to or greater than the predetermined value, the patients are administered with a medicament or preparation containing AKR1C3 enzyme-activated prodrug.

9. A method for treating cancer or tumor, which comprises the step of administrating a medicament or preparation containing AKR1C3 enzyme-activated prodrug; and the step of regulating NRF2 content or expression level, where the NRF2 content or expression level is regulated to be equal to or greater than the predetermined value, the patient is administered with a medicament or preparation containing AKR1C3 enzyme-activated prodrug.

**10.** Pharmaceutical use of AKR1C3 enzyme-activated prodrug in the manufacture of a medicament for treating cancer, **characterized in that**:

the tumor or cancer tissue of the patient is detected to comprise a NRF2 content or expression level that is equal to or greater than the predetermined value; or
the patient is detected to comprise a NRF2 content or expression level that is equal to or greater than the predetermined value.

**11.** A medicament comprising AKR1C3 enzyme-activated prodrug compound, which is used to treat cancer patients,

wherein the tumor or cancer tissue of the patients is detected to comprise a NRF2 content or expression level that is equal to or greater than the predetermined value; or
the patients are detected to comprise a NRF2 content or expression level that is equal to or greater than the predetermined value.

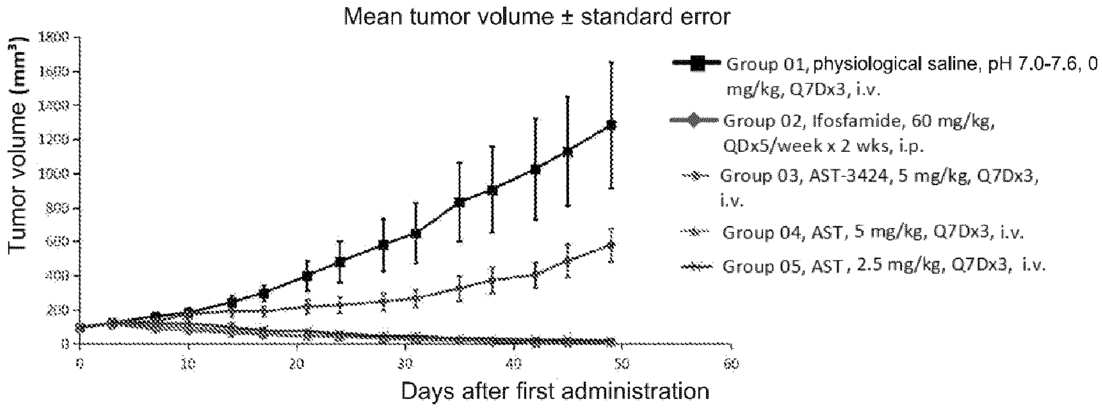


Fig. 1

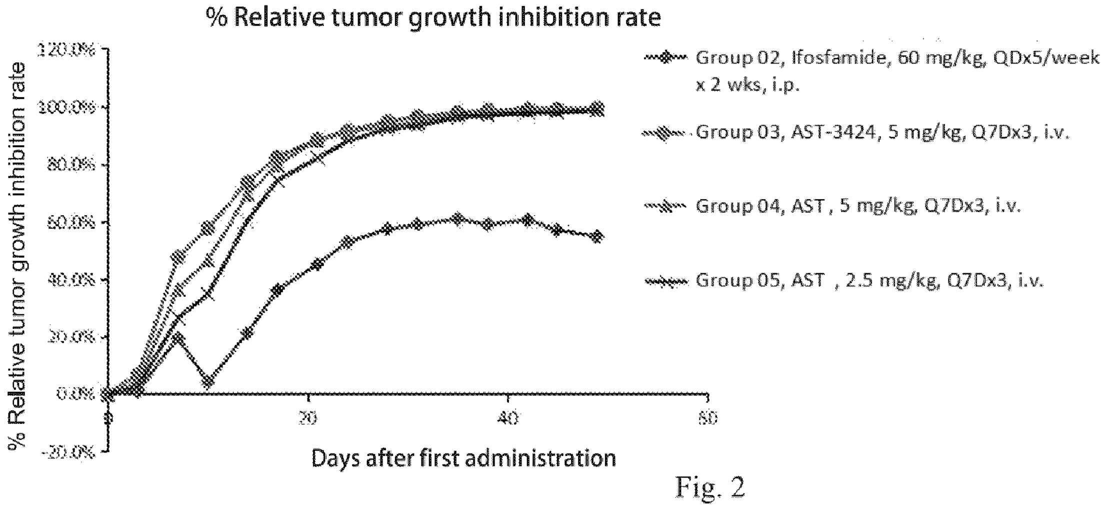


Fig. 2

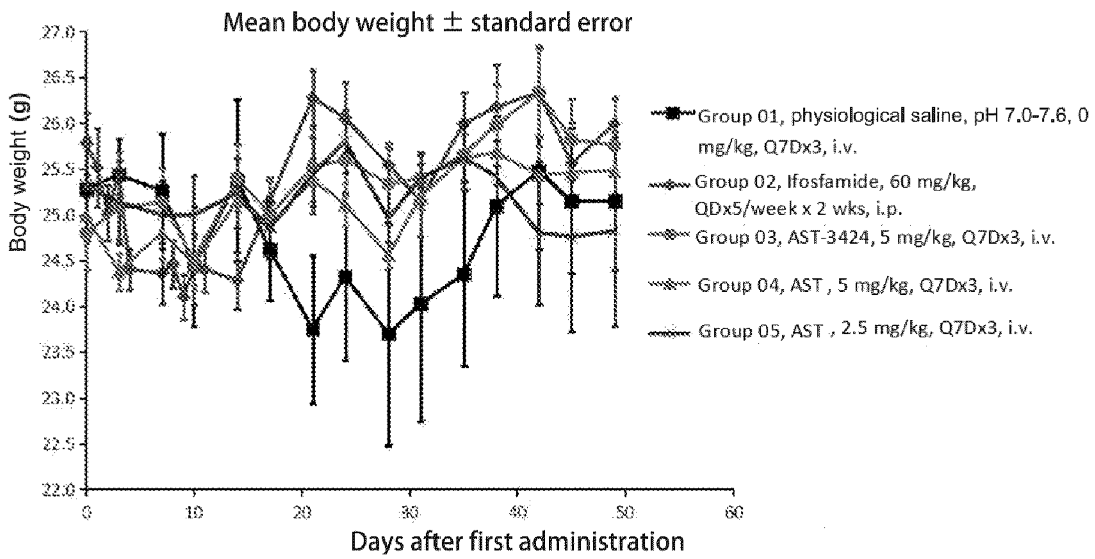


Fig. 3

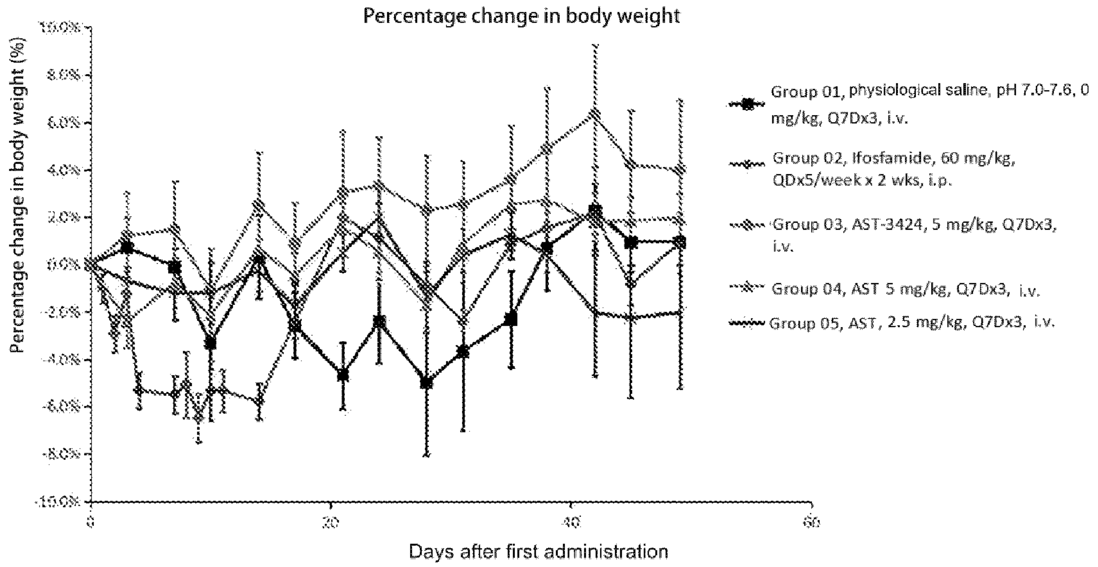


Fig. 4

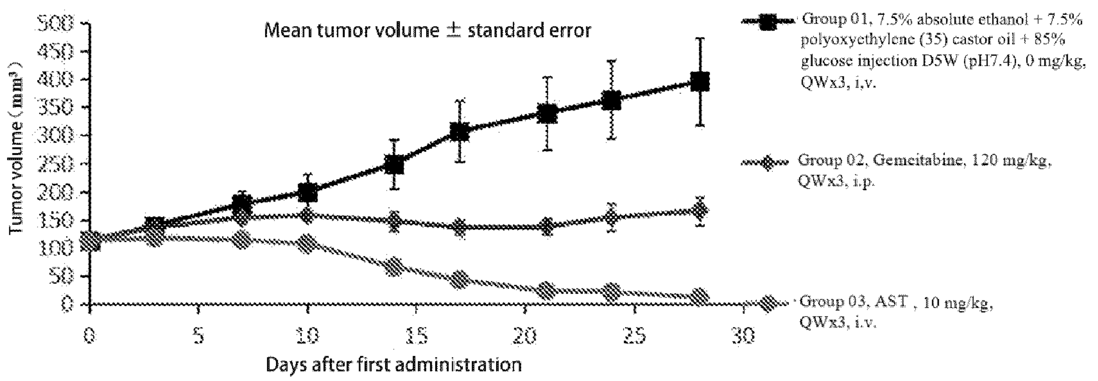


Fig. 5

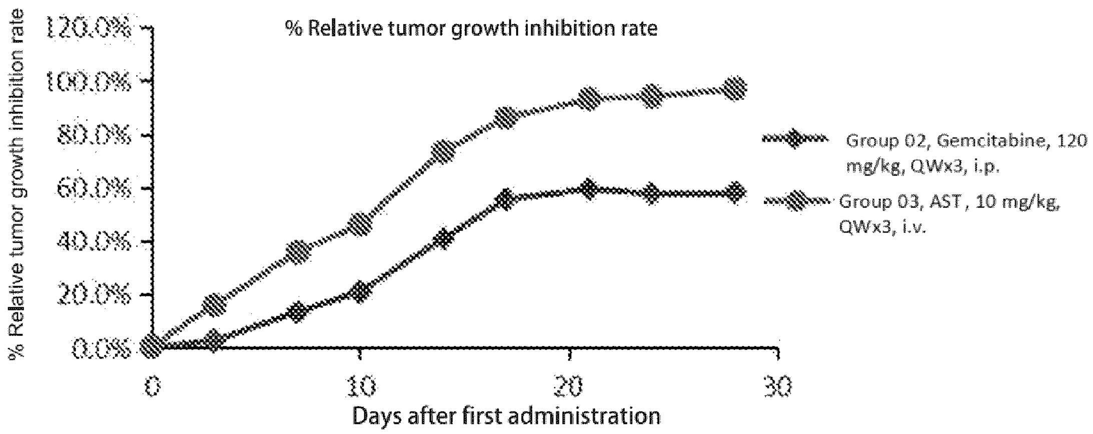


Fig. 6

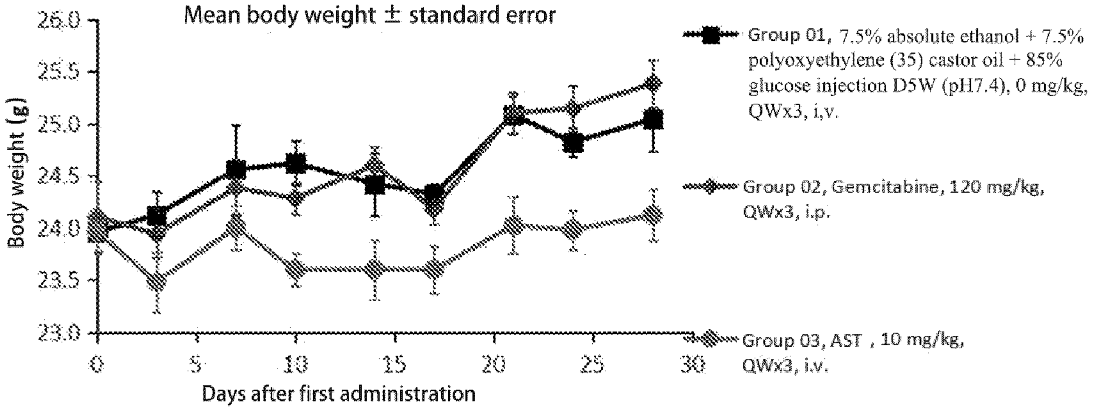


Fig. 7

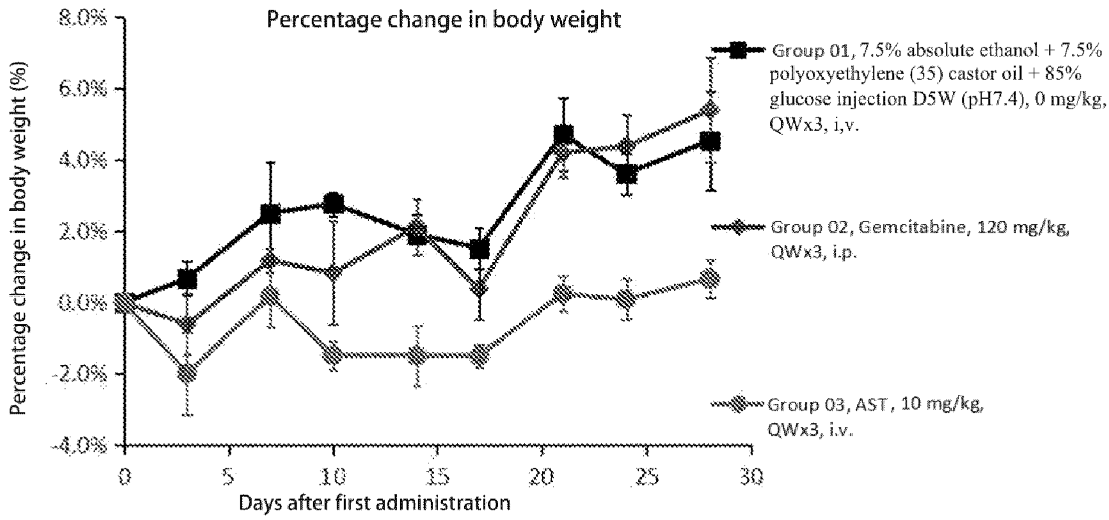


Fig. 8

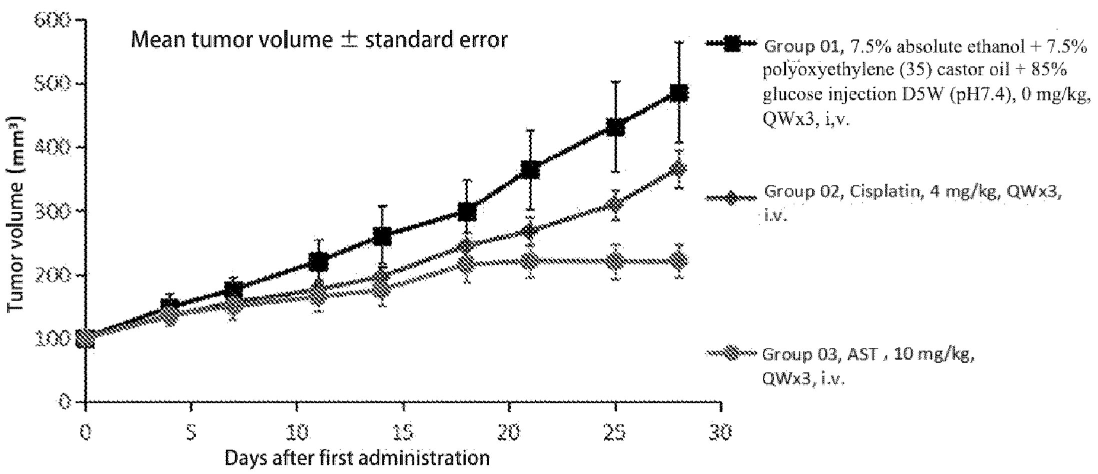


Fig. 9

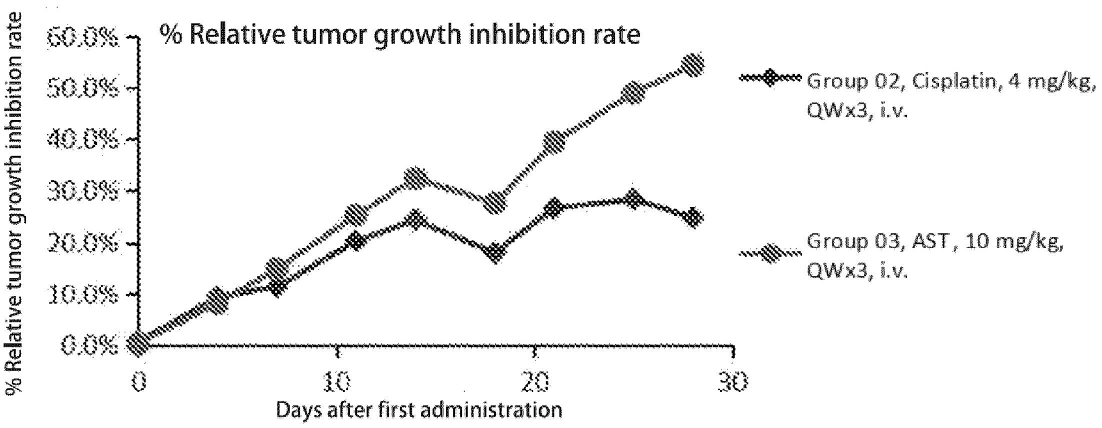


Fig. 10

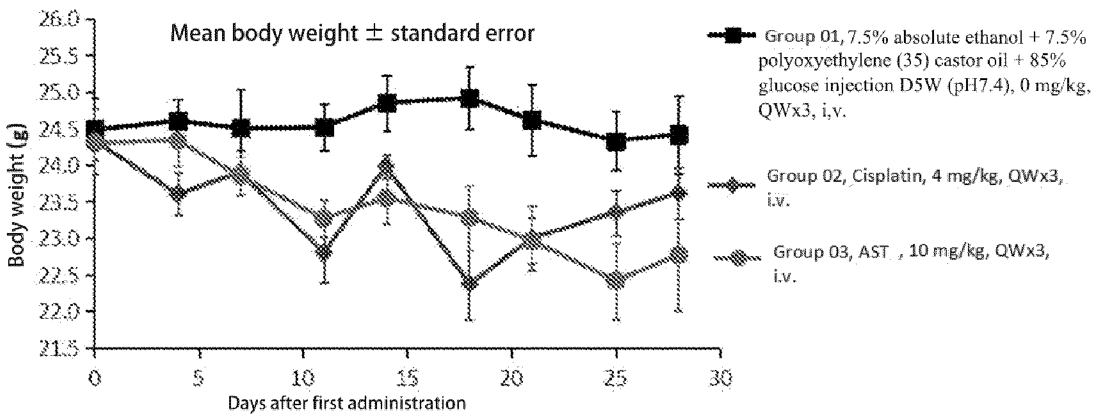


Fig. 11

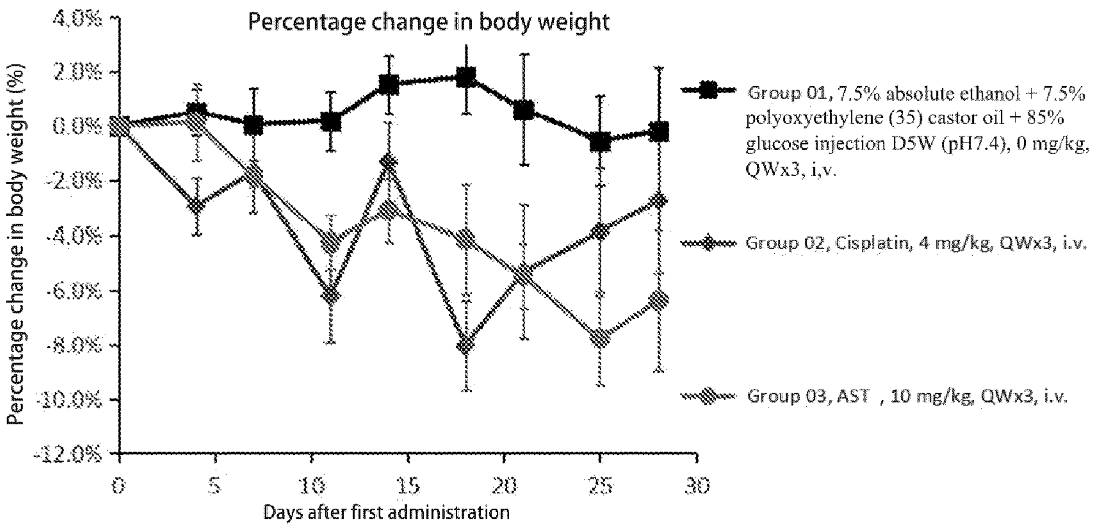


Fig. 12

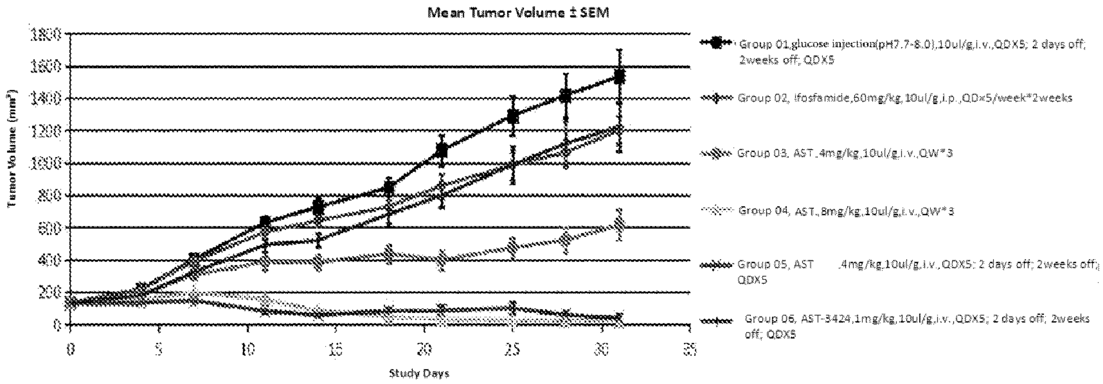


Fig. 13

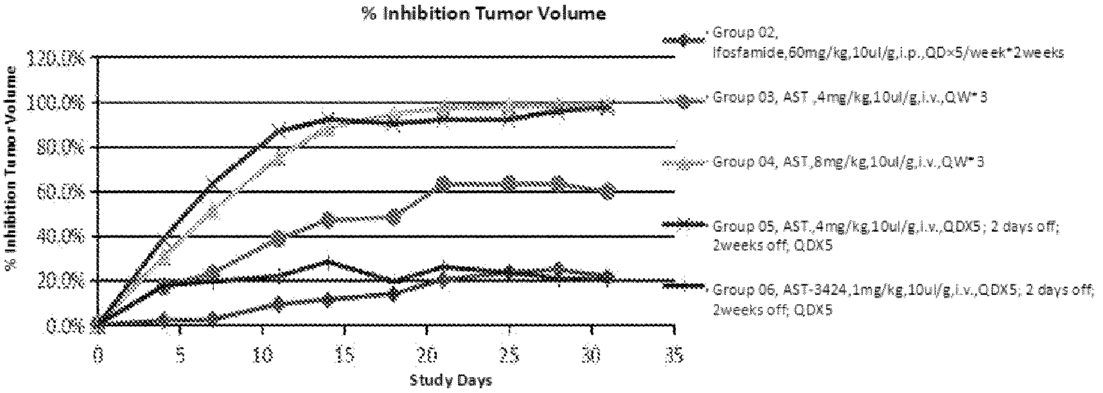


Fig. 14

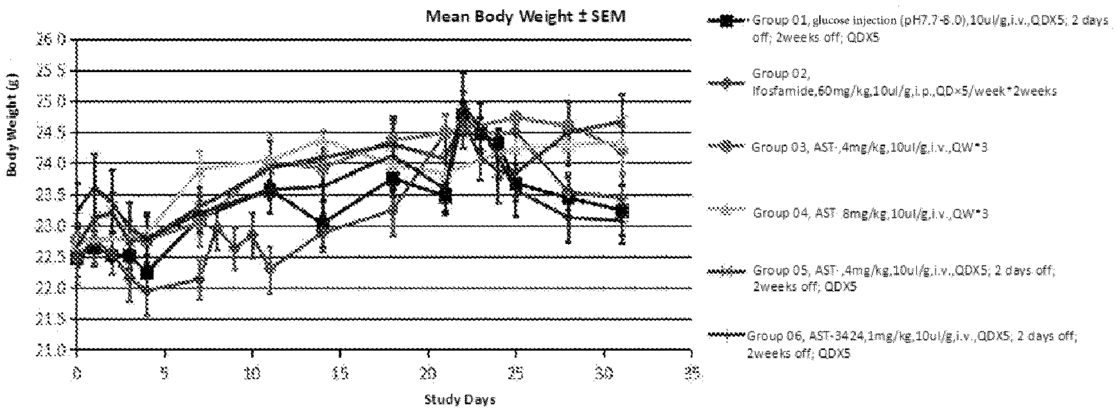


Fig. 15

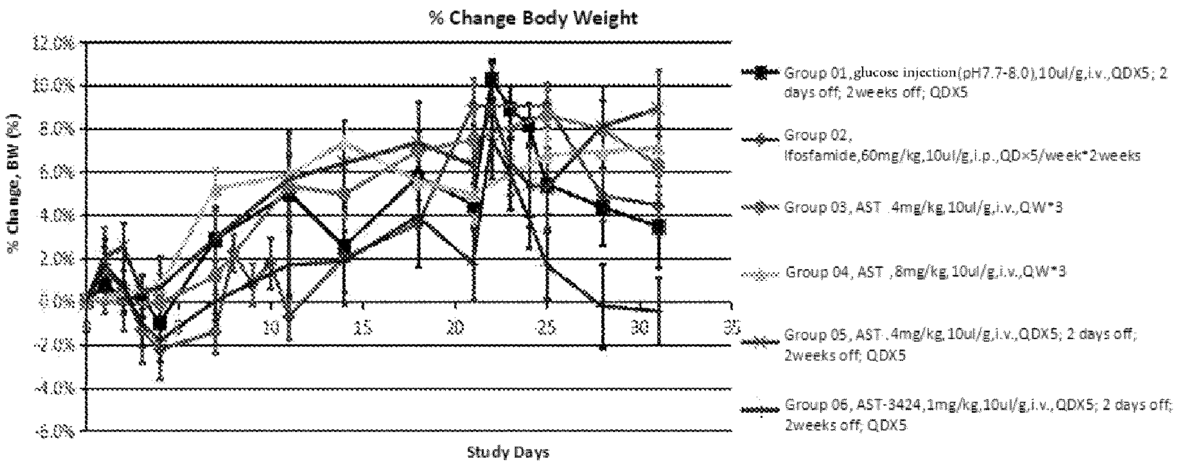
% Change Body Weight
12.0%
10.0%
8.0%
6.0%
4.0%
2.0%
0.0%
-2.0%
-4.0%
-6.0%
% Change, BW (%)
10
15
20
25
30
35
Study Days
Group 01, glucose injection(pH7.7-8.0),10ul/g,i.v.,QDX5; 2 days off; 2weeks off; QDX5
Group 02, Ifosfamide,60mg/kg,10ul/g,i.p.,QD×5/week*2weeks
Group 03, AST .4mg/kg,10ul/g,i.v.,QW*3
Group 04, AST ,8mg/kg,10ul/g,i.v.,QW*3
Group 05, AST .4mg/kg,10ul/g,i.v.,QDX5; 2 days off; 2weeks off; QDX5
Group 06, AST-3424,1mg/kg,10ul/g,i.v.,QDX5; 2 days off; 2weeks off; QDX5

Fig. 16

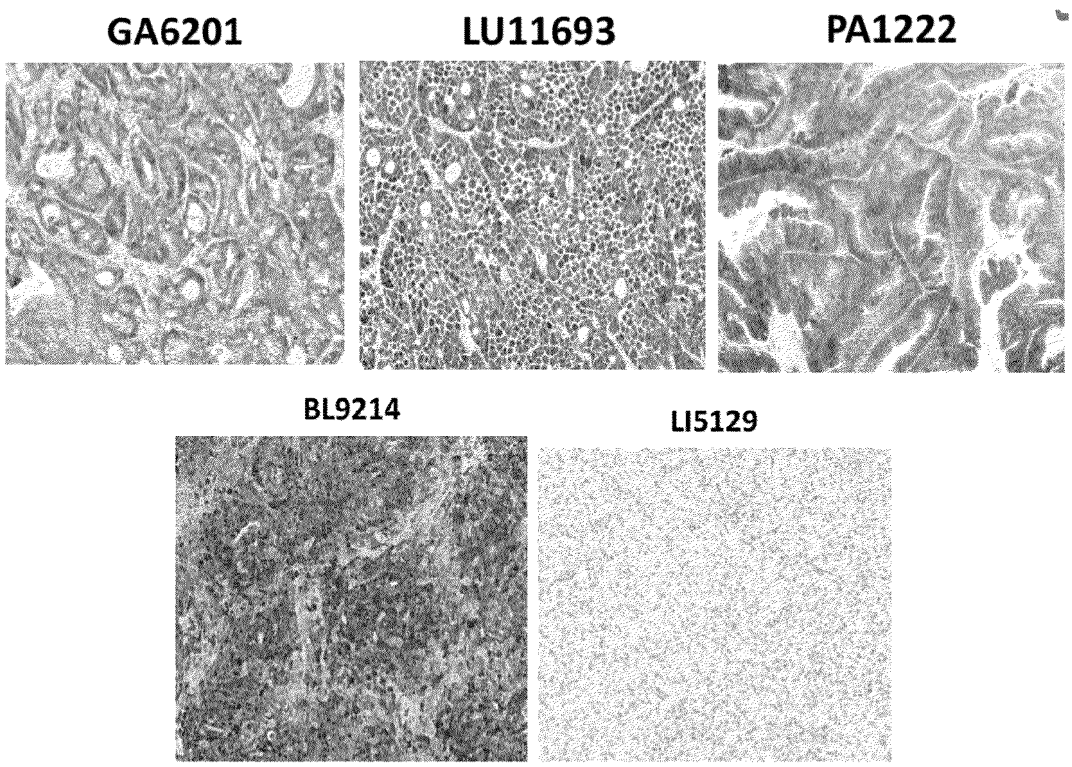
GA6201
LU11693
PA1222
BL9214
LI5129

Fig. 17

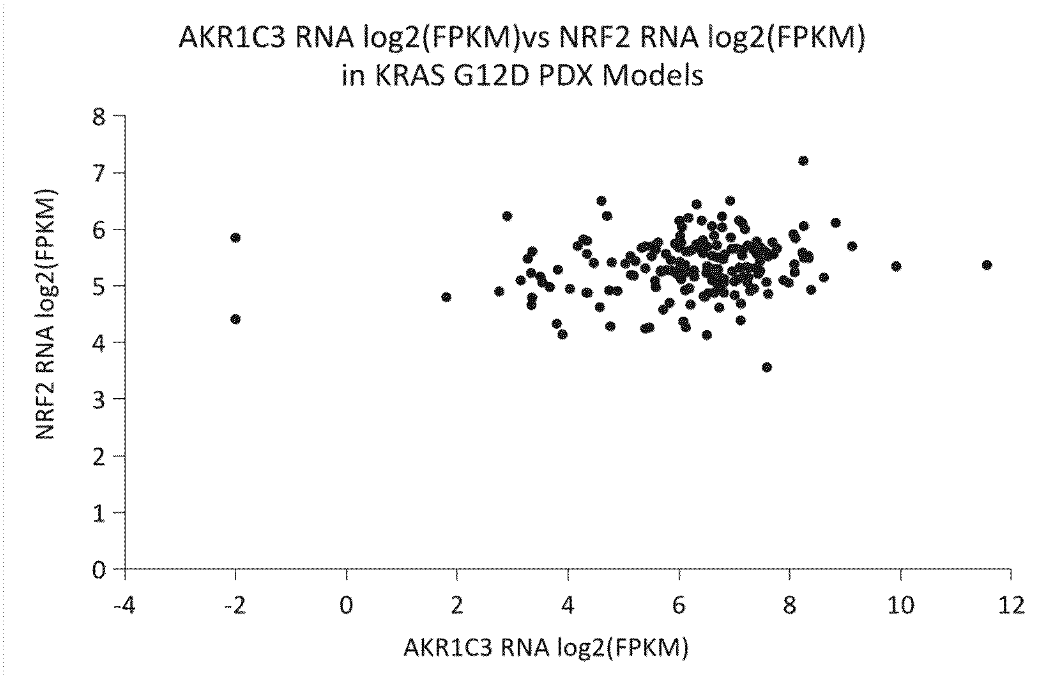
AKR1C3 RNA log2(FPKM)vs NRF2 RNA log2(FPKM)
in KRAS G12D PDX Models
NRF2 RNA log2(FPKM)
AKR1C3 RNA log2(FPKM)
0
1
2
3
4
5
6
7
8
-4
-2
0
2
4
6
8
10
12

Fig. 18

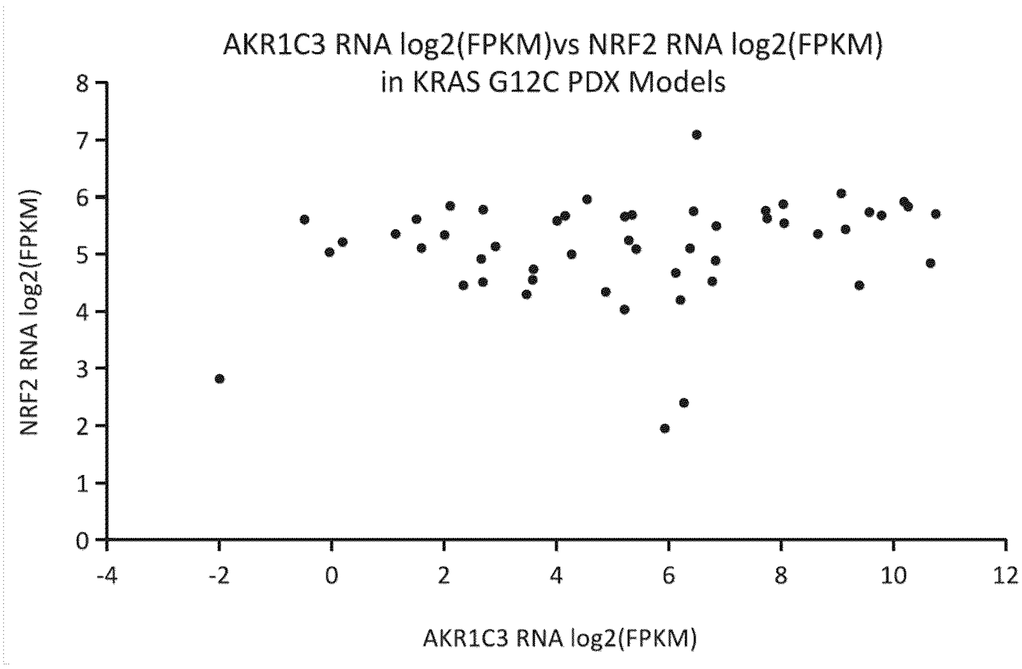
AKR1C3 RNA log2(FPKM)vs NRF2 RNA log2(FPKM)
in KRAS G12C PDX Models
NRF2 RNA log2(FPKM)
AKR1C3 RNA log2(FPKM)
0
1
2
3
4
5
6
7
8
-4
-2
0
2
4
6
8
10
12

Fig. 19

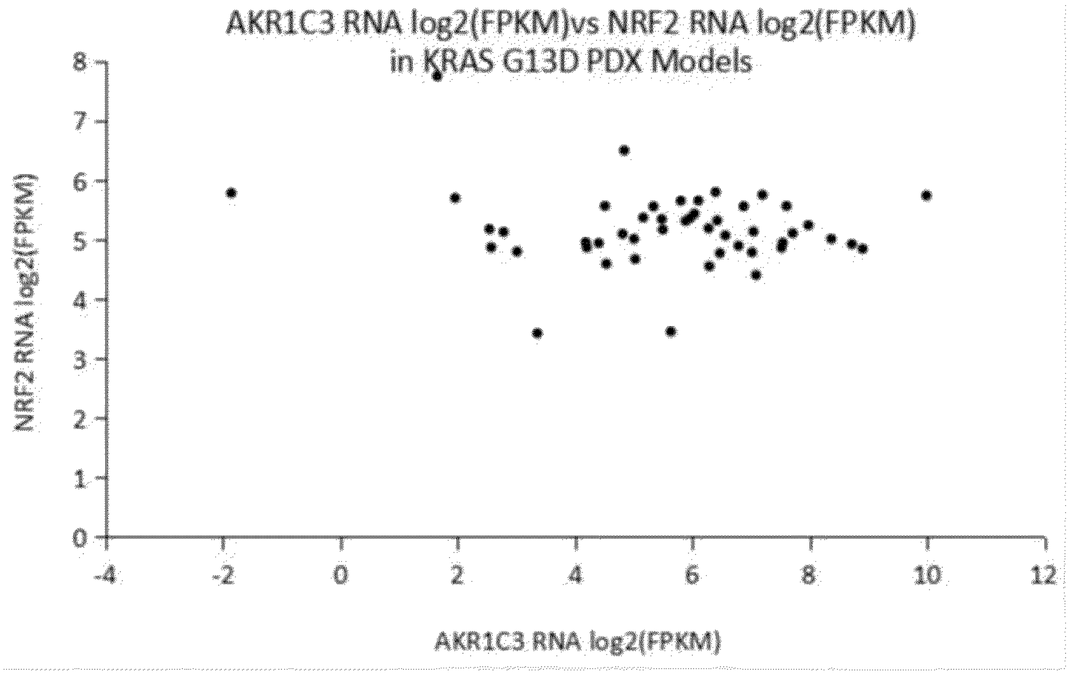
AKR1C3 RNA log2(FPKM)vs NRF2 RNA log2(FPKM)
in KRAS G13D PDX Models
NRF2 RNA log2(FPKM)
AKR1C3 RNA log2(FPKM)
0
1
2
3
4
5
6
7
8
-4
-2
0
2
4
6
8
10
12

Fig. 20

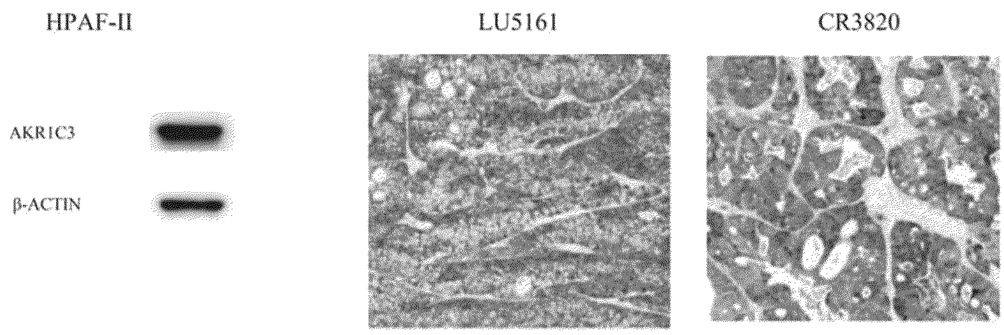
HPAF-II
AKR1C3
β-ACTIN
LU5161
CR3820

Fig. 21

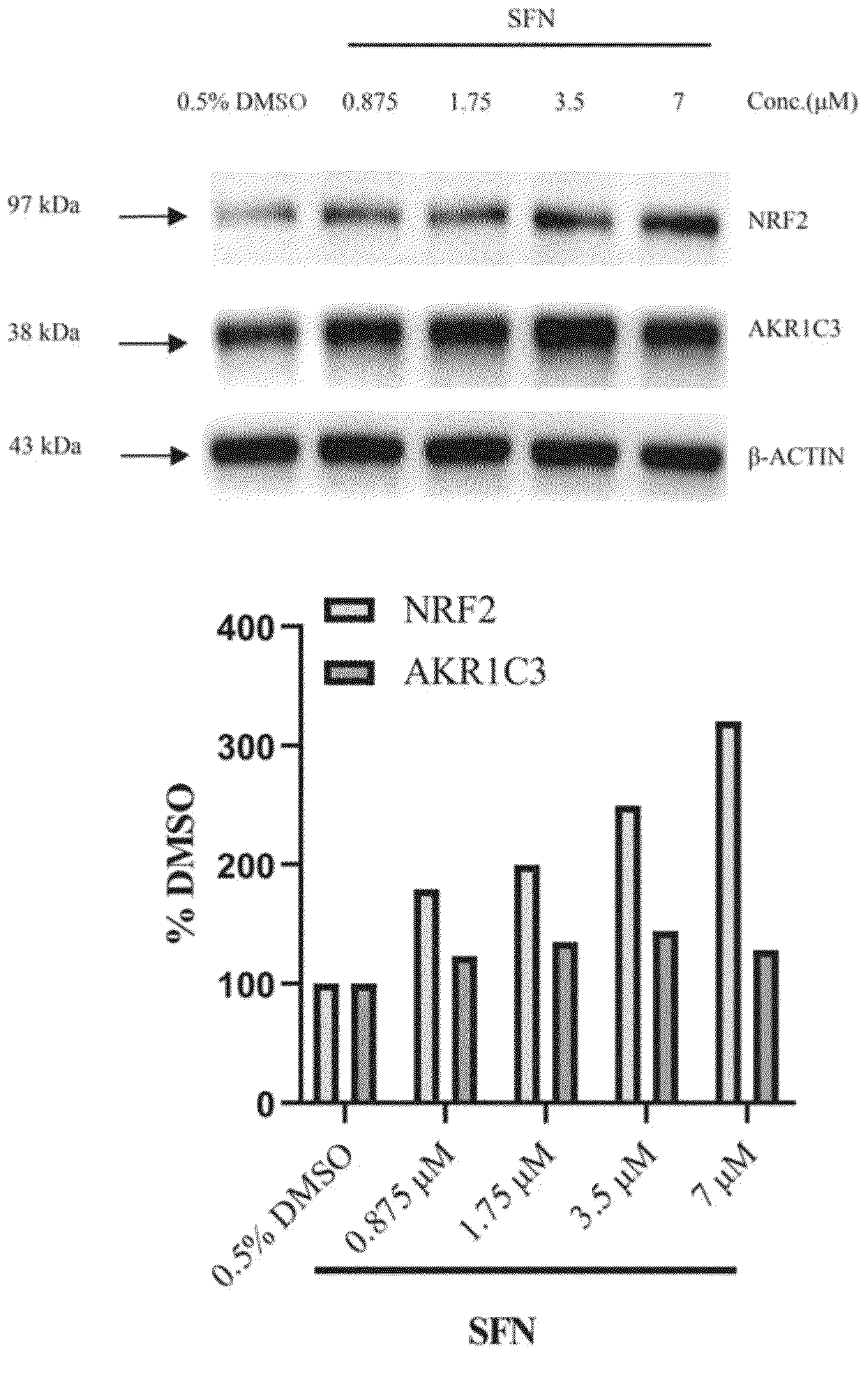
SFN
0.5% DMSO
0.875
1.75
3.5
7
Conc.(μM)
97 kDa
NRF2
38 kDa
AKR1C3
43 kDa
β-ACTIN
NRF2
AKR1C3
400
300
200
100
0
% DMSO
0.5% DMSO
0.875 μM
1.75 μM
3.5 μM
7 μM
SFN

Fig. 22

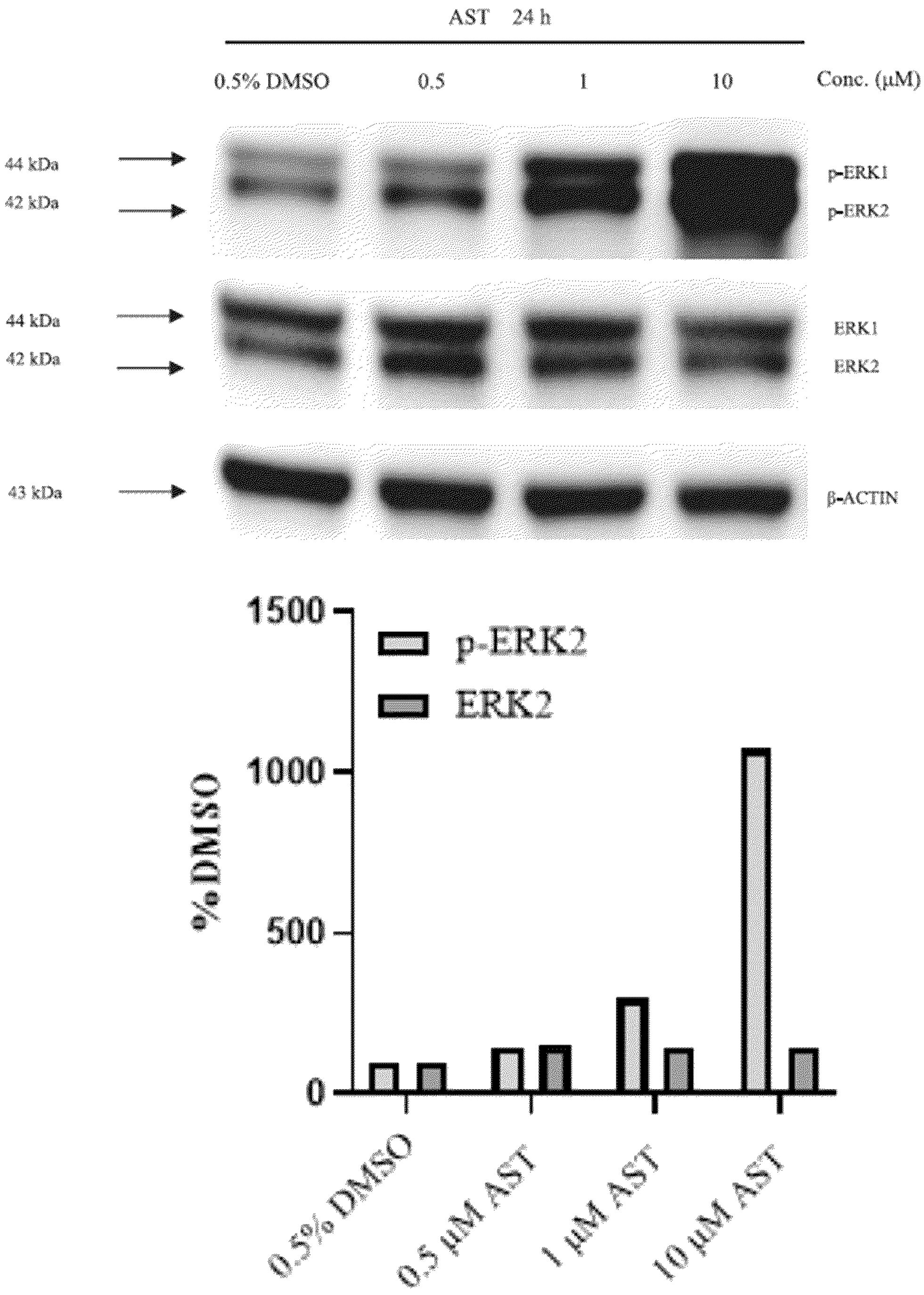
AST 24 h
0.5% DMSO
0.5
1
10
Conc. (μM)
44 kDa
42 kDa
p-ERK1
p-ERK2
44 kDa
42 kDa
ERK1
ERK2
43 kDa
β-ACTIN
1500
1000
500
0
% DMSO
p-ERK2
ERK2
0.5% DMSO
0.5 μM AST
1 μM AST
10 μM AST

Fig. 23

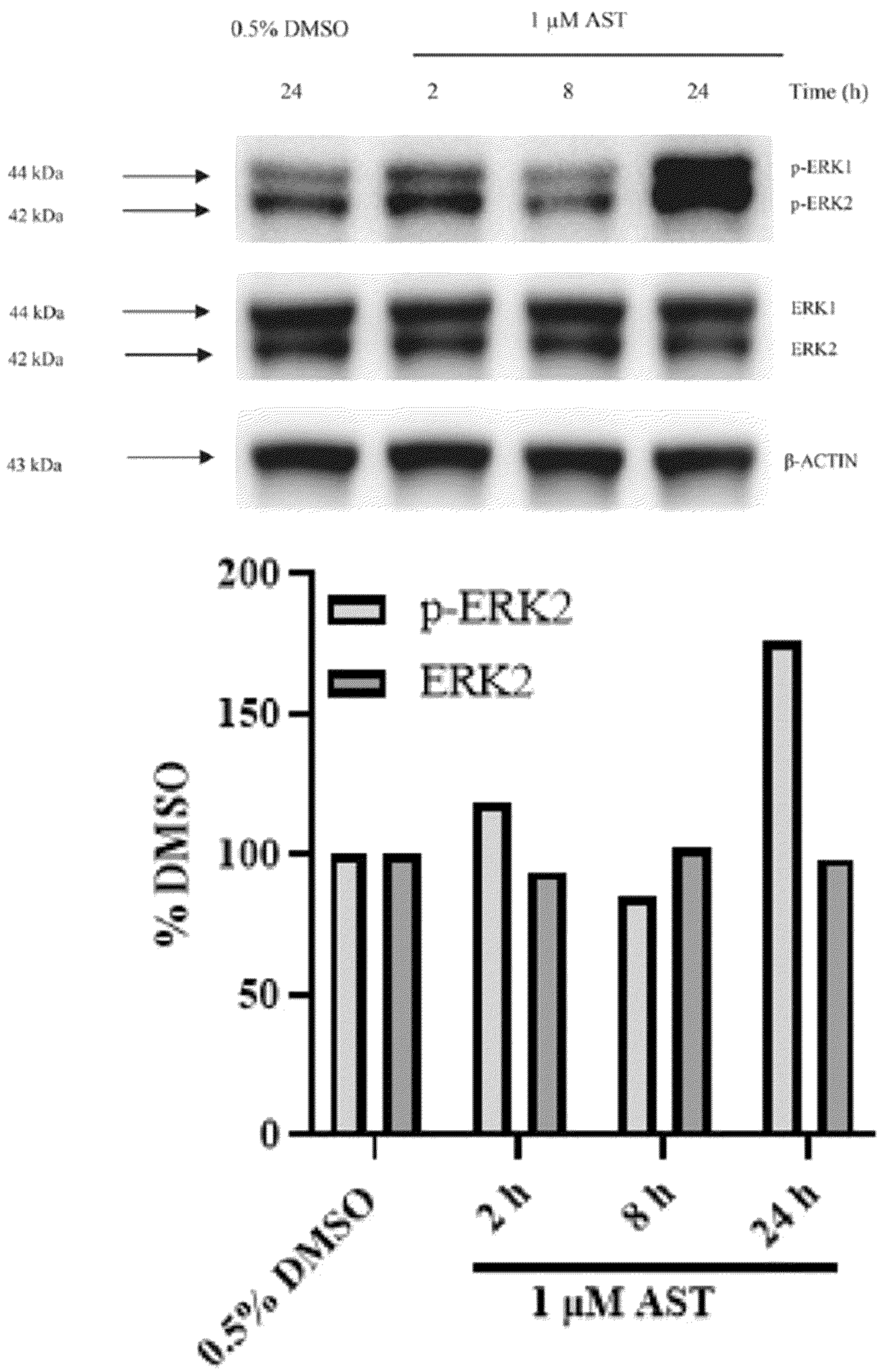
0.5% DMSO
1 µM AST
24
2
8
24
Time (h)
44 kDa
42 kDa
p-ERK1
p-ERK2
44 kDa
42 kDa
ERK1
ERK2
43 kDa
β-ACTIN
200
150
100
50
0
% DMSO
p-ERK2
ERK2
0.5% DMSO
2 h
8 h
24 h
1 µM AST

Fig. 24

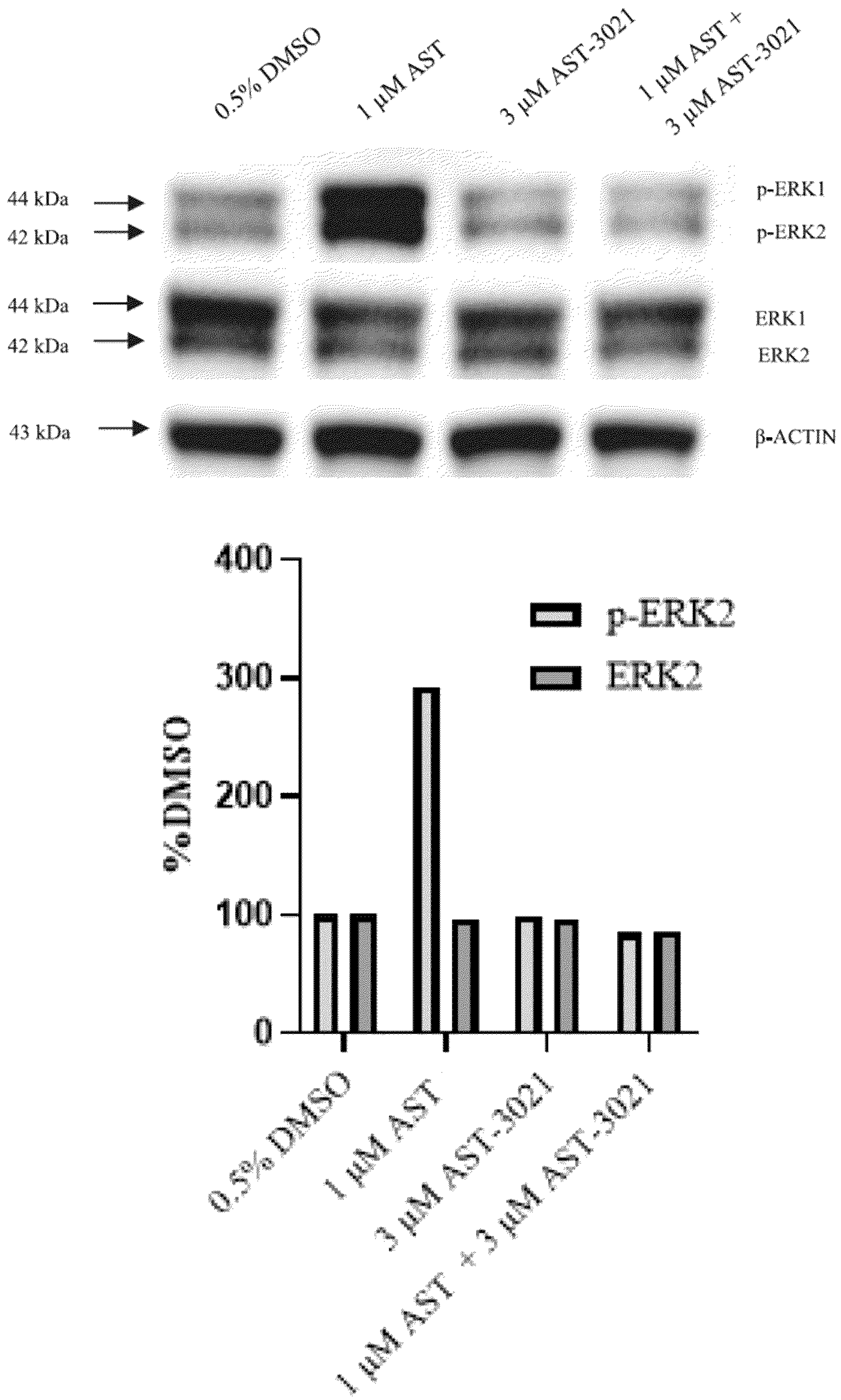
0.5% DMSO
1 µM AST
3 µM AST-3021
1 µM AST + 3 µM AST-3021
44 kDa
42 kDa
p-ERK1
p-ERK2
44 kDa
42 kDa
ERK1
ERK2
43 kDa
β-ACTIN
400
300
200
100
0
%DMSO
p-ERK2
ERK2
0.5% DMSO
1 µM AST
3 µM AST-3021
1 µM AST + 3 µM AST-3021

Fig. 25

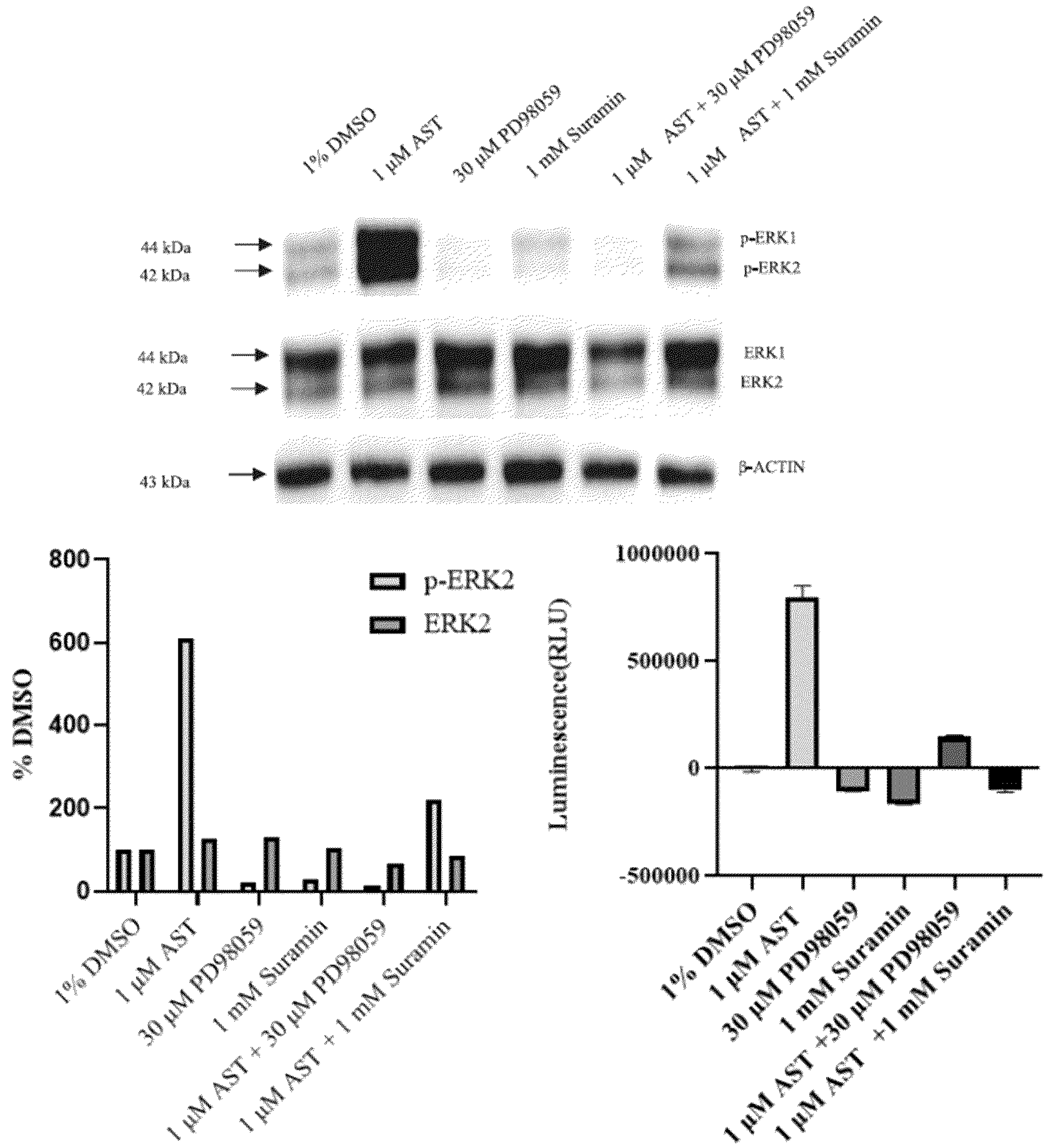
1% DMSO
1 µM AST
30 µM PD98059
1 mM Suramin
1 µM AST + 30 µM PD98059
1 µM AST + 1 mM Suramin
44 kDa
42 kDa
p-ERK1
p-ERK2
44 kDa
42 kDa
ERK1
ERK2
43 kDa
β-ACTIN
% DMSO
800
600
400
200
0
p-ERK2
ERK2
1% DMSO
1 µM AST
30 µM PD98059
1 mM Suramin
1 µM AST + 30 µM PD98059
1 µM AST + 1 mM Suramin
Luminescence(RLU)
1000000
500000
0
-500000
1% DMSO
1 µM AST
30 µM PD98059
1 mM Suramin
1 µM AST +30 µM PD98059
1 µM AST +1 mM Suramin

Fig. 26

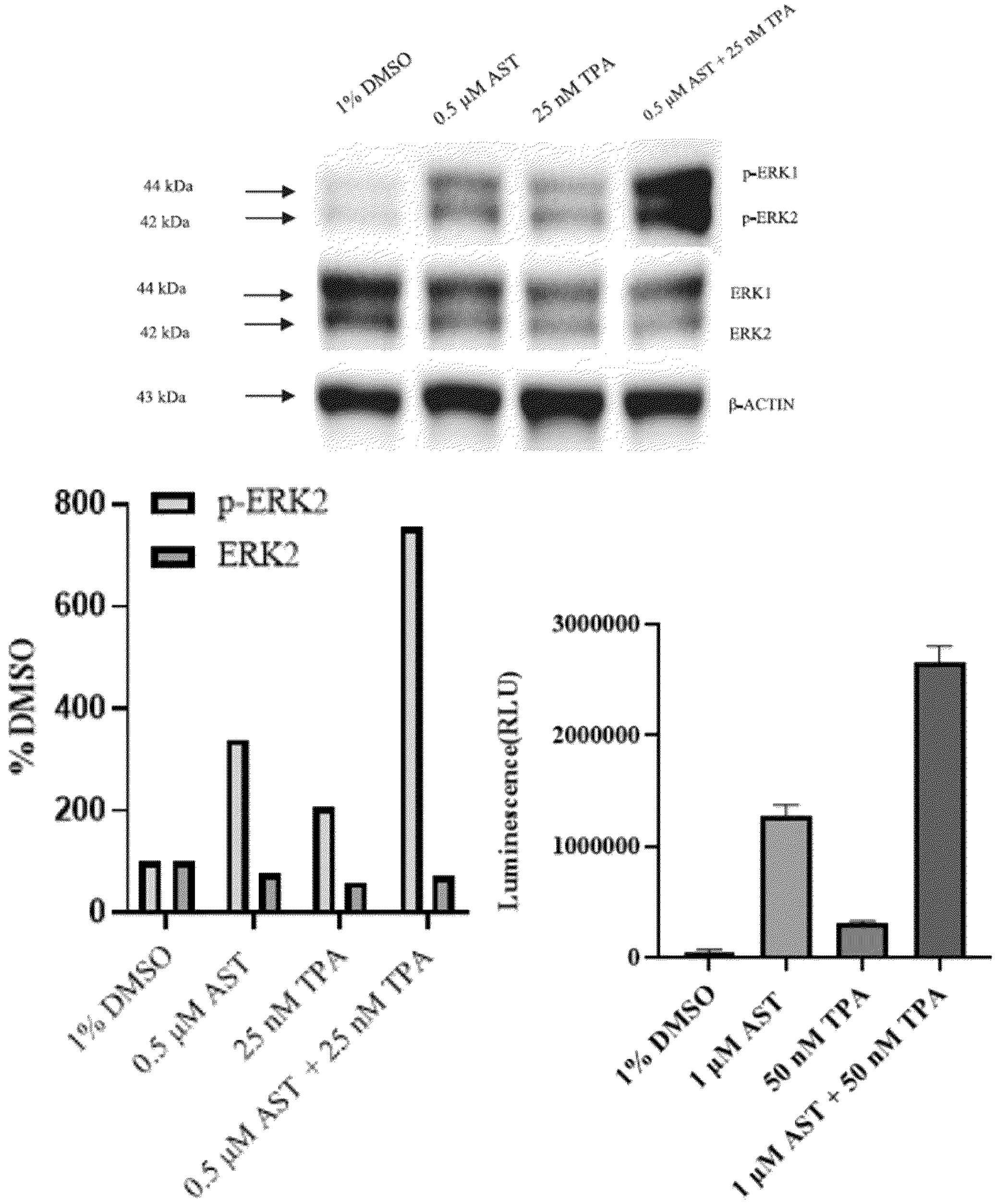
1% DMSO
0.5 μM AST
25 nM TPA
0.5 μM AST + 25 nM TPA
44 kDa
42 kDa
p-ERK1
p-ERK2
44 kDa
42 kDa
ERK1
ERK2
43 kDa
β-ACTIN
800
600
400
200
0
% DMSO
p-ERK2
ERK2
1% DMSO
0.5 μM AST
25 nM TPA
0.5 μM AST + 25 nM TPA
3000000
2000000
1000000
0
Luminescence(RLU)
1% DMSO
1 μM AST
50 nM TPA
1 μM AST + 50 nM TPA

Fig. 27

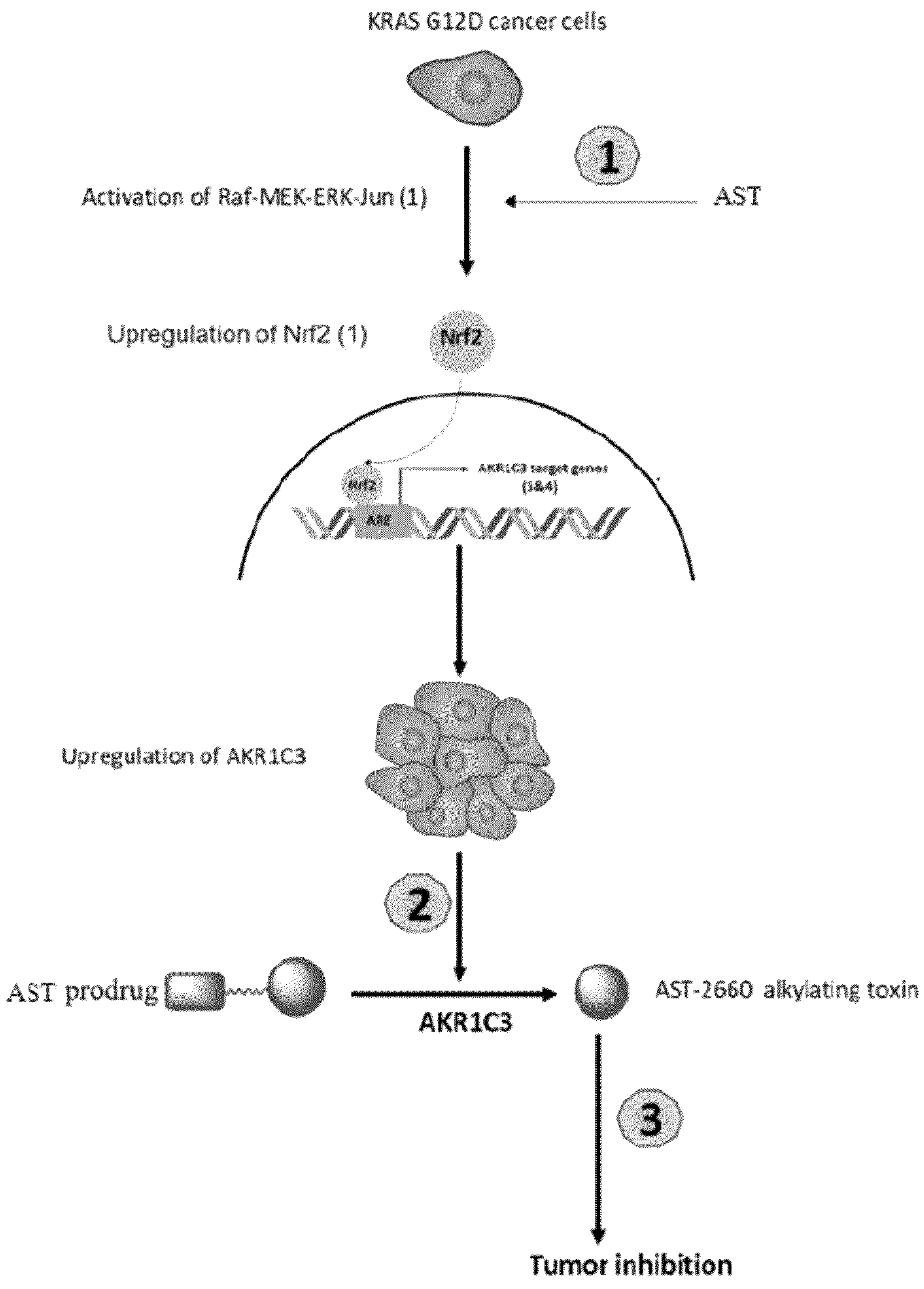
KRAS G12D cancer cells
1
Activation of Raf-MEK-ERK-Jun (1)
AST
Upregulation of Nrf2 (1)
Nrf2
Nrf2
AKR1C3 target genes
(3&4)
ARE
Upregulation of AKR1C3
2
AST prodrug
AKR1C3
AST-2660 alkylating toxin
3
Tumor inhibition

Fig. 28

**INTERNATIONAL SEARCH REPORT**

International application No.
**PCT/CN2023/099294**

**A. CLASSIFICATION OF SUBJECT MATTER**

A61K 31/675(2006.01)i; A61K 31/495(2006.01)i; A61K 31/496(2006.01)i; A61K 31/472(2006.01)i; A61K 31/4375(2006.01)i; A61K 31/445(2006.01)i; A61K 31/5377(2006.01)i; A61K 31/18(2006.01)i; A61K 31/255(2006.01)i; A61K 31/551(2006.01)i; A61K 31/136(2006.01)i; A61K 31/4709(2006.01)i; A61K 31/7068(2006.01)i; A61K 31/337(2006.01)i; A61K 31/4523(2006.01)i; A61K 31/7072(2006.01)i; A61K 31/704(2006.01)i; A61K 31/4745(2006.01)i; A61K 31/517(2006.01)i; A61K 31/519(2006.01)i; A61K 31/404(2006.01)i; A61K 38/07(2006.01)i; A61K 38/08(2019.01)i; A61K 31/14(2006.01)i; A61K 31/7048(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNABS; CNTXT; DWPI; VEN; ENTXTC; ENTXT; WPABS; CNKI; VCN; CJFD; 万方, WANFANG; ISI Web of Science; 读秀学术, Duxiu Scholar; Springer; STNext: 深圳艾欣达伟医药科技有限公司, AKR1C3酶, NRF2, G12D, 突变, 肿瘤, 癌症, 前药, OBI-3424, AST-3424, Ascentawits Pharmaceuticals, mutation, tumor, cancer, prodrug

**C. DOCUMENTS CONSIDERED TO BE RELEVANT**

| Category* | Citation of document, with indication, where appropriate, of the relevant passages | Relevant to claim No. |
|---|---|---|
| X | MENG, Fanying et al. "A Novel Selective AKR1C3-activated Prodrug AST-3424/OBI-3424 Exhibits Broad Anti-tumor Activity" *Am J Cancer Res,* Vol. 11, No. (7), 30 July 2021 (2021-07-30), pages 3645-3659<br>page 3650, figure 1, and page 3656, left-hand column, paragraphs 2-3, and right-hand column, paragraphs 1-2 | 1-7, 10-11 |
| X | CN 112904026 A (ASCENTAWITS PHARMACEUTICALS, LTD.) 04 June 2021 (2021-06-04)<br>claims 1-20 | 1-7, 10-11 |
| X | CN 114206870 A (NOVARTIS AG) 18 March 2022 (2022-03-18)<br>claims 1-31 | 1-7, 10-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 September 2023** | **15 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/099294

**C. DOCUMENTS CONSIDERED TO BE RELEVANT**

| Category* | Citation of document, with indication, where appropriate, of the relevant passages | Relevant to claim No. |
|---|---|---|
| X | WO 2021083310 A1 (ASCENTAWITS PHARMACEUTICALS, LTD.) 06 May 2021 (2021-05-06)<br>claims 1-29 | 1-7, 10-11 |
| A | 张玉 等 (ZHANG, Yu et al.). "AKR1C3与恶性肿瘤的研究进展 (Progression of AKR1C3 and Malignant Tumor)"<br>*临床肿瘤学杂志 (Chinese Clinical Oncology),*<br>Vol. 26, No. (10), 31 October 2021 (2021-10-31), pages 935-941<br>page 936, right-hand column, paragraphs 2-3 | 1-7, 10-11 |

Form PCT/ISA/210 (second sheet) (July 2022)

**INTERNATIONAL SEARCH REPORT**

International application No.
**PCT/CN2023/099294**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-5, 8, 9**
because they relate to subject matter not required to be searched by this Authority, namely:

The subject matter of claims 1-5 relates to a method for treatment of cancer patients with an AKR1C3-activated prodrug. The subject matter of claim 8 relates to an AKR1C3-activated prodrug treating cancer patients. Claim 9 relates to a method for treating cancer or tumors. However, the above-mentioned methods have an ultimate purpose for treating diseases in a living human or animal body, which falls within the category of methods for disease diagnosis and treatment (PCT Rule 39.1(4)).

With regard to claims 1-5, the reasonably expected amendments are made as follows:

Claim 1: the use of an AKR1C3-activated prodrug in the preparation of a drug for treating cancer, ...

Claims 2-5: the pharmaceutical use according to claim ..., ...

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/099294**

| Patent document cited in search report | Publication date (day/month/year) | Patent family member(s) | Publication date (day/month/year) |
|---|---|---|---|
| CN 112904026 A | 04 June 2021 | None | |
| CN 114206870 A | 18 March 2022 | US 2022315582 A1 | 06 October 2022 |
| | | CA 3139940 A1 | 14 January 2021 |
| | | PE 20220343 A1 | 14 March 2022 |
| | | MX 2022000961 A | 14 February 2022 |
| | | ECSP 22005621 A | 25 February 2022 |
| | | TW 202120507 A | 01 June 2021 |
| | | UY 38806 A | 26 February 2021 |
| | | CU 20220005 A7 | 08 September 2022 |
| | | EP 4013500 A1 | 22 June 2022 |
| | | IL 287792 A | 01 January 2022 |
| | | JP 2021527113 A | 11 October 2021 |
| | | JP 7008161 B2 | 25 January 2022 |
| | | DOP 2022000010 A | 15 March 2022 |
| | | CR 20220031 A | 09 February 2022 |
| | | WO 2021005586 A1 | 14 January 2021 |
| | | CO 2022000370 A2 | 28 January 2022 |
| | | KR 20220025845 A | 03 March 2022 |
| | | BR 112022001308 A2 | 17 May 2022 |
| | | CL 2022000201 A1 | 18 November 2022 |
| | | JOP 20220020 A1 | 30 January 2023 |
| | | AU 2020309846 A1 | 06 January 2022 |
| WO 2021083310 A1 | 06 May 2021 | EP 4053135 A1 | 07 September 2022 |
| | | US 2022387345 A1 | 08 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016021581 W
- WO 2016145092 A1
- CN 2016800150788
- CN 107530556 A
- US 2016062114 W
- WO 2017087428 A
- CN 2016800446081
- CN 108290911 A
- US 2016025665 W
- WO 2016161342 A
- CN 2016800200132
- CN 108136214 A
- NZ 2019050030 W
- WO 2019190331 A
- CN 2019800234236
- CN 111918864 A
- CN 2020120281 W
- WO 2021068952 A1
- CN 2020089692 W
- WO 2020228685 A1
- WO 2019190331 A1
- WO 2016161342 A3
- CN 2021118597 W
- WO 2022057838 A1
- CN 2022098082 W
- WO 2022258043 A1
- CN 202210585771
- CN 115403579 A
- WO 2020057285 A
- WO 2021005586 A1
- CN 202080053804
- CN 114206870 A
- WO 2017087428 A1
- WO 2019062919 A1
- WO 2021008520 A1
- WO 2020228685 A9
- WO 2021043275 A1
- CN 2022120817 W
- WO 2023046060 A1


### Non-patent literature cited in the description


- *AACR-NCI-EORTC Annual Meeting*, 2017
- *Clin Cancer Res*, 2019, vol. 25, 4493-503
- *Am J Cancer Res*, 2021, vol. 11 (7), 3645-3659
- Safety, Pharmacokinetics, and Clinical Activity of OBI-3424, an AKR1C3-Activated Prodrug, in Patients with Advanced or Metastatic Solid Tumors: A Phase 1 Dose-Escalation Study. *J Clin Oncol*, 2022, vol. 40 (16)
- *Nature*, 2011, vol. 475, 106
- *Cancer Res*, 2014, vol. 74, 7430
- *Chem Res Toxicol*, 2017, vol. 30, 162
- *Cancer*, 2019, vol. 11, 1715
- **WU S et al.** Nrf2 in cancers: A double-edged sword. *Cancer Med.*, 2019, vol. 8 (5), 2252-2267
- **JIANG T et al.** p62 links autophagy and Nrf2 signaling. *Free Radic Biol Med.*, 2015, vol. 88, 199-204
- World Conference on Lung Cancer. Squibb Inc, 2017
- *Bioorganic and Medicinal Chemistry*, 2014, 962-977
- **PRIOR IA** ; **HOOD FE** ; **HARTLEY JL**. The Frequency of Ras Mutations in Cancer. *Cancer Res*, 2020, vol. 80, 2969-2974
- **PARK HE** ; **YOO SY** ; **CHO NY** ; **BAE JM** ; **HAN SW** ; **LEE HS** ; **PARK KJ** ; **KIM TY** ; **KANG GH**. Tumor microenvironment-adjusted prognostic implications of the KRAS mutation subtype in patients with stage III colorectal cancer treated with adjuvant FOLFOX. *Sci Rep*, 2021, vol. 11, 14609
- **PATRICELLI MP** ; **JANES MR** ; **LI LS** ; **HANSEN R** ; **PETERS U** ; **KESSLER LV** ; **CHEN Y** ; **KUCHARSKI JM** ; **FENG J** ; **ELY T**. Selective Inhibition of Oncogenic KRAS Output with Small Molecules Targeting the Inactive State. *Cancer Discov*, 2016, vol. 6, 316-329
- **MENG, F.** ; **LI, W. F.** ; **JUNG, D.** ; **WANG, C. C.** ; **QI, T.** ; **SHIA, C. S.** ; **HSU, R. Y.** ; **HSIEH, Y. C.** ; **DUAN, J.** A novel selective AKR1C3-activated prodrug AST-3424/OBI-3424 exhibits broad anti-tumor activity. *American journal of cancer research*, 2021, vol. 11 (7), 3645-3659
- **FLANAGAN et al.** *Bioorganic and Medicinal Chemistry*, 2014, 962-977
- **WANG X** ; **MARTINDALE JL** ; **HOLBROOK NJ**. Requirement for ERK activation in cisplatin-induced apoptosis. *J Biol Chem*, 2000, vol. 275, 39435-39443